# EUROPEAN PATENT SPECIFICATION

(11) **EP 3 387 136 B1**
(45) Date of publication and mention of the grant of the patent: **05.01.2022**
(21) Application number: 16822120.8
(22) Date of filing: 09.12.2016
(51) Int. Cl.: C12N 15/81, C07K 14/395, C12N 9/10, C12N 9/90, C12N 15/82, C12P 15/00, A23L 27/30

(54) **PRODUCTION OF STEVIOL GLYCOSIDES IN RECOMBINANT HOSTS**
ERZEUGUNG VON STEVIOLGLYCOSIDEN IN REKOMBINANTEN WIRTEN
PRODUCTION DE GLYCOSIDES DE STÉVIOL DANS DES HÔTES RECOMBINANTS

(30) Priority: 10.12.2015 US 201562265895 P
(43) Date of publication of application: 17.10.2018
(73) Proprietor: Evolva SA, 4153 Reinach (CH)
(72) Inventor: DYEKJAER, Jane Dannow, 4153 Reinach (CH); HOUGHTON-LARSEN, Jens, 3460 Birkeroed (DK); DOUCHIN, Veronique, 2000 Frederiksberg (DK)
(74) Representative: Forrest, Stuart
(86) International application number: PCT/EP2016/080516
(87) International publication number: WO 2017/098017

(56) References cited:
- EP-A1- 2 902 410
- WO-A1-2011/140329
- WO-A1-2011/153378
- WO-A1-2014/191580
- WO-A1-2015/011209
- WO-A1-2015/014959
- WO-A1-2015/016393
- WO-A1-2016/023844
- WO-A2-2013/022989
- WO-A2-2014/122227
- HITESH KUMAR ET AL: "A comprehensive analysis of fifteen genes of steviol glycosides biosynthesis pathway in(Bertoni)", GENE, ELSEVIER, AMSTERDAM, NL, vol. 492, no. 1, 4 October 2011 (2011-10-04), pages 276-284, XP028345135, ISSN: 0378-1119, DOI: 10.1016/J.GENE.2011.10.015 [retrieved on 2011-10-20]
- SUDESH KUMAR YADAV ET AL: "Steviol Glycosides from Stevia: Biosynthesis Pathway Review and their Application in Foods and Medicine", CRITICAL REVIEWS IN FOOD SCIENCE AND NUTRITION, vol. 52, no. 11, 1 November 2012 (2012-11-01), pages 988-998, XP055351385, USA ISSN: 1040-8398, DOI: 10.1080/10408398.2010.519447

## Description

### Field of the Invention

This disclosure relates to recombinant production of steviol glycosides and steviol glycoside precursors in recombinant hosts. In particular, this disclosure relates to production of steviol glycosides comprising Steviol-13-*O*-Glucoside (13-SMG), Steviol-19-*O*-Glucoside (19-SMG), Steviol-1,2-Bioside, Steviol-1,3-Bioside, 1,2-Stevioside, 1,3-Stevioside, Rubusoside (Rubu), Rebaudioside A (RebA), Rebaudioside B (RebB), Rebaudioside C (RebC), Rebaudioside D (RebD), Rebaudioside E (RebE), Rebaudioside F (RebF), Rebaudioside M (RebM), Rebaudioside Q (RebQ), Rebaudioside I (Rebl), dulcoside A, tri-glucosylated steviol glycosides, tetra-glycosylated steviol glycosides, penta-glucosylated steviol glycosides, hexa-glucosylated steviol glycosides hepta-glucosylated steviol glycosides, or isomers thereof in recombinant hosts.

### Description of Related Art

Sweeteners are well known as ingredients used most commonly in the food, beverage, or confectionary industries. The sweetener can either be incorporated into a final food product during production or for stand-alone use, when appropriately diluted, as a tabletop sweetener or an at-home replacement for sugars in baking. Sweeteners include natural sweeteners such as sucrose, high fructose corn syrup, molasses, maple syrup, and honey and artificial sweeteners such as aspartame, saccharine, and sucralose. Stevia extract is a natural sweetener that can be isolated and extracted from a perennial shrub, *Stevia rebaudiana.* Stevia is commonly grown in South America and Asia for commercial production of stevia extract. Stevia extract, purified to various degrees, is used commercially as a high intensity sweetener in foods and in blends or alone as a tabletop sweetener.

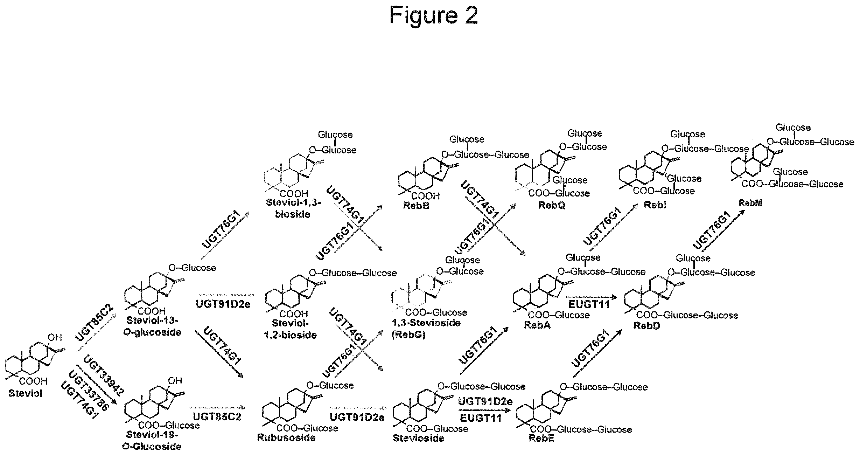

Chemical structures for several steviol glycosides are shown in Figure 2, including the diterpene steviol and various steviol glycosides. Extracts of the Stevia plant generally comprise steviol glycosides that contribute to the sweet flavor, although the amount of each steviol glycoside often varies, *inter alia,* among different production batches.

As recovery and purification of steviol glycosides from the Stevia plant have proven to be labor intensive and inefficient, there remains a need for a recombinant production system that can accumulate high yields of desired steviol glycosides, such as RebD and RebM. There also remains a need for improved production of steviol glycosides in recombinant hosts for commercial uses. As well, there remains a need for identifying enzymes selective towards particular substrates to produce one or more specific steviol glycosides. In some aspects, there remains a need to increase the catalytic capability of enzymes with 19-*O* glycosylation activity in order to produce higher yields of steviol glycosides.

### SUMMARY OF THE INVENTION

It is against the above background that the present invention provides certain advantages and advancements over the prior art.

Although this invention as disclosed herein is not limited to specific advantages or functionalities, the invention provides a recombinant host cell comprising a recombinant gene encoding a polypeptide capable of glycosylating steviol or a steviol glycoside at its C-19 carboxyl group and having at least 80% sequence identity to the amino acid sequence set forth in SEQ ID NO:2 or 119, wherein the recombinant host cell is capable of producing the steviol glycoside or a steviol glycoside composition.

In some aspects, the recombinant host cell further comprises:
(a) a gene encoding a polypeptide capable of synthesizing geranylgeranyl pyrophosphate (GGPP) from farnesyl diphosphate (FPP) and isopentenyl diphosphate (IPP);
(b) a gene encoding a polypeptide capable of synthesizing *ent*-copalyl diphosphate from GGPP;
(c) a gene encoding an a polypeptide capable of synthesizing *ent*-kaurene from *ent*-copalyl pyrophosphate;
(d) a gene encoding a polypeptide capable of synthesizing *ent*-kaurenoic acid from *ent*-kaurene;
(e) a gene encoding a polypeptide capable of reducing cytochrome P450 complex; and
(f) a gene encoding a polypeptide capable of synthesizing steviol from *ent-*kaurenoic acid;
(g) a gene encoding a polypeptide capable of glycosylating steviol or a steviol glycoside at its C-13 hydroxyl group;
(h) a gene encoding a polypeptide capable of beta 1,3 glycosylation of the C3' of the 13-*O*-glucose, 19-*O*-glucose, or both 13-*O*-glucose and 19-*O-*glucose of a steviol glycoside;
(i) a gene encoding a polypeptide capable of glycosylating steviol or a steviol glycoside at its C-19 carboxyl group;
(j) a gene encoding a polypeptide capable of beta 1,2 glycosylation of the C2' of the 13-*O*-glucose, 19-*O*-glucose, or both 13-*O*-glucose and 19-*O-*glucose of a steviol glycoside; and/or
(k) a gene encoding a bifunctional polypeptide capable of synthesizing *ent-*copalyl diphosphate from GGPP and synthesizing *ent-kaurene* from *ent-*copalyl pyrophosphate;
wherein at least one of the genes is a recombinant gene.

In some aspects of the recombinant host cell disclosed herein:
(a) the polypeptide capable of synthesizing GGPP comprises a polypeptide having at least 70% identity to the amino acid sequence set forth in SEQ ID NO:20, SEQ ID NO:22, SEQ ID NO:24, SEQ ID NO:26, SEQ ID NO:28, SEQ ID NO:30, SEQ ID NO:32, or SEQ ID NO:116;
(b) the polypeptide capable of synthesizing *ent*-copalyl diphosphate comprises a polypeptide having at least 70% identity to the amino acid sequence set forth in SEQ ID NO:34, SEQ ID NO:36, SEQ ID NO:38, SEQ ID NO:40, or SEQ ID NO:42;
(c) the polypeptide capable of synthesizing *ent*-kaurene comprises a polypeptide having at least 70% identity to the amino acid sequence set forth in SEQ ID NO:44, SEQ ID NO:46, SEQ ID NO:48, SEQ ID NO:50, or SEQ ID NO:52;
(d) the polypeptide capable of synthesizing *ent*-kaurenoic acid comprises a polypeptide having at least 70% identity to the amino acid sequence set forth in SEQ ID NO:60, SEQ ID NO:62, SEQ ID NO:66, SEQ ID NO:68, SEQ ID NO:70, SEQ ID NO:72, SEQ ID NO:74, SEQ ID NO:76, or SEQ ID NO:117;
(e) the polypeptide capable of reducing cytochrome P450 complex comprises a polypeptide having at least 70% identity to the amino acid sequence set forth in SEQ ID NO:78, SEQ ID NO:80, SEQ ID NO:82, SEQ ID NO:84, SEQ ID NO:86, SEQ ID NO:88, SEQ ID NO:90, SEQ ID NO:92;
(f) the polypeptide capable of synthesizing steviol comprises a polypeptide having at least 70% identity to the amino acid sequence set forth in SEQ ID NO:94, SEQ ID NO:97, SEQ ID NO:100, SEQ ID NO:101, SEQ ID NO:102, SEQ ID NO:103, SEQ ID NO:104, SEQ ID NO:106, SEQ ID NO:108, SEQ ID NO:110, SEQ ID NO:112, or SEQ ID NO:114;
(g) the polypeptide capable of glycosylating steviol or a steviol glycoside at its C-13 hydroxyl group thereof comprises a polypeptide having at least 55% identity to the amino acid sequence set forth in SEQ ID NO:7;
(h) the polypeptide capable of beta 1,3 glycosylation of the C3' of the 13-*O-*glucose, 19-*O*-glucose, or both 13-*O*-glucose and 19-*O*-glucose of a steviol glycoside comprises a polypeptide having at least 50% identity to the amino acid sequence set forth in SEQ ID NO:9;
(i) the polypeptide capable of glycosylating steviol or a steviol glycoside at its C-19 carboxyl group thereof comprises a polypeptide having at least 55% identity to the amino acid sequence set forth in SEQ ID NO:4;
(j) the polypeptide capable of beta 1,2 glycosylation of the C2' of the 13-*O-*glucose, 19-*O*-glucose, or both 13-*O*-glucose and 19-*O*-glucose of a steviol glycoside comprises a polypeptide having 80% or greater identity to the amino acid sequence set forth in SEQ ID NO:11; a polypeptide having 80% or greater identity to the amino acid sequence set forth in SEQ ID NO:13; or a polypeptide having at least 65% identity to the amino acid sequence set forth in SEQ ID NO:16; and
(k) the bifunctional polypeptide capable of synthesizing *ent*-copalyl diphosphate from GGPP and synthesizing *ent*-kaurene from *ent*-copalyl pyrophosphate comprises a polypeptide having at least 50% identity to the amino acid sequence set forth in SEQ ID NO:54, SEQ ID NO:56, or SEQ ID NO:58.

In some aspects, the recombinant host cell disclosed herein comprises a plant cell, a mammalian cell, an insect cell, a fungal cell, an algal cell, an archeal cell or a bacterial cell.

In some aspects, the bacterial cell comprises *Escherichia* bacteria cells, *Lactobacillus* cells, *Lactococcus, Cornebacterium* cells, *Acetobacter* cells, *Acinetobacter* cells, or *Pseudomonas* cells.

In some aspects, the fungal cell comprises a yeast cell.

In some aspects, the yeast cell is a cell from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous,* or *Candida albicans* species.

In some aspects the yeast cell is a *Saccharomycete.*

In some aspects, the yeast cell is a cell from the *Saccharomyces cerevisiae* species.

The invention further provides a method of producing a steviol glycoside or a steviol glycoside composition, comprising growing the recombinant host cell disclosed herein in a cell culture medium, under conditions in which the genes are expressed, wherein the steviol glycoside or the steviol glycoside composition is produced by the recombinant host cell.

The invention further provides a method for producing a steviol glycoside or a steviol glycoside composition, comprising whole-cell bioconversion of a plant-derived or synthetic steviol and/or steviol glycosides in a cell culture medium using a recombinant polypeptide capable of glycosylating steviol or a steviol glycoside at its C-19 carboxyl group and having at least 80% sequence identity to the amino acid sequence set forth in SEQ IN NO:2 or 119 produced by the recombinant host cell disclosed herein, and synthesizing the steviol glycoside thereby.

In some aspects, the methods disclosed herein further comprises isolating the steviol glycoside or the steviol glycoside composition.

In some aspects of the methods disclosed herein, the isolating step comprises:
(a) providing the cell culture medium comprising the steviol glycoside or the steviol glycoside composition;
(b) separating a liquid phase of the cell culture medium from a solid phase of the cell culture to obtain a supernatant comprising the steviol glycoside or the steviol glycoside composition;
(c) providing one or more adsorbent resins, comprising providing the adsorbent resins in a packed column; and
(d) contacting the supernatant of step (b) with the one or more adsorbent resins in order to obtain at least a portion of the steviol glycoside or the steviol glycoside composition, thereby isolating the steviol glycoside or the steviol glycoside composition.

In some aspects of the methods disclosed herein, the isolating step comprises:
(a) providing the cell culture comprising the steviol glycoside or the steviol glycoside composition;
(b) separating a liquid phase of the cell culture from a solid phase of the cell culture to obtain a supernatant comprising the steviol glycoside or the steviol glycoside composition;
(c) providing one or more ion exchange or ion exchange or reversed-phase chromatography columns; and
(d) contacting the supernatant of step (b) with the one or more ion exchange or ion exchange or reversed-phase chromatography columns in order to obtain at least a portion of the steviol glycoside or the steviol glycoside composition.

In some aspects of the methods disclosed herein, the isolating step comprises:
(a) providing the cell culture comprising the steviol glycoside or the steviol glycoside composition;
(b) separating a liquid phase of the cell culture from a solid phase of the cell culture to obtain a supernatant comprising the steviol glycoside or the steviol glycoside composition;
(c) crystallizing or extracting the steviol glycoside, thereby isolating the steviol glycoside or the steviol glycoside composition.

In some aspects, the methods disclosed herein further comprise recovering the steviol glycoside alone or as a composition comprising the steviol glycoside.

In some aspects of the methods disclosed herein, the recovered composition is enriched for the steviol glycoside, relative to a glycoside composition from a Stevia plant and has a reduced level of Stevia plant-derived components relative to a plant-derived Stevia extract.

In some aspects of the method disclosed herein, the recovered composition has a reduced level of Stevia plant-derived components relative to a plant-derived Stevia extract.

In some aspects of the methods disclosed herein, the cell culture medium comprises:
(a) the steviol glycoside or the steviol glycoside composition produced by the recombinant host cell disclosed herein or whole-cell bioconversion of a plant-derived or synthetic steviol and/or steviol glycosides,
(b) glucose, fructose, and/or sucrose; and/or
(c) supplemental nutrients comprising trace metals, vitamins, salts, yeast nitrogen base (YNB), and/or amino acids.

In some aspects of the method disclosed herein, the steviol glycoside is produced in a permeabilized recombinant host cell which has been transformed with the gene encoding the polypeptide capable of glycosylating steviol or a steviol glycoside at its C-19 carboxyl group.

In some aspects of the methods disclosed herein, the recombinant host comprises a plant cell, a mammalian cell, an insect cell, a fungal cell, an algal cell or a bacterial cell.

In some aspects of the methods disclosed herein, the bacterial cell comprises *Escherichia* cells, *Lactobacillus* cells, *Lactococcus* cells, *Cornebacterium* cells, *Acetobacter* cells, *Acinetobacter* cells, or *Pseudomonas* cells.

In some aspects of the methods disclosed herein, the fungal cell comprises a yeast cell.

In some aspects of the methods disclosed herein, the yeast cell is a cell from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous,* or *Candida albicans* species.

In some aspects of the methods disclosed herein, the yeast cell is a *Saccharomycete.*

In some aspects of the methods disclosed herein, the yeast cell is a cell from the *Saccharomyces cerevisiae* species.

The invention further provides *in vitro* method for producing steviol-19-*O*-glucoside (19-SMG), comprising adding a recombinant polypeptide capable of glycosylating steviol or a steviol glycoside at its C-19 carboxyl group and having at least 80% sequence identity to the amino acid sequence set forth in SEQ IN NO:2 or 119 and a plant-derived or synthetic steviol to a reaction mixture; and synthesizing 19-SMG thereby.

In some aspects of the methods disclosure herein, the reaction mixture comprises:
(a) one or more 19-SMG;
(b) the recombinant polypeptide;
(c) glucose, fructose, and/or sucrose, uridine diphosphate (UDP)-glucose, UDP-rhamnose, UDP-xylose, and/or N-acetyl-glucosamine; and/or
(d) reaction buffer and/or salts.

In some aspects of the methods disclosed herein, the steviol glycoside comprises steviol-13-*O*-glucoside (13-SMG), steviol-1,2-bioside, steviol-1,3-bioside, steviol-19-*O*-glucoside (19-SMG), stevioside, 1,3-stevioside, rubusoside, Rebaudioside A (RebA), Rebaudioside B (RebB), Rebaudioside C (RebC), Rebaudioside D (RebD), Rebaudioside E (RebE), Rebaudioside F (RebF), Rebaudioside M (RebM), Rebaudioside Q (RebQ), Rebaudioside I (Rebl), dulcoside A, or isomers thereof.

The disclosure further provides a cell culture medium, comprising:
(a) the recombinant host cells disclosed herein; and
(b) one or more steviol glycosides produced by the recombinant host cells disclosed herein;
wherein one or more steviol glycosides is present at a concentration of at least 0.1 mg/liter of the cell culture medium.

The disclosure further provides a cell culture medium, comprising:
(a) the recombinant host cells disclosed herein; and
(b) one or more steviol glycosides produced by the recombinant host cells disclosed herein;
wherein one or more steviol glycosides is present at a concentration of at least 0.1 mg/liter of the cell culture medium and the cell culture medium further comprises glucose, sucrose, UDP-glucose, UDP-rhamnose, UDP-xylose, N-acetyl-glucosamine, and/or YNB.

The disclosure further provides a cell culture medium, comprising:
(a) the recombinant host cells disclosed herein; and
(b) one or more steviol glycosides produced by the recombinant host cells disclosed herein;
   wherein one or more steviol glycosides is present at a concentration of at least 0.1 mg/liter of the culture medium and the cell culture medium further comprises glucose, sucrose, UDP-glucose, UDP-rhamnose, UDP-xylose, N-acetyl-glucosamine, and/or YNB; and
   wherein the cell culture medium has a reduced level of Stevia plant-derived components relative to a plant-derived Stevia extract.

The invention further provides a cell culture medium, comprising:
(a) the recombinant host cells disclosed herein; and
(b) one or more steviol glycosides produced by the recombinant host cells disclosed herein;
   wherein one or more steviol glycosides is present at a concentration of at least 0.1 mg/liter of the cell culture medium and the cell culture medium further comprises glucose, sucrose, UDP-glucose, UDP-rhamnose, UDP-xylose, N-acetyl-glucosamine, and/or YNB; and
   wherein the cell culture medium is enriched for 19-SMG relative to a steviol glycoside extract from Stevia plant and has a reduced level of Stevia plant-derived components relative to a plant-derived Stevia extract.

The disclosure further provides a cell lysate comprising one or more steviol glycosides produced by the recombinant host cells disclosed herein, and the cell lysate further comprises glucose, sucrose, UDP-glucose, UDP-rhamnose, UDP-xylose, N-acetyl-glucosamine, and/or YNB.

The invention further provides a steviol glycoside produced by the recombinant host cells disclosed herein.

The invention further provides a steviol glycoside produced by the methods disclosed herein.

The disclosure further provides a sweetener composition, comprising a steviol glycoside produced by the recombinant host cells or the method disclosed herein.

The disclosure further provides a food product comprising the sweetener composition disclosed herein.

The disclosure further provides a beverage or a beverage concentration comprising the sweetener composition disclosed herein.

The invention further provides a composition of steviol glycosides produced by a recombinant host disclosed herein, wherein the relative levels of steviol glycosides in the composition correspond to the relative levels of steviol glycosides in the recombinant host.

In some aspects, the composition further comprises an increased level of 19-SMG relative to a composition of steviol glycosides produced by a corresponding recombinant host cell lacking the gene encoding a polypeptide capable of glycosylating steviol or a steviol glycoside at its C-19 carboxyl group and having at least 80% sequence identity to the amino acid sequence set forth in SEQ IN NO:2 or 119.

These and other features and advantages of the present invention will be more fully understood from the following detailed description taken together with the accompanying claims. It is noted that the scope of the claims is defined by the recitations therein and not by the specific discussion of features and advantages set forth in the present description.

### BRIEF DESCRIPTION OF THE DRAWINGS

The following detailed description of the embodiments of the present invention can be best understood when read in conjunction with the following drawings, where like structure is indicated with like reference numerals and in which:

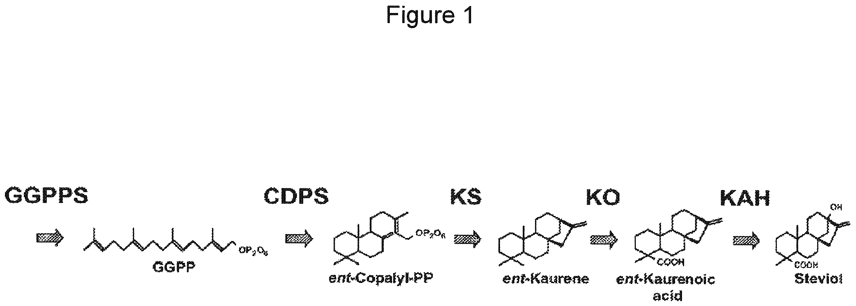

Figure 1 shows a schematic of an engineered biosynthetic pathway for producing steviol in yeast from geranylgeranyl diphosphate using a polypeptide capable of synthesizing geranylgeranyl pyrophosphate (GGPP) from farnesyl diphosphate (FPP) and isopentenyl diphosphate (IPP) (e.g., geranylgeranyl diphosphate synthase (GGPPS)); a polypeptide capable of synthesizing *ent*-copalyl diphosphate from GGPP (*e*.*g*., ent-copalyl diphosphate synthase (CDPS)); a polypeptide capable of synthesizing *ent*-kaurene from *ent*-copalyl pyrophosphate (*e*.*g*., kaurene synthase (KS)); a polypeptide capable of synthesizing *ent*-kaurenoic acid from *ent*-kaurene (*e*.*g*., kaurene oxidase (KO)); a polypeptide capable of reducing cytochrome P450 complex (*e*.*g*., cytochrome P450 reductase (CPR)) (not shown); and a polypeptide capable of synthesizing steviol from *ent*-kaurenoic acid (*e*.*g*., steviol synthase (KAH)).
Figure 2 shows representative steviol glycoside glycosylation reactions catalyzed by suitable uridine 5'-diphospho (UDP) glycosyl transferases (UGT) enzymes and chemical structures for several steviol glycoside compounds.
Figure 3 shows 19-SMG accumulation (in area-under-the-curve; AUC) by a steviol-fed *S. cerevisiae* strain expressing UGT33942 (SEQ ID NO:1, SEQ ID NO:2).
Figure 4 shows liquid chromatography-mass spectrometry (LC-MS) selected-ion recording (SIR) traces, corresponding to the mass of 19-SMG, of samples from a steviol-fed S. *cerevisiae* control strain (top) and a steviol-fed *S. cerevisiae* strain expressing UGT33786 (SEQ ID NO:118, SEQ ID NO:119) (bottom).

### DETAILED DESCRIPTION OF THE INVENTION

Before describing the present invention in detail, a number of terms will be defined. As used herein, the singular forms "a," "an," and "the" include plural referents unless the context clearly dictates otherwise. For example, reference to a "nucleic acid" means one or more nucleic acids.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that can or cannot be utilized in a particular embodiment of the present invention.

For the purposes of describing and defining the present invention it is noted that the term "substantially" is utilized herein to represent the inherent degree of uncertainty that can be attributed to any quantitative comparison, value, measurement, or other representation. The term "substantially" is also utilized herein to represent the degree by which a quantitative representation can vary from a stated reference without resulting in a change in the basic function of the subject matter at issue.

Methods well known to those skilled in the art can be used to construct genetic expression constructs and recombinant cells according to this invention. These methods include *in vitro* recombinant DNA techniques, synthetic techniques, *in vivo* recombination techniques, and polymerase chain reaction (PCR) techniques. See, for example, techniques as described in Green & Sambrook, 2012, MOLECULAR CLONING: A LABORATORY MANUAL, Fourth Edition, Cold Spring Harbor Laboratory, New York; Ausubel et al., 1989, CURRENT PROTOCOLS IN MOLECULAR BIOLOGY, Greene Publishing Associates and Wiley Interscience, New York, and PCR Protocols: A Guide to Methods and Applications (Innis et al., 1990, Academic Press, San Diego, CA).

As used herein, the terms "polynucleotide", "nucleotide", "oligonucleotide", and "nucleic acid" can be used interchangeably to refer to nucleic acid comprising DNA, RNA, derivatives thereof, or combinations thereof, in either single-stranded or double-stranded embodiments depending on context as understood by the skilled worker.

As used herein, the terms "microorganism," "microorganism host," "microorganism host cell," "host," and "host cell" can be used interchangeably. As used herein, the terms "recombinant host" or "recombinant microorganism" are intended to refer to a host, the genome of which has been augmented by at least one DNA sequence. Such DNA sequences include but are not limited to genes that are not naturally present, DNA sequences that are not normally transcribed into RNA or translated into a protein ("expressed"), and other genes or DNA sequences which one desires to introduce into a host. It will be appreciated that typically the genome of a recombinant host described herein is augmented through stable introduction of one or more recombinant genes. Generally, introduced DNA is not originally resident in the host that is the recipient of the DNA, but it is within the scope of this disclosure to isolate a DNA segment from a given host, and to subsequently introduce one or more additional copies of that DNA into the same host, e.g., to enhance production of the product of a gene or alter the expression pattern of a gene. In some instances, the introduced DNA will modify or even replace an endogenous gene or DNA sequence by, *e*.*g*., homologous recombination or site-directed mutagenesis. Suitable recombinant hosts include microorganisms.

As used herein, the term "recombinant gene" refers to a gene or DNA sequence that is introduced into a recipient host, regardless of whether the same or a similar gene or DNA sequence may already be present in such a host. "Introduced," or "augmented" in this context, is known in the art to mean introduced or augmented by the hand of man. Thus, a recombinant gene can be a DNA sequence from another species or can be a DNA sequence that originated from or is present in the same species but has been incorporated into a host by recombinant methods to form a recombinant host. It will be appreciated that a recombinant gene that is introduced into a host can be identical to a DNA sequence that is normally present in the host being transformed, and is introduced to provide one or more additional copies of the DNA to thereby permit overexpression or modified expression of the gene product of that DNA. In some aspects, said recombinant genes are encoded by cDNA. In other embodiments, recombinant genes are synthetic and/or codon-optimized for expression in S. *cerevisiae.*

As used herein, the term "engineered biosynthetic pathway" refers to a biosynthetic pathway that occurs in a recombinant host, as described herein. In some aspects, one or more steps of the biosynthetic pathway do not naturally occur in an unmodified host. In some embodiments, a heterologous version of a gene is introduced into a host that comprises an endogenous version of the gene.

As used herein, the term "endogenous" gene refers to a gene that originates from and is produced or synthesized within a particular organism, tissue, or cell. In some embodiments, the endogenous gene is a yeast gene. In some embodiments, the gene is endogenous to *S*. *cerevisiae,* including, but not limited to *S*. *cerevisiae* strain S288C. In some embodiments, an endogenous yeast gene is overexpressed. As used herein, the term "overexpress" is used to refer to the expression of a gene in an organism at levels higher than the level of gene expression in a wild type organism. *See, e.g.,* Prelich, 2012, Genetics 190:841-54. In some embodiments, an endogenous yeast gene is deleted. *See, e.g*., Giaever & Nislow, 2014, Genetics 197(2):451-65. As used herein, the terms "deletion," "deleted," "knockout," and "knocked out" can be used interchangeably to refer to an endogenous gene that has been manipulated to no longer be expressed in an organism, including, but not limited to, S. *cerevisiae.*

As used herein, the terms "heterologous sequence" and "heterologous coding sequence" are used to describe a sequence derived from a species other than the recombinant host. In some embodiments, the recombinant host is an *S*. *cerevisiae* cell, and a heterologous sequence is derived from an organism other than *S*. *cerevisiae.* A heterologous coding sequence, for example, can be from a prokaryotic microorganism, a eukaryotic microorganism, a plant, an animal, an insect, or a fungus different than the recombinant host expressing the heterologous sequence. In some embodiments, a coding sequence is a sequence that is native to the host.

A "selectable marker" can be one of any number of genes that complement host cell auxotrophy, provide antibiotic resistance, or result in a color change. Linearized DNA fragments of the gene replacement vector then are introduced into the cells using methods well known in the art (see below). Integration of the linear fragments into the genome and the disruption of the gene can be determined based on the selection marker and can be verified by, for example, PCR or Southern blot analysis. Subsequent to its use in selection, a selectable marker can be removed from the genome of the host cell by, *e*.*g*., Cre-LoxP systems (see, *e*.*g*., Gossen et al., 2002, Ann. Rev. Genetics 36:153-173 and U.S. 2006/0014264). Alternatively, a gene replacement vector can be constructed in such a way as to include a portion of the gene to be disrupted, where the portion is devoid of any endogenous gene promoter sequence and encodes none, or an inactive fragment of, the coding sequence of the gene.

As used herein, the terms "variant" and "mutant" are used to describe a protein sequence that has been modified at one or more amino acids, compared to the wild-type sequence of a particular protein.

As used herein, the term "inactive fragment" is a fragment of the gene that encodes a protein having, *e*.*g*., less than about 10% (*e*.*g*., less than about 9%, less than about 8%, less than about 7%, less than about 6%, less than about 5%, less than about 4%, less than about 3%, less than about 2%, less than about 1%, or 0%) of the activity of the protein produced from the full-length coding sequence of the gene. Such a portion of a gene is inserted in a vector in such a way that no known promoter sequence is operably linked to the gene sequence, but that a stop codon and a transcription termination sequence are operably linked to the portion of the gene sequence. This vector can be subsequently linearized in the portion of the gene sequence and transformed into a cell. By way of single homologous recombination, this linearized vector is then integrated in the endogenous counterpart of the gene with inactivation thereof.

As used herein, the term "steviol glycoside" refers to Rebaudioside A (RebA) (CAS # 58543-16-1), Rebaudioside B (RebB) (CAS # 58543-17-2), Rebaudioside C (RebC) (CAS # 63550-99-2), Rebaudioside D (RebD) (CAS # 63279-13-0), Rebaudioside E (RebE) (CAS # 63279-14-1), Rebaudioside F (RebF) (CAS # 438045-89-7), Rebaudioside M (RebM) (CAS # 1220616-44-3), Rubusoside (CAS # 63849-39-4), Dulcoside A (CAS # 64432-06-0), Rebaudioside I (Rebl) (MassBank Record: FU000332), Rebaudioside Q (RebQ), 1,2-Stevioside (CAS # 57817-89-7), 1,3-Stevioside (RebG), 1,2-bioside (MassBank Record: FU000299), 1,3-bioside, Steviol-13-*O*-glucoside (13-SMG), Steviol-19-*O*-glucoside (19-SMG), a tri-glucosylated steviol glycoside, a tetra-glycosylated steviol glycoside, a penta-glucosylated steviol glycoside, a hexa-glucosylated steviol glycoside, a hepta-glucosylated steviol glycoside, and isomers thereof. *See* Figure 2; *see also,* Steviol Glycosides Chemical and Technical Assessment 69th JECFA, 2007, prepared by Harriet Wallin, Food Agric. Org.

As used herein, the terms "steviol glycoside precursor" and "steviol glycoside precursor compound" are used to refer to intermediate compounds in the steviol glycoside biosynthetic pathway. Steviol glycoside precursors include, but are not limited to, geranylgeranyl diphosphate (GGPP), ent-copalyl-diphosphate, ent-kaurene, ent-kaurenol, ent-kaurenal, ent-kaurenoic acid, and steviol. See Figure 1. In some embodiments, steviol glycoside precursors are themselves steviol glycoside compounds. For example, 19-SMG, rubusoside, stevioside, and RebE are steviol glycoside precursors of RebM. *See* Figure 2. Steviol glycosides and/or steviol glycoside precursors can be produced *in vivo (i.e.,* in a recombinant host), *in vitro (i.e.,* enzymatically), or by whole cell bioconversion. As used herein, the terms "produce" and "accumulate" can be used interchangeably to describe synthesis of steviol glycosides and steviol glycoside precursors *in vivo, in vitro,* or by whole cell bioconversion.

Recombinant steviol glycoside-producing *Saccharomyces cerevisiae (S. cerevisiae)* strains are described in WO 2011/153378, WO 2013/022989, WO 2014/122227, and WO 2014/122328. Methods of producing steviol glycosides in recombinant hosts, by whole cell bioconversion, and *in vitro* are also described in WO 2011/153378, WO 2013/022989, WO 2014/122227, and WO 2014/122328.

In some embodiments, steviol glycosides are produced *in vivo* through expression of one or more enzymes involved in the steviol glycoside biosynthetic pathway in a recombinant host. For example, a steviol-producing recombinant host expressing a recombinant gene encoding a polypeptide capable of glycosylating steviol or a steviol glycoside at its C-19 carboxyl group and having at least 80% sequence identity to the amino acid sequence set forth in SEQ IN NO:2 or 119 (UDP-glycosyltransferase (UGT) polypeptide structurally similar to members of the UGT85 family capable of glycosyiating steviol or a steviol glycoside at its C-19 carboxyl group) can produce a steviol glycoside *in vivo.*

In some embodiments, a recombinant host comprising a gene encoding a polypeptide capable of synthesizing geranylgeranyl pyrophosphate (GGPP) from farnesyl diphosphate (FPP) and isopentenyl diphosphate (IPP) (*e*.*g*., geranylgeranyl diphosphate synthase (GGPPS)); a gene encoding a polypeptide capable of synthesizing *ent*-copalyl diphosphate from GGPP (*e*.*g*., ent-copalyl diphosphate synthase (CDPS)); a gene encoding a polypeptide capable of synthesizing *ent*-kaurene from *ent*-copalyl pyrophosphate (*e*.*g*., kaurene synthase (KS)); a gene encoding a polypeptide capable of synthesizing *ent*-kaurenoic acid from ent-kaurene (*e*.*g*., kaurene oxidase (KO)); a gene encoding a polypeptide capable of reducing cytochrome P450 complex (*e*.*g*., cytochrome P450 reductase (CPR); for example, but not limited to a polypeptide capable of electron transfer from NADPH to cytochrome P450 complex during conversion of NADPH to NADP⁺, which is utilized as a cofactor for terpenoid biosynthesis); and a gene encoding a polypeptide capable of synthesizing steviol from *ent-*kaurenoic acid (*e*.*g*., steviol synthase (KAH)); and/or a gene encoding a bifunctional polypeptide capable of synthesizing *ent*-copalyl diphosphate from GGPP and synthesizing ent-kaurene from *ent*-copalyl pyrophosphate (*e*.*g*., an ent-copalyl diphosphate synthase (CDPS) - ent-kaurene synthase (KS) polypeptide) can produce steviol *in vivo. See, e.g.,* Figure 1. The skilled worker will appreciate that one or more of these genes can be endogenous to the host provided that at least one (and in some embodiments, all) of these genes is a recombinant gene introduced into the recombinant host.

In some embodiments, steviol glycosides and/or steviol glycoside precursors are produced *in vivo* through expression of a recombinant gene encoding a polypeptide capable of glycosylating steviol or a steviol glycoside at its C-19 carboxyl group and having at least 80% sequence identity to the amino acid sequence set forth in SEQ IN NO:2 or 119 and one or more enzymes involved in the steviol glycoside biosynthetic pathway in a recombinant host. For example, a steviol-producing recombinant host comprising a gene encoding a polypeptide capable of glycosylating steviol or a steviol glycoside at its C-19 carboxyl group and having at least 80% sequence identity to the amino acid sequence set forth in SEQ IN NO:2 or 119, a gene encoding a polypeptide capable of synthesizing geranylgeranyl pyrophosphate (GGPP) from farnesyl diphosphate (FPP) and isopentenyl diphosphate (IPP); a gene encoding a polypeptide capable of synthesizing *ent*-copalyl diphosphate from GGPP; a gene encoding a polypeptide capable of synthesizing *ent*-kaurene from *ent*-copalyl pyrophosphate; a gene encoding a polypeptide capable of synthesizing *ent*-kaurenoic acid from *ent*-kaurene; a gene encoding a polypeptide capable of reducing cytochrome P450 complex; and/or a gene encoding a bifunctional polypeptide capable of synthesizing *ent*-copalyl diphosphate from GGPP and synthesizing *ent*-kaurene from *ent*-copalyl pyrophosphate; a gene encoding a polypeptide capable of glycosylating steviol or a steviol glycoside at its C-13 hydroxyl group (*e*.*g*., UGT85C2 polypeptide); a gene encoding a polypeptide capable of beta 1,3 glycosylation of the C3' of the 13-*O*-glucose, 19-*O*-glucose, or both 13-*O*-glucose and 19-*O*-glucose of a steviol glycoside (e.g., UGT76G1 polypeptide); a gene encoding a polypeptide capable of glycosylating steviol or a steviol glycoside at its C-19 carboxyl group *(e.g.,* UGT74G1 polypeptide); and/or a gene encoding a polypeptide capable of beta 1,2 glycosylation of the C2' of the 13-*O*-glucose, 19-*O-*glucose, or both 13-*O*-glucose and 19-*O*-glucose of a steviol glycoside (*e*.*g*., UGT91D2 and EUGT11 polypeptide) can produce a steviol glycoside and/or a steviol glycoside precursor *in vivo. See, e.g.,* Figures 1 and 2. The skilled worker will appreciate that one or more of these genes can be endogenous to the host provided that at least one (and in some embodiments, all) of these genes is a recombinant gene introduced into the recombinant host.

In some aspects, the polypeptide capable of synthesizing geranylgeranyl pyrophosphate (GGPP) from farnesyl diphosphate (FPP) and isopentenyl diphosphate (IPP) comprises a polypeptide having the amino acid sequence set forth in SEQ ID NO:20 (which can be encoded by the nucleotide sequence set forth in SEQ ID NO:19), SEQ ID NO:22 (encoded by the nucleotide sequence set forth in SEQ ID NO:21), SEQ ID NO:24 (encoded by the nucleotide sequence set forth in SEQ ID NO:23), SEQ ID NO:26 (encoded by the nucleotide sequence set forth in SEQ ID NO:25), SEQ ID NO:28 (encoded by the nucleotide sequence set forth in SEQ ID NO:27), SEQ ID NO:30 (encoded by the nucleotide sequence set forth in SEQ ID NO:29), SEQ ID NO:32 (encoded by the nucleotide sequence set forth in SEQ ID NO:31), or SEQ ID NO:116 (encoded by the nucleotide sequence set forth in SEQ ID NO:115).

In some aspects, the polypeptide capable of synthesizing *ent*-copalyl diphosphate from GGPP comprises a polypeptide having the amino acid sequence set forth in SEQ ID NO:34 (which can be encoded by the nucleotide sequence set forth in SEQ ID NO:33), SEQ ID NO:36 (encoded by the nucleotide sequence set forth in SEQ ID NO:35), SEQ ID NO:38 (encoded by the nucleotide sequence set forth in SEQ ID NO:37), SEQ ID NO:40 (encoded by the nucleotide sequence set forth in SEQ ID NO:39), or SEQ ID NO:42 (encoded by the nucleotide sequence set forth in SEQ ID NO:41). In some embodiments, the polypeptide capable of synthesizing *ent*-copalyl diphosphate from GGPP lacks a chloroplast transit peptide.

In some aspects, the polypeptide capable of synthesizing *ent*-kaurene from *ent-*copalyl pyrophosphate comprises a polypeptide having the amino acid sequence set forth in SEQ ID NO:44 (which can be encoded by the nucleotide sequence set forth in SEQ ID NO:43), SEQ ID NO:46 (encoded by the nucleotide sequence set forth in SEQ ID NO:45), SEQ ID NO:48 (encoded by the nucleotide sequence set forth in SEQ ID NO:47), SEQ ID NO:50 (encoded by the nucleotide sequence set forth in SEQ ID NO:49), or SEQ ID NO:52 (encoded by the nucleotide sequence set forth in SEQ ID NO:51).

In some embodiments, a recombinant host comprises a gene encoding a bifunctional polypeptide capable of synthesizing *ent*-copalyl diphosphate from GGPP and synthesizing *ent-*kaurene from *ent*-copalyl pyrophosphate. In some aspects, the bifunctional polypeptide comprises a polypeptide having the amino acid sequence set forth in SEQ ID NO:54 (which can be encoded by the nucleotide sequence set forth in SEQ ID NO:53), SEQ ID NO:56 (encoded by the nucleotide sequence set forth in SEQ ID NO:55), or SEQ ID NO:58 (encoded by the nucleotide sequence set forth in SEQ ID NO:57).

In some aspects, the polypeptide capable of synthesizing *ent*-kaurenoic acid from *ent*-kaurene comprises a polypeptide having the amino acid sequence set forth in SEQ ID NO:60 (which can be encoded by the nucleotide sequence set forth in SEQ ID NO:59), SEQ ID NO:62 (encoded by the nucleotide sequence set forth in SEQ ID NO:61), SEQ ID NO:66 (encoded by the nucleotide sequence set forth in SEQ ID NO:65), SEQ ID NO:68 (encoded by the nucleotide sequence set forth in SEQ ID NO:67), SEQ ID NO:70 (encoded by the nucleotide sequence set forth in SEQ ID NO:69), SEQ ID NO:72 (encoded by the nucleotide sequence set forth in SEQ ID NO:71), SEQ ID NO:74 (encoded by the nucleotide sequence set forth in SEQ ID NO:73), SEQ ID NO:76 (encoded by the nucleotide sequence set forth in SEQ ID NO:75), or SEQ ID NO:117 (encoded by the nucleotide sequence set forth in SEQ ID NO:63 or SEQ ID NO:64).

In some aspects, the polypeptide capable of reducing cytochrome P450 complex comprises a polypeptide having the amino acid sequence set forth in SEQ ID NO:78 (which can be encoded by the nucleotide sequence set forth in SEQ ID NO:77), SEQ ID NO:80 (encoded by the nucleotide sequence set forth in SEQ ID NO:79), SEQ ID NO:82 (encoded by the nucleotide sequence set forth in SEQ ID NO:81), SEQ ID NO:84 (encoded by the nucleotide sequence set forth in SEQ ID NO:83), SEQ ID NO:86 (encoded by the nucleotide sequence set forth in SEQ ID NO:85), SEQ ID NO:88 (encoded by the nucleotide sequence set forth in SEQ ID NO:87), SEQ ID NO:90 (encoded by the nucleotide sequence set forth in SEQ ID NO:89), or SEQ ID NO:92 (encoded by the nucleotide sequence set forth in SEQ ID NO:91).

In some aspects, the polypeptide capable of synthesizing steviol from *ent*-kaurenoic acid comprises a polypeptide having the amino acid sequence set forth in SEQ ID NO:94 (which can be encoded by the nucleotide sequence set forth in SEQ ID NO:93), SEQ ID NO:97 (encoded by the nucleotide sequence set forth in SEQ ID NO:95 or SEQ ID NO:96), SEQ ID NO:100 (encoded by the nucleotide sequence set forth in SEQ ID NO:98 or SEQ ID NO:99), SEQ ID NO:101, SEQ ID NO:102, SEQ ID NO:103, SEQ ID NO:104, SEQ ID NO:106 (encoded by the nucleotide sequence set forth in SEQ ID NO:105), SEQ ID NO:108 (encoded by the nucleotide sequence set forth in SEQ ID NO:107), SEQ ID NO:110 (encoded by the nucleotide sequence set forth in SEQ ID NO:109), SEQ ID NO:112 (encoded by the nucleotide sequence set forth in SEQ ID NO:111), or SEQ ID NO:114 (encoded by the nucleotide sequence set forth in SEQ ID NO:113).

In some embodiments, a recombinant host comprises a nucleic acid encoding a polypeptide capable of glycosylating steviol or a steviol glycoside at its C-13 hydroxyl group (*e*.*g*., UGT85C2 polypeptide) (SEQ ID NO:7), a nucleic acid encoding a polypeptide capable of beta 1,3 glycosylation of the C3' of the 13-*O*-glucose, 19-*O*-glucose, or both 13-*O*-glucose and 19-*O*-glucose of a steviol glycoside (e.g., UGT76G1 polypeptide) (SEQ ID NO:9), a nucleic acid encoding a polypeptide capable of glycosylating steviol or a steviol glycoside at its C-19 carboxyl group (*e*.*g*., UGT74G1 polypeptide) (SEQ ID NO:4), a nucleic acid encoding a polypeptide capable of beta 1,2 glycosylation of the C2' of the 13-*O*-glucose, 19-*O*-glucose, or both 13-*O*-glucose and 19-*O*-glucose of a steviol glycoside *(e.g.,* EUGT11 polypeptide) (SEQ ID NO:16), a nucleic acid encoding a polypeptide capable of glycosylating steviol or a steviol glycoside at its C-19 carboxyl group (e.g., UGT33942 polypeptide) (SEQ ID NO:2), and/or a nucleic acid encoding a polypeptide capable of glycosylating steviol or a steviol glycoside at its C-19 carboxyl group (e.g., UGT33786 polypeptide) (SEQ ID NO:119). In some aspects, the polypeptide capable of beta 1,2 glycosylation of the C2' of the 13-*O*-glucose, 19-*O*-glucose, or both 13-*O*-glucose and 19-*O*-glucose of a steviol glycoside (e.g., UGT91D2 polypeptide) can be a UGT91D2e polypeptide (SEQ ID NO:11) or a UGT91D2e-b polypeptide (SEQ ID NO:13).

In some aspects, the polypeptide capable of glycosylating steviol or a steviol glycoside at its C-13 hydroxyl group is encoded by the nucleotide sequence set forth in SEQ ID NO:5 or SEQ ID NO:6, the polypeptide capable of beta 1,3 glycosylation of the C3' of the 13-0-glucose, 19-*O*-glucose, or both 13-*O*-glucose and 19-*O*-glucose of a steviol glycoside is encoded by the nucleotide sequence set forth in SEQ ID NO:8, the polypeptide capable of glycosylating steviol or a steviol glycoside at its C-19 carboxyl group is encoded by the nucleotide sequence set forth in SEQ ID NO:3, the polypeptide capable of beta 1,2 glycosylation of the C2' of the 13-*O*-glucose, 19-*O*-glucose, or both 13-*O*-glucose and 19-*O*-glucose of a steviol glycoside is encoded by the nucleotide sequence set forth in SEQ ID NO: 10,12,14 or 15, the UGT33942 polypeptide is encoded by the nucleotide sequence set forth in SEQ ID NO:1, and the UGT33786 polypeptide is encoded by the nucleotide sequence set forth in SEQ ID NO:118. The skilled worker will appreciate that expression of these genes may be necessary to produce a particular steviol glycoside but that one or more of these genes can be endogenous to the host provided that at least one (and in some embodiments, all) of these genes is a recombinant gene introduced into the recombinant host.

In a particular embodiment, a steviol-producing recombinant microorganism comprises exogenous nucleic acids encoding a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O*-glucose and 19-*O*-glucose of a steviol glycoside, or a polypeptide capable of beta 1,2 glycosylation of the C2' of the 13-*O*-glucose, 19-O-glucose, or both 13-*O*-glucose and 19-*O*-glucose of a steviol glycoside polypeptides.

In another particular embodiment, a steviol-producing recombinant microorganism comprises exogenous nucleic acids encoding a polypeptide capable of glycosylating steviol or a steviol glycoside at its C-13 hydroxyl group; a polypeptide capable of beta 1,3 glycosylation of the C3' of the 13-*O*-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-0-glucose, 19-O-glucose, or both 13-*O*-glucose and 19-*O*-glucose of a steviol glycoside.

In another particular embodiment, a steviol-producing recombinant microorganism comprises exogenous nucleic acids encoding a polypeptide capable of glycosylating steviol or a steviol glycoside at its C-13 hydroxyl group; a polypeptide capable of beta 1,3 glycosylation of the C3' of the 13-*O*-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-0-glucose, 19-O-glucose, or both 13-*O*-glucose and 19-*O*-glucose of a steviol glycoside and/or UGT33942 polypeptide.

In yet another particular embodiment, a steviol-producing recombinant microorganism comprises exogenous nucleic acids encoding a polypeptide capable of glycosylating steviol or a steviol glycoside at its C-13 hydroxyl group; a polypeptide capable of beta 1,3 glycosylation of the C3' of the 13-*O*-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-*O*-glucose, 19-O-glucose, or both 13-*O*-glucose and 19-*O*-glucose of a steviol glycoside, and/or UGT33786 polypeptides.

In yet another particular embodiment, a steviol-producing recombinant microorganism comprises exogenous nucleic acids encoding a polypeptide capable of glycosylating steviol or a steviol glycoside at its C-13 hydroxyl group; a polypeptide capable of beta 1,3 glycosylation of the C3' of the 13-*O*-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-*O*-glucose, 19-O-glucose, or both 13-*O*-glucose and 19-*O*-glucose of a steviol glycoside, and/or UGT33942 polypeptide and UGT33786 polypeptides.

In some embodiments, steviol glycosides and/or steviol glycoside precursors are produced *in vivo* through expression in a recombinant host of one or more enzymes capable of reactions found in the steviol glycoside biosynthetic pathway. For example, a steviol-producing recombinant host expressing one or more of a gene encoding a polypeptide capable of synthesizing geranylgeranyl pyrophosphate (GGPP) from farnesyl diphosphate (FPP) and isopentenyl diphosphate (IPP), a gene encoding a polypeptide capable of synthesizing *ent-*copalyl diphosphate from GGPP, a gene encoding a polypeptide capable of synthesizing *ent-*kaurene from *ent*-copalyl pyrophosphate, a gene encoding a polypeptide capable of synthesizing *ent*-kaurenoic acid from *ent*-kaurene, a gene encoding a polypeptide capable of synthesizing steviol from *ent*-kaurenoic acid, a gene encoding a polypeptide capable of reducing cytochrome P450 complex, and/or a gene encoding a bifunctional polypeptide capable of synthesizing *ent*-copalyl diphosphate from GGPP and synthesizing *ent*-kaurene from *ent*-copalyl pyrophosphate, and one or more of a gene encoding a polypeptide capable of beta 1,3 glycosylation of the C3' of the 13-*O*-glucose, 19-*O*-glucose, or both 13-*O*-glucose and 19-*O-*glucose of a steviol glycoside, a polypeptide capable of beta 1,2 glycosylation of the C2' of the 13-*O*-glucose, 19-*O*-glucose, or both 13-*O*-glucose and 19-*O*-glucose of a steviol glycoside, and a polypeptide capable of glycosylating steviol or a steviol glycoside at its C-13 hydroxyl group or a polypeptide capable of glycosylating steviol or a steviol glycoside at its C-19 carboxyl group, can produce a steviol glycoside and/or steviol glycoside precursors *in vivo.* The skilled worker will appreciate that one or more of these genes can be endogenous to the host provided that at least one (and in some embodiments, all) of these genes is a recombinant gene introduced into the recombinant host.

In some embodiments, steviol glycosides and/or steviol glycoside precursors are produced *in vivo* through expression in a recombinant host of a recombinant gene encoding a UDP-glycosyltransferase (UGT) polypeptide structurally similar to members of the UGT85 family capable of glycosylating steviol or a steviol glycoside at its C-19 carboxyl group *(e.g.,* UGT33942 and UGT33786), and one or more enzymes capable of reactions found in the steviol glycoside biosynthetic pathway. For example, a steviol-producing recombinant host expressing a UDP-glycosyltransferase (UGT) polypeptide structurally similar to members of the UGT85 family capable of glycosylating steviol or a steviol glycoside at its C-19 carboxyl group *(e.g.,* UGT33942 and UGT33786), and one or more of a gene encoding a polypeptide capable of synthesizing geranylgeranyl pyrophosphate (GGPP) from farnesyl diphosphate (FPP) and isopentenyl diphosphate (IPP), a gene encoding a polypeptide capable of synthesizing *ent-*copalyl diphosphate from GGPP, a gene encoding a polypeptide capable of synthesizing *ent-*kaurene from *ent*-copalyl pyrophosphate, a gene encoding a polypeptide capable of synthesizing *ent*-kaurenoic acid from *ent*-kaurene, a gene encoding a polypeptide capable of synthesizing steviol from *ent*-kaurenoic acid, a gene encoding a polypeptide capable of reducing cytochrome P450 complex, and/or a gene encoding a bifunctional polypeptide capable of synthesizing *ent*-copalyl diphosphate from GGPP and synthesizing *ent*-kaurene from *ent*-copalyl pyrophosphate, and one or more of a gene encoding a polypeptide capable of beta 1,3 glycosylation of the C3' of the 13-*O*-glucose, 19-*O*-glucose, or both 13-*O*-glucose and 19-0-glucose of a steviol glycoside, a polypeptide capable of beta 1,2 glycosylation of the C2' of the 13-*O*-glucose, 19-*O*-glucose, or both 13-*O*-glucose and 19-*O*-glucose of a steviol glycoside, and a polypeptide capable of glycosylating steviol or a steviol glycoside at its C-13 hydroxyl group or a polypeptide capable of glycosylating steviol or a steviol glycoside at its C-19 carboxyl group, can produce a steviol glycoside *in vivo.* The skilled worker will appreciate that one or more of these genes can be endogenous to the host provided that at least one (and in some embodiments, all) of these genes is a recombinant gene introduced into the recombinant host.

In another example, a recombinant host expressing a gene encoding a polypeptide capable of synthesizing geranylgeranyl pyrophosphate (GGPP) from farnesyl diphosphate (FPP) and isopentenyl diphosphate (IPP), a gene encoding a polypeptide capable of synthesizing *ent*-copalyl diphosphate from GGPP, a gene encoding a polypeptide capable of synthesizing *ent*-kaurene from *ent*-copalyl pyrophosphate, a gene encoding a polypeptide capable of synthesizing *ent*-kaurenoic acid from *ent*-kaurene, a gene encoding a polypeptide capable of synthesizing steviol from *ent*-kaurenoic acid, a gene encoding a polypeptide capable of reducing cytochrome P450 complex, and/or a gene encoding a bifunctional polypeptide capable of synthesizing *ent*-copalyl diphosphate from GGPP and synthesizing *ent*-kaurene from *ent*-copalyl pyrophosphate can produce steviol *in vivo.* The skilled worker will appreciate that one or more of these genes can be endogenous to the host provided that at least one (and in some embodiments, all) of these genes is a recombinant gene introduced into the recombinant host.

In another example, a recombinant host expressing a recombinant gene encoding a UDP-glycosyltransferase (UGT) polypeptide structurally similar to members of the UGT85 family capable of glycosylating steviol or a steviol glycoside at its C-19 carboxyl group (e.g., UGT33942 and UGT33786), a gene encoding a polypeptide capable of synthesizing geranylgeranyl pyrophosphate (GGPP) from farnesyl diphosphate (FPP) and isopentenyl diphosphate (IPP), a gene encoding a polypeptide capable of synthesizing *ent*-copalyl diphosphate from GGPP, a gene encoding a polypeptide capable of synthesizing *ent*-kaurene from *ent*-copalyl pyrophosphate, a gene encoding a polypeptide capable of synthesizing *ent-*kaurenoic acid from *ent*-kaurene, a gene encoding a polypeptide capable of synthesizing steviol from *ent*-kaurenoic acid, a gene encoding a polypeptide capable of reducing cytochrome P450 complex, and/or a gene encoding a bifunctional polypeptide capable of synthesizing *ent*-copalyl diphosphate from GGPP and synthesizing *ent-kaurene* from *ent*-copalyl pyrophosphate can produce a steviol glycoside *in vivo.* The skilled worker will appreciate that one or more of these genes can be endogenous to the host provided that at least one (and in some embodiments, all) of these genes is a recombinant gene introduced into the recombinant host.

In another example, a recombinant host expressing a gene encoding a polypeptide capable of synthesizing geranylgeranyl pyrophosphate (GGPP) from farnesyl diphosphate (FPP) and isopentenyl diphosphate (IPP), a gene encoding a polypeptide capable of synthesizing *ent*-copalyl diphosphate from GGPP, a gene encoding a polypeptide capable of synthesizing ent-kaurene from *ent*-copalyl pyrophosphate, a gene encoding a polypeptide capable of synthesizing *ent*-kaurenoic acid from *ent*-kaurene, a gene encoding a polypeptide capable of synthesizing steviol from *ent*-kaurenoic acid, a gene encoding a polypeptide capable of reducing cytochrome P450 complex, and/or a bifunctional polypeptide capable of synthesizing *ent*-copalyl diphosphate from GGPP and synthesizing *ent*-kaurene from *ent*-copalyl pyrophosphate and one or more of a gene encoding a polypeptide capable of beta 1,3 glycosylation of the C3' of the 13-*O*-glucose, 19-*O*-glucose, or both 13-*O*-glucose and 19-*O-*glucose of a steviol glycoside, a polypeptide capable of beta 1,2 glycosylation of the C2' of the 13-*O*-glucose, 19-*O*-glucose, or both 13-*O*-glucose and 19-*O*-glucose of a steviol glycoside, and a polypeptide capable of glycosylating steviol or a steviol glycoside at its C-13 hydroxyl group or a polypeptide capable of glycosylating steviol or a steviol glycoside at its C-19 carboxyl group, can produce a steviol glycoside *in vivo.* The skilled worker will appreciate that one or more of these genes can be endogenous to the host provided that at least one (and in some embodiments, all) of these genes is a recombinant gene introduced into the recombinant host.

In another example, a recombinant host expressing a recombinant gene encoding a UDP-glycosyltransferase (UGT) polypeptide structurally similar to members of the UGT85 family capable of glycosylating steviol or a steviol glycoside at its C-19 carboxyl group (*e.g.,* UGT33942 and UGT33786), a gene encoding a polypeptide capable of synthesizing geranylgeranyl pyrophosphate (GGPP) from farnesyl diphosphate (FPP) and isopentenyl diphosphate (IPP), a gene encoding a polypeptide capable of synthesizing *ent*-copalyl diphosphate from GGPP, a gene encoding a polypeptide capable of synthesizing *ent*-kaurene from *ent*-copalyl pyrophosphate, a gene encoding a polypeptide capable of synthesizing *ent-*kaurenoic acid from *ent*-kaurene, a gene encoding a polypeptide capable of synthesizing steviol from *ent*-kaurenoic acid, a gene encoding polypeptide a capable of reducing cytochrome P450 complex, and/or a bifunctional polypeptide capable of synthesizing *ent*-copalyl diphosphate from GGPP and synthesizing *ent*-kaurene from *ent*-copalyl pyrophosphate and one or more of a gene encoding a polypeptide capable of beta 1,3 glycosylation of the C3' of the 13-*O*-glucose, 19-*O*-glucose, or both 13-*O*-glucose and 19-*O*-glucose of a steviol glycoside, a polypeptide capable of beta 1,2 glycosylation of the C2' of the 13-*O*-glucose, 19-*O*-glucose, or both 13-*O-*glucose and 19-*O*-glucose of a steviol glycoside, and a polypeptide capable of glycosylating steviol or a steviol glycoside at its C-13 hydroxyl group or a polypeptide capable of glycosylating steviol or a steviol glycoside at its C-19 carboxyl group, can produce a steviol glycoside *in vivo.* The skilled worker will appreciate that one or more of these genes can be endogenous to the host provided that at least one (and in some embodiments, all) of these genes is a recombinant gene introduced into the recombinant host.

In some embodiments, the steviol glycoside comprises steviol-13-*O*-glucoside (13-SMG), steviol-1,2-bioside, steviol-1,3-bioside, steviol-19-*O*-glucoside (19-SMG), stevioside, 1,3-stevioside, rubusoside, Rebaudioside A (RebA), Rebaudioside B (RebB), Rebaudioside C (RebC), Rebaudioside D (RebD), Rebaudioside E (RebE), Rebaudioside F (RebF), Rebaudioside M (RebM), Rebaudioside Q (RebQ), Rebaudioside I (Rebl), dulcoside A, or isomers thereof.

In some aspects, the polypeptide capable of synthesizing geranylgeranyl pyrophosphate (GGPP) from farnesyl diphosphate (FPP) and isopentenyl diphosphate (IPP) comprises a polypeptide having the amino acid sequence set forth in SEQ ID NO:20 (which can be encoded by the nucleotide sequence set forth in SEQ ID NO:19), SEQ ID NO:22 (encoded by the nucleotide sequence set forth in SEQ ID NO:21), SEQ ID NO:24 (encoded by the nucleotide sequence set forth in SEQ ID NO:23), SEQ ID NO:26 (encoded by the nucleotide sequence set forth in SEQ ID NO:25), SEQ ID NO:28 (encoded by the nucleotide sequence set forth in SEQ ID NO:27), SEQ ID NO:30 (encoded by the nucleotide sequence set forth in SEQ ID NO:29), SEQ ID NO:32 (encoded by the nucleotide sequence set forth in SEQ ID NO:31), or SEQ ID NO:116 (encoded by the nucleotide sequence set forth in SEQ ID NO:115).

In some aspects, the polypeptide capable of synthesizing *ent*-copalyl diphosphate from GGPP comprises a polypeptide having the amino acid sequence set forth in SEQ ID NO:34 (which can be encoded by the nucleotide sequence set forth in SEQ ID NO:33), SEQ ID NO:36 (encoded by the nucleotide sequence set forth in SEQ ID NO:35), SEQ ID NO:38 (encoded by the nucleotide sequence set forth in SEQ ID NO:37), SEQ ID NO:40 (encoded by the nucleotide sequence set forth in SEQ ID NO:39), or SEQ ID NO:42 (encoded by the nucleotide sequence set forth in SEQ ID NO:41). In some embodiments, the polypeptide capable of synthesizing *ent*-copalyl diphosphate from GGPP lacks a chloroplast transit peptide.

In some aspects, the polypeptide capable of synthesizing *ent*-kaurene from *ent-*copalyl pyrophosphate comprises a polypeptide having the amino acid sequence set forth in SEQ ID NO:44 (which can be encoded by the nucleotide sequence set forth in SEQ ID NO:43), SEQ ID NO:46 (encoded by the nucleotide sequence set forth in SEQ ID NO:45), SEQ ID NO:48 (encoded by the nucleotide sequence set forth in SEQ ID NO:47), SEQ ID NO:50 (encoded by the nucleotide sequence set forth in SEQ ID NO:49), or SEQ ID NO:52 (encoded by the nucleotide sequence set forth in SEQ ID NO:51).

In some aspects, the polypeptide capable of synthesizing *ent*-kaurenoic acid from *ent*-kaurene comprises a polypeptide having the amino acid sequence set forth in SEQ ID NO:60 (which can be encoded by the nucleotide sequence set forth in SEQ ID NO:59), SEQ ID NO:62 (encoded by the nucleotide sequence set forth in SEQ ID NO:61), SEQ ID NO:66 (encoded by the nucleotide sequence set forth in SEQ ID NO:65), SEQ ID NO:68 (encoded by the nucleotide sequence set forth in SEQ ID NO:67), SEQ ID NO:70 (encoded by the nucleotide sequence set forth in SEQ ID NO:69), SEQ ID NO:72 (encoded by the nucleotide sequence set forth in SEQ ID NO:71), SEQ ID NO:74 (encoded by the nucleotide sequence set forth in SEQ ID NO:73), SEQ ID NO:76 (encoded by the nucleotide sequence set forth in SEQ ID NO:75), or SEQ ID NO:117 (encoded by the nucleotide sequence set forth in SEQ ID NO:63 or SEQ ID NO:64).

In some aspects, the polypeptide capable of reducing cytochrome P450 complex comprises a polypeptide having the amino acid sequence set forth in SEQ ID NO:78 (which can be encoded by the nucleotide sequence set forth in SEQ ID NO:77), SEQ ID NO:80 (encoded by the nucleotide sequence set forth in SEQ ID NO:79), SEQ ID NO:82 (encoded by the nucleotide sequence set forth in SEQ ID NO:81), SEQ ID NO:84 (encoded by the nucleotide sequence set forth in SEQ ID NO:83), SEQ ID NO:86 (encoded by the nucleotide sequence set forth in SEQ ID NO:85), SEQ ID NO:88 (encoded by the nucleotide sequence set forth in SEQ ID NO:87), SEQ ID NO:90 (encoded by the nucleotide sequence set forth in SEQ ID NO:89), or SEQ ID NO:92 (encoded by the nucleotide sequence set forth in SEQ ID NO:91).

In some aspects, the polypeptide capable of synthesizing steviol from *ent*-kaurenoic acid comprises a polypeptide having the amino acid sequence set forth in SEQ ID NO:94 (which can be encoded by the nucleotide sequence set forth in SEQ ID NO:93), SEQ ID NO:97 (encoded by the nucleotide sequence set forth in SEQ ID NO:95 or SEQ ID NO:96), SEQ ID NO:100 (encoded by the nucleotide sequence set forth in SEQ ID NO:98 or SEQ ID NO:99), SEQ ID NO:101, SEQ ID NO:102, SEQ ID NO:103, SEQ ID NO:104, SEQ ID NO:106 (encoded by the nucleotide sequence set forth in SEQ ID NO:105), SEQ ID NO:108 (encoded by the nucleotide sequence set forth in SEQ ID NO:107), SEQ ID NO:110 (encoded by the nucleotide sequence set forth in SEQ ID NO:109), SEQ ID NO:112 (encoded by the nucleotide sequence set forth in SEQ ID NO:111), or SEQ ID NO:114 (encoded by the nucleotide sequence set forth in SEQ ID NO:113).

In some aspects, the bifunctional polypeptide capable of synthesizing *ent*-copalyl diphosphate from GGPP and synthesizing *ent*-kaurene from *ent*-copalyl pyrophosphate comprises a polypeptide having at least 50% identity to the amino acid sequence set forth in SEQ ID NO:54, SEQ ID NO:56, or SEQ ID NO:58.

In some aspects, the polypeptide capable of glycosylating steviol or a steviol glycoside at its C-13 hydroxyl group comprises a polypeptide having the amino acid sequence set forth in SEQ ID NO:7 (which can be encoded by the nucleotides sequence set forth in SEQ ID NO:5 or SEQ ID NO:6). In some aspects, the polypeptide capable of beta 1,3 glycosylation of the C3' of the 13-*O*-glucose, 19-*O*-glucose, or both 13-*O*-glucose and 19-*O*-glucose of a steviol glycoside comprises a polypeptide having a sequence set forth in SEQ ID NO:9 (encoded by the nucleotide sequence set forth in SEQ ID NO:8). In some aspects, the polypeptide capable of glycosylating steviol or a steviol glycoside at its C-19 carboxyl group comprises a polypeptide having a sequence set forth in SEQ ID NO:2, SEQ ID NO:4, or SEQ ID NO:119 (encoded by the nucleotide sequence set forth in SEQ ID NO:1, SEQ ID NO:3, or SEQ ID NO:118). In some aspects, the polypeptide capable of beta 1,2 glycosylation of the C2' of the 13-*O*-glucose, 19-*O*-glucose, or both 13-*O*-glucose and 19-*O*-glucose of a steviol glycoside comprises a polypeptide having the amino acid sequence set forth in SEQ ID NO:16, SEQ ID NO:11, or SEQ ID NO:13 (encoded by the nucleotide sequence set forth in SEQ ID NO:10, SEQ ID NO:12, SEQ ID NO:14, or SEQ ID NO:15).

In some embodiments, the polypeptide capable of synthesizing geranylgeranyl pyrophosphate (GGPP) from farnesyl diphosphate (FPP) and isopentenyl diphosphate (IPP) is a geranylgeranyl diphosphate synthase (GGPPS) polypeptide. In some embodiments, the polypeptide capable of synthesizing *ent*-copalyl diphosphate from GGPP is an *ent*-copalyl diphosphate synthase (CDPS) polypeptide. In some embodiments, the polypeptide capable of synthesizing ent-kaurene from *ent*-copalyl pyrophosphate is an ent-kaurene synthase (KS) polypeptide. In some embodiments, the polypeptide capable of synthesizing *ent*-kaurenoic acid from *ent*-kaurene is an *ent*-kaurene oxidase (KO) polypeptide. In some embodiments, the polypeptide capable of synthesizing steviol from *ent*-kaurenoic acid is an *ent*-kaurenoic acid hydroxylase (KAH) polypeptide. In some embodiments, the polypeptide capable of reducing cytochrome P450 complex is a cytochrome P450 reductase (CPR) polypeptide. In some embodiments, the polypeptide capable of beta 1,3 glycosylation of the C3' of the 13-*O*-glucose, 19-*O*-glucose, or both 13-*O*-glucose and 19-*O*-glucose of a steviol glycoside is a polypeptide capable of beta 1,3 glycosylation of the C3' of the 13-*O*-glucose, 19-*O*-glucose, or both 13-*O-*glucose and 19-*O*-glucose of a steviol glycoside. In some embodiments, the polypeptide capable of glycosylating steviol or a steviol glycoside at its C-13 hydroxyl group thereof is a polypeptide capable of glycosylating steviol or a steviol glycoside at its C-13 hydroxyl group. In some embodiments, the bifunctional polypeptide capable of synthesizing *ent*-copalyl diphosphate from GGPP and synthesizing *ent*-kaurene from *ent*-copalyl pyrophosphate is an *ent*-copalyl diphosphate synthase (CDPS) - *ent*-kaurene synthase (KS) polypeptide. In some embodiments, the polypeptide capable of glycosylating steviol or a steviol glycoside at its C-19 carboxyl group is a UGT74G1 polypeptide, a UGT33942 polypeptide, or a UGT33786 polypeptide. In some embodiments, the polypeptide capable of beta 1,2 glycosylation of the C2' of the 13-*O*-glucose, 19-*O*-glucose, or both 13-*O*-glucose and 19-*O*-glucose of a steviol glycoside is a UGT91D2 polypeptide such as UGT91D2e or UGT91D2e-b, or a EUGT11 polypeptide.

In certain embodiments, the steviol glycoside is RebA, RebB, RebD, and/or RebM. RebA can be synthesized, for example, in a steviol-producing recombinant microorganism expressing a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O*-glucose and 19-*O*-glucose of a steviol glycoside, polypeptide capable of beta 1,2 glycosylation of the C2' of the 13-*O*-glucose, 19-*O*-glucose, or both 13-*O-*glucose and 19-*O*-glucose of a steviol glycoside, and i) a polypeptide capable of glycosylating steviol or a steviol glycoside at its C-19 carboxyl group, ii) UGT33942, and/or iii) UGT33786. RebB can be synthesized, for example, in a steviol-producing recombinant microorganism expressing a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O*-glucose and 19-*O*-glucose of a steviol glycoside, and polypeptide capable of beta 1,2 glycosylation of the C2' of the 13-*O*-glucose, 19-*O*-glucose, or both 13-*O-*glucose and 19-*O*-glucose of a steviol glycoside. RebD can be synthesized, for example, in a steviol-producing recombinant microorganism expressing a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O*-glucose and 19-*O-*glucose of a steviol glycoside, polypeptide capable of beta 1,2 glycosylation of the C2' of the 13-O-glucose, 19-*O*-glucose, or both 13-*O*-glucose and 19-*O*-glucose of a steviol glycoside, and i) a polypeptide capable of glycosylating steviol or a steviol glycoside at its C-19 carboxyl group, ii) UGT33942, and/or iii) UGT33786. RebM can be synthesized, for example, in a steviol-producing recombinant microorganism expressing a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O*-glucose and 19-*O*-glucose of a steviol glycoside, polypeptide capable of beta 1,2 glycosylation of the C2' of the 13-*O*-glucose, 19-*O*-glucose, or both 13-*O*-glucose and 19-*O*-glucose of a steviol glycoside, and i) a polypeptide capable of glycosylating steviol or a steviol glycoside at its C-19 carboxyl group, ii) UGT33942, and/or iii) UGT33786 (see Figure 2).

In certain embodiments, the steviol glycoside is RebA, RebB, RebD, and/or RebM. RebA can be synthesized in a steviol-producing recombinant microorganism expressing a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O*-glucose and 19-*O*-glucose of a steviol glycoside, polypeptide capable of beta 1,2 glycosylation of the C2' of the 13-*O*-glucose, 19-*O*-glucose, or both 13-*O*-glucose and 19-*O-*glucose of a steviol glycoside, and i) a polypeptide capable of glycosylating steviol or a steviol glycoside at its C-19 carboxyl group, ii) UGT33942, and/or iii) UGT33786; RebB can be synthesized in a steviol-producing recombinant microorganism expressing a polypeptide capable of glycosylating steviol or a steviol glycoside at its C-13 hydroxyl group, a polypeptide capable of beta 1,3 glycosylation of the C3' of the 13-*O*-glucose, 19-*O*-glucose, or both 13-*O-*glucose and 19-*O*-glucose of a steviol glycoside, and polypeptide capable of beta 1,2 glycosylation of the C2' of the 13-*O*-glucose, 19-*O*-glucose, or both 13-*O*-glucose and 19-*O-*glucose of a steviol glycoside; RebD can be synthesized in a steviol-producing recombinant microorganism expressing a polypeptide capable of glycosylating steviol or a steviol glycoside at its C-13 hydroxyl group; a polypeptide capable of beta 1,3 glycosylation of the C3' of the 13-O-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-*O*-glucose, 19-*O-*glucose, or both 13-*O*-glucose and 19-*O*-glucose of a steviol glycoside, and i) a polypeptide capable of glycosylating steviol or a steviol glycoside at its C-19 carboxyl group, ii) UGT33942, and/or iii) UGT33786; RebM can be synthesized in a steviol-producing recombinant microorganism expressing a polypeptide capable of glycosylating steviol or a steviol glycoside at its C-13 hydroxyl group; a polypeptide capable of beta 1,3 glycosylation of the C3' of the 13-O-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-*O*-glucose, 19-*O-*glucose, or both 13-*O*-glucose and 19-*O*-glucose of a steviol glycoside, and i) a polypeptide capable of glycosylating steviol or a steviol glycoside at its C-19 carboxyl group, ii) UGT33942, and/or iii) UGT33786; wherein the steviol-producing host expresses one or more of a gene encoding a polypeptide capable of synthesizing geranylgeranyl pyrophosphate (GGPP) from farnesyl diphosphate (FPP) and isopentenyl diphosphate (IPP) (*e*.*g*., geranylgeranyl diphosphate synthase (GGPPS)); a gene encoding a polypeptide capable of synthesizing *ent-*copalyl diphosphate from GGPP (*e*.*g*., ent-copalyl diphosphate synthase (CDPS)); a gene encoding a polypeptide capable of synthesizing *ent*-kaurene from *ent*-copalyl pyrophosphate (*e*.*g*., kaurene synthase (KS)); a gene encoding a polypeptide capable of synthesizing *ent-*kaurenoic acid from *ent*-kaurene (*e*.*g*., kaurene oxidase (KO)); a gene encoding a polypeptide capable of reducing cytochrome P450 complex (*e*.*g*., cytochrome P450 reductase (CPR)); and a gene encoding a polypeptide capable of synthesizing steviol from *ent*-kaurenoic acid (*e*.*g*., steviol synthase (KAH)); and/or a gene encoding a bifunctional polypeptide capable of synthesizing *ent*-copalyl diphosphate from GGPP and synthesizing *ent*-kaurene from *ent*-copalyl pyrophosphate (*e*.*g*., an ent-copalyl diphosphate synthase (CDPS) - *ent*-kaurene synthase (KS) polypeptide), and a gene encoding a UGT polypeptide can produce a steviol glycoside and/or steviol glycoside precursors *in vivo.*

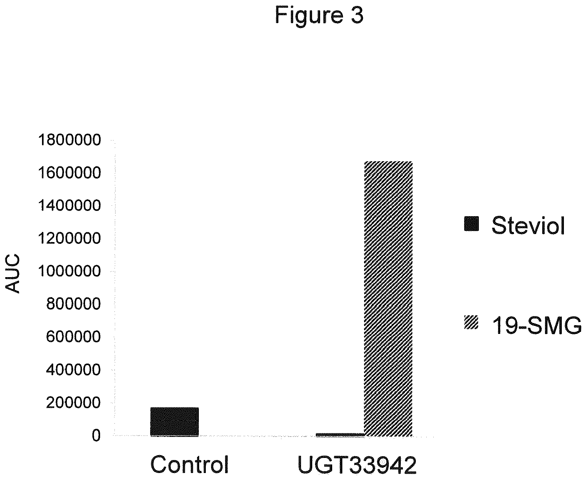

In some embodiments, an S. *cerevisiae* strain expressing a gene encoding a UGT33942 polypeptide (SEQ ID NO:1, SEQ ID NO:2) converts steviol to 19-SMG. UGT33942 is a UGT polypeptide with high structural similarity to members of the UGT85 family and catalyzes glycosylation of the 19-0 position of steviol and steviol glycosides. See Example 1 and Figure 3. In some embodiments, UGT33942 catalyzes conversion of i) steviol-1,2-bioside to stevioside, ii) steviol-1,3-bioside to 1,3-stevioside, iii) 13-SMG to rubusoside, and/or iv) RebB to RebA.

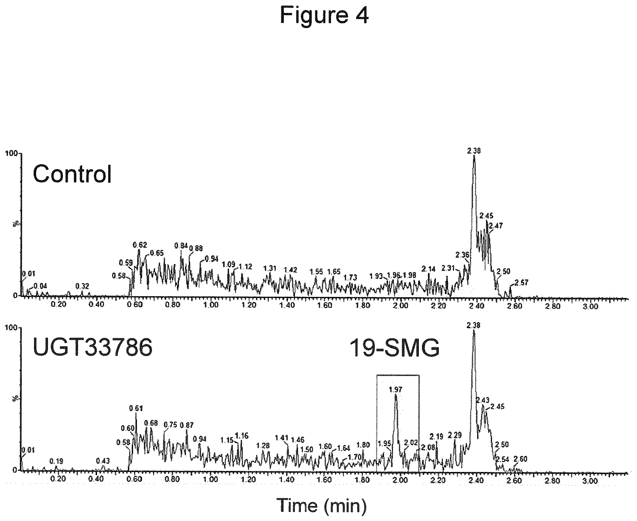

In some embodiments, an S. *cerevisiae* strain expressing a gene encoding a UGT33786 polypeptide (SEQ ID NO:118, SEQ ID NO:119) converts steviol to 19-SMG. UGT33786 is a UGT polypeptide with high structural similarity to members of the UGT85 family and catalyzes glycosylation of the 19-0 position of steviol and steviol glycosides. *See* Example 2 and Figure 4. In some embodiments, UGT33786 catalyzes conversion of i) steviol-1,2-bioside to stevioside, ii) steviol-1,3-bioside to 1,3-stevioside, iii) 13-SMG to rubusoside, and/or iv) RebB to RebA.

In some embodiments, steviol glycosides and/or steviol glycoside precursors are produced through contact of a steviol glycoside precursor with one or more enzymes involved in the steviol glycoside pathway *in vitro.* For example, contacting steviol with a UGT polypeptide can result in production of a steviol glycoside *in vitro.* In some embodiments, a steviol glycoside precursor is produced through contact of an upstream steviol glycoside precursor with one or more enzymes involved in the steviol glycoside pathway *in vitro.* For example, contacting ent-kaurenoic acid with a polypeptide capable of synthesizing steviol from *ent*-kaurenoic acid can result in production of steviol *in vitro.*

In some embodiments, steviol glycosides are produced *in vitro* through contact of a steviol glycoside and/or a steviol glycoside precursor with a polypeptide capable of glycosylating steviol or a steviol glycoside at its C-19 carboxyl group. For example, contacting steviol with a polypeptide capable of glycosylating steviol or a steviol glycoside at its C-19 carboxyl group can result in production of a steviol glycoside *in vitro.* In some embodiments, the polypeptide capable of glycosylating steviol or a steviol glycoside at its C-19 carboxyl group is a UGT33786 polypeptide. In some embodiments, the UGT33942 polypeptide comprises a polypeptide having at least 80% identity to the amino acid sequence set forth in SEQ ID NO:2. In some embodiments, the UGT33786 polypeptide comprises a polypeptide having at least 80% identity to the amino acid sequence set forth in SEQ ID NO:119. In some embodiments, the steviol glycoside produced is 19-SMG. In some embodiments, the contact occurs in a reaction mixture comprising 19-SMG, a UDP-glycosyltransferase (UGT) polypeptide structurally similar to members of the UGT85 family capable of glycosylating steviol or a steviol glycoside at its C-19 carboxyl group (e.g., UGT33942 and UGT33786), uridine diphosphate (UDP)-glucose, UDP-rhamnose, UDP-xylose, and/or N-acetyl-glucosamine, and/or reaction buffer and/or salts.

In some embodiments, a steviol glycoside or steviol glycoside precursor is produced by whole cell bioconversion. For whole cell bioconversion to occur, a host cell expressing one or more enzymes involved in the steviol glycoside pathway takes up and modifies the steviol glycoside or steviol glycoside precursor in the cell; following modification *in vivo,* the steviol glycoside or steviol glycoside precursor remains in the cell and/or is excreted into the cell culture medium. For example, a recombinant host cell expressing a gene encoding a UGT polypeptide can take up steviol and glycosylate steviol in the cell; following glycosylation *in vivo,* a steviol glycoside can be excreted into the cell culture medium. In some embodiments, the cell is permeabilized to take up a substrate to be modified or to excrete a modified product. In another example, a recombinant host cell expressing a gene encoding a UGT polypeptide can take up steviol and glycosylate steviol in the cell; following glycosylation *in vivo,* a steviol glycoside can be excreted into the cell culture medium. A permeabilized recombinant host cell can then be added to the cell culture medium to take up the excreted steviol glycoside to be further modified and to excrete a further modified product.

In some embodiments, the UGT polypeptide can be a polypeptide capable of glycosylating steviol or a steviol glycoside at its C-13 hydroxyl group; a polypeptide capable of beta 1,3 glycosylation of the C3' of the 13-*O*-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-*O*-glucose, 19-O-glucose, or both 13-*O*-glucose and 19-*O*-glucose of a steviol glycoside, and i) a polypeptide capable of glycosylating steviol or a steviol glycoside at its C-19 carboxyl group and/or ii) a UDP-glycosyltransferase (UGT) polypeptide structurally similar to members of the UGT85 family capable of glycosylating steviol or a steviol glycoside at its C-19 carboxyl group (*e*.*g*., UGT33942 and UGT33786).

In another example, a steviol-producing recombinant host expressing one or more of a gene encoding a polypeptide capable of synthesizing geranylgeranyl pyrophosphate (GGPP) from farnesyl diphosphate (FPP) and isopentenyl diphosphate (IPP) (*e*.*g*., geranylgeranyl diphosphate synthase (GGPPS)); a gene encoding a polypeptide capable of synthesizing *ent-*copalyl diphosphate from GGPP (*e*.*g*., ent-copalyl diphosphate synthase (CDPS)); a gene encoding a polypeptide capable of synthesizing *ent*-kaurene from *ent*-copalyl pyrophosphate (*e*.*g*., kaurene synthase (KS)); a gene encoding a polypeptide capable of synthesizing *ent-*kaurenoic acid from *ent*-kaurene (*e*.*g*., kaurene oxidase (KO)); a gene encoding a polypeptide capable of reducing cytochrome P450 complex (*e*.*g*., cytochrome P450 reductase (CPR)); and a gene encoding a polypeptide capable of synthesizing steviol from *ent*-kaurenoic acid (*e*.*g*., steviol synthase (KAH)); and/or a gene encoding a bifunctional polypeptide capable of synthesizing *ent*-copalyl diphosphate from GGPP and synthesizing *ent*-kaurene from *ent*-copalyl pyrophosphate (e.g., an ent-copalyl diphosphate synthase (CDPS) - *ent*-kaurene synthase (KS) polypeptide) can produce steviol in the cell; following production *in vivo,* steviol can be excreted into the cell culture medium. A permeabilized recombinant host cell expressing a gene encoding a UGT polypeptide capable of glycosylating steviol or a steviol glycoside *in vivo* can then be added to the cell culture medium to take up the excreted steviol to be modified and to excrete a modified product.

In some embodiments, the UGT polypeptide can be a polypeptide capable of glycosylating steviol or a steviol glycoside at its C-13 hydroxyl group; a polypeptide capable of beta 1,3 glycosylation of the C3' of the 13-*O*-glucose, 19-*O*-glucose, or both 13-*O*-glucose and 19-*O*-glucose of a steviol glycoside; a polypeptide capable of glycosylating steviol or a steviol glycoside at its C-19 carboxyl group; and a polypeptide capable of beta 1,2 glycosylation of the C2' of the 13-*O*-glucose, 19-O-glucose, or both 13-*O*-glucose and 19-*O*-glucose of a steviol glycoside, and i) a polypeptide capable of glycosylating steviol or a steviol glycoside at its C-19 carboxyl group and/or ii) a UDP-glycosyltransferase (UGT) polypeptide structurally similar to members of the UGT85 family capable of glycosylating steviol or a steviol glycoside at its C-19 carboxyl group (e.g., UGT33942 and UGT33786).

In some embodiments, a steviol glycoside or steviol glycoside precursor is produced by whole cell bioconversion in a host cell expressing of one or more enzymes involved in the steviol glycoside biosynthetic pathway in a recombinant host. For example, a host cell expressing a recombinant gene encoding a polypeptide capable of glycosylating steviol or a steviol glycoside at its C-19 carboxyl group can modify steviol glycosides and/or steviol glycoside precursors in *vivo.*

In some embodiments, a polypeptide capable of glycosylating steviol or a steviol glycoside at its C-19 carboxyl group can be displayed on the surface of the recombinant host cells disclosed herein by fusing it with the anchoring motifs. The polypeptide capable of glycosylating steviol or a steviol glycoside at its C-19 carboxyl group to be displayed - the passenger protein - can be fused to an anchoring motif - the carrier protein - by N-terminal fusion, C-terminal fusion or sandwich fusion. Such cell-surface display can be used for production of steviol glycosides and steviol glycoside precursors by whole cell bioconversion.

In some embodiments, the polypeptide capable of glycosylating steviol or a steviol glycoside at its C-19 carboxyl group is a UGT33942 polypeptide. In some embodiments, the polypeptide capable of glycosylating steviol or a steviol glycoside at its C-19 carboxyl group is a UGT33786 polypeptide. In some embodiments, the UGT33942 polypeptide comprises a polypeptide having at least 80% identity to the amino acid sequence set forth in SEQ ID NO:2. In some embodiments, the UGT33786 polypeptide comprises a polypeptide having at least 80% identity to the amino acid sequence set forth in SEQ ID NO:119.

In some embodiments, a steviol glycoside or steviol glycoside precursor is produced by whole cell bioconversion in a host cell expressing of one or more enzymes involved in the steviol glycoside biosynthetic pathway in a recombinant host. For example, a steviol-producing recombinant host expressing one or more of a gene encoding a polypeptide capable of synthesizing geranylgeranyl pyrophosphate (GGPP) from farnesyl diphosphate (FPP) and isopentenyl diphosphate (IPP) (e.g., geranylgeranyl diphosphate synthase (GGPPS)); a gene encoding a polypeptide capable of synthesizing *ent*-copalyl diphosphate from GGPP (*e*.*g*., ent-copalyl diphosphate synthase (CDPS)); a gene encoding a polypeptide capable of synthesizing *ent*-kaurene from *ent*-copalyl pyrophosphate (*e*.*g*., kaurene synthase (KS)); a gene encoding a polypeptide capable of synthesizing *ent*-kaurenoic acid from *ent*-kaurene (*e*.*g*., kaurene oxidase (KO)); a gene encoding a polypeptide capable of reducing cytochrome P450 complex *(e.g.,* cytochrome P450 reductase (CPR)); and a gene encoding a polypeptide capable of synthesizing steviol from *ent*-kautenoic acid (e.g., steviol synthase (KAH)); and/or a gene encoding a bifunctional polypeptide capable of synthesizing *ent*-copalyl diphosphate from GGPP and synthesizing *ent*-kaurene from *ent*-copalyl pyrophosphate (e.g., an ent-copalyl diphosphate synthase (CDPS) - *ent*-kaurene synthase (KS) polypeptide), and a gene encoding a UGT can modify steviol glycosides and/or steviol glycoside precursors in *vivo. See, e.g.,* Figures 1 and 2. The skilled worker will appreciate that one or more of these genes can be endogenous to the host provided that at least one (and in some embodiments, all) of these genes is a recombinant gene introduced into the recombinant host.

In some embodiments, a steviol glycoside or steviol glycoside precursor is produced by whole cell bioconversion in a host cell expressing a polypeptide capable of glycosylating steviol or a steviol glycoside at its C-19 carboxyl group (e.g., UGT33942 and UGT33786) and one or more enzymes involved in the steviol glycoside biosynthetic pathway in a recombinant host. For example, a steviol-producing recombinant host expressing a UDP-glycosyltransferase (UGT) polypeptide structurally similar to members of the UGT85 family capable of glycosylating steviol or a steviol glycoside at its C-19 carboxyl group (e.g., UGT33942 and UGT33786) and one or more of a gene encoding a polypeptide capable of synthesizing geranylgeranyl pyrophosphate (GGPP) from farnesyl diphosphate (FPP) and isopentenyl diphosphate (IPP) *(e.g.,* geranylgeranyl diphosphate synthase (GGPPS)); a gene encoding a polypeptide capable of synthesizing *ent*-copalyl diphosphate from GGPP (*e*.*g*., ent-copalyl diphosphate synthase (CDPS)); a gene encoding a polypeptide capable of synthesizing *ent*-kaurene from *ent*-copalyl pyrophosphate (*e*.*g*., kaurene synthase (KS)); a gene encoding a polypeptide capable of synthesizing *ent*-kaurenoic acid from *ent-kaurene* (*e*.*g*., kaurene oxidase (KO)); a gene encoding a polypeptide capable of reducing cytochrome P450 complex (*e*.*g*., cytochrome P450 reductase (CPR)); and a gene encoding a polypeptide capable of synthesizing steviol from *ent-*kaurenoic acid (*e*.*g*., steviol synthase (KAH)); and/or a gene encoding a bifunctional polypeptide capable of synthesizing *ent*-copalyl diphosphate from GGPP and synthesizing *ent*-kaurene from *ent*-copalyl pyrophosphate (*e*.*g*., an ent-copalyl diphosphate synthase (CDPS) - *ent*-kaurene synthase (KS) polypeptide), and a gene encoding a UGT polypeptide can modify steviol glycosides and/or steviol glycoside precursors in *vivo.*

In some embodiments, the steviol glycoside comprises steviol-13-*O*-glucoside (13-SMG), steviol-1,2-bioside, steviol-1,3-bioside, steviol-19-*O*-glucoside (19-SMG), stevioside, 1,3-stevioside, rubusoside, Rebaudioside A (RebA), Rebaudioside B (RebB), Rebaudioside C (RebC), Rebaudioside D (RebD), Rebaudioside E (RebE), Rebaudioside F (RebF), Rebaudioside M (RebM), Rebaudioside Q (RebQ), Rebaudioside I (Rebl), dulcoside A, or isomers thereof.

In some embodiments, steviol, one or more steviol glycoside precursors, and/or one or more steviol glycosides are produced by co-culturing of two or more hosts. In some embodiments, one or more hosts, each expressing one or more enzymes involved in the steviol glycoside pathway, produce steviol, one or more steviol glycoside precursors, and/or one or more steviol glycosides. For example, a host comprising a polypeptide capable of synthesizing geranylgeranyl pyrophosphate (GGPP) from farnesyl diphosphate (FPP) and isopentenyl diphosphate (IPP), a polypeptide capable of synthesizing *ent*-copalyl diphosphate from GGPP, a polypeptide capable of synthesizing *ent*-kaurenoic acid from *ent*-kaurene, a polypeptide capable of synthesizing *ent*-kaurene from *ent*-copalyl pyrophosphate, a polypeptide capable of synthesizing steviol from *ent*-kaurenoic acid, and/or a polypeptide capable of reducing cytochrome P450 complex and a host comprising one or more UGTs produce one or more steviol glycosides.

In some embodiments, the steviol glycoside comprises steviol-13-*O*-glucoside (13-SMG), steviol-1,2-bioside, steviol-1,3-bioside, steviol-19-*O*-glucoside (19-SMG), stevioside, 1,3-stevioside, rubusoside, Rebaudioside A (RebA), Rebaudioside B (RebB), Rebaudioside C (RebC), Rebaudioside D (RebD), Rebaudioside E (RebE), Rebaudioside F (RebF), Rebaudioside M (RebM), Rebaudioside Q (RebQ), Rebaudioside I (Rebl), dulcoside A, or isomers thereof.

In some embodiments, a steviol glycoside or steviol glycoside precursor composition produced *in vivo, in vitro,* or by whole cell bioconversion does not comprise or comprises a reduced amount or reduced level of plant-derived components than a Stevia extract from, *inter alia,* a Stevia plant. Plant-derived components can contribute to off-flavors and include pigments, lipids, proteins, phenolics, saccharides, spathulenol and other sesquiterpenes, labdane diterpenes, monoterpenes, decanoic acid, 8,11,14-eicosatrienoic acid, 2-methyloctadecane, pentacosane, octacosane, tetracosane, octadecanol, stigmasterol, β-sitosterol, α- and β-amyrin, lupeol, β-amryin acetate, pentacyclic triterpenes, centauredin, quercitin, epi-alpha-cadinol, carophyllenes and derivatives, beta-pinene, beta-sitosterol, and gibberellin. In some embodiments, the plant-derived components referred to herein are non-glycoside compounds.

As used herein, the terms "detectable amount," "detectable concentration," "measurable amount," and "measurable concentration" refer to a level of steviol glycosides measured in AUC, µM/OD₆₀₀, mg/L, µM, or mM. Steviol glycoside production (*i*.*e*., total, supernatant, and/or intracellular steviol glycoside levels) can be detected and/or analyzed by techniques generally available to one skilled in the art, for example, but not limited to, liquid chromatography-mass spectrometry (LC-MS), thin layer chromatography (TLC), high-performance liquid chromatography (HPLC), ultraviolet-visible spectroscopy/ spectrophotometry (UV-Vis), mass spectrometry (MS), and nuclear magnetic resonance spectroscopy (NMR).

As used herein, the term "undetectable concentration" refers to a level of a compound that is too low to be measured and/or analyzed by techniques such as TLC, HPLC, UV-Vis, MS, or NMR. In some embodiments, a compound of an "undetectable concentration" is not present in a steviol glycoside or steviol glycoside precursor composition.

As used herein, the terms "or" and "and/or" is utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z." In some embodiments, "and/or" is used to refer to the exogenous nucleic acids that a recombinant cell comprises, wherein a recombinant cell comprises one or more exogenous nucleic acids selected from a group. In some embodiments, "and/or" is used to refer to production of steviol glycosides and/or steviol glycoside precursors. In some embodiments, "and/or" is used to refer to production of steviol glycosides, wherein one or more steviol glycosides are produced. In some embodiments, "and/or" is used to refer to production of steviol glycosides, wherein one or more steviol glycosides are produced through one or more of the following steps: culturing a recombinant microorganism, synthesizing one or more steviol glycosides in a recombinant microorganism, and/or isolating one or more steviol glycosides.

### Functional Homologs

Functional homologs of the polypeptides described above are also suitable for use in producing steviol glycosides in a recombinant host. A functional homolog is a polypeptide that has sequence similarity to a reference polypeptide, and that carries out one or more of the biochemical or physiological function(s) of the reference polypeptide. A functional homolog and the reference polypeptide can be a natural occurring polypeptide, and the sequence similarity can be due to convergent or divergent evolutionary events. As such, functional homologs are sometimes designated in the literature as homologs, or orthologs, or paralogs. Variants of a naturally occurring functional homolog, such as polypeptides encoded by mutants of a wild type coding sequence, can themselves be functional homologs. Functional homologs can also be created via site-directed mutagenesis of the coding sequence for a polypeptide, or by combining domains from the coding sequences for different naturally-occurring polypeptides ("domain swapping"). Techniques for modifying genes encoding functional polypeptides described herein are known and include, *inter alia,* directed evolution techniques, site-directed mutagenesis techniques and random mutagenesis techniques, and can be useful to increase specific activity of a polypeptide, alter substrate specificity, alter expression levels, alter subcellular location, or modify polypeptide-polypeptide interactions in a desired manner. Such modified polypeptides are considered functional homologs. The term "functional homolog" is sometimes applied to the nucleic acid that encodes a functionally homologous polypeptide.

Functional homologs can be identified by analysis of nucleotide and polypeptide sequence alignments. For example, performing a query on a database of nucleotide or polypeptide sequences can identify homologs of steviol glycoside biosynthesis polypeptides. Sequence analysis can involve BLAST, Reciprocal BLAST, or PSI-BLAST analysis of non-redundant databases using a UGT amino acid sequence as the reference sequence. Amino acid sequence is, in some instances, deduced from the nucleotide sequence. Those polypeptides in the database that have greater than 40% sequence identity are candidates for further evaluation for suitability as a steviol glycoside biosynthesis polypeptide. Amino acid sequence similarity allows for conservative amino acid substitutions, such as substitution of one hydrophobic residue for another or substitution of one polar residue for another. If desired, manual inspection of such candidates can be carried out in order to narrow the number of candidates to be further evaluated. Manual inspection can be performed by selecting those candidates that appear to have domains present in steviol glycoside biosynthesis polypeptides, *e*.*g*., conserved functional domains. In some embodiments, nucleic acids and polypeptides are identified from transcriptome data based on expression levels rather than by using BLAST analysis.

Conserved regions can be identified by locating a region within the primary amino acid sequence of a steviol glycoside biosynthesis polypeptide that is a repeated sequence, forms some secondary structure (e.g., helices and beta sheets), establishes positively or negatively charged domains, or represents a protein motif or domain. *See, e.g.,* the Pfam web site describing consensus sequences for a variety of protein motifs and domains on the World Wide Web at sanger.ac.uk/Software/Pfam/ and pfam.janelia.org/. The information included at the Pfam database is described in Sonnhammer et al., Nucl. Acids Res., 26:320-322 (1998); Sonnhammer et al.. Proteins, 28:405-420 (1997); and Bateman et al., Nucl. Acids Res., 27:260-262 (1999). Conserved regions also can be determined by aligning sequences of the same or related polypeptides from closely related species. Closely related species preferably are from the same family. In some embodiments, alignment of sequences from two different species is adequate to identify such homologs.

Typically, polypeptides that exhibit at least about 40% amino acid sequence identity are useful to identify conserved regions. Conserved regions of related polypeptides exhibit at least 45% amino acid sequence identity (e.g., at least 50%, at least 60%, at least 70%, at least 80%, or at least 90% amino acid sequence identity). In some embodiments, a conserved region exhibits at least 92%, 94%, 96%, 98%, or 99% amino acid sequence identity.

For example, polypeptides suitable for producing steviol in a recombinant host include functional homologs of UGTs.

Methods to modify the substrate specificity of, for example, a UGT, are known to those skilled in the art, and include without limitation site-directed/rational mutagenesis approaches, random directed evolution approaches and combinations in which random mutagenesis/saturation techniques are performed near the active site of the enzyme. For example see Osmani et al., 2009, Phytochemistry 70: 325-347.

A candidate sequence typically has a length that is from 80% to 200% of the length of the reference sequence, *e.g*., 82, 85, 87, 89, 90, 93, 95, 97, 99, 100, 105, 110, 115, 120, 130, 140, 150, 160, 170, 180, 190, or 200% of the length of the reference sequence. A functional homolog polypeptide typically has a length that is from 95% to 105% of the length of the reference sequence, *e*.*g*., 90, 93, 95, 97, 99, 100, 105, 110, 115, or 120% of the length of the reference sequence, or any range between. A% identity for any candidate nucleic acid or polypeptide relative to a reference nucleic acid or polypeptide can be determined as follows. A reference sequence (*e*.*g*., a nucleic acid sequence or an amino acid sequence described herein) is aligned to one or more candidate sequences using the computer program ClustalW (version 1.83, default parameters), which allows alignments of nucleic acid or polypeptide sequences to be carried out across their entire length (global alignment). Chenna et al., 2003, Nucleic Acids Res. 31(13):3497-500.

ClustalW calculates the best match between a reference and one or more candidate sequences, and aligns them so that identities, similarities and differences can be determined. Gaps of one or more residues can be inserted into a reference sequence, a candidate sequence, or both, to maximize sequence alignments. For fast pairwise alignment of nucleic acid sequences, the following default parameters are used: word size: 2; window size: 4; scoring method: % age; number of top diagonals: 4; and gap penalty: 5. For multiple alignment of nucleic acid sequences, the following parameters are used: gap opening penalty: 10.0; gap extension penalty: 5.0; and weight transitions: yes. For fast pairwise alignment of protein sequences, the following parameters are used: word size: 1; window size: 5; scoring method:% age; number of top diagonals: 5; gap penalty: 3. For multiple alignment of protein sequences, the following parameters are used: weight matrix: blosum; gap opening penalty: 10.0; gap extension penalty: 0.05; hydrophilic gaps: on; hydrophilic residues: Gly, Pro, Ser, Asn, Asp, Gin, Glu, Arg, and Lys; residue-specific gap penalties: on. The ClustalW output is a sequence alignment that reflects the relationship between sequences. ClustalW can be run, for example, at the Baylor College of Medicine Search Launcher site on the World Wide Web (searchlauncher.bcm.tmc.edu/multi-align/multi-align.html) and at the European Bioinformatics Institute site on the World Wide Web (ebi.ac.uk/clustalw).

To determine % identity of a candidate nucleic acid or amino acid sequence to a reference sequence, the sequences are aligned using ClustalW, the number of identical matches in the alignment is divided by the length of the reference sequence, and the result is multiplied by 100. It is noted that the % identity value can be rounded to the nearest tenth. For example, 78.11, 78.12, 78.13, and 78.14 are rounded down to 78.1, while 78.15, 78.16, 78.17, 78.18, and 78.19 are rounded up to 78.2.

It will be appreciated that functional UGT proteins *(e.g.,* a polypeptide capable of glycosylating steviol or a steviol glycoside at its C-19 carboxyl group) can include additional amino acids that are not involved in the enzymatic activities carried out by the enzymes. In some embodiments, UGT proteins are fusion proteins. The terms "chimera," "fusion polypeptide," "fusion protein," "fusion enzyme," "fusion construct," "chimeric protein," "chimeric polypeptide," "chimeric construct," and "chimeric enzyme" can be used interchangeably herein to refer to proteins engineered through the joining of two or more genes that code for different proteins. In some embodiments, a nucleic acid sequence encoding a UGT polypeptide *(e.g.,* a polypeptide capable of glycosylating steviol or a steviol glycoside at its C-19 carboxyl group) can include a tag sequence that encodes a "tag" designed to facilitate subsequent manipulation *(e.g.,* to facilitate purification or detection), secretion, or localization of the encoded polypeptide. Tag sequences can be inserted in the nucleic acid sequence encoding the polypeptide such that the encoded tag is located at either the carboxyl or amino terminus of the polypeptide. Nonlimiting examples of encoded tags include green fluorescent protein (GFP), human influenza hemagglutinin (HA), glutathione S transferase (GST), polyhistidine-tag (HIS tag), and Flag^{™} tag (Kodak, New Haven, CT). Other examples of tags include a chloroplast transit peptide, a mitochondrial transit peptide, an amyloplast peptide, signal peptide, or a secretion tag.

In some embodiments, a fusion protein is a protein altered by domain swapping. As used herein, the term "domain swapping" is used to describe the process of replacing a domain of a first protein with a domain of a second protein. In some embodiments, the domain of the first protein and the domain of the second protein are functionally identical or functionally similar. In some embodiments, the structure and/or sequence of the domain of the second protein differs from the structure and/or sequence of the domain of the first protein. In some embodiments, a UGT polypeptide (*e*.*g*., a polypeptide capable of glycosylating steviol or a steviol glycoside at its C-19 carboxyl group) is altered by domain swapping.

In some embodiments, a fusion protein is a protein altered by circular permutation, which consists in the covalent attachment of the ends of a protein that would be opened elsewhere afterwards. Thus, the order of the sequence is altered without causing changes in the amino acids of the protein. In some embodiments, a targeted circular permutation can be produced, for example but not limited to, by designing a spacer to join the ends of the original protein. Once the spacer has been defined, there are several possibilities to generate permutations through generally accepted molecular biology techniques, for example but not limited to, by producing concatemers by means of PCR and subsequent amplification of specific permutations inside the concatemer or by amplifying discrete fragments of the protein to exchange to join them in a different order. The step of generating permutations can be followed by creating a circular gene by binding the fragment ends and cutting back at random, thus forming collections of permutations from a unique construct. In some embodiments, a UGT polypeptide (*e*.*g*., a polypeptide capable of glycosylating steviol or a steviol glycoside at its C-19 carboxyl group) is altered by circular permutation.

In some embodiments, the polypeptide capable of glycosylating steviol or a steviol glycoside at its C-19 carboxyl group is a UGT33942 polypeptide. In some embodiments, the polypeptide capable of glycosylating steviol or a steviol glycoside at its C-19 carboxyl group is a UGT33786 polypeptide. In some embodiments, the UGT33942 polypeptide comprises a polypeptide having at least 80% identity to the amino acid sequence set forth in SEQ ID NO:2. In some embodiments, the UGT33786 polypeptide comprises a polypeptide having at least 80% identity to the amino acid sequence set forth in SEQ ID NO:119.

### Steviol and Steviol Glycoside Biosynthesis Nucleic Acids

A recombinant gene encoding a polypeptide described herein comprises the coding sequence for that polypeptide, operably linked in sense orientation to one or more regulatory regions suitable for expressing the polypeptide. Because many microorganisms are capable of expressing multiple gene products from a polycistronic mRNA, multiple polypeptides can be expressed under the control of a single regulatory region for those microorganisms, if desired. A coding sequence and a regulatory region are considered to be operably linked when the regulatory region and coding sequence are positioned so that the regulatory region is effective for regulating transcription or translation of the sequence. Typically, the translation initiation site of the translational reading frame of the coding sequence is positioned between one and about fifty nucleotides downstream of the regulatory region for a monocistronic gene.

In many cases, the coding sequence for a polypeptide described herein is identified in a species other than the recombinant host, *i*.*e*., is a heterologous nucleic acid. Thus, if the recombinant host is a microorganism, the coding sequence can be from other prokaryotic or eukaryotic microorganisms, from plants or from animals. In some case, however, the coding sequence is a sequence that is native to the host and is being reintroduced into that organism. A native sequence can often be distinguished from the naturally occurring sequence by the presence of non-natural sequences linked to the exogenous nucleic acid, *e*.*g*., non-native regulatory sequences flanking a native sequence in a recombinant nucleic acid construct. In addition, stably transformed exogenous nucleic acids typically are integrated at positions other than the position where the native sequence is found. "Regulatory region" refers to a nucleic acid having nucleotide sequences that influence transcription or translation initiation and rate, and stability and/or mobility of a transcription or translation product. Regulatory regions include, without limitation, promoter sequences, enhancer sequences, response elements, protein recognition sites, inducible elements, protein binding sequences, 5' and 3' untranslated regions (UTRs), transcriptional start sites, termination sequences, polyadenylation sequences, introns, and combinations thereof. A regulatory region typically comprises at least a core (basal) promoter. A regulatory region also may include at least one control element, such as an enhancer sequence, an upstream element or an upstream activation region (UAR). A regulatory region is operably linked to a coding sequence by positioning the regulatory region and the coding sequence so that the regulatory region is effective for regulating transcription or translation of the sequence. For example, to operably link a coding sequence and a promoter sequence, the translation initiation site of the translational reading frame of the coding sequence is typically positioned between one and about fifty nucleotides downstream of the promoter. A regulatory region can, however, be positioned as much as about 5,000 nucleotides upstream of the translation initiation site, or about 2,000 nucleotides upstream of the transcription start site.

The choice of regulatory regions to be included depends upon several factors, including, but not limited to, efficiency, selectability, inducibility, desired expression level, and preferential expression during certain culture stages. It is a routine matter for one of skill in the art to modulate the expression of a coding sequence by appropriately selecting and positioning regulatory regions relative to the coding sequence. It will be understood that more than one regulatory region may be present, *e.g*., introns, enhancers, upstream activation regions, transcription terminators, and inducible elements.

One or more genes can be combined in a recombinant nucleic acid construct in "modules" useful for a discrete aspect of steviol and/or steviol glycoside production. Combining a plurality of genes in a module, particularly a polycistronic module, facilitates the use of the module in a variety of species. For example, a steviol biosynthesis gene cluster, or a UGT gene cluster, can be combined in a polycistronic module such that, after insertion of a suitable regulatory region, the module can be introduced into a wide variety of species. As another example, a UGT gene cluster can be combined such that each UGT coding sequence is operably linked to a separate regulatory region, to form a UGT module. Such a module can be used in those species for which monocistronic expression is necessary or desirable. In addition to genes useful for steviol or steviol glycoside production, a recombinant construct typically also contains an origin of replication, and one or more selectable markers for maintenance of the construct in appropriate species.

It will be appreciated that because of the degeneracy of the genetic code, a number of nucleic acids can encode a particular polypeptide; i.e., for many amino acids, there is more than one nucleotide triplet that serves as the codon for the amino acid. Thus, codons in the coding sequence for a given polypeptide can be modified such that optimal expression in a particular host is obtained, using appropriate codon bias tables for that host (*e*.*g*., microorganism). As isolated nucleic acids, these modified sequences can exist as purified molecules and can be incorporated into a vector or a virus for use in constructing modules for recombinant nucleic acid constructs.

In some cases, it is desirable to inhibit one or more functions of an endogenous polypeptide in order to divert metabolic intermediates towards steviol or steviol glycoside biosynthesis. For example, it may be desirable to downregulate synthesis of sterols in a yeast strain in order to further increase steviol or steviol glycoside production, *e*.*g*., by downregulating squalene epoxidase. As another example, it may be desirable to inhibit degradative functions of certain endogenous gene products, *e*.*g*., glycohydrolases that remove glucose moieties from secondary metabolites or phosphatases as discussed herein. In such cases, a nucleic acid that overexpresses the polypeptide or gene product may be included in a recombinant construct that is transformed into the strain. Alternatively, mutagenesis can be used to generate mutants in genes for which it is desired to increase or enhance function.

### Host Microorganisms

Recombinant hosts can be used to express polypeptides for producing steviol glycosides, including mammalian, insect, plant, and algal cells. A number of prokaryotes and eukaryotes are also suitable for use in constructing the recombinant microorganisms described herein, *e*.*g*., gram-negative bacteria, yeast, and fungi. A species and strain selected for use as a steviol glycoside production strain is first analyzed to determine which production genes are endogenous to the strain and which genes are not present. Genes for which an endogenous counterpart is not present in the strain are advantageously assembled in one or more recombinant constructs, which are then transformed into the strain in order to supply the missing function(s).

Typically, the recombinant microorganism is grown in a fermenter at a temperature(s) for a period of time, wherein the temperature and period of time facilitate the production of a steviol glycoside. The constructed and genetically engineered microorganisms provided by the invention can be cultivated using conventional fermentation processes, including, *inter alia,* chemostat, batch, fed-batch cultivations, semi-continuous fermentations such as draw and fill, continuous perfusion fermentation, and continuous perfusion cell culture. Depending on the particular microorganism used in the method, other recombinant genes such as isopentenyl biosynthesis genes and terpene synthase and cyclase genes may also be present and expressed. Levels of substrates and intermediates, *e*.*g*., isopentenyl diphosphate, dimethylallyl diphosphate, GGPP, ent-kaurene and ent-kaurenoic acid, can be determined by extracting samples from culture media for analysis according to published methods.

Carbon sources of use in the instant method include any molecule that can be metabolized by the recombinant host cell to facilitate growth and/or production of the steviol glycosides. Examples of suitable carbon sources include, but are not limited to, sucrose (e.g., as found in molasses), fructose, xylose, ethanol, glycerol, glucose, cellulose, starch, cellobiose or other glucose-comprising polymer. In embodiments employing yeast as a host, for example, carbons sources such as sucrose, fructose, xylose, ethanol, glycerol, and glucose are suitable. The carbon source can be provided to the host organism throughout the cultivation period or alternatively, the organism can be grown for a period of time in the presence of another energy source, *e*.*g*., protein, and then provided with a source of carbon only during the fed-batch phase.

After the recombinant microorganism has been grown in culture for the period of time, wherein the temperature and period of time facilitate the production of a steviol glycoside, steviol and/or one or more steviol glycosides can then be recovered from the culture using various techniques known in the art. In some embodiments, a permeabilizing agent can be added to aid the feedstock entering into the host and product getting out. For example, a crude lysate of the cultured microorganism can be centrifuged to obtain a supernatant. The resulting supernatant can then be applied to a chromatography column, *e.g.,* a C-18 column, and washed with water to remove hydrophilic compounds, followed by elution of the compound(s) of interest with a solvent such as methanol. The compound(s) can then be further purified by preparative HPLC. *See also,* WO 2009/140394.

It will be appreciated that the various genes and modules discussed herein can be present in two or more recombinant hosts rather than a single host. When a plurality of recombinant hosts is used, they can be grown in a mixed culture to accumulate steviol and/or steviol glycosides.

Alternatively, the two or more hosts each can be grown in a separate culture medium and the product of the first culture medium, *e.g.,* steviol, can be introduced into second culture medium to be converted into a subsequent intermediate, or into an end product such as, for example, RebA. The product produced by the second, or final host is then recovered. It will also be appreciated that in some embodiments, a recombinant host is grown using nutrient sources other than a culture medium and utilizing a system other than a fermenter.

Exemplary prokaryotic and eukaryotic species are described in more detail below. However, it will be appreciated that other species can be suitable. For example, suitable species can be in a genus such as *Agaricus, Aspergillus, Bacillus, Candida, Corynebacterium, Eremothecium, Escherichia, Fusarium*/*Gibberella, Kluyveromyces, Laetiporus, Lentinus, Phaffia, Phanerochaete, Pichia, Physcomitrella, Rhodoturula, Saccharomyces, Schizosaccharomyces, Sphaceloma, Xanthophyllomyces* or *Yarrowia.* Exemplary species from such genera include *Lentinus tigrinus, Laetiporus sulphureus, Phanerochaete chrysosporium, Pichia pastoris, Cyberlindnera jadinii, Physcomitrella patens, Rhodoturula glutinis, Rhodoturula mucilaginosa, Phaffia rhodozyma, Xanthophyllomyces dendrorhous, Fusarium fujikuroi*/*Gibberella fujikuroi, Candida utilis, Candida glabrata, Candida albicans,* and *Yarrowia lipolytica.*

In some embodiments of the recombinant hosts as otherwise described herein, a microorganism can be a prokaryote such as *Escherichia* bacteria cells, for example, *Escherichia coli* cells; *Lactobacillus* bacteria cells; *Lactococcus* bacteria cells; *Cornebacterium* bacteria cells; *Acetobacter* bacteria cells; *Acinetobacter* bacteria cells; or *Pseudomonas* bacterial cells.

In some embodiments of the recombinant hosts as otherwise described herein, a microorganism can be an Ascomycete such as *Gibberella fujikuroi, Kluyveromyces lactis, Schizosaccharomyces pombe, Aspergillus niger, Yarrowia lipolytica, Ashbya gossypii,* or S. *cerevisiae.*

In some embodiments of the recombinant hosts as otherwise described herein, a microorganism can be an algal cell such as *Blakeslea trispora, Dunaliella salina, Haematococcus pluvialis, Chlorella sp., Undaria pinnatifida, Sargassum, Laminaria japonica, Scenedesmus almeriensis* species.

In some embodiments of the recombinant hosts as otherwise described herein, a microorganism can be a cyanobacterial cell such as *Blakeslea trispora, Dunaliella salina, Haematococcus pluvialis, Chlorella sp., Undaria pinnatifida, Sargassum, Laminaria japonica, Scenedesmus almeriensis.*

### Saccharomyces spp.

*Saccharomyces* is a widely used chassis organism in synthetic biology, and can be used as the recombinant microorganism platform of a recombinant host as otherwise described herein. For example, there are libraries of mutants, plasmids, detailed computer models of metabolism and other information available for *S*. *cerevisiae,* allowing for rational design of various modules to enhance product yield. Methods are known for making recombinant microorganisms.

### Aspergillus spp.

*Aspergillus* species such as *A. oryzae, A. niger* and *A. sojae* are widely used microorganisms in food production and can also be used as the recombinant microorganism platform of a recombinant host as otherwise described herein. Nucleotide sequences are available for genomes of *A*. *nidulans, A. fumigatus, A. oryzae, A. clavatus, A. flavus, A. niger,* and *A*. *terreus,* allowing rational design and modification of endogenous pathways to enhance flux and increase product yield. Metabolic models have been developed for *Aspergillus,* as well as transcriptomic studies and proteomics studies. *A*. *niger* is cultured for the industrial production of a number of food ingredients such as citric acid and gluconic acid, and thus species such as *A*. *niger* are generally suitable for producing steviol glycosides.

### E. coli

*E. coli,* another widely used platform organism in synthetic biology, can also be used as the recombinant microorganism platform of a recombinant host as otherwise described herein. Similar to *Saccharomyces,* there are libraries of mutants, plasmids, detailed computer models of metabolism and other information available for *E. coli,* allowing for rational design of various modules to enhance product yield. Methods similar to those described above for *Saccharomyces* can be used to make recombinant *E. coli* microorganisms.

### Agaricus, Gibberella, and Phanerochaete spp.

*Agaricus, Gibberella,* and *Phanerochaete* spp. can be useful because they are known to produce large amounts of isoprenoids in culture. Thus, the terpene precursors for producing large amounts of steviol glycosides are already produced by endogenous genes. Thus, modules comprising recombinant genes for steviol glycoside biosynthesis polypeptides can be introduced into species from such genera without the necessity of introducing mevalonate or MEP pathway genes. Accordingly, *Agaricus, Gibberella,* and *Phanerochaete* spp. can be used as the recombinant microorganism platform of a recombinant host as otherwise described herein.

### Arxula adeninivorans (Blastobotrys adeninivorans)

*Arxula adeninivorans* is dimorphic yeast (it grows as budding yeast like the baker's yeast up to a temperature of 42°C, above this threshold it grows in a filamentous form) with unusual biochemical characteristics. It can grow on a wide range of substrates and can assimilate nitrate. It has successfully been applied to the generation of strains that can produce natural plastics or the development of a biosensor for estrogens in environmental samples. Accordingly, *Arxula adeninivorans* can be used as the recombinant microorganism platform of a recombinant host as otherwise described herein.

### Yarrowia lipolytica

*Yarrowia lipolytica* is dimorphic yeast (*see Arxula adeninivorans*) and belongs to the family Hemiascomycetes. The entire genome of *Yarrowia lipolytica* is known. *Yarrowia* species is aerobic and considered to be non-pathogenic. *Yarrowia* is efficient in using hydrophobic substrates (e.g. alkanes, fatty acids, oils) and can grow on sugars. It has a high potential for industrial applications and is an oleaginous microorganism. *Yarrowia lipolyptica* can accumulate lipid content to approximately 40% of its dry cell weight and is a model organism for lipid accumulation and remobilization. *See e.g.,* Nicaud, 2012, Yeast 29(10):409-18; Beopoulos et al., 2009, Biochimie 91(6):692-6; Bankar et al., 2009, Appl Microbiol Biotechnol. 84(5):847-65. Accordingly, *Yarrowia lipolytica* can be used as the recombinant microorganism platform of a recombinant host as otherwise described herein.

### Rhodotorula sp.

*Rhodotorula* is unicellular, pigmented yeast. The oleaginous red yeast, *Rhodotorula glutinis,* has been shown to produce lipids and carotenoids from crude glycerol (Saenge et al., 2011, Process Biochemistry 46(1):210-8). *Rhodotorula toruloides* strains have been shown to be an efficient fed-batch fermentation system for improved biomass and lipid productivity (Li et al., 2007, Enzyme and Microbial Technology 41:312-7). Accordingly, *Rhodotorula* can be used as the recombinant microorganism platform of a recombinant host as otherwise described herein.

### Rhodosporidium toruloides

*Rhodosporidium toruloides* is oleaginous yeast and useful for engineering lipid-production pathways (See e.g. Zhu et al., 2013, Nature Commun. 3:1112; Ageitos et al., 2011, Applied Microbiology and Biotechnology 90(4):1219-27). Accordingly, *Rhodosporidium toruloides* can be used as the recombinant microorganism platform of a recombinant host as otherwise described herein.

### Candida boidinii

*Candida boidinii* is methylotrophic yeast (it can grow on methanol). Like other methylotrophic species such as *Hansenula polymorpha* and *Pichia pastoris,* it provides an excellent platform for producing heterologous proteins. Yields in a multigram range of a secreted foreign protein have been reported. A computational method, IPRO, recently predicted mutations that experimentally switched the cofactor specificity of *Candida boidinii* xylose reductase from NADPH to NADH. *See, e.g.,* Mattanovich et al., 2012, Methods Mol Biol. 824:329-58; Khoury et al., 2009, Protein Sci. 18(10):2125-38. Accordingly, *Candida boidinii* can be used as the recombinant microorganism platform of a recombinant host as otherwise described herein.

### Hansenula polymorpha (Pichia angusta)

*Hansenula polymorpha* is methylotrophic yeast *(see Candida boidinii).* It can furthermore grow on a wide range of other substrates; it is thermo-tolerant and can assimilate nitrate (see also *Kluyveromyces lactis).* It has been applied to producing hepatitis B vaccines, insulin and interferon alpha-2a for the treatment of hepatitis C, furthermore to a range of technical enzymes. *See, e.g.,* Xu et al., 2014, Virol Sin. 29(6):403-9. Accordingly, *Hansenula polymorpha* can be used as the recombinant microorganism platform of a recombinant host as otherwise described herein.

### Kluyveromyces lactis

*Kluyveromyces lactis* is yeast regularly applied to the production of kefir. It can grow on several sugars, most importantly on lactose which is present in milk and whey. It has successfully been applied among others for producing chymosin (an enzyme that is usually present in the stomach of calves) for producing cheese. Production takes place in fermenters on a 40,000 L scale. *See, e.g.,* van Ooyen et al., 2006, FEMS Yeast Res. 6(3):381-92. Accordingly, *Kluyveromyces lactis* can be used as the recombinant microorganism platform of a recombinant host as otherwise described herein.

### Pichia pastoris

*Pichia pastoris* is methylotrophic yeast *(see Candida boidinii* and *Hansenula polymorpha).* It provides an efficient platform for producing foreign proteins. Platform elements are available as a kit and it is worldwide used in academia for producing proteins. Strains have been engineered that can produce complex human N-glycan (yeast glycans are similar but not identical to those found in humans). See, *e.g.,* Piirainen et al., 2014, N Biotechnol. 31(6):532-7. Accordingly, *Pichia pastoris* can be used as the recombinant microorganism platform of a recombinant host as otherwise described herein.

### Physcomitrella spp.

*Physcomitrella mosses,* when grown in suspension culture, have characteristics similar to yeast or other fungal cultures. This genera can be used for producing plant secondary metabolites, which can be difficult to produce in other types of cells. Accordingly, *Physcomitrella mosses* can be used as the recombinant microorganism platform of a recombinant host as otherwise described herein.

### Steviol Glycoside Compositions

Steviol glycosides do not necessarily have equivalent performance in different food systems. It is therefore desirable to have the ability to direct the synthesis to steviol glycoside compositions of choice. Recombinant hosts described herein can produce compositions that are selectively enriched for specific steviol glycosides (*e*.*g*., 19-SMG, RebD or RebM) and have a consistent taste profile. As used herein, the term "enriched" is used to describe a steviol glycoside composition with an increased proportion of a particular steviol glycoside, compared to a steviol glycoside composition (extract) from a Stevia plant. Thus, the recombinant hosts described herein can facilitate the production of compositions that are tailored to meet the sweetening profile desired for a given food product and that have a proportion of each steviol glycoside that is consistent from batch to batch. In some embodiments, hosts described herein do not produce or produce a reduced amount of undesired plant-derived components found in Stevia extracts. Thus, steviol glycoside compositions produced by the recombinant hosts described herein are distinguishable from compositions derived from Stevia plants.

The amount of an individual steviol glycoside *(e.g.,* RebA, RebB, RebD, or RebM) accumulated can be from about 1 to about 7,000 mg/L, *e.g.,* about 1 to about 10 mg/L, about 3 to about 10 mg/L, about 5 to about 20 mg/L, about 10 to about 50 mg/L, about 10 to about 100 mg/L, about 25 to about 500 mg/L, about 100 to about 1,500 mg/L, or about 200 to about 1,000 mg/L, at least about 1,000 mg/L, at least about 1,200 mg/L, at least about at least 1,400 mg/L, at least about 1,600 mg/L, at least about 1,800 mg/L, at least about 2,800 mg/L, or at least about 7,000 mg/L. In some aspects, the amount of an individual steviol glycoside can exceed 7,000 mg/L. The amount of a combination of steviol glycosides (*e*.*g*., RebA, RebB, RebD, or RebM) accumulated can be from about 1 mg/L to about 7,000 mg/L, *e*.*g*., about 200 to about 1,500, at least about 2,000 mg/L, at least about 3,000 mg/L, at least about 4,000 mg/L, at least about 5,000 mg/L, at least about 6,000 mg/L, or at least about 7,000 mg/L. In some aspects, the amount of a combination of steviol glycosides can exceed 7,000 mg/L. In general, longer culture times will lead to greater amounts of product. Thus, the recombinant microorganism can be cultured for from 1 day to 7 days, from 1 day to 5 days, from 3 days to 5 days, about 3 days, about 4 days, or about 5 days.

It will be appreciated that the various genes and modules discussed herein can be present in two or more recombinant microorganisms rather than a single microorganism. When a plurality of recombinant microorganisms is used, they can be grown in a mixed culture to produce steviol and/or steviol glycosides. For example, a first microorganism can comprise one or more biosynthesis genes for producing a steviol glycoside precursor, while a second microorganism comprises steviol glycoside biosynthesis genes. The product produced by the second, or final microorganism is then recovered. It will also be appreciated that in some embodiments, a recombinant microorganism is grown using nutrient sources other than a culture medium and utilizing a system other than a fermenter.

Alternatively, the two or more microorganisms each can be grown in a separate culture medium and the product of the first culture medium, *e*.*g*., steviol, can be introduced into second culture medium to be converted into a subsequent intermediate, or into an end product such as RebA. The product produced by the second, or final microorganism is then recovered. It will also be appreciated that in some embodiments, a recombinant microorganism is grown using nutrient sources other than a culture medium and utilizing a system other than a fermenter.

Steviol glycosides and compositions obtained by the methods disclosed herein can be used to make food products, dietary supplements and sweetener compositions. *See, e.g.,* WO 2011/153378, WO 2013/022989, WO 2014/122227, and WO 2014/122328.

For example, substantially pure steviol or steviol glycoside such as RebM or RebD can be included in food products such as ice cream, carbonated beverages, fruit juices, yogurts, baked goods, chewing gums, hard and soft candies, and sauces. Substantially pure steviol or steviol glycoside can also be included in non-food products such as pharmaceutical products, medicinal products, dietary supplements and nutritional supplements. Substantially pure steviol or steviol glycosides may also be included in animal feed products for both the agriculture industry and the companion animal industry. Alternatively, a mixture of steviol and/or steviol glycosides can be made by culturing recombinant microorganisms separately, each producing a specific steviol or steviol glycoside, recovering the steviol or steviol glycoside in substantially pure form from each microorganism and then combining the compounds to obtain a mixture comprising each compound in the desired proportion. The recombinant microorganisms described herein permit more precise and consistent mixtures to be obtained compared to current Stevia products.

In another alternative, a substantially pure steviol or steviol glycoside can be incorporated into a food product along with other sweeteners, e.g. saccharin, dextrose, sucrose, fructose, erythritol, aspartame, sucralose, monatin, or acesulfame potassium. The weight ratio of steviol or steviol glycoside relative to other sweeteners can be varied as desired to achieve a satisfactory taste in the final food product. *See, e.g.,* U.S. 2007/0128311. In some embodiments, the steviol or steviol glycoside may be provided with a flavor (e.g., citrus) as a flavor modulator.

Compositions produced by a recombinant microorganism described herein can be incorporated into food products. For example, a steviol glycoside composition produced by a recombinant microorganism can be incorporated into a food product in an amount ranging from about 20 mg steviol glycoside/kg food product to about 1800 mg steviol glycoside/kg food product on a dry weight basis, depending on the type of steviol glycoside and food product. For example, a steviol glycoside composition produced by a recombinant microorganism can be incorporated into a dessert, cold confectionary (*e*.*g*., ice cream), dairy product (*e*.*g*., yogurt), or beverage (*e*.*g*., a carbonated beverage) such that the food product has a maximum of 500 mg steviol glycoside/kg food on a dry weight basis. A steviol glycoside composition produced by a recombinant microorganism can be incorporated into a baked good (*e*.*g*., a biscuit) such that the food product has a maximum of 300 mg steviol glycoside/kg food on a dry weight basis. A steviol glycoside composition produced by a recombinant microorganism can be incorporated into a sauce *(e.g.,* chocolate syrup) or vegetable product (*e*.*g*., pickles) such that the food product has a maximum of 1000 mg steviol glycoside/kg food on a dry weight basis. A steviol glycoside composition produced by a recombinant microorganism can be incorporated into bread such that the food product has a maximum of 160 mg steviol glycoside/kg food on a dry weight basis. A steviol glycoside composition produced by a recombinant microorganism, plant, or plant cell can be incorporated into a hard or soft candy such that the food product has a maximum of 1600 mg steviol glycoside/kg food on a dry weight basis. A steviol glycoside composition produced by a recombinant microorganism, plant, or plant cell can be incorporated into a processed fruit product (*e*.*g*., fruit juices, fruit filling, jams, and jellies) such that the food product has a maximum of 1000 mg steviol glycoside/kg food on a dry weight basis. In some embodiments, a steviol glycoside composition produced herein is a component of a pharmaceutical composition. *See, e.g*., Steviol Glycosides Chemical and Technical Assessment 69th JECFA, 2007, prepared by Harriet Wallin, Food Agric. Org.; EFSA Panel on Food Additives and Nutrient Sources added to Food (ANS), "Scientific Opinion on the safety of steviol glycosides for the proposed uses as a food additive," 2010, EFSA Journal 8(4):1537; U.S. Food and Drug Administration GRAS Notice 323; U.S Food and Drug Administration GRAS Notice 329; WO 2011/037959; WO 2010/146463; WO 2011/046423; and WO 2011/056834.

For example, such a steviol glycoside composition can have from 90-99 weight % RebA and an undetectable amount of Stevia plant-derived components relative to a plant-derived Stevia extract, and be incorporated into a food product at from 25-1600 mg/kg, *e*.*g*., 100-500 mg/kg, 25-100 mg/kg, 250-1000 mg/kg, 50-500 mg/kg or 500-1000 mg/kg on a dry weight basis.

Such a steviol glycoside composition can be a RebB-enriched composition having greater than 3 weight % RebB and be incorporated into the food product such that the amount of RebB in the product is from 25-1600 mg/kg, *e.g.,* 100-500 mg/kg, 25-100 mg/kg, 250-1000 mg/kg, 50-500 mg/kg or 500-1000 mg/kg on a dry weight basis. Typically, the RebB-enriched composition has an undetectable amount of Stevia plant-derived components relative to a plant-derived Stevia extract.

Such a steviol glycoside composition can be a RebD-enriched composition having greater than 3 weight % RebD and be incorporated into the food product such that the amount of RebD in the product is from 25-1600 mg/kg, *e.g.,* 100-500 mg/kg, 25-100 mg/kg, 250-1000 mg/kg, 50-500 mg/kg or 500-1000 mg/kg on a dry weight basis. Typically, the RebD-enriched composition has an undetectable amount of Stevia plant-derived components relative to a plant-derived Stevia extract.

Such a steviol glycoside composition can be a RebE-enriched composition having greater than 3 weight % RebE and be incorporated into the food product such that the amount of RebE in the product is from 25-1600 mg/kg, *e.g.,* 100-500 mg/kg, 25-100 mg/kg, 250-1000 mg/kg, 50-500 mg/kg or 500-1000 mg/kg on a dry weight basis. Typically, the RebE-enriched composition has an undetectable amount of Stevia plant-derived components relative to a plant-derived Stevia extract.

Such a steviol glycoside composition can be a RebM-enriched composition having greater than 3 weight % RebM and be incorporated into the food product such that the amount of RebM in the product is from 25-1600 mg/kg, *e*.*g*., 100-500 mg/kg, 25-100 mg/kg, 250-1000 mg/kg, 50-500 mg/kg or 500-1000 mg/kg on a dry weight basis. Typically, the RebM-enriched composition has an undetectable amount of Stevia plant-derived components relative to a plant-derived Stevia extract.

In some embodiments, a substantially pure steviol or steviol glycoside is incorporated into a tabletop sweetener or "cup-for-cup" product. Such products typically are diluted to the appropriate sweetness level with one or more bulking agents, *e.g.*, maltodextrins, known to those skilled in the art. Steviol glycoside compositions enriched for RebA, RebB, RebD, RebE, or RebM, can be package in a sachet, for example, at from 10,000 to 30,000 mg steviol glycoside/kg product on a dry weight basis, for tabletop use. In some embodiments, a steviol glycoside produced *in vitro, in vivo,* or by whole cell bioconversion

The invention will be further described in the following examples, which do not limit the scope of the invention described in the claims.

### EXAMPLES

The Examples that follow are illustrative of specific embodiments of the invention, and various uses thereof. They are set forth for explanatory purposes only, and are not to be taken as limiting the invention.

### Example 1. Engineering of S. cerevisiae strain comprising UGT33942

UGT33942 was cloned and overexpressed in an *S. cerevisiae* strain. The S. *cerevisiae* strain transformed with a UGT33942 expression plasmid was grown for 24 h in synthetic complete medium (SC medium) lacking uracil to select for plasmid being there at 30°C. After 24 h of growth, steviol was added to the culture to a final concentration of 40µM. Samples for LC-MS were prepared 24 h after addition of steviol by mixing 1 volume of culture with one volume of DMSO, placing them at 80°C for 10 min, and centrifuging in an Eppendorff centrifuge 5415D at maximum speed. An aliquot of the resulting supernatant was then transferred to a new vial and subjected to LC-MS analysis.

LC-MS analysis was performed on Waters ACQUITY UPLC (Waters Corporation, Milford, MA) with coupled to a Waters ACQUITY ESI (electrospray ionization)-TQD triple quadropole mass spectrometer. Compound separation was achieved on Waters ACQUITY UPLC^{®} BEH C18 column (2.1 x 50 mm, 1.7 µm particles, 130 Å pore size) equipped with ACQUITY UPLC BEH C18 VanGuard pre-column (130 Å, 1.7 µm, 2.1 mm x 5 mm) by using a gradient of the two mobile phases: A (H₂O with 0.1% formic acid) and B (Acetonitrile with 0.1% formic acid), increasing B from 20% to 50% between 0.3 to 2.0 min and up to 100% at 2.01 min, holding at 100 % for 0.6 min, and re-equilibrating for 0.6 min. The flow rate was 0.6 mL/min, and the column temperature was 55°C. The MS acquisition was in negative ion-mode using Single Ion Monitoring (SIM) mode. Steviol glycoside identification was done by comparison with known standards.

Area-under-the-curve (AUC) values for LC-MS derived peaks corresponding to steviol and 19-SMG were determined for the *S. cerevisiae* strain overexpressing UGT33942 and for a control *S. cerevisiae* strain comprising an empty vector. Results are shown in Figure 3. The majority of the steviol fed to the *S. cerevisiae* strain overexpressing UGT33942 was converted to 19-SMG, while the control yeast strain did not convert the fed steviol. Thus, UGT33942 glucosylates steviol on the 19*-O-*position resulting in production of 19-SMG. In *S*. *rebaudiana*, a UGT of the UGT74G family catalyzes 19-*O* glycosylation; however, UGT33942 is a UGT polypeptide with high structural similarity to members of the UGT85 family. The activity of UGT33942 is surprising and unexpected because members of the UGT85 family have not been previously characterized as having predominantly 19-*O* glycosylation activity.

### Example 2. Engineering of S. cerevisiae strain comprising UGT33786

UGT33786 was cloned and overexpressed in an *S. cerevisiae* strain. The *S*. *cerevisiae* strain transformed with a UGT33786 expression plasmid was grown for 24 h in synthetic complete medium (SC medium) lacking uracil to select for plasmid being there and to maintain the plasmid at 30°C. After 24 h of growth, steviol was added to the culture to a final concentration of 40µM. Samples for LC-MS were prepared 24 h after addition of steviol by mixing 1 volume of culture with one volume of DMSO, placing them at 80°C for 10 min, and centrifuging in an Eppendorff centrifuge 5415D at maximum speed. An aliquot of the resulting supernatant was then transferred to a new vial and subjected to LC-MS analysis.

LC-MS analysis was performed according to the method described in Example 1. Area-under-the-curve (AUC) values for LC-MS derived peaks corresponding to steviol and 19-SMG were determined for the *S*. *cerevisiae* strain overexpressing UGT33786 and for a control *S. cerevisiae* strain comprising an empty vector. Results are shown in Figure 4. Portion of the steviol fed to the *S*. *cerevisiae* strain overexpressing UGT33786 was converted to 19-SMG, while the control yeast strain did not convert the fed steviol. Thus, UGT33786 glucosylates steviol on the 19-*O*-position, resulting in production of 19-SMG. As for UGT33944, UGT33786 is a UGT polypeptide with high structural similarity to members of the UGT85 family. Thus, the 19-*O* glycosylation activity of UGT33944 is also surprising and unexpected.

Having described the invention in detail and by reference to specific embodiments thereof, it will be apparent that modifications and variations are possible without departing from the scope of the invention defined in the appended claims. More specifically, although some aspects of the present invention are identified herein as particularly advantageous, it is contemplated that the present invention is not necessarily limited to these particular aspects of the invention.

The scope of the invention is defined by the appended claims.

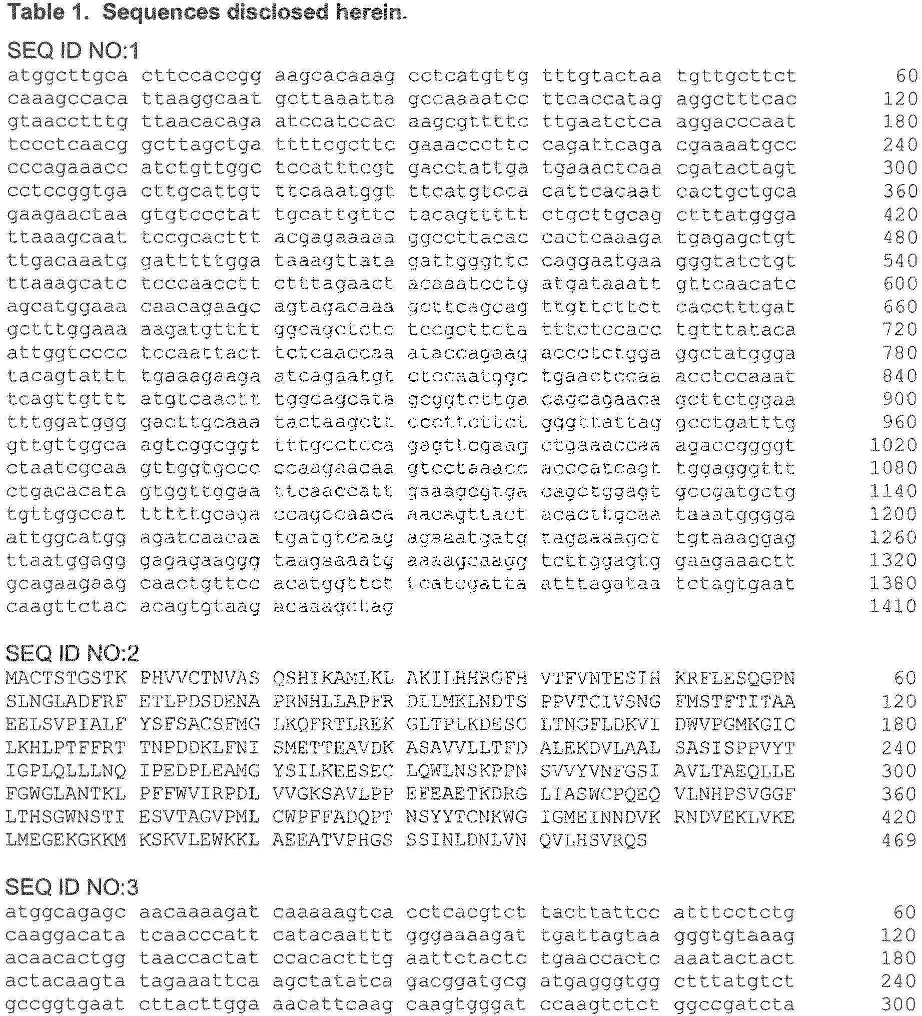

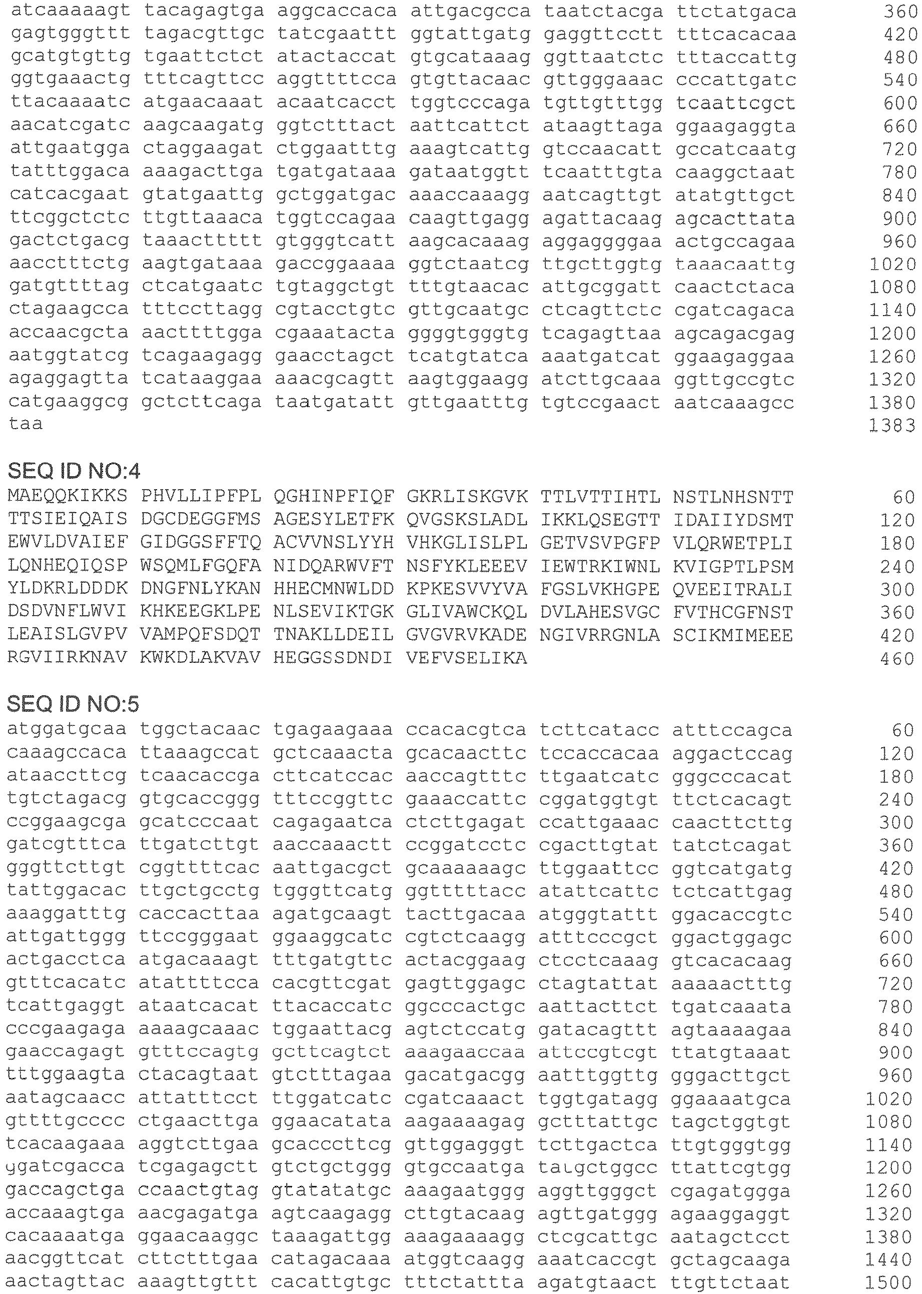

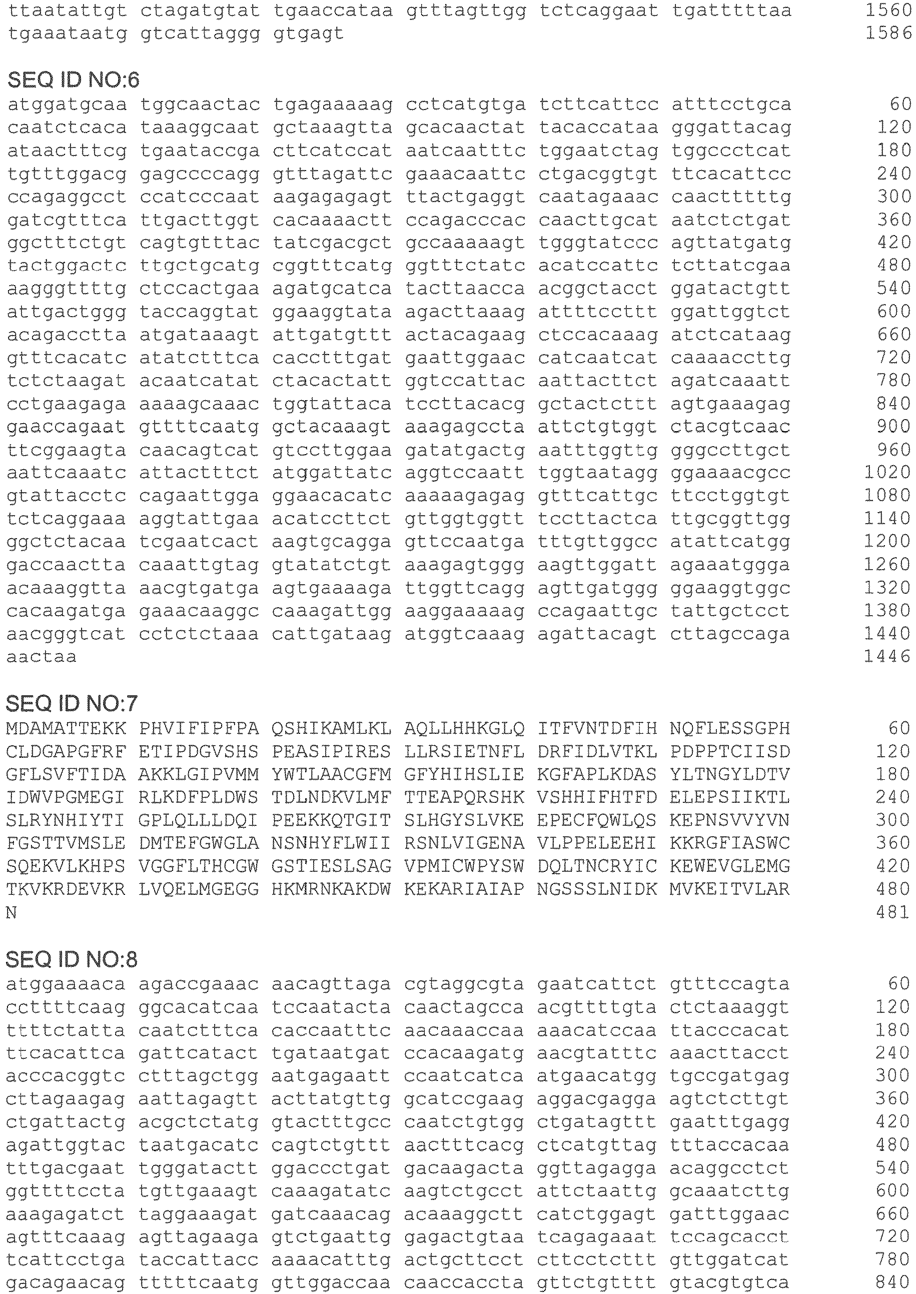

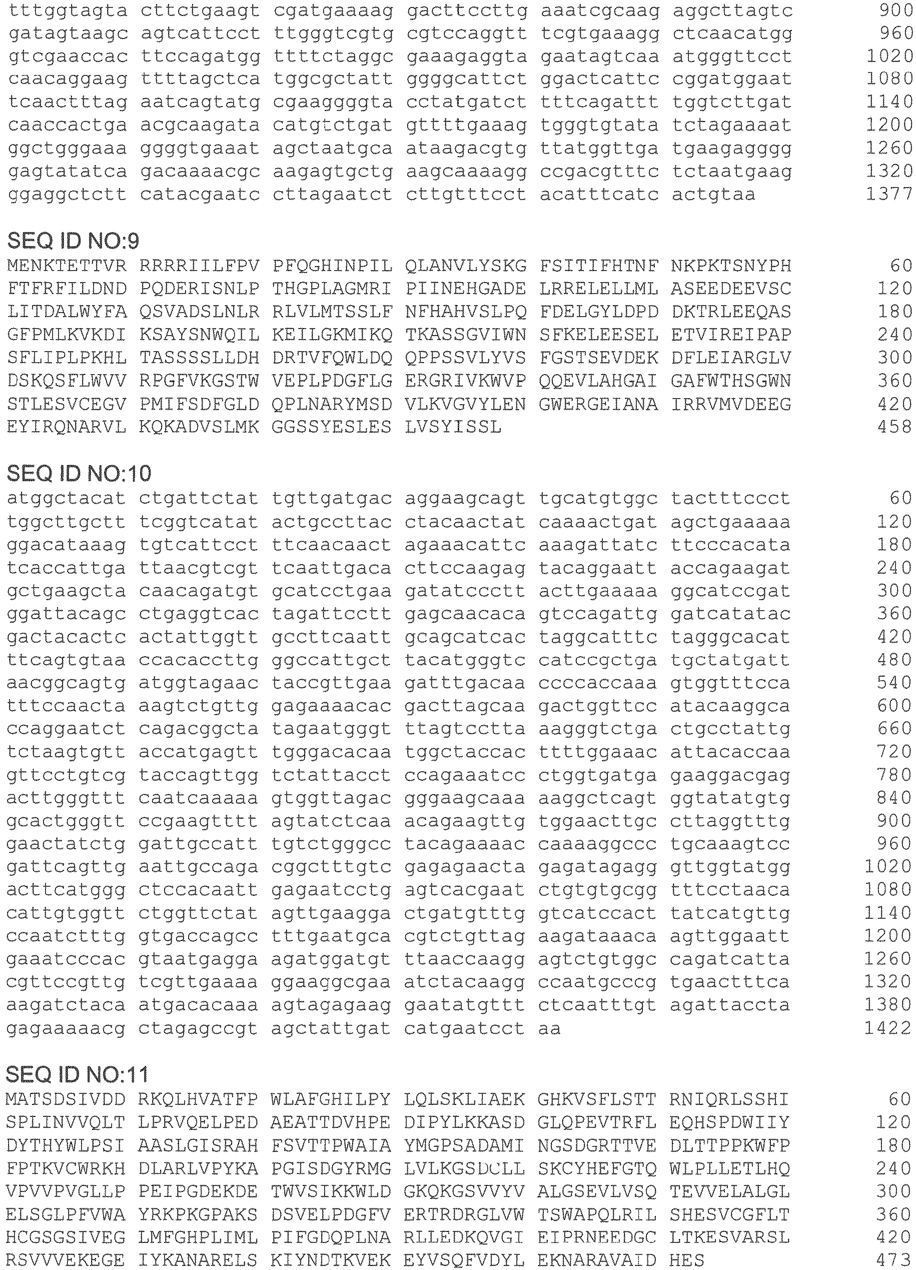

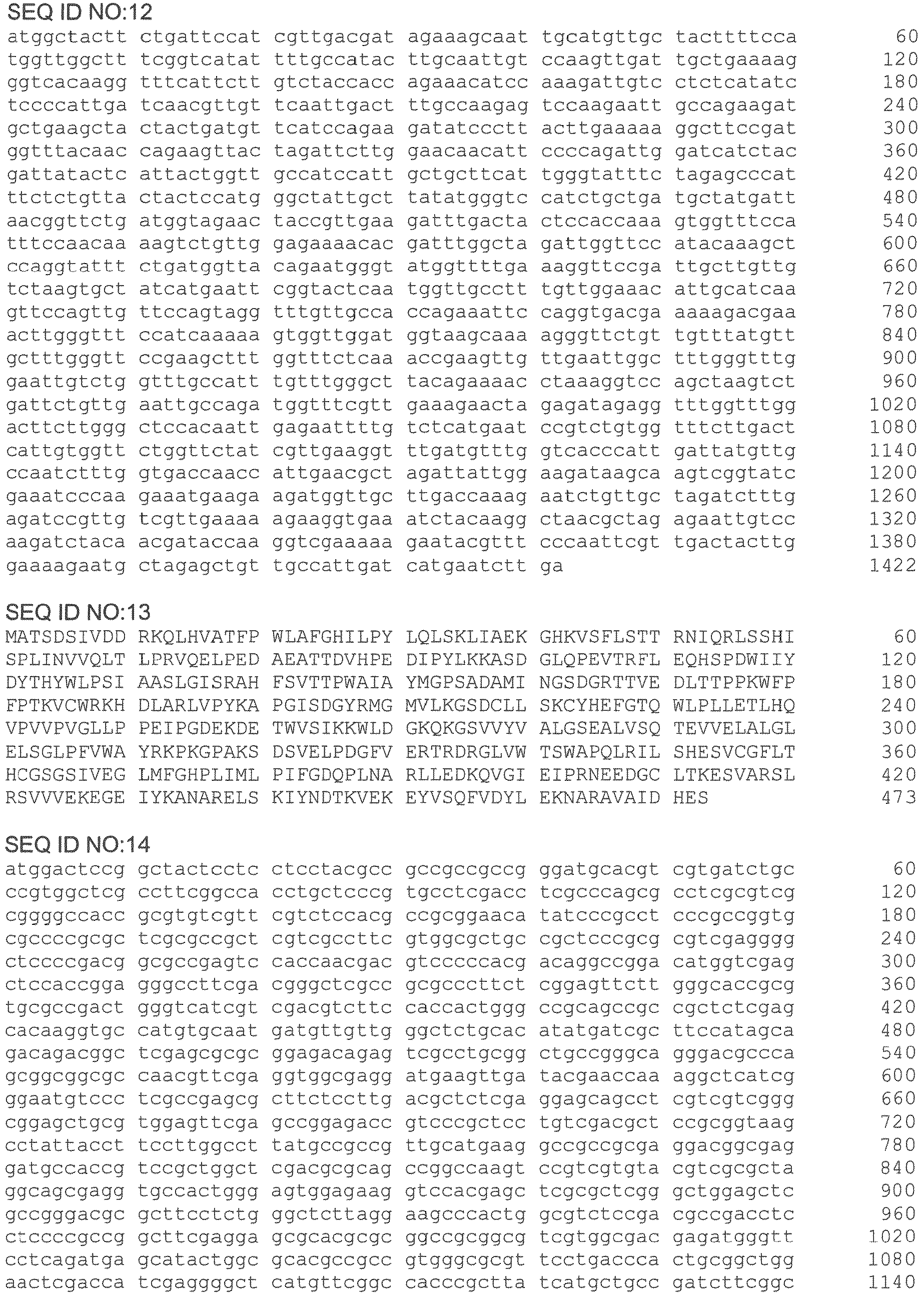

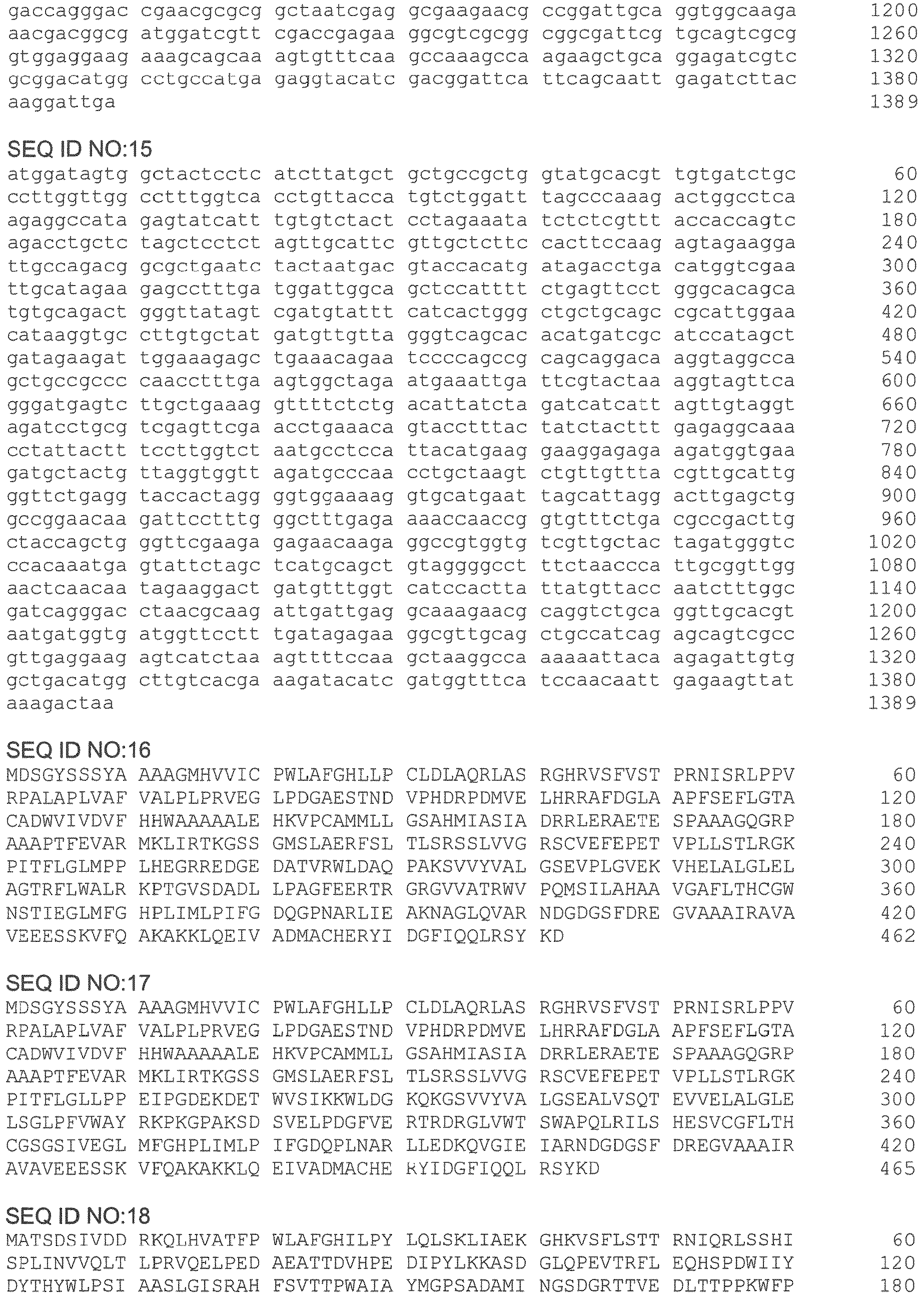

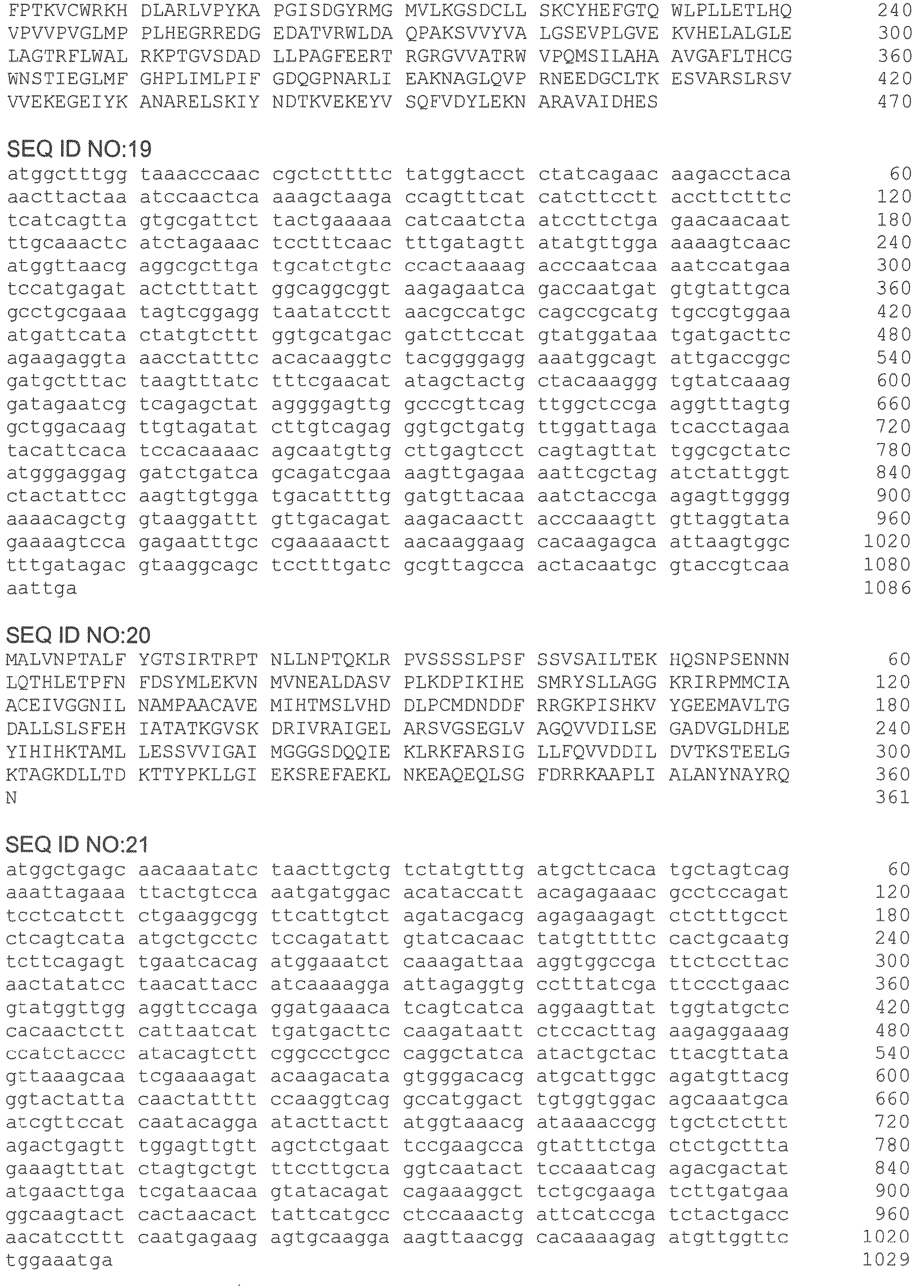

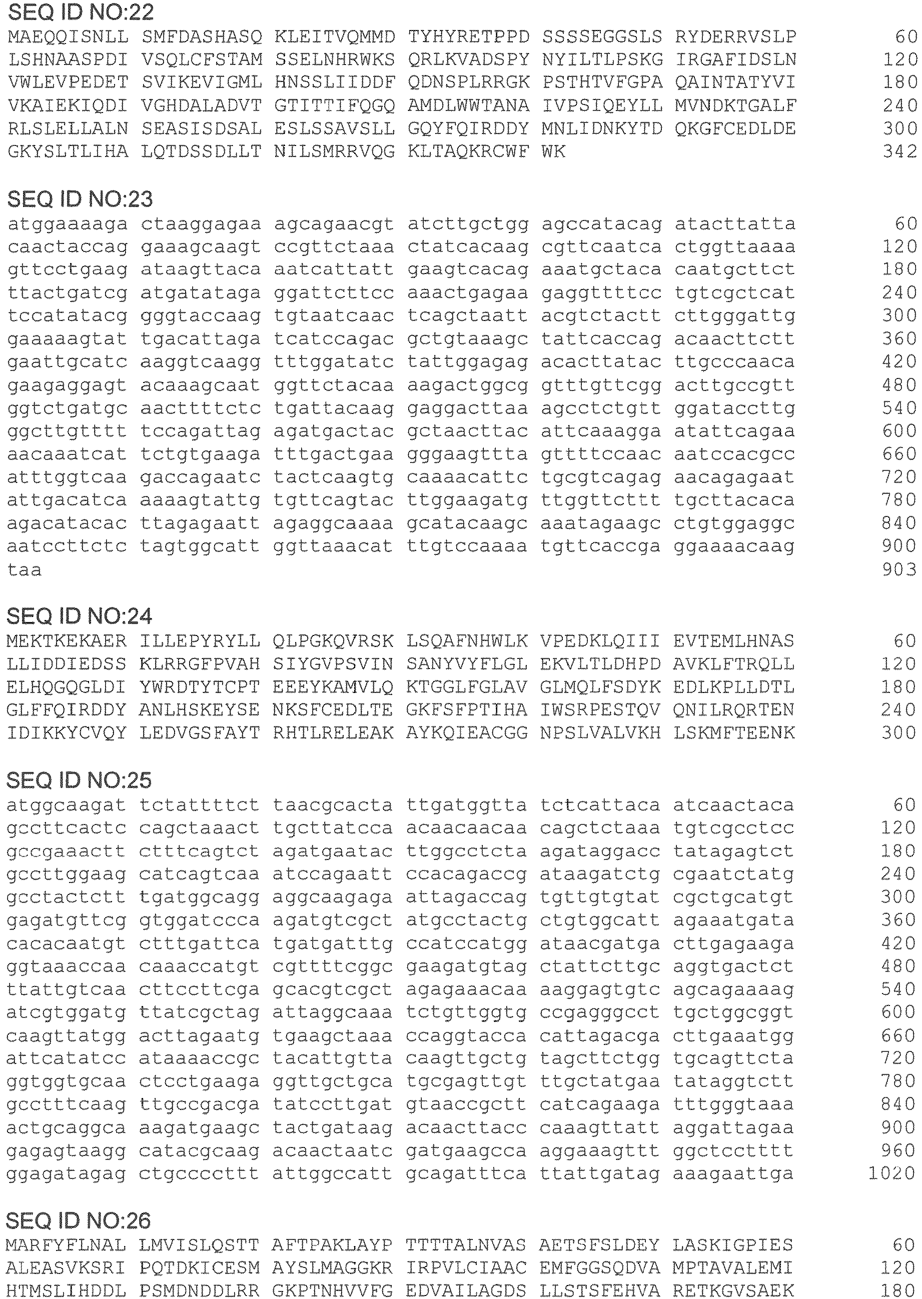

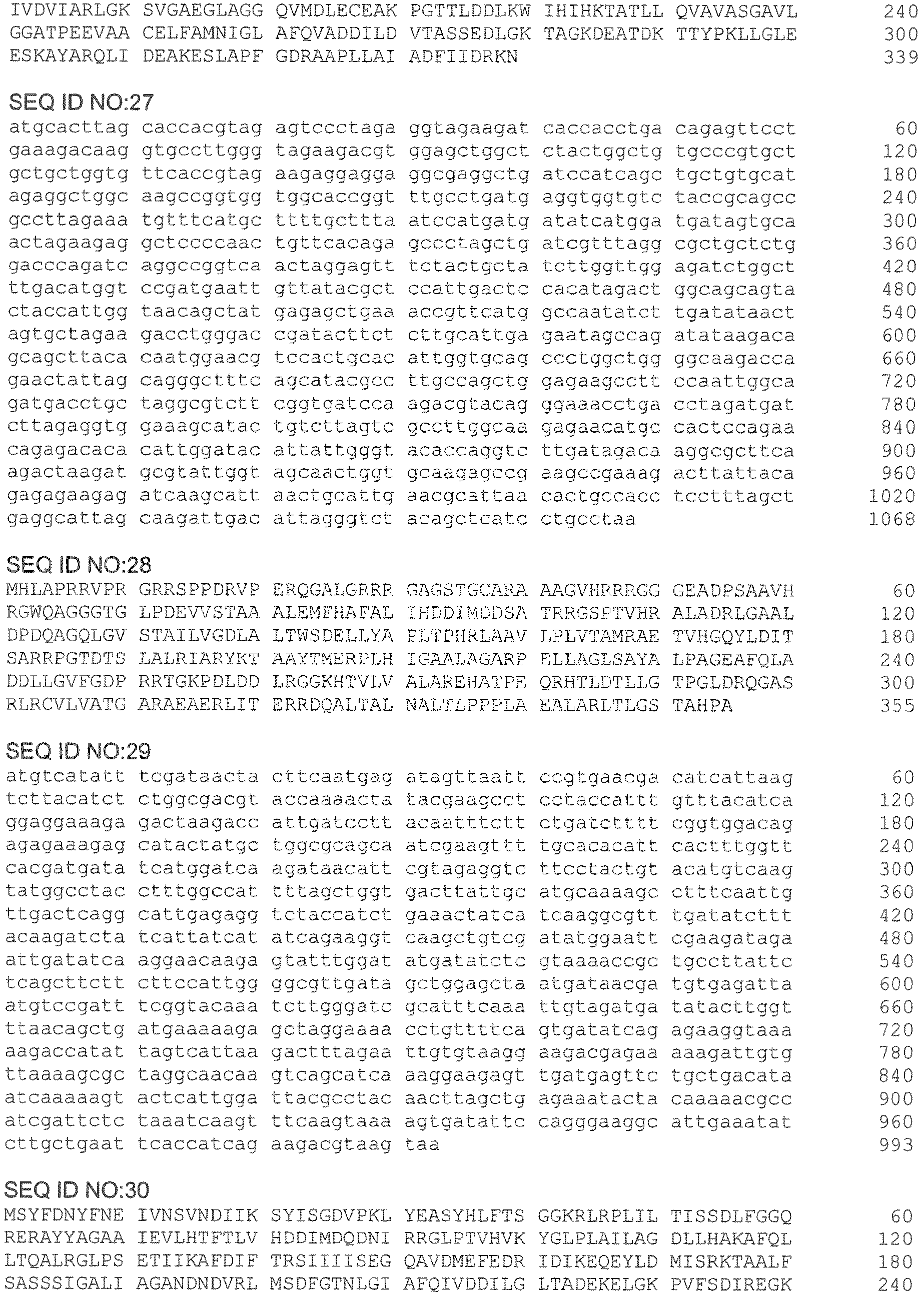

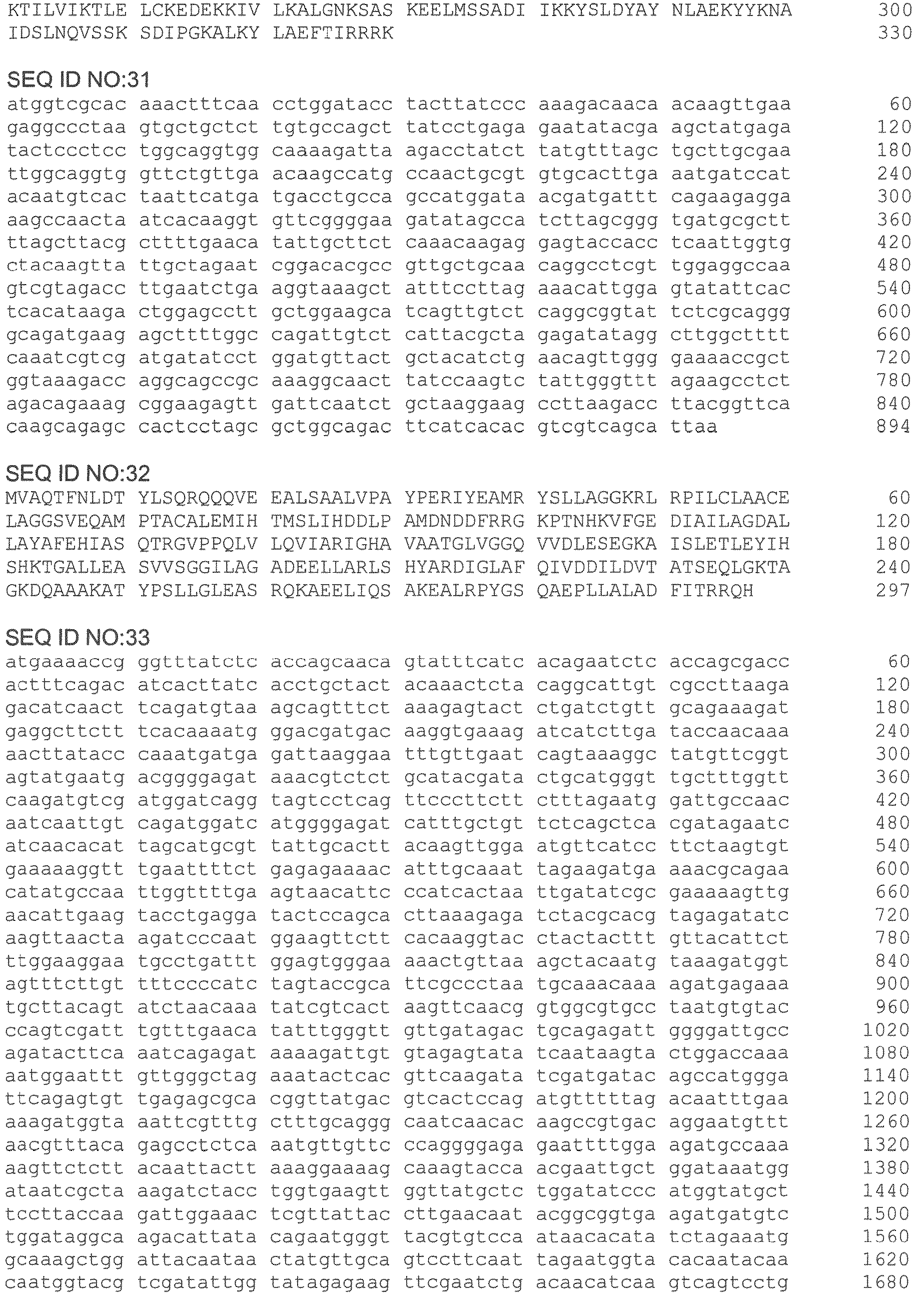

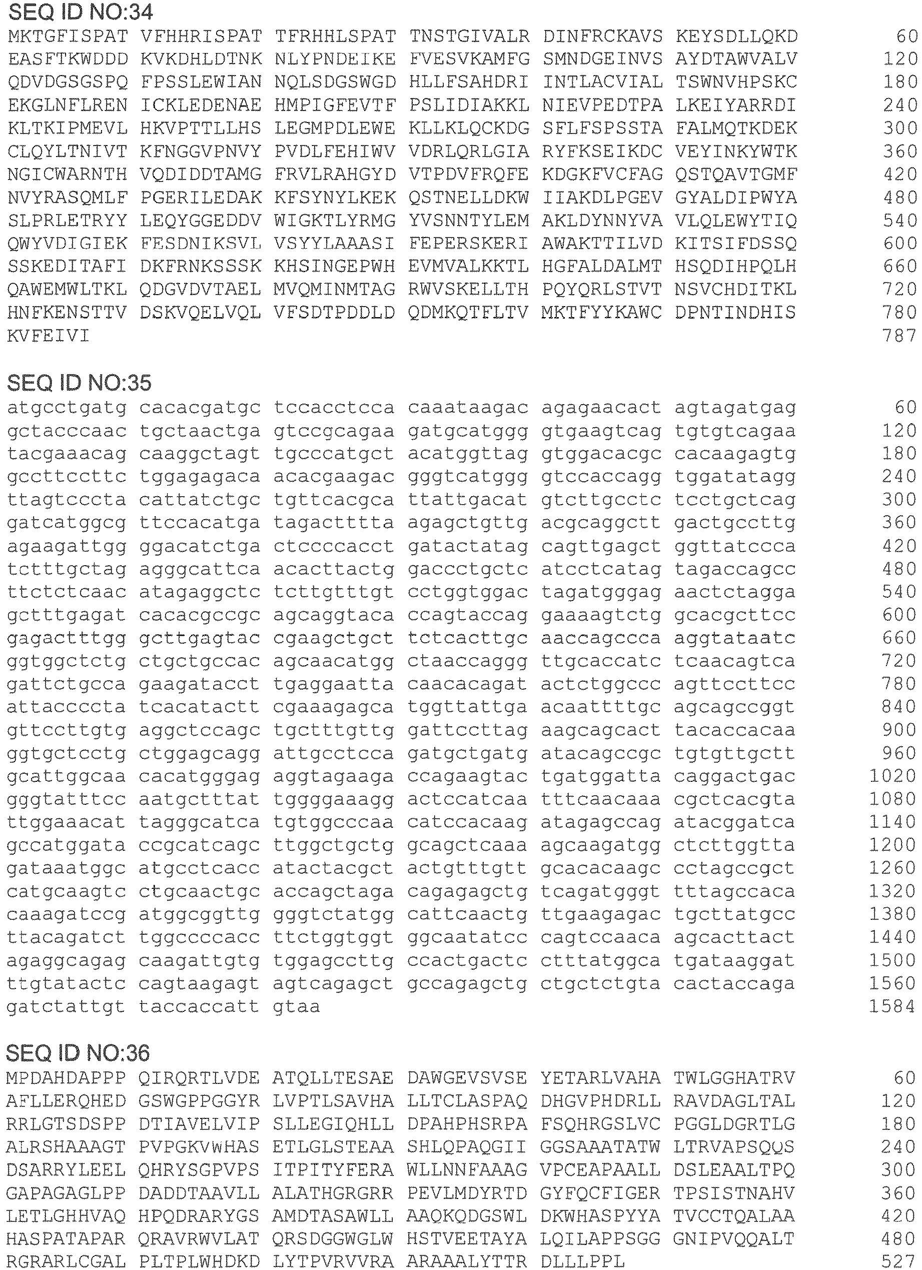

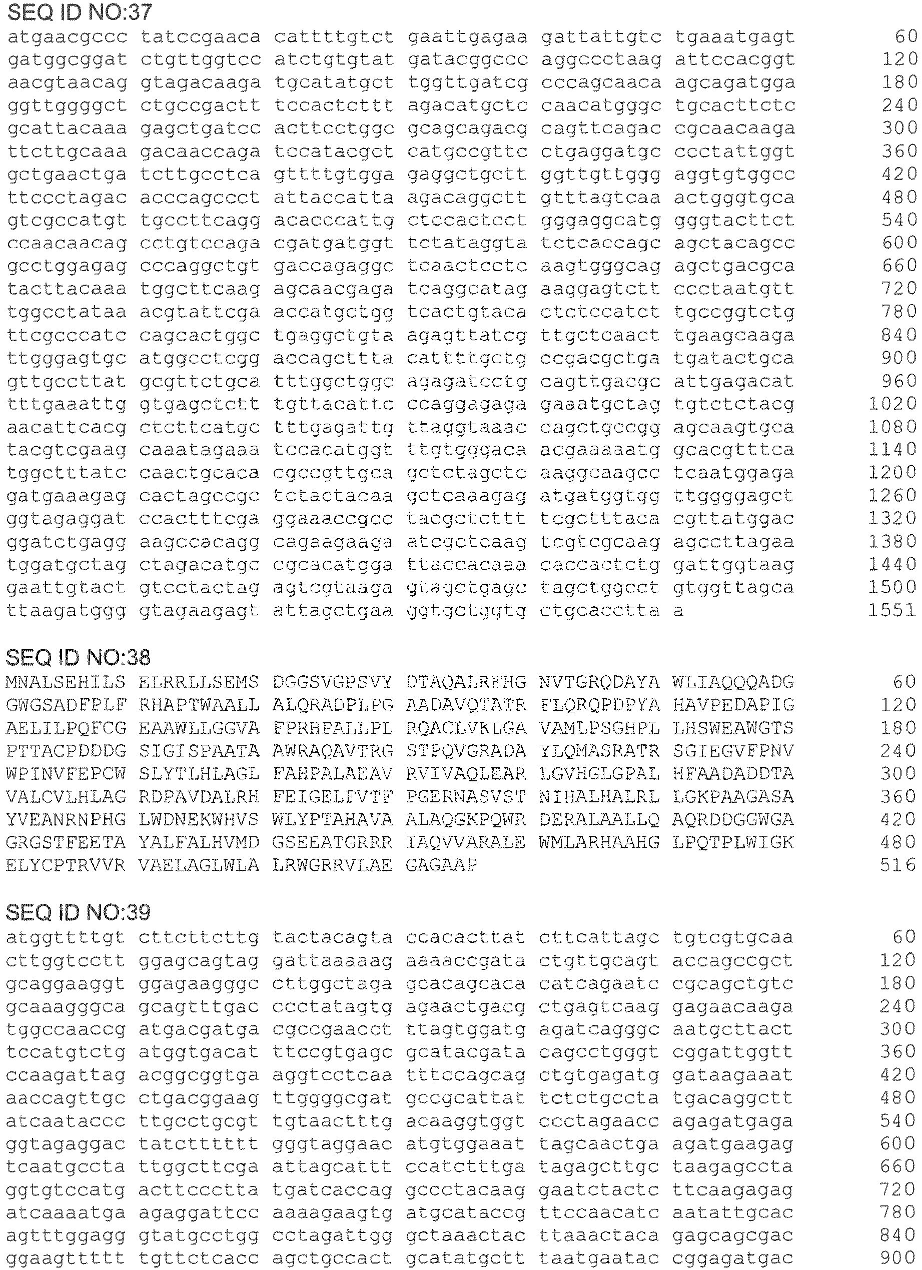

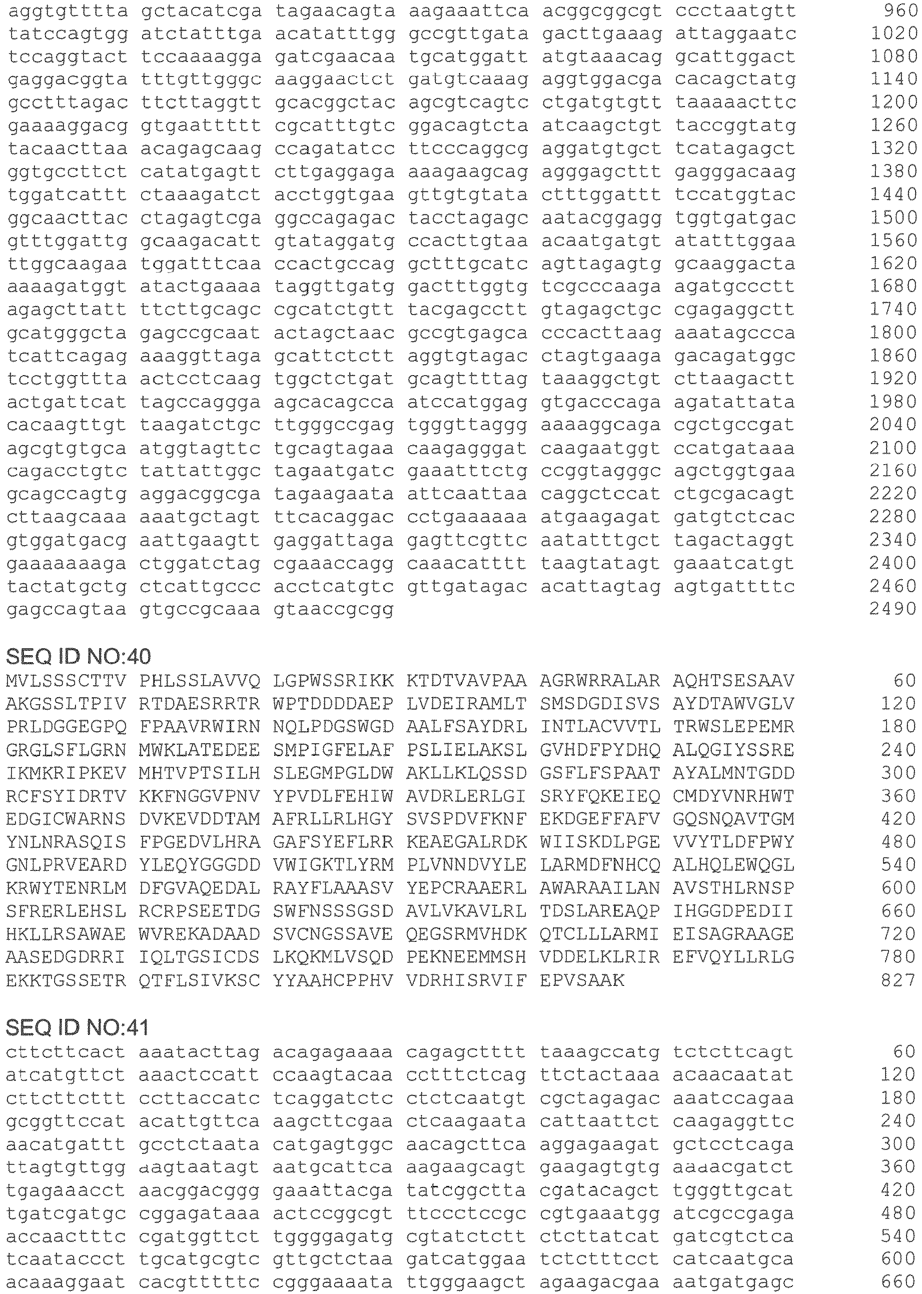

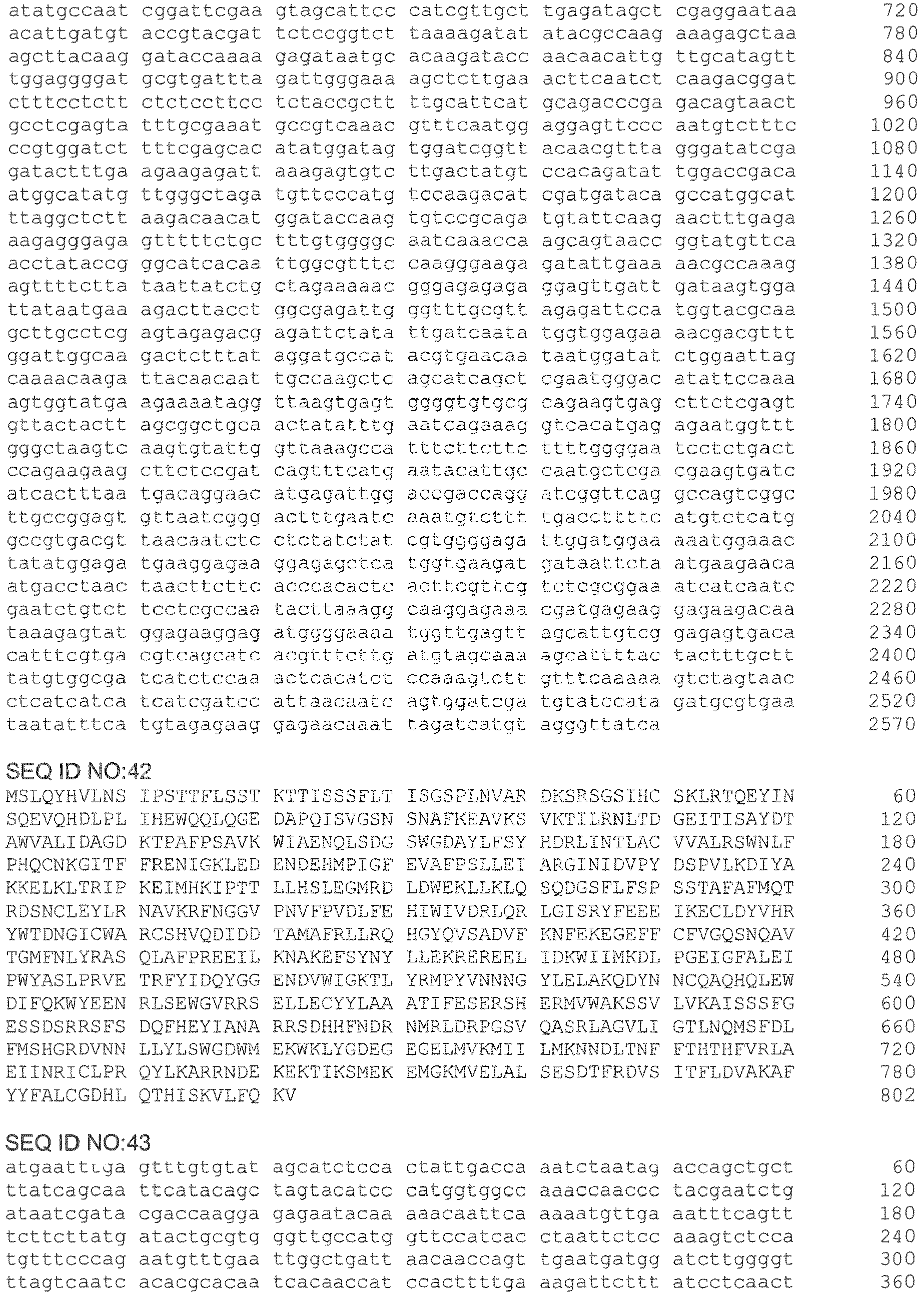

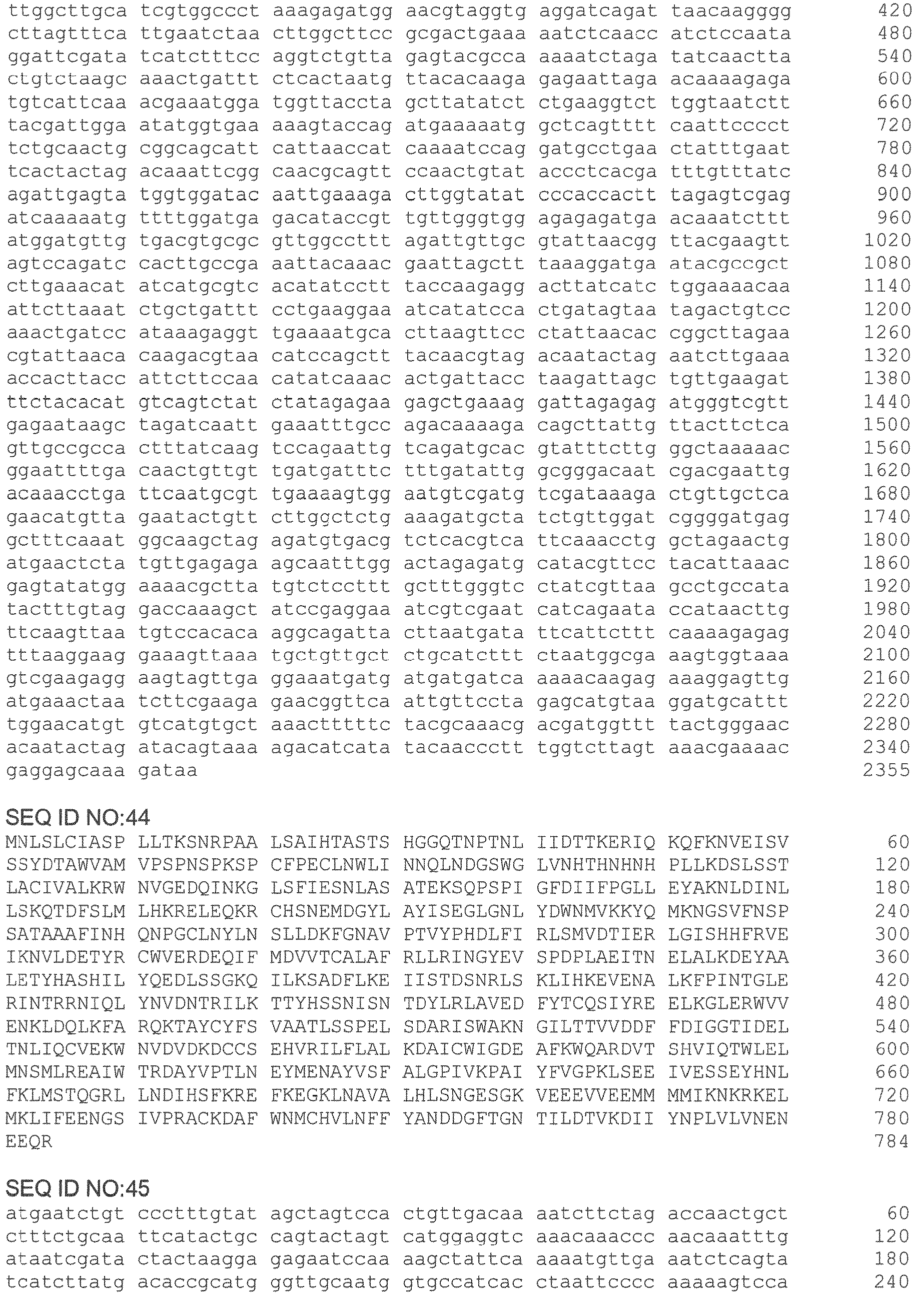

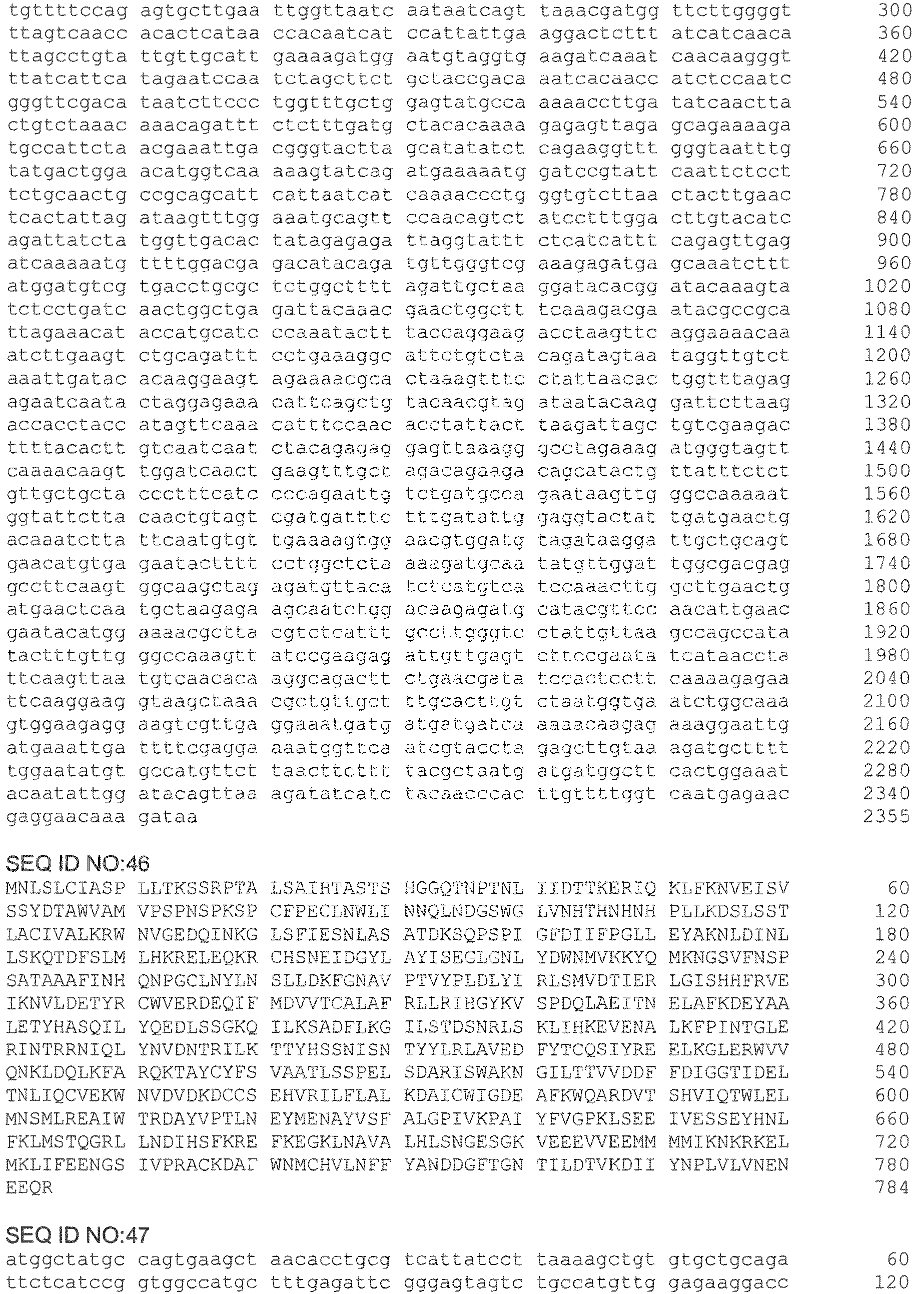

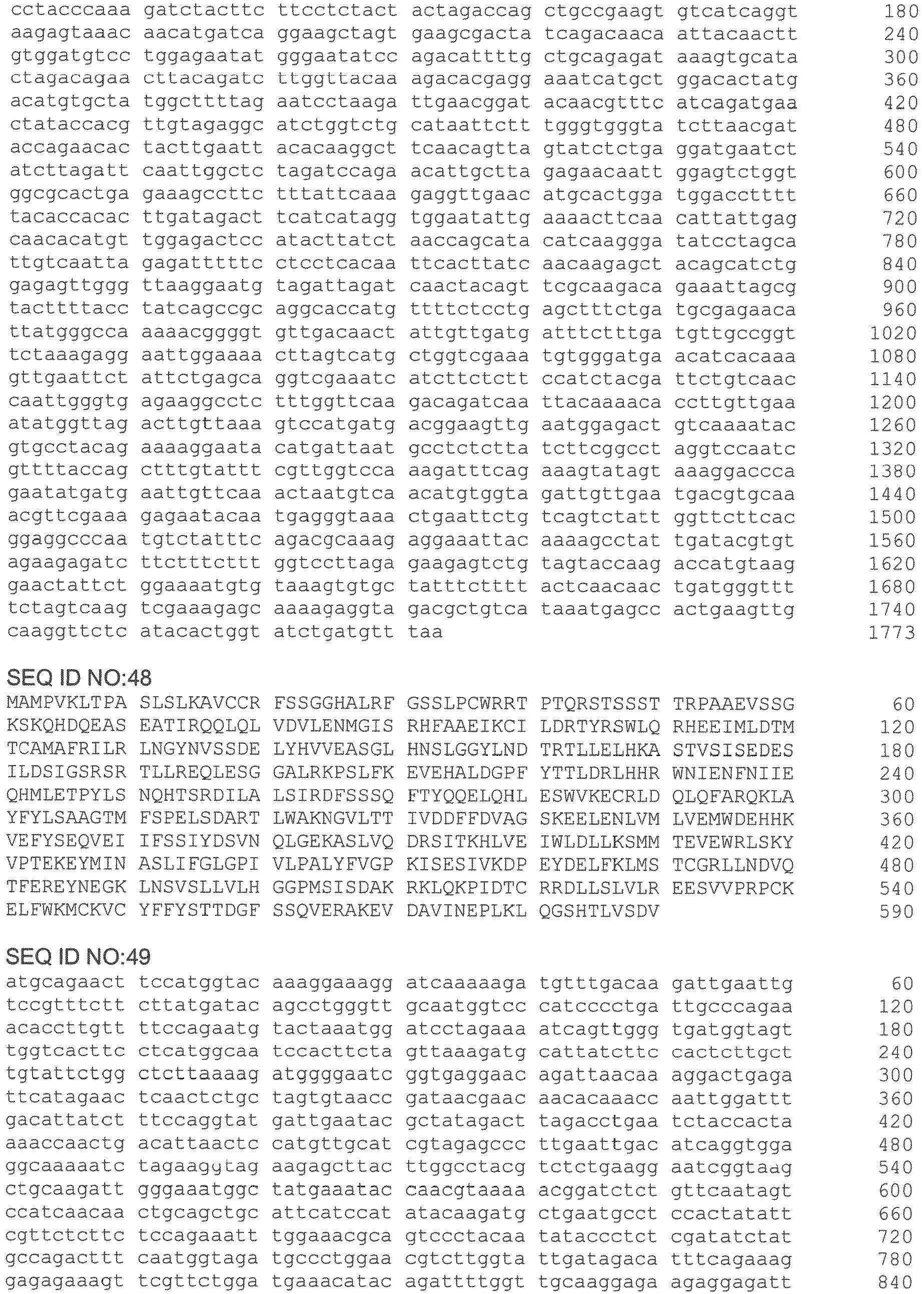

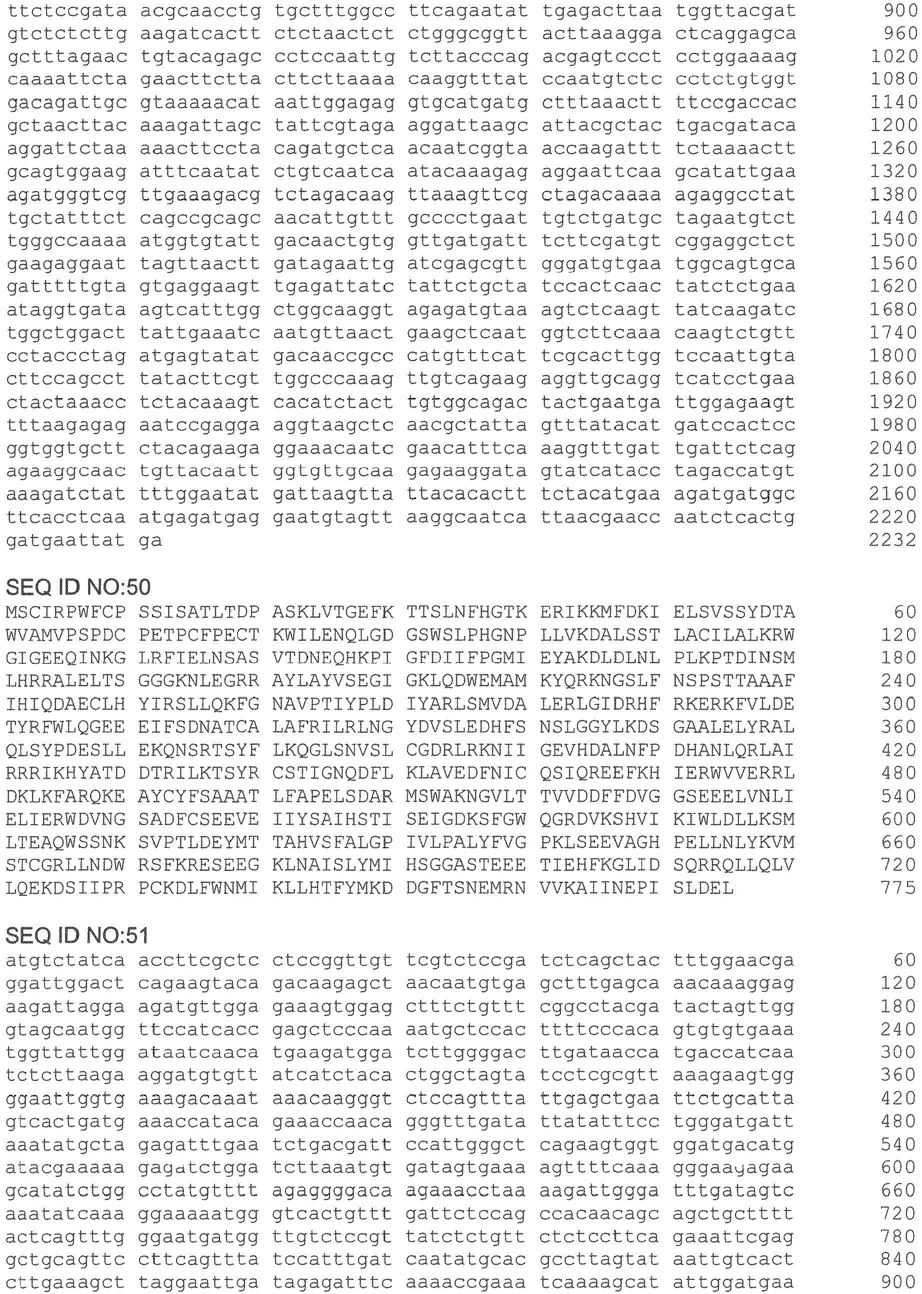

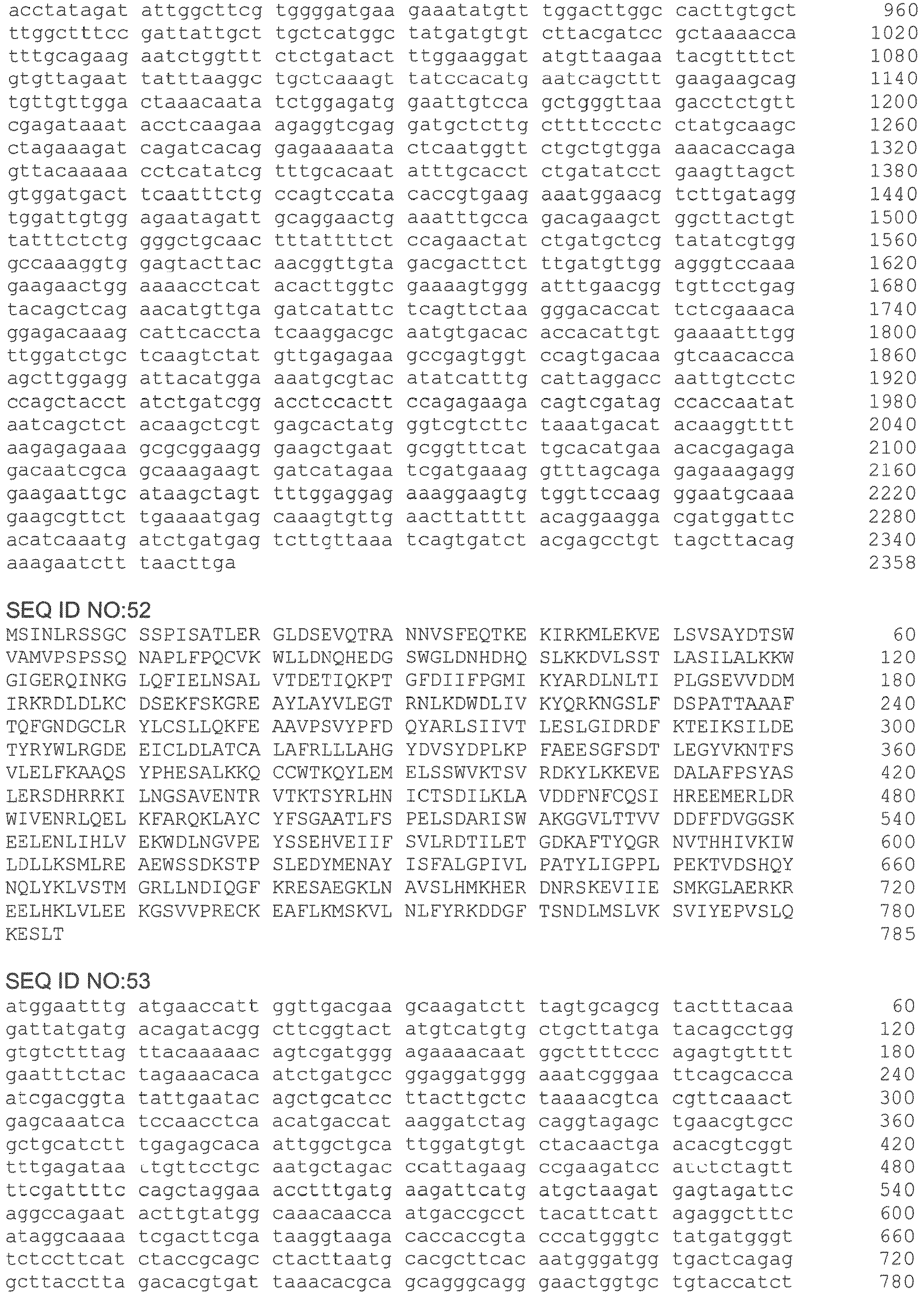

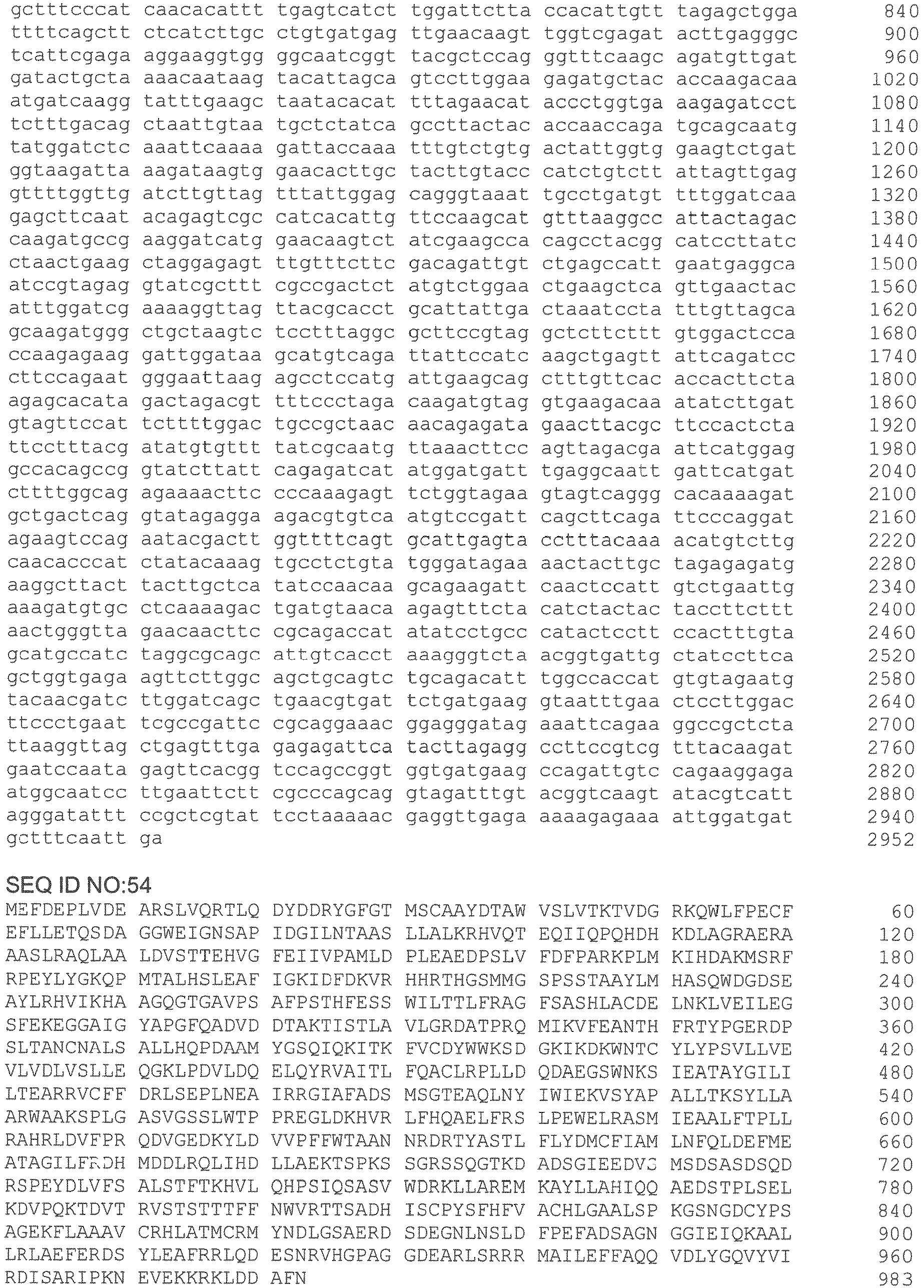

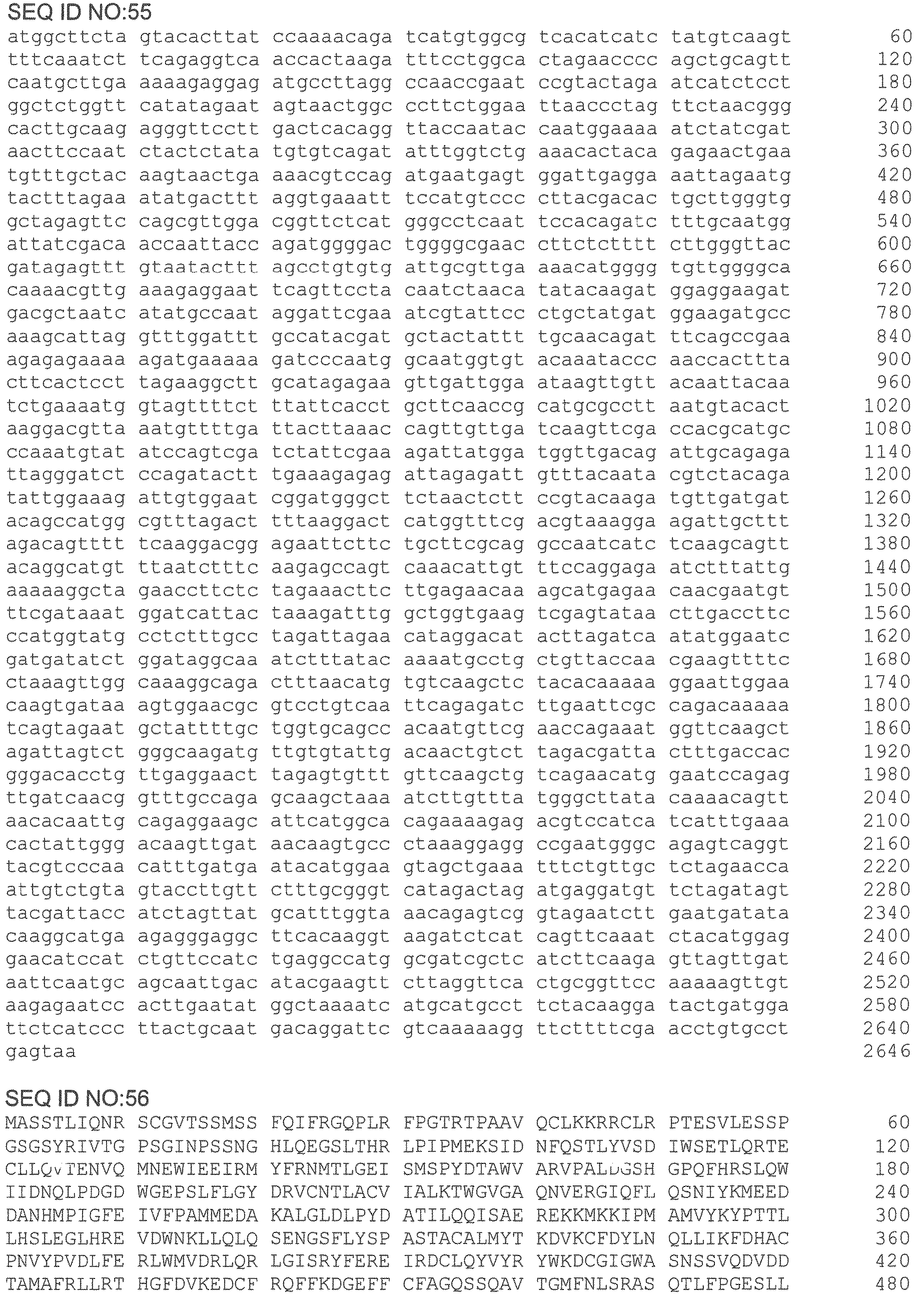

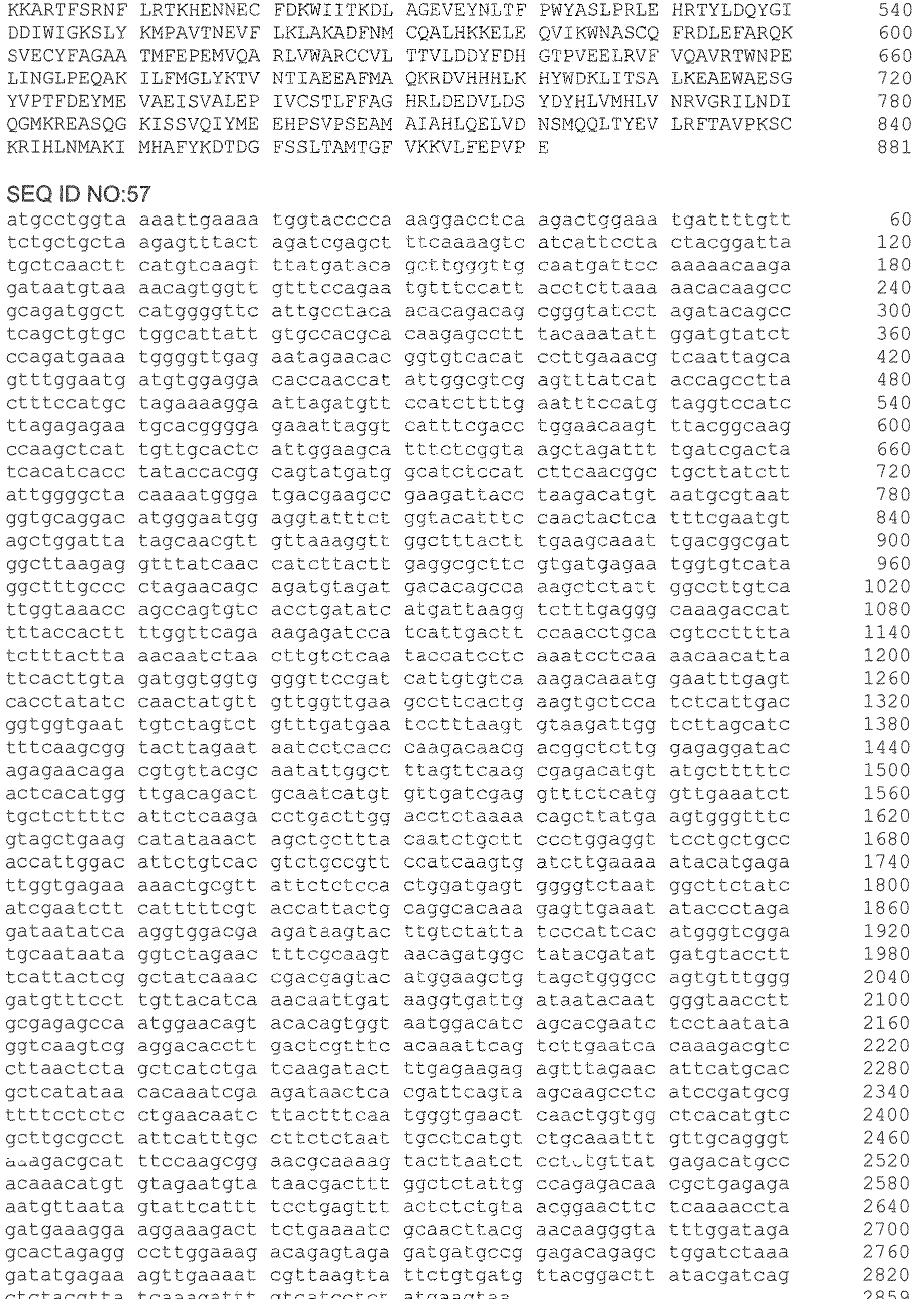

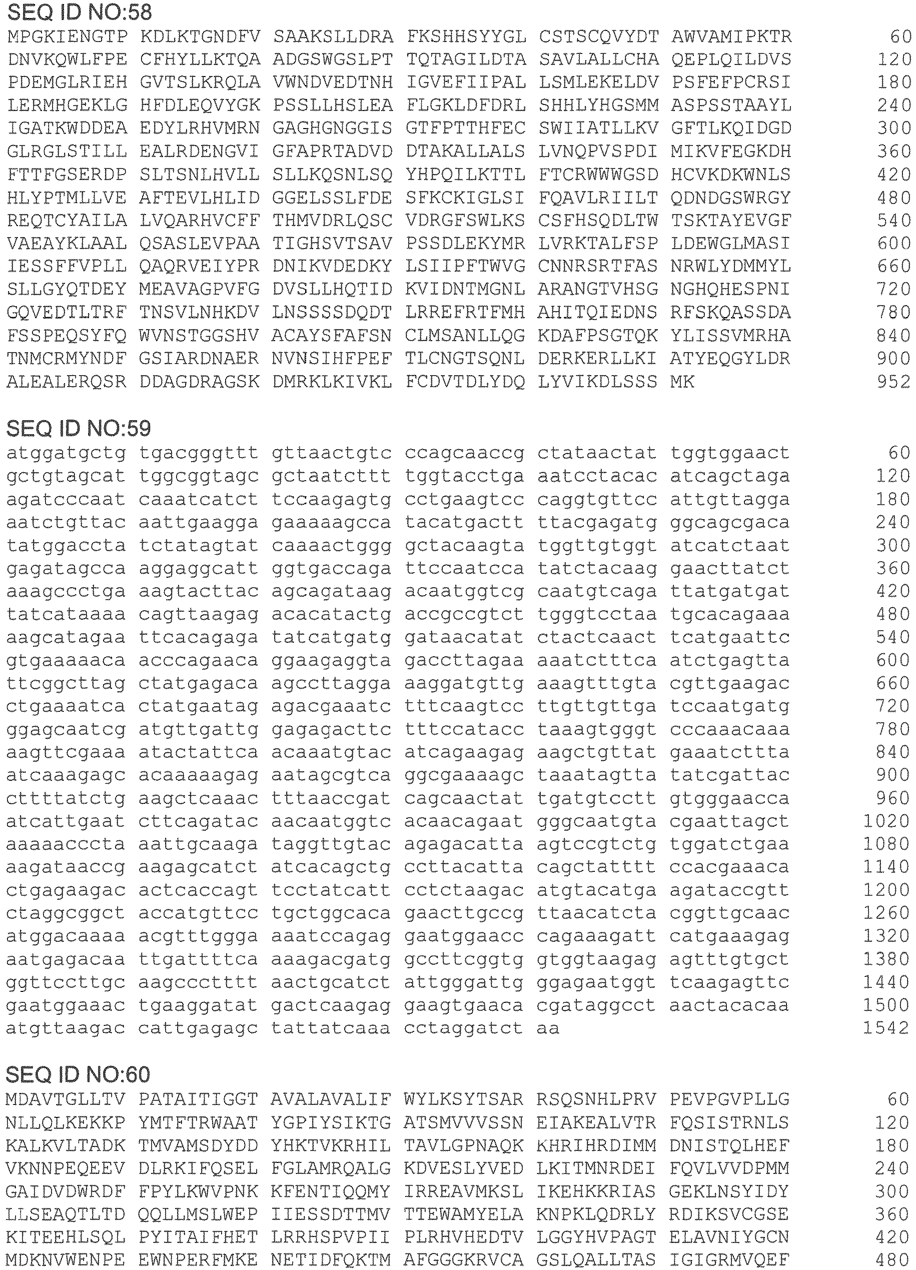

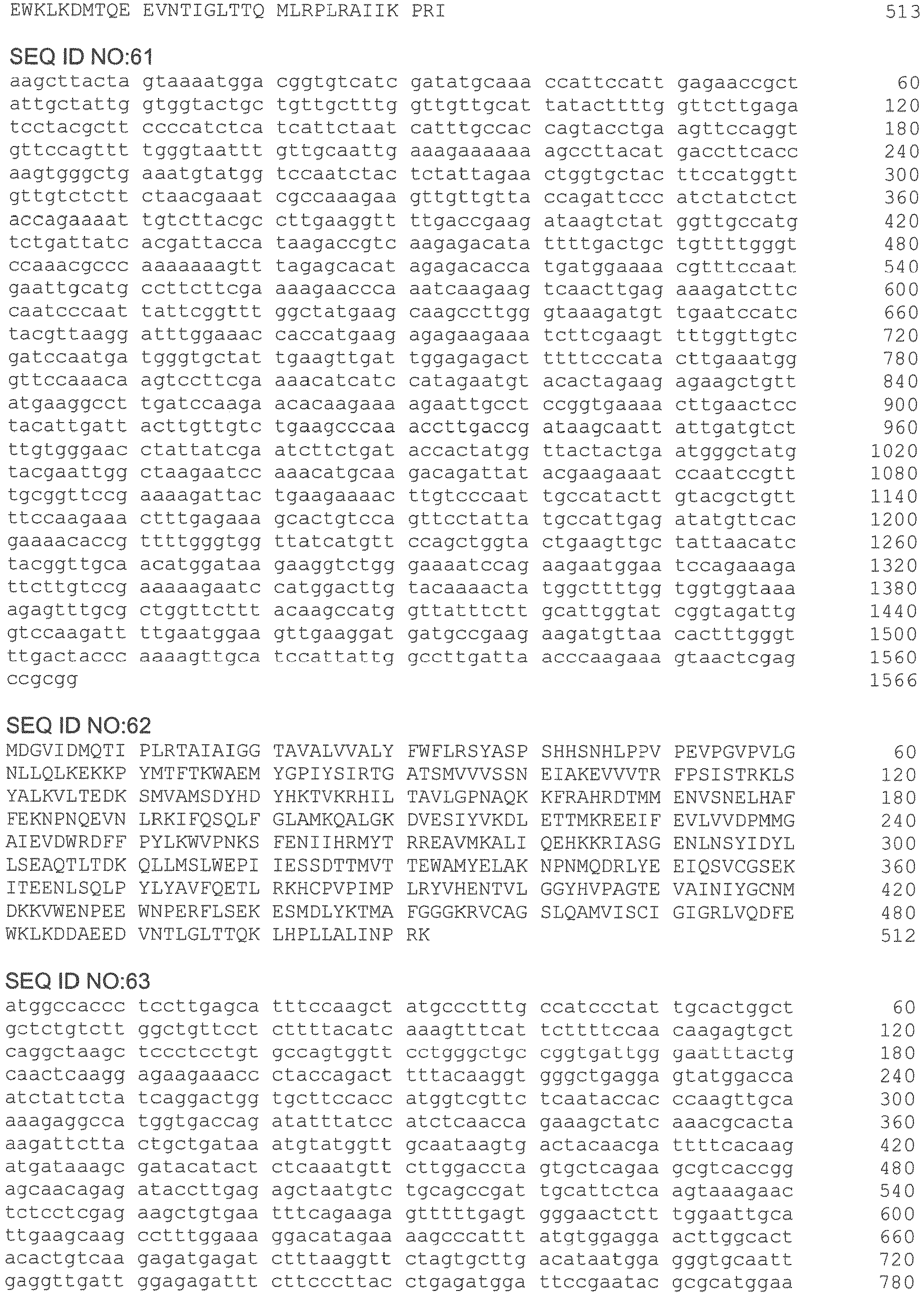

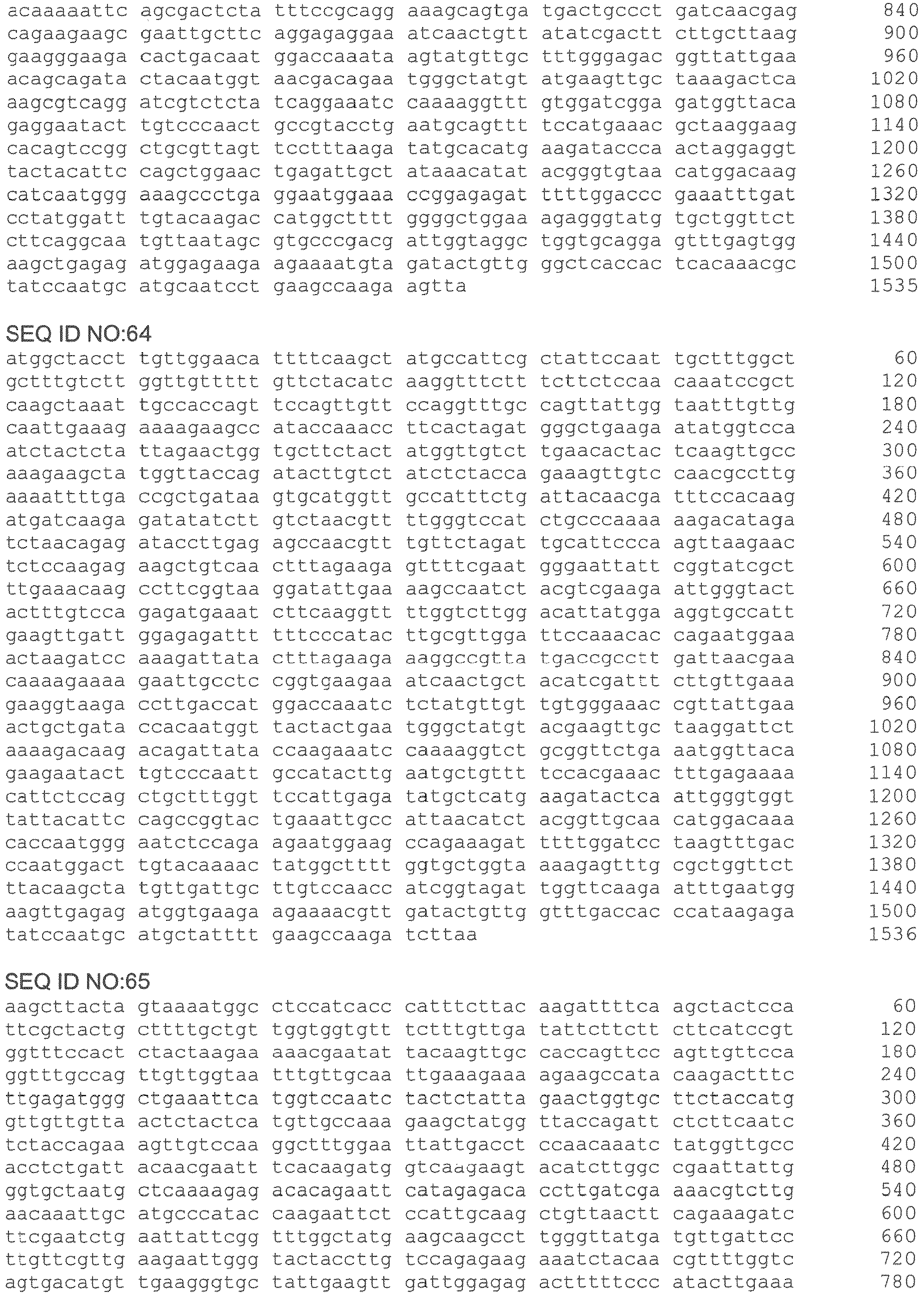

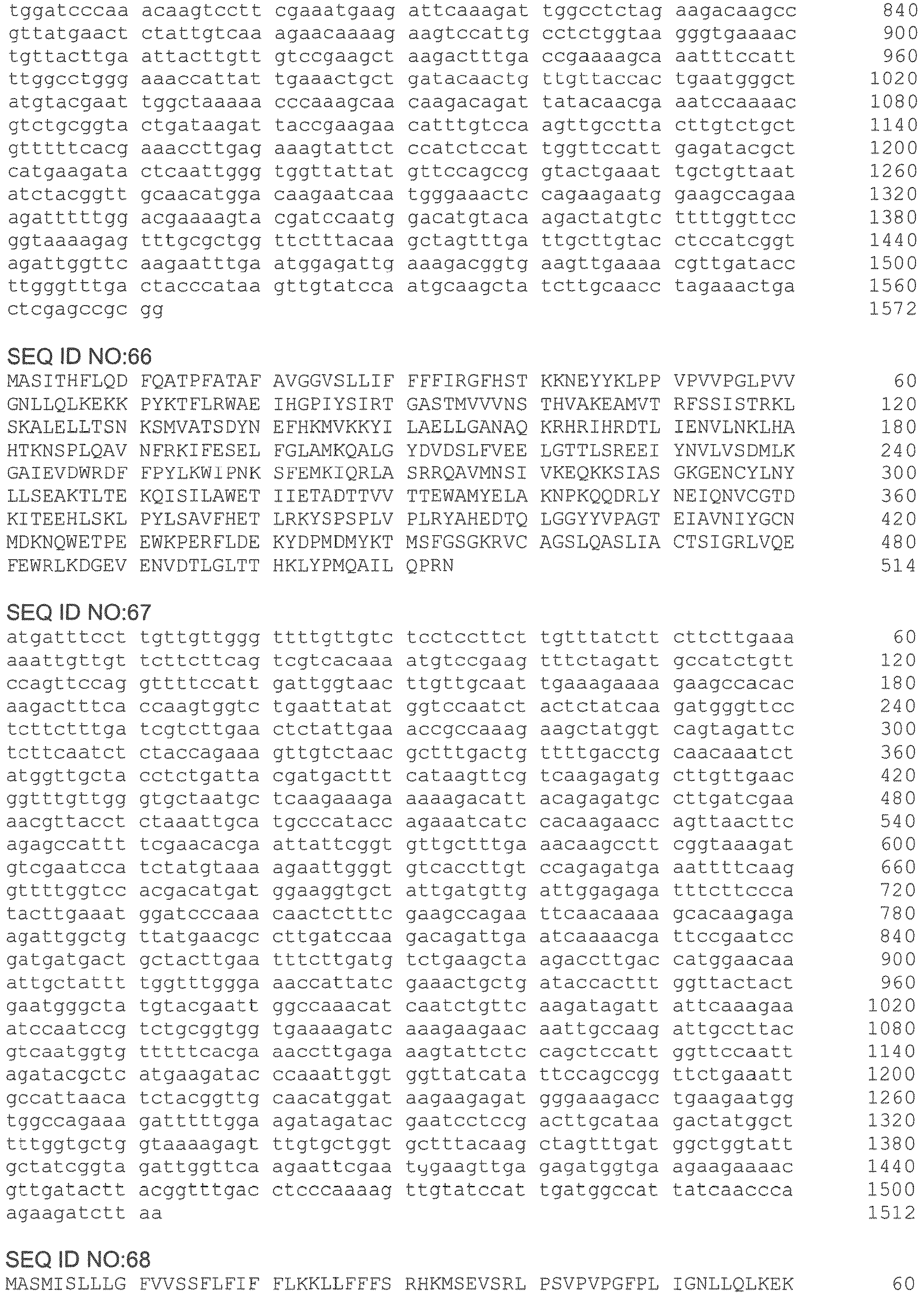

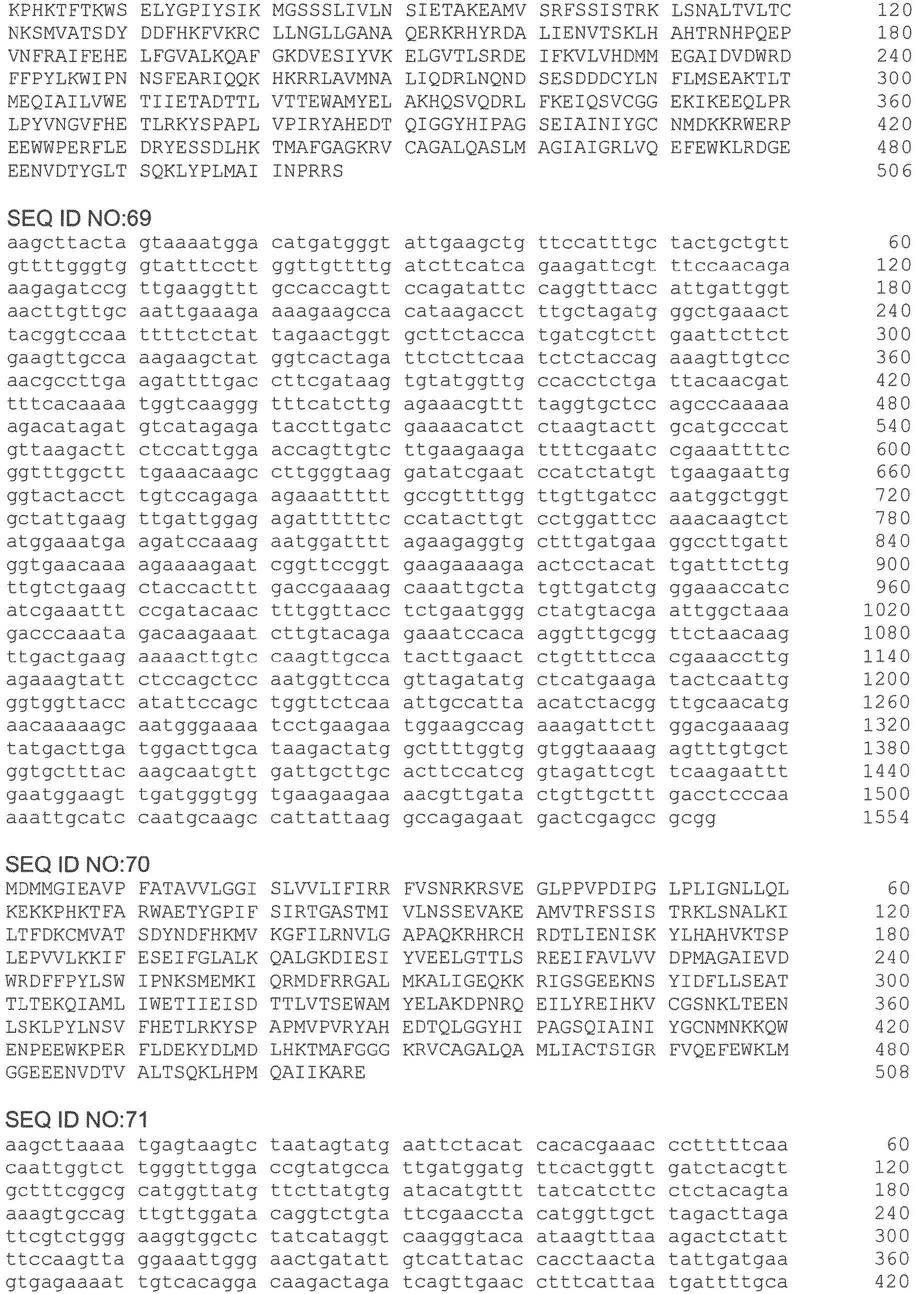

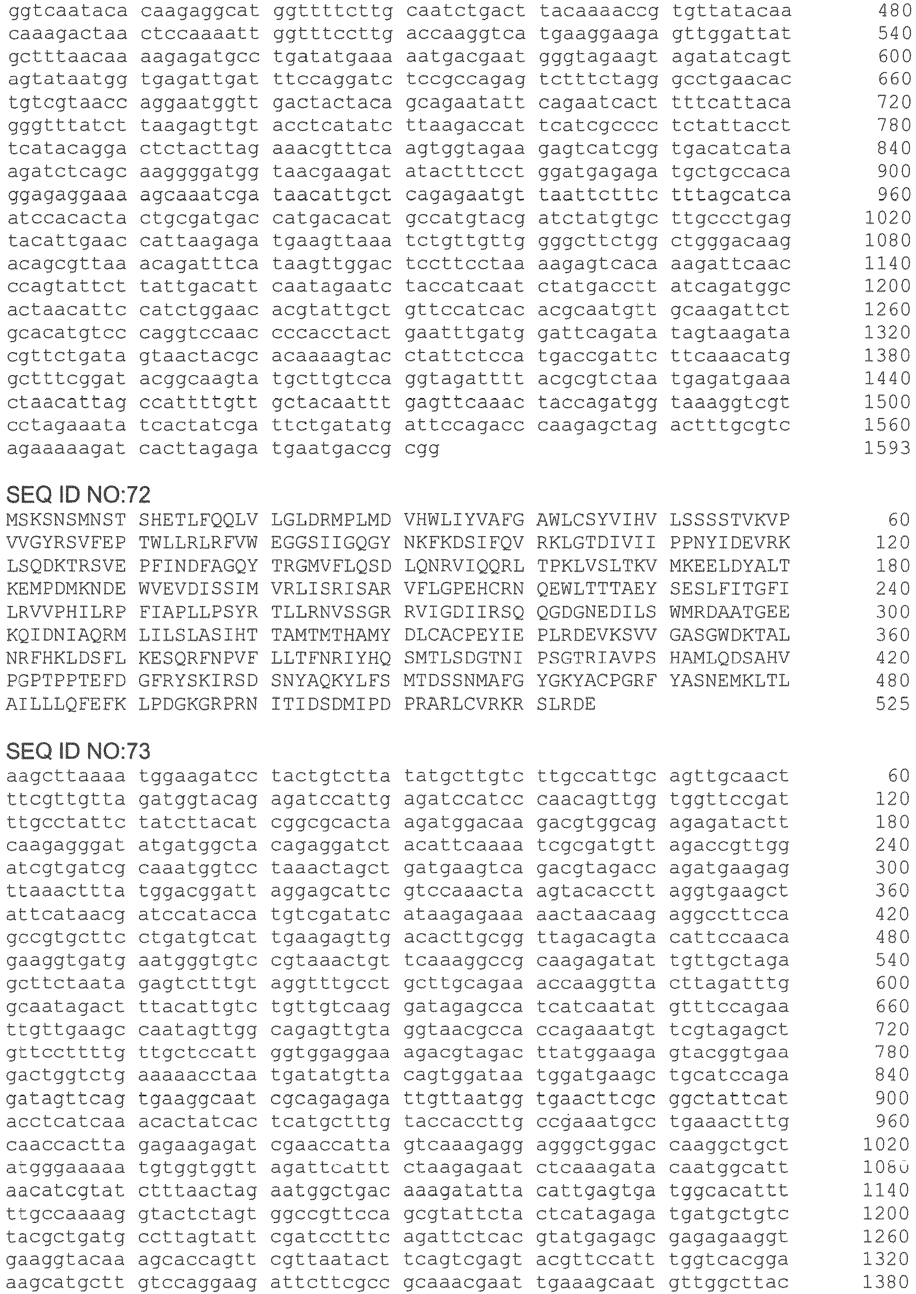

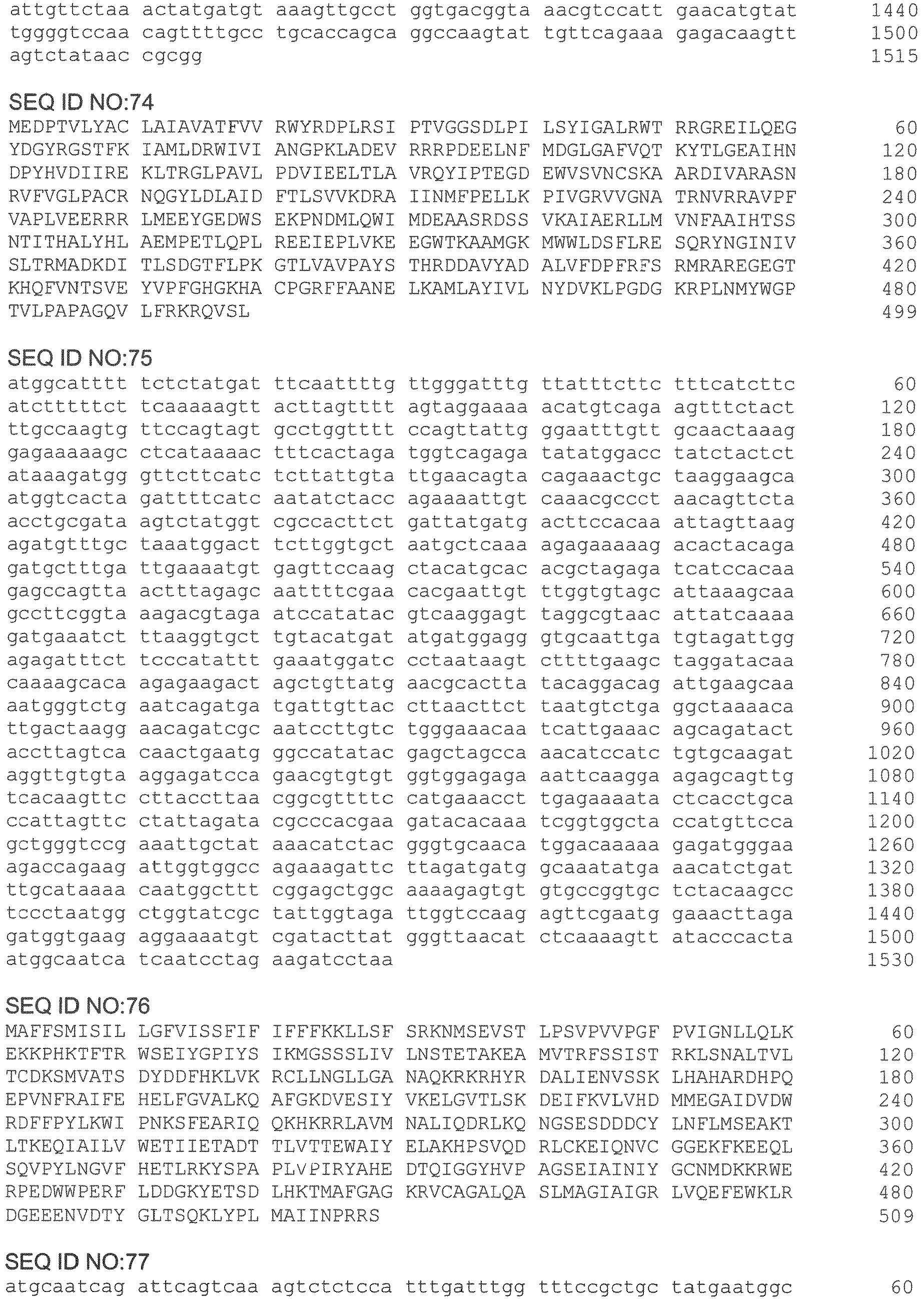

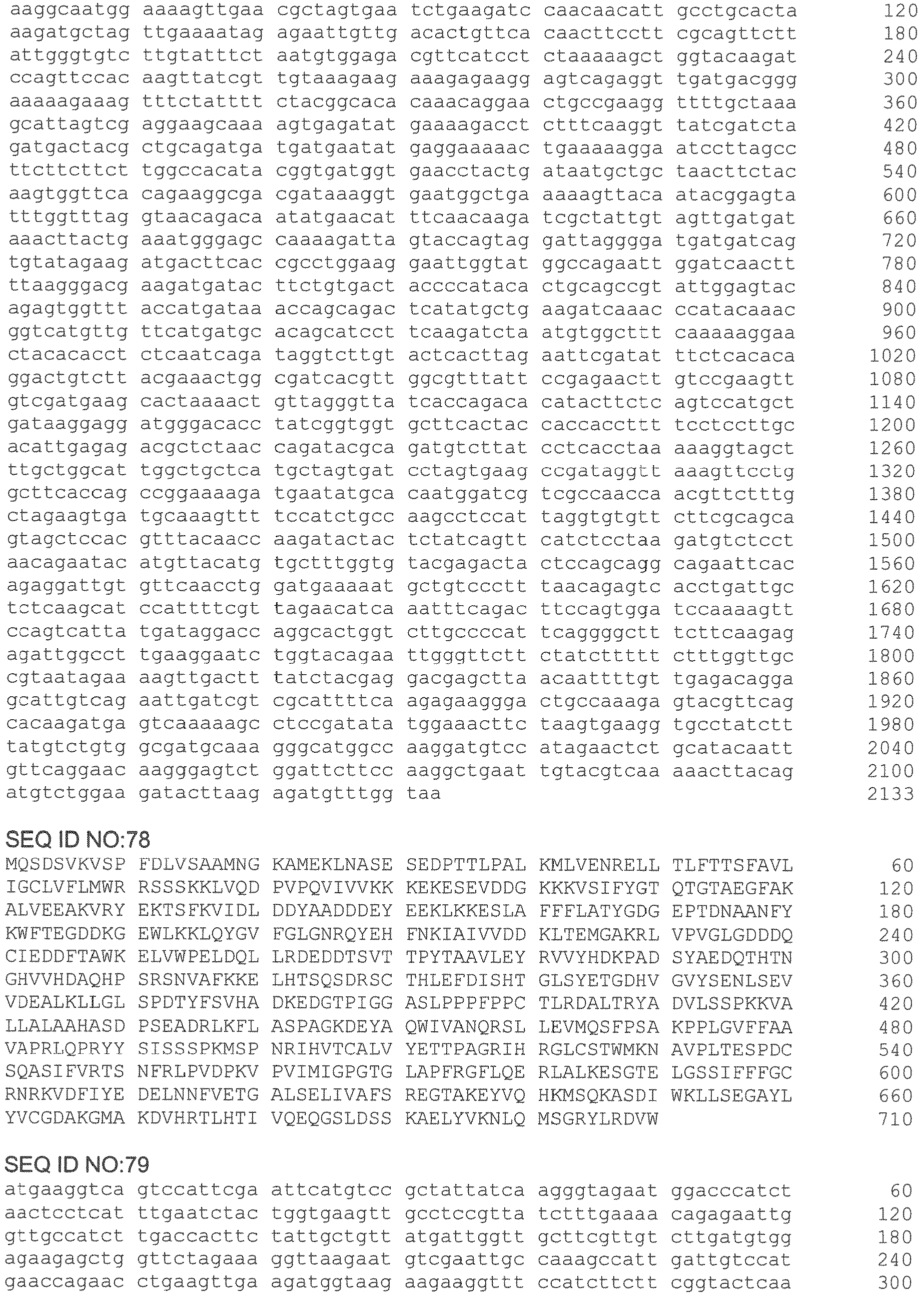

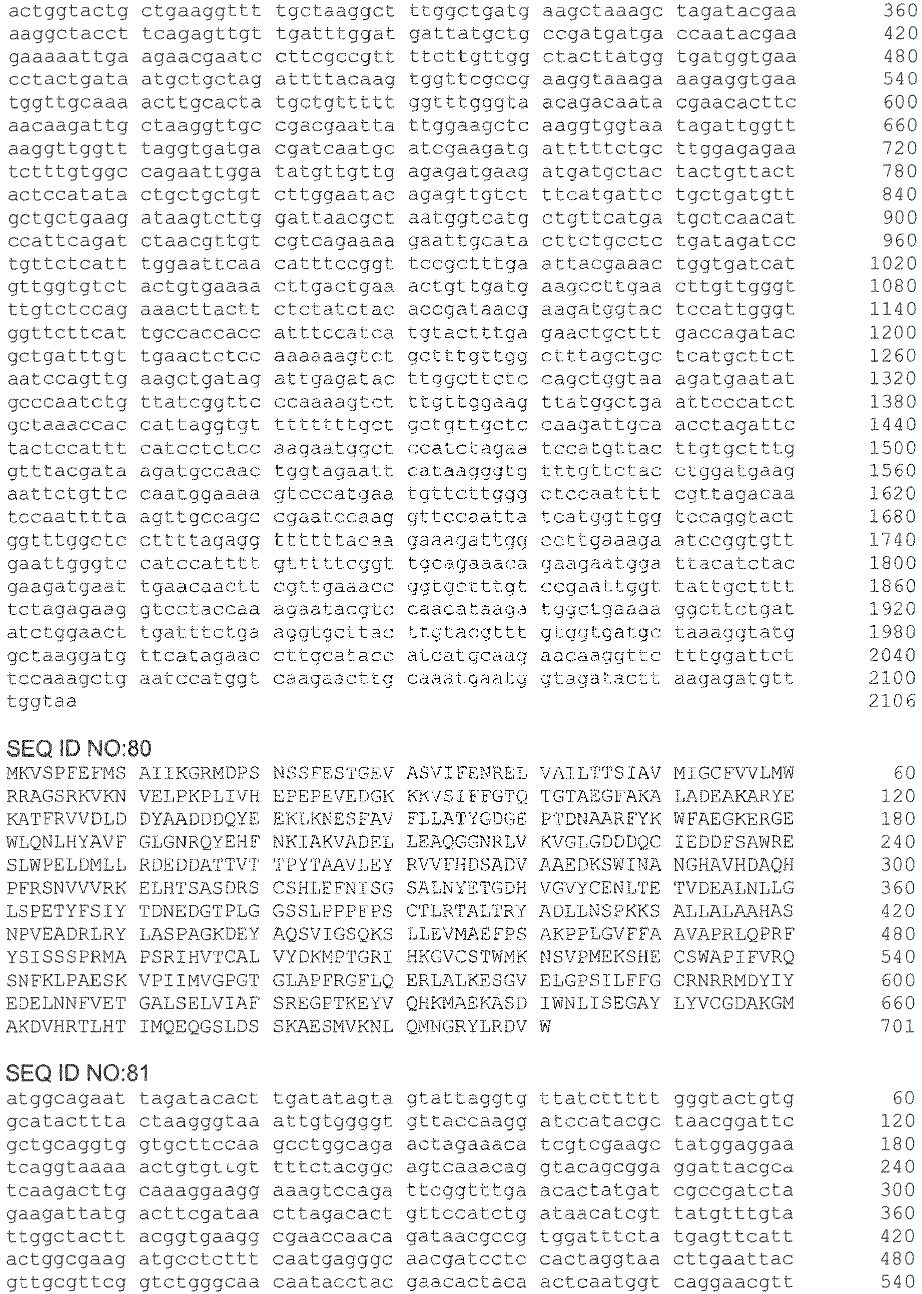

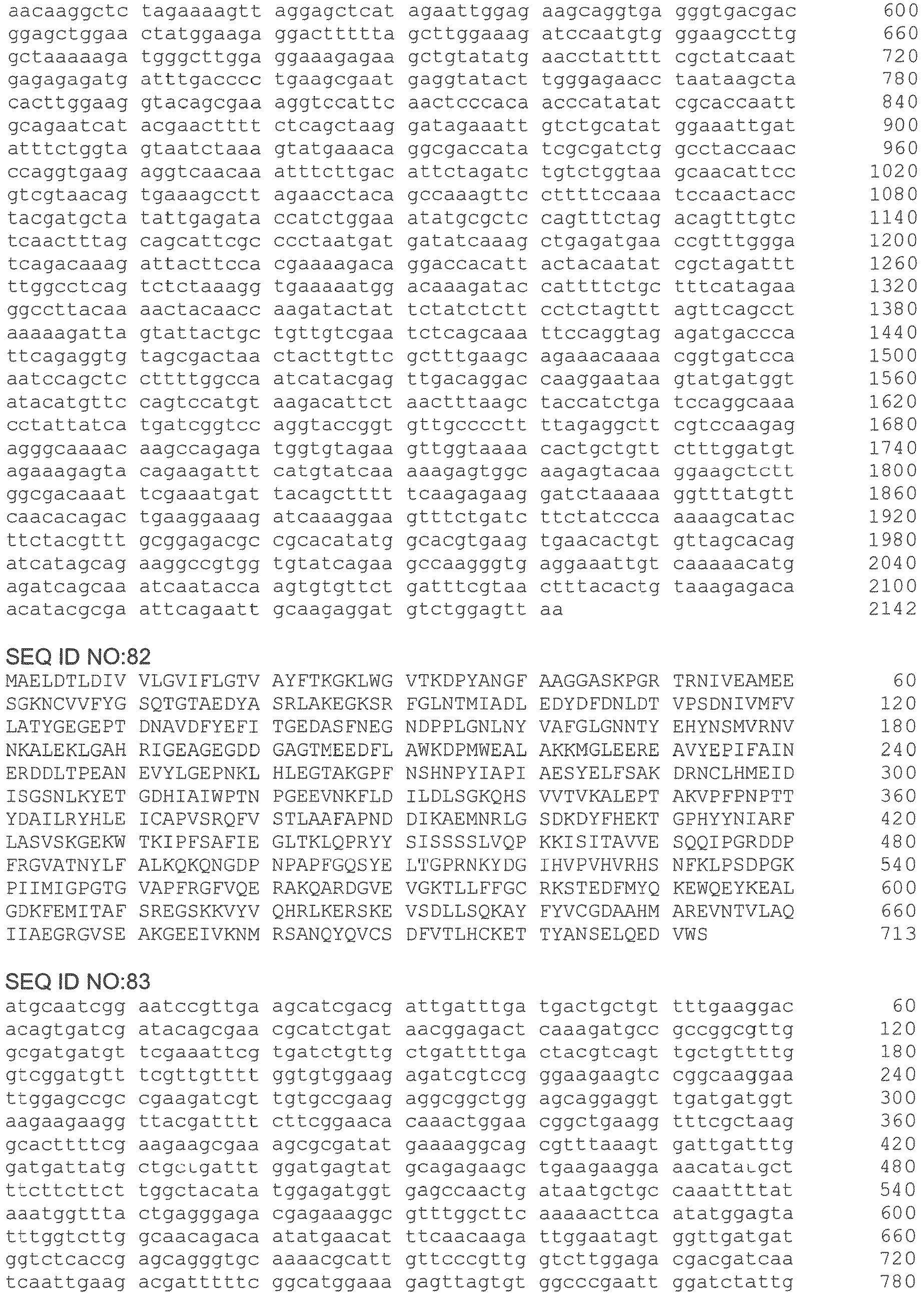

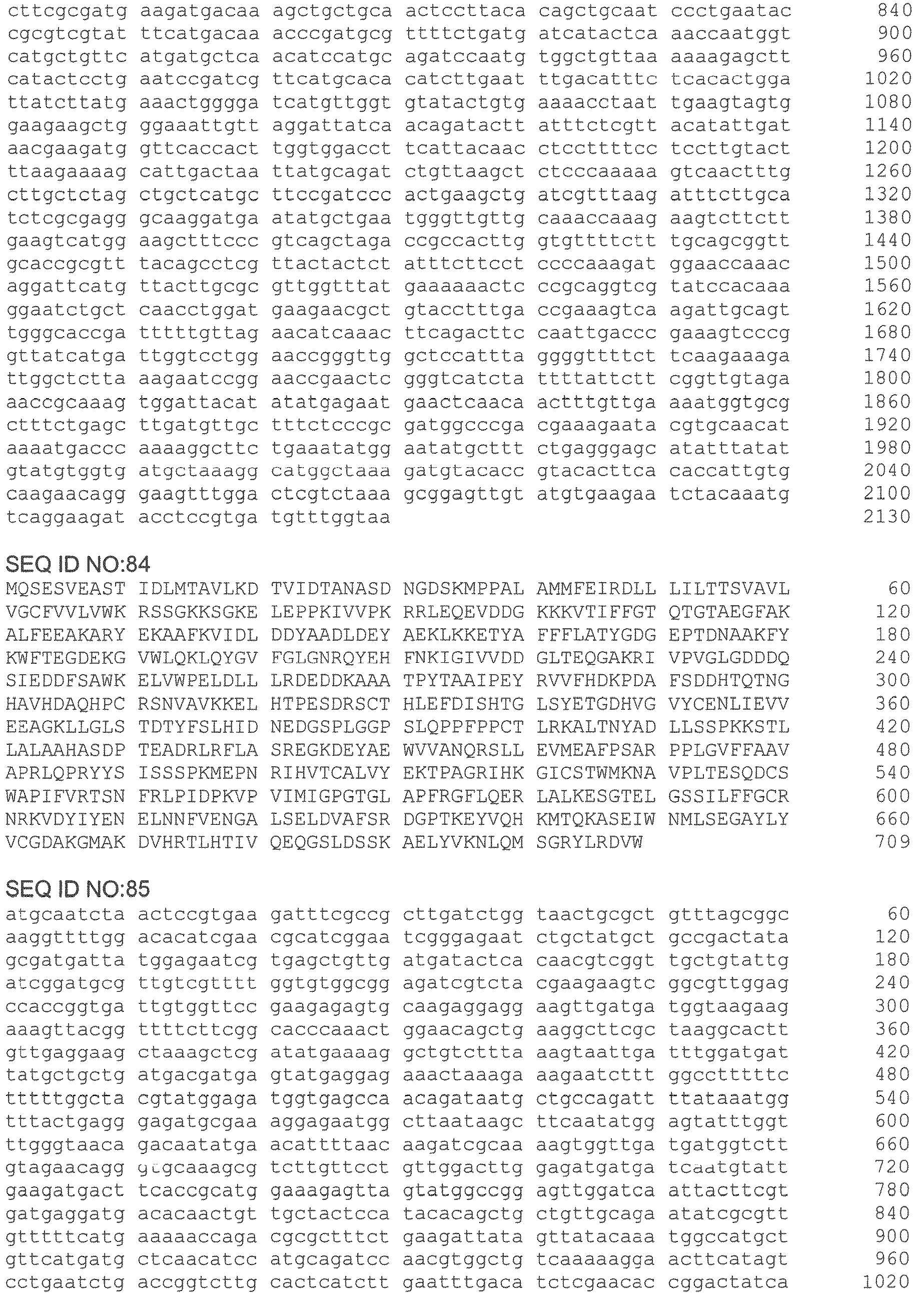

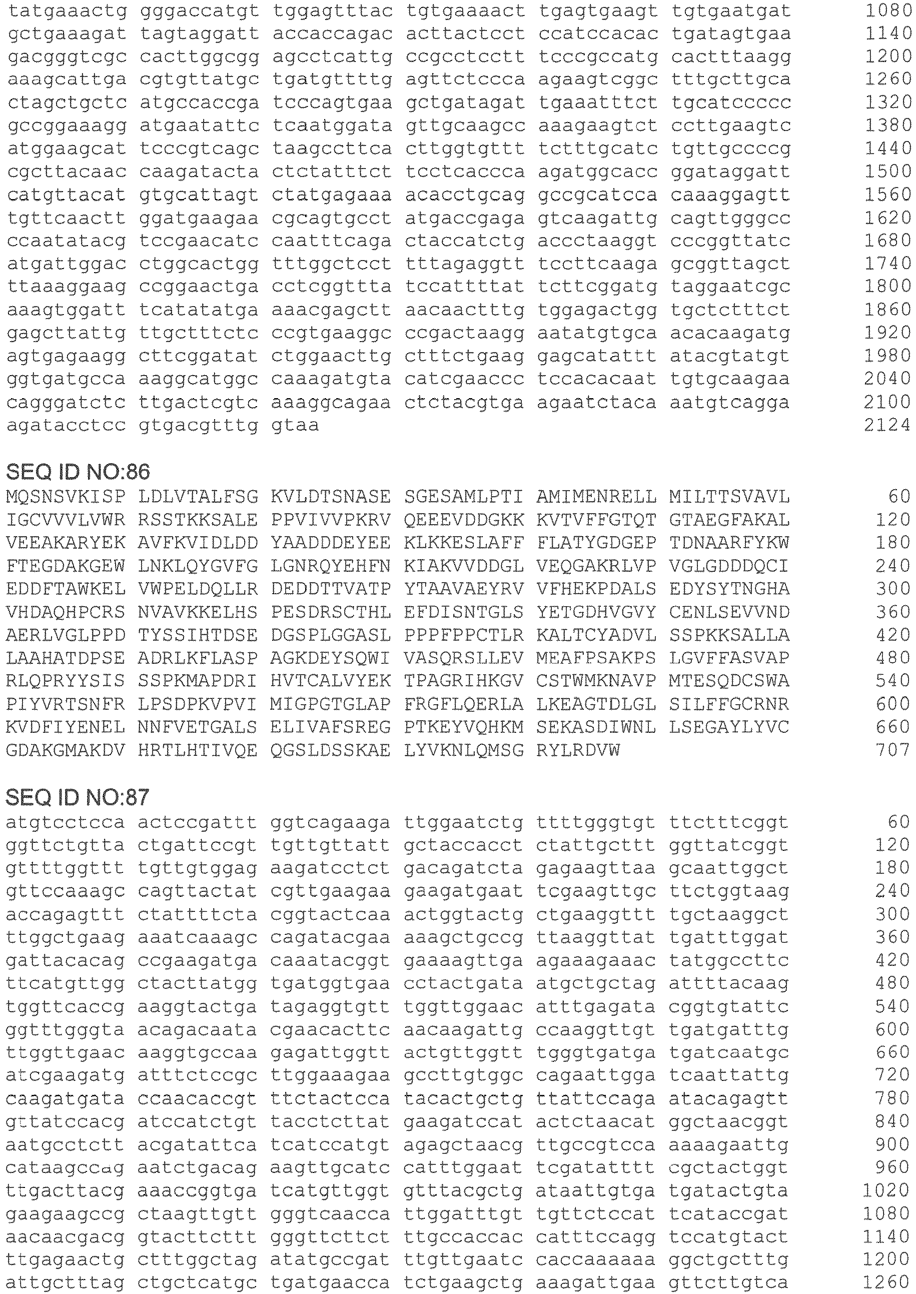

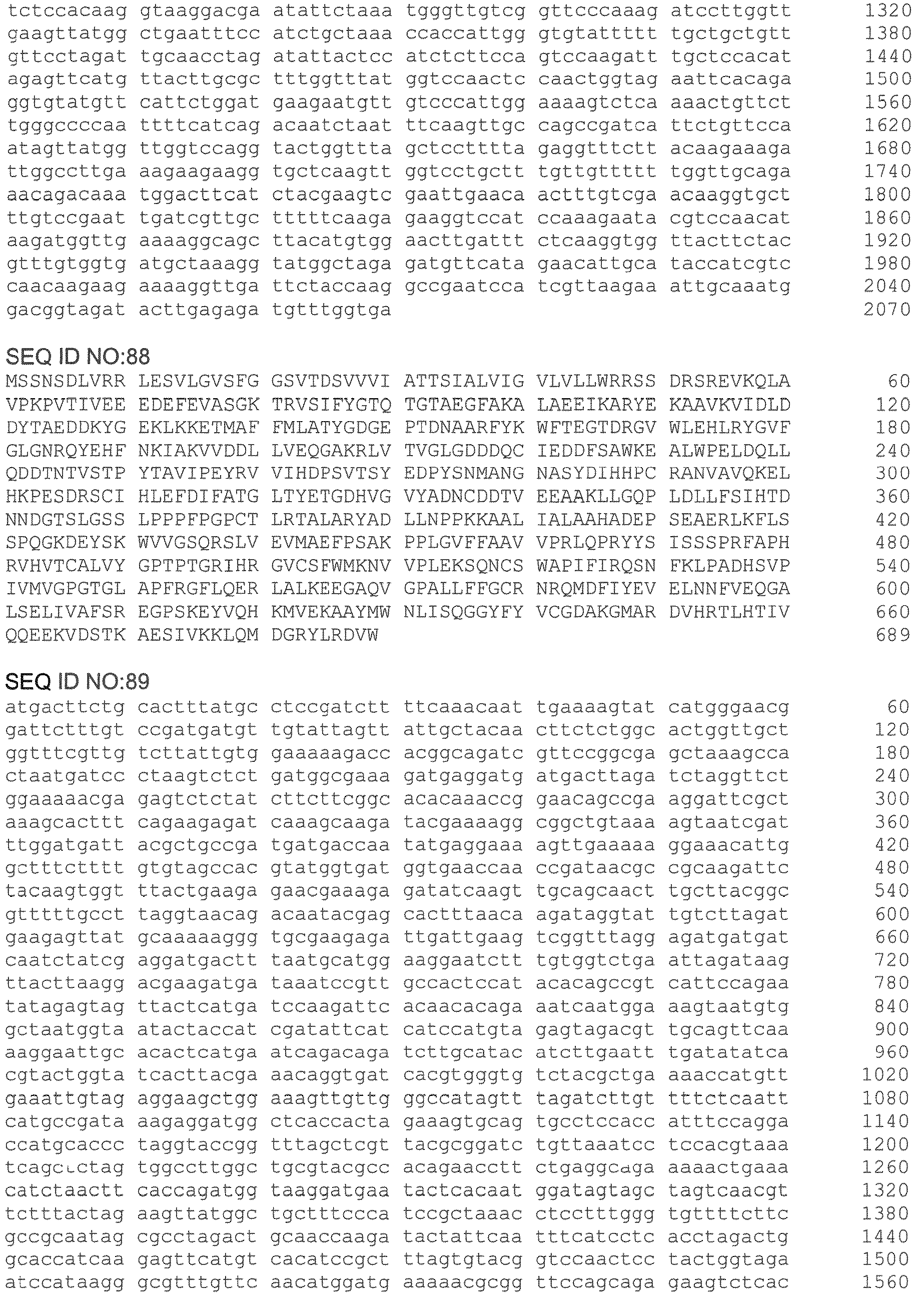

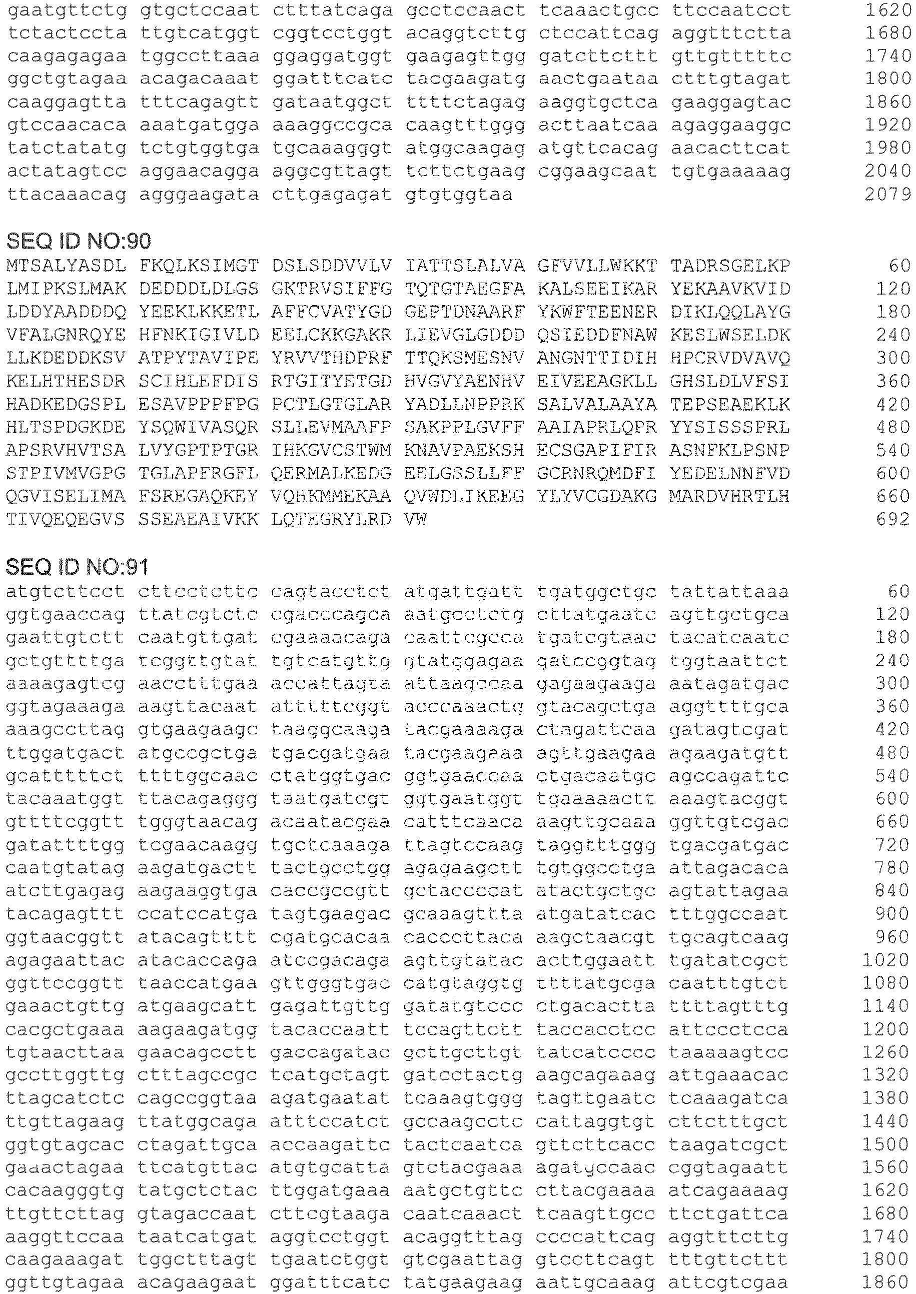

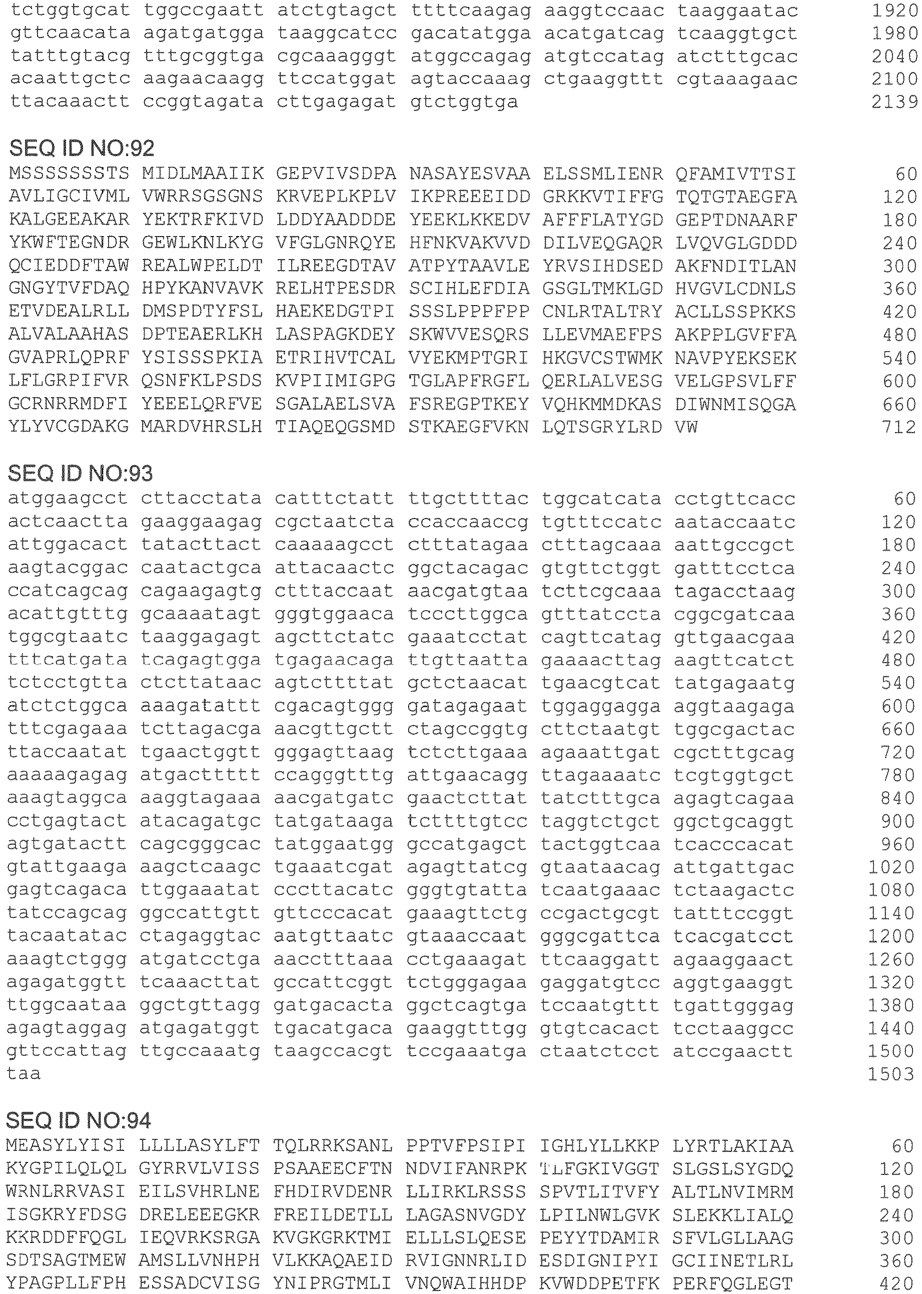

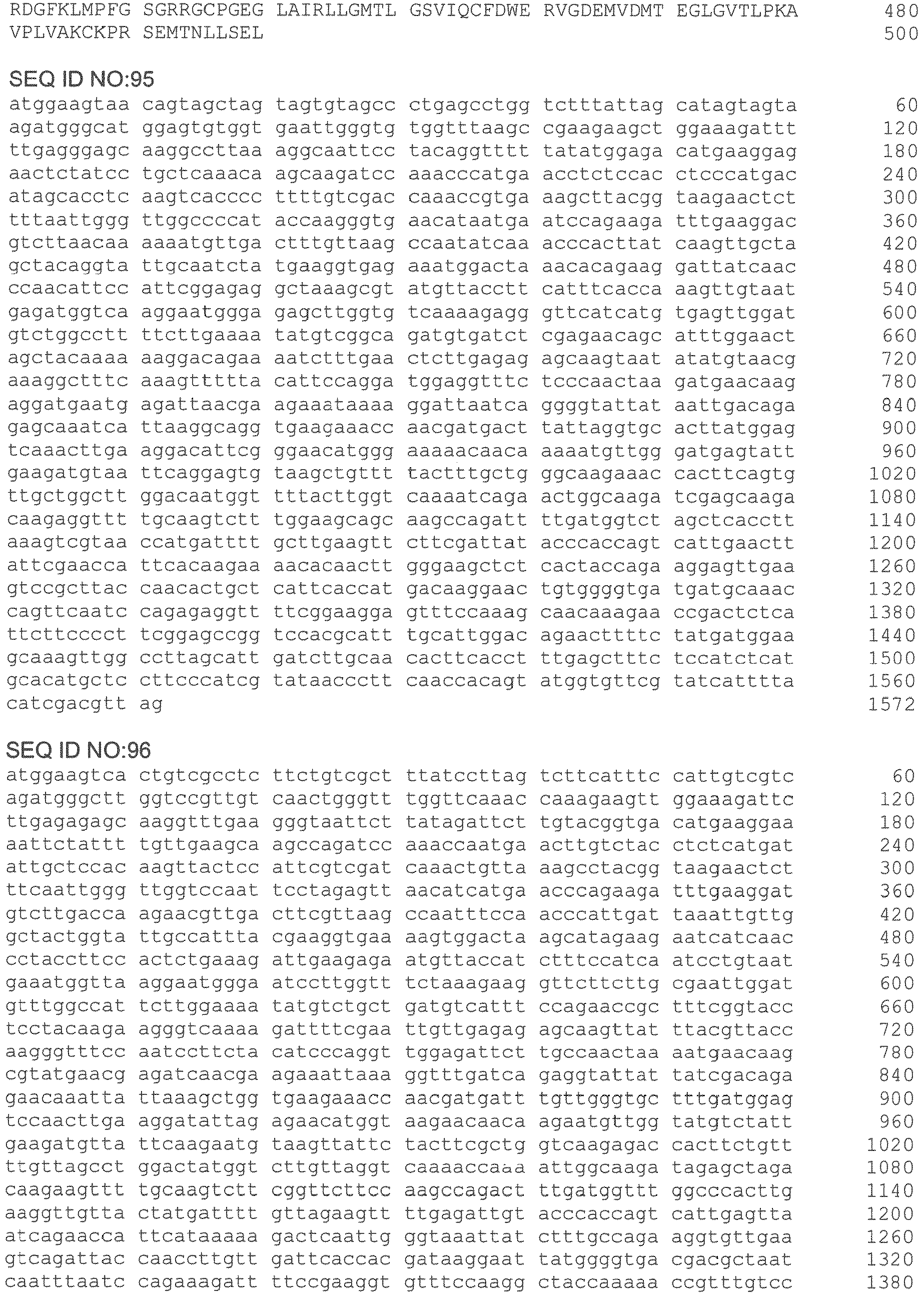

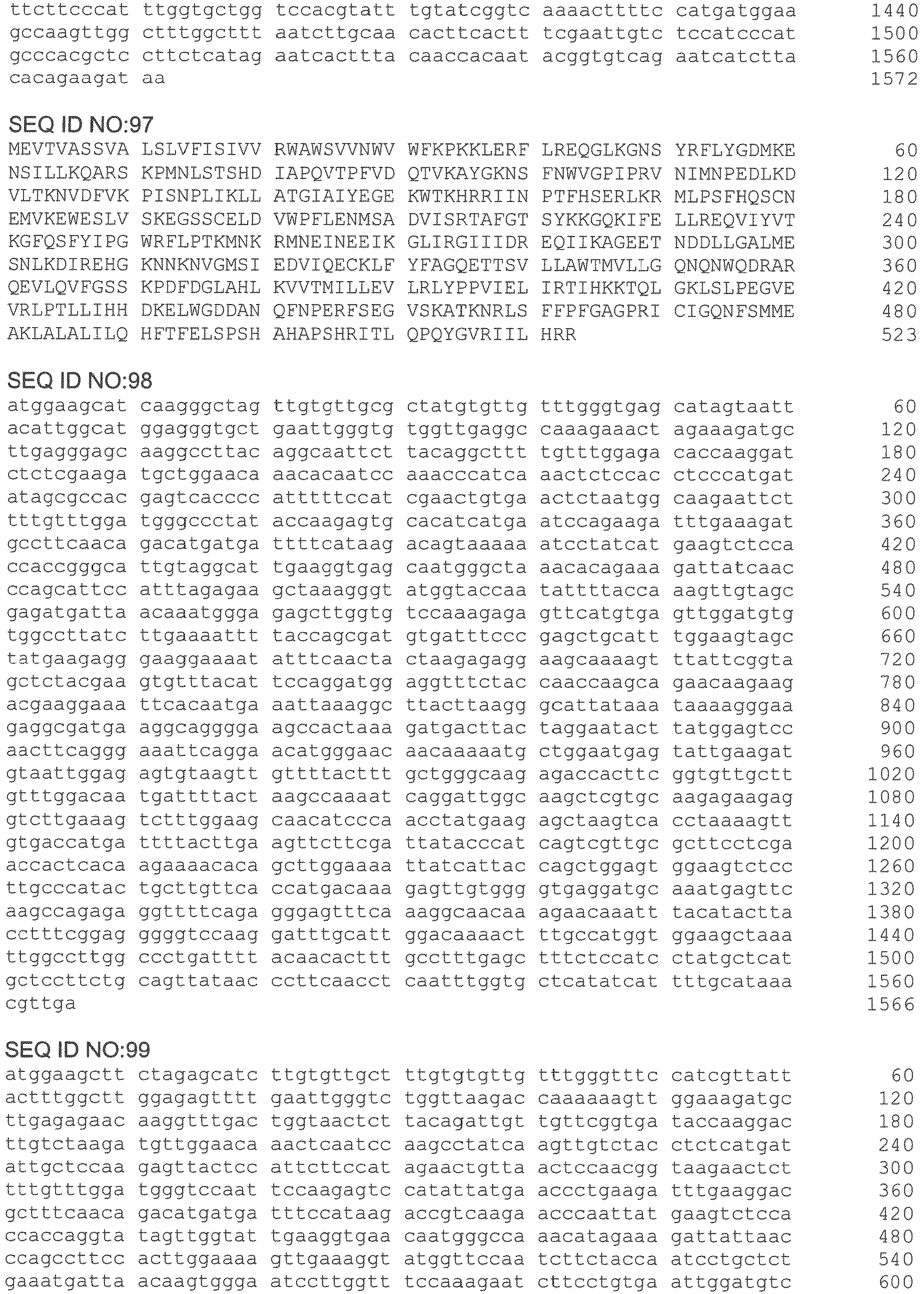

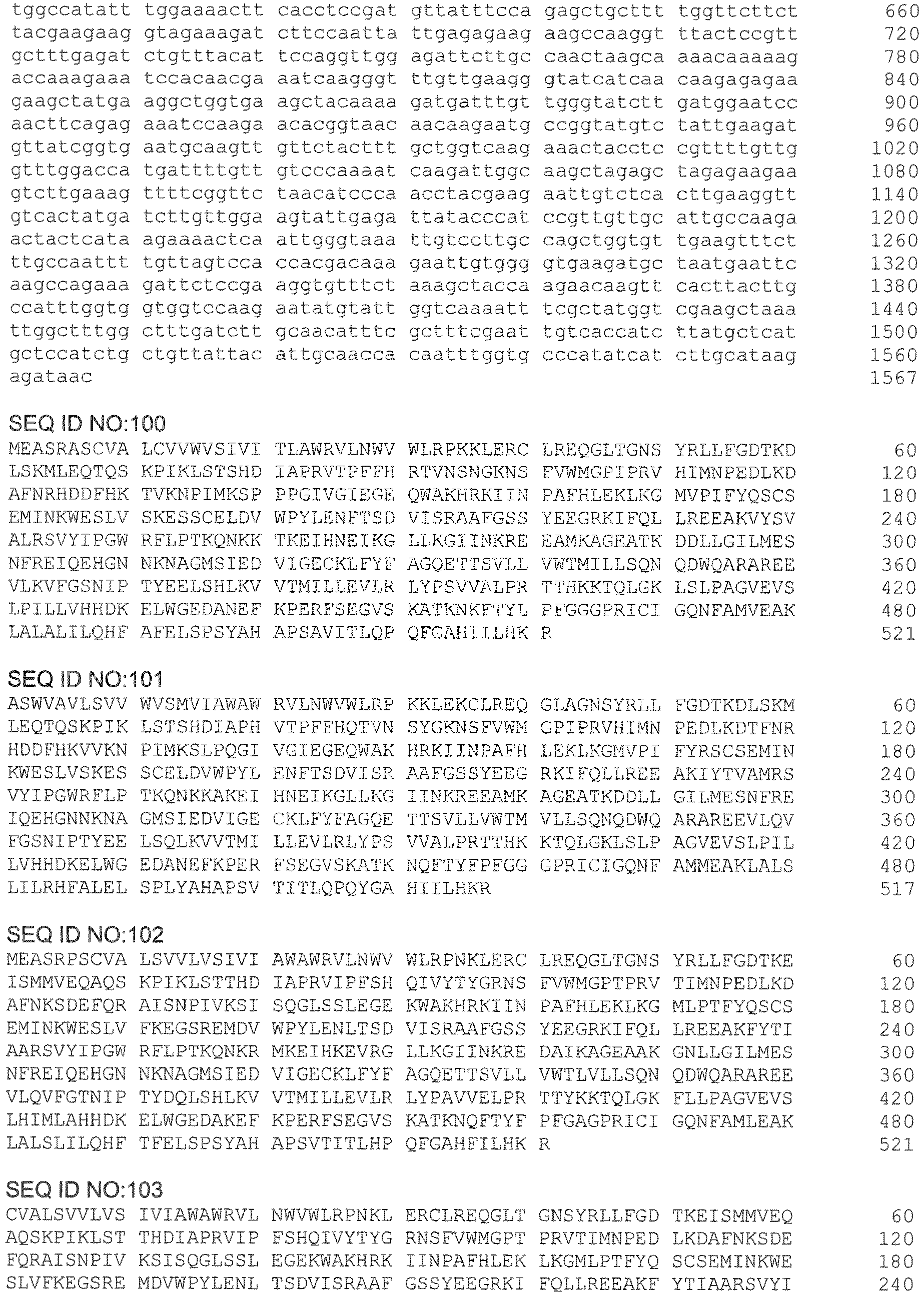

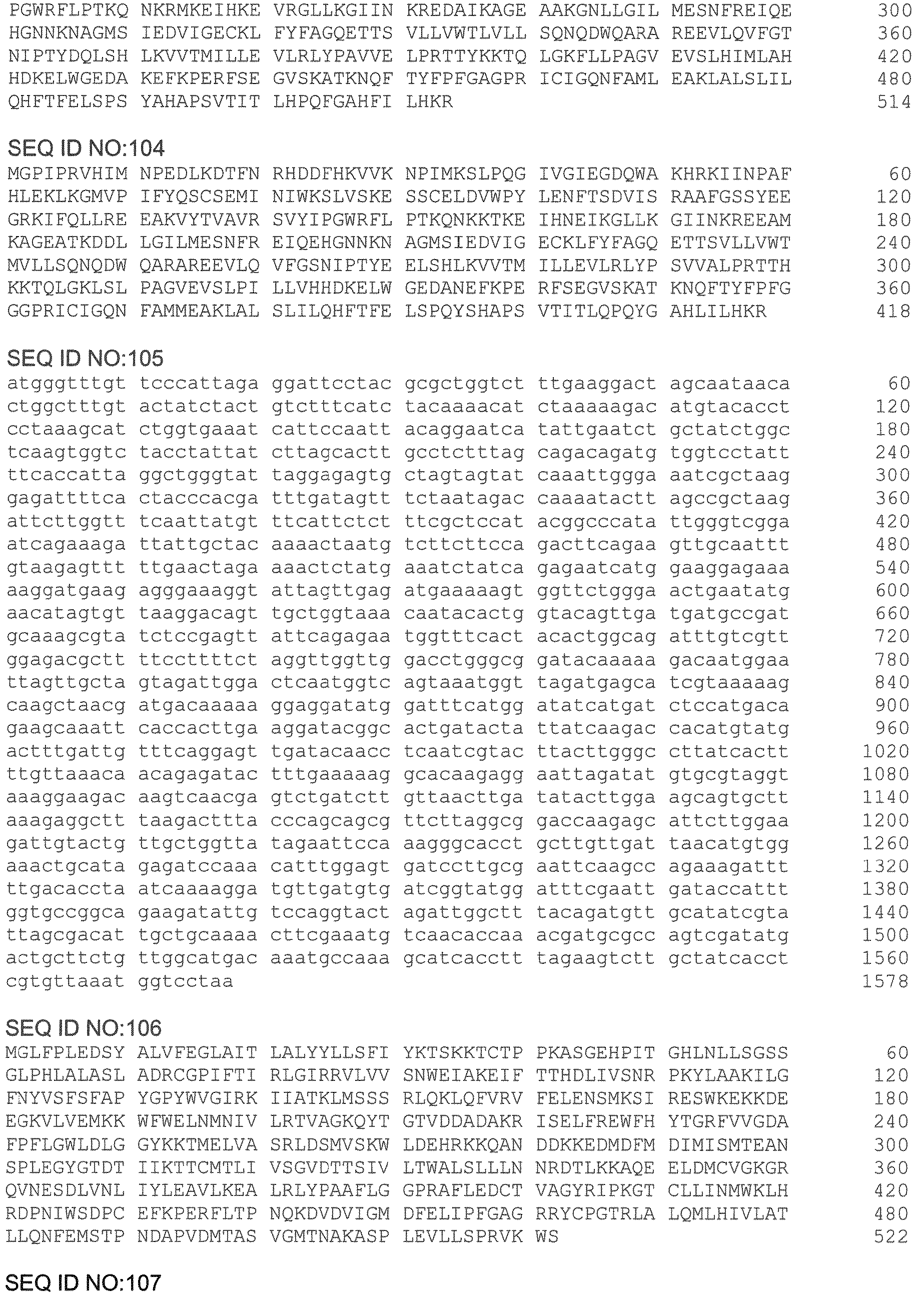

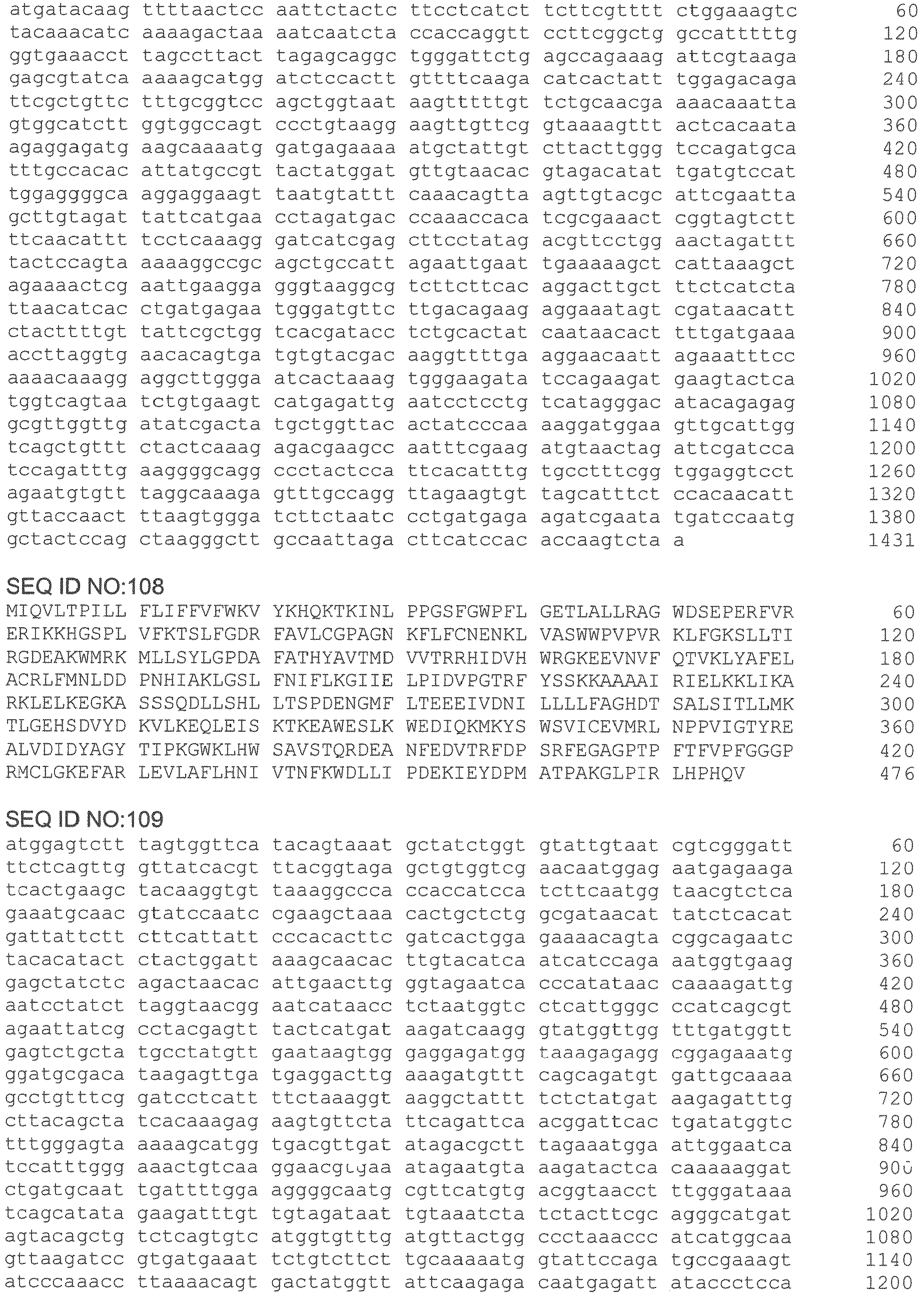

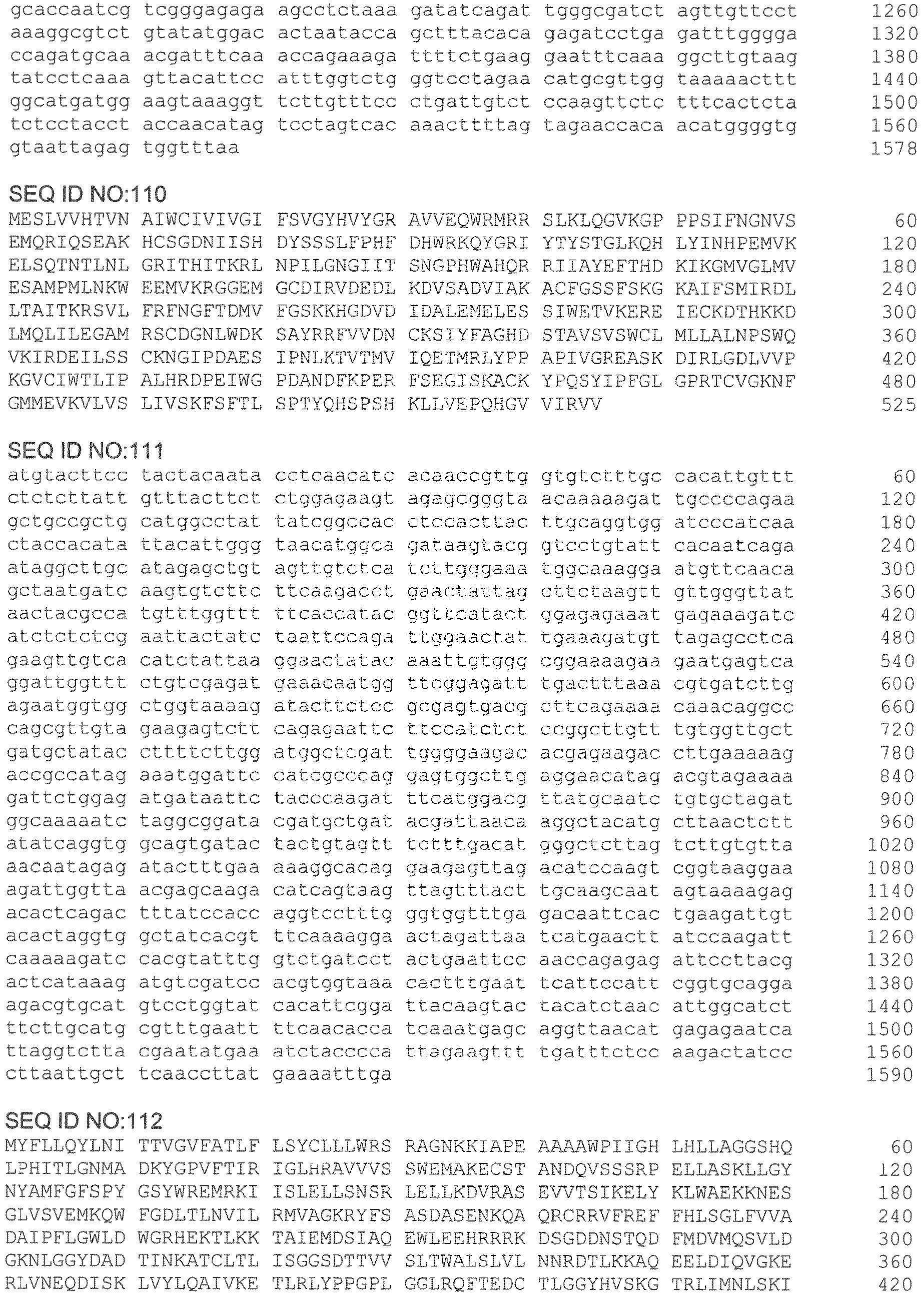

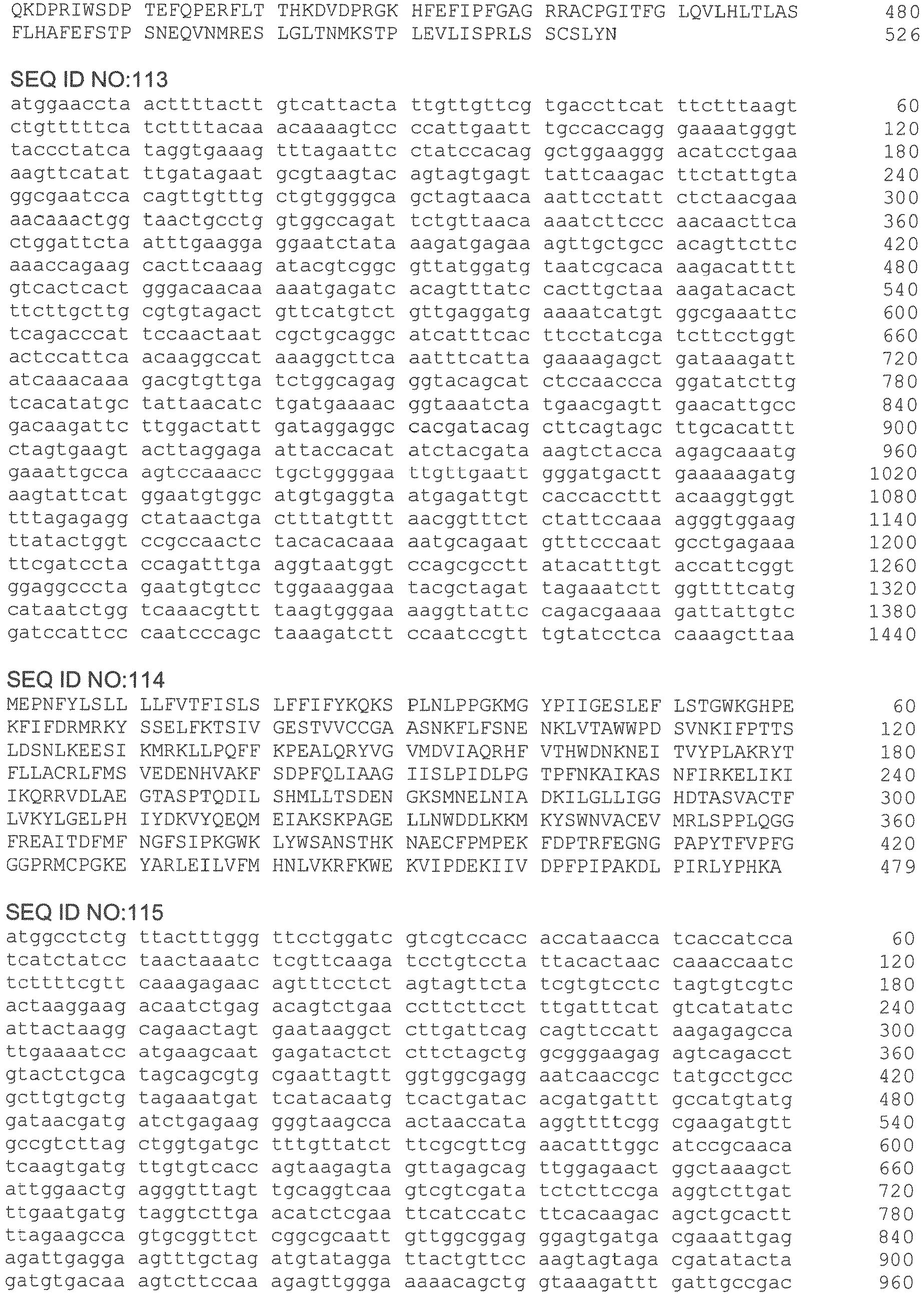

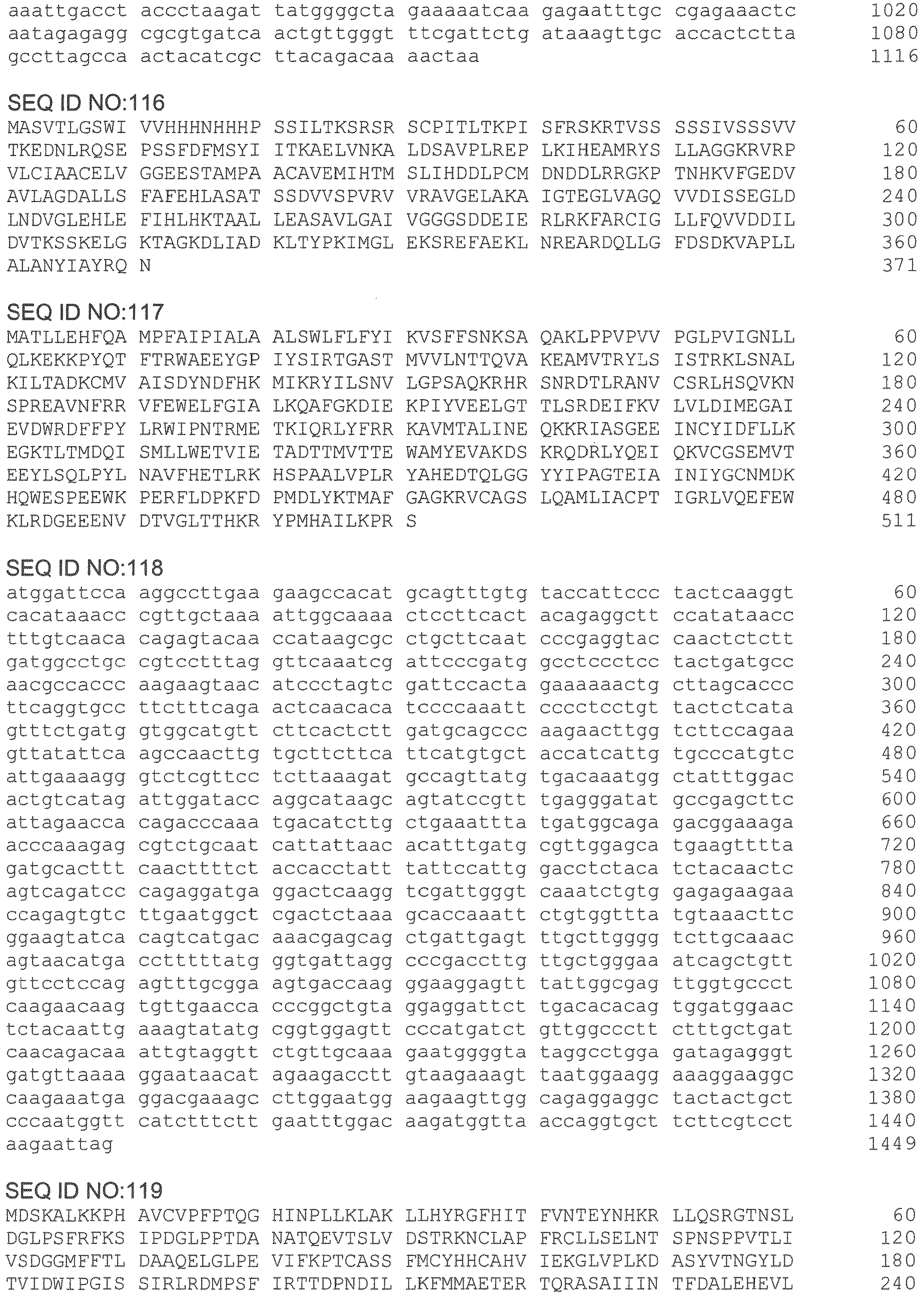

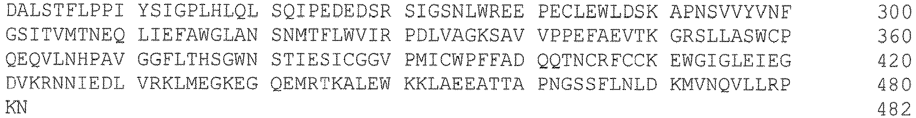

## Claims

1. A recombinant host cell, comprising a recombinant gene encoding a polypeptide catalyzing glycosylation of steviol, steviol-1,2-bioside, steviol-1,3-bioside, steviol-13-*O*-glucoside (13-SMG), and/or Rebaudioside B (RebB) at its C-19 carboxyl group and having at least 80% sequence identity to the amino acid sequence set forth in SEQ ID NO:2 or 119, wherein the recombinant host cell is producing a steviol glycoside or a steviol glycoside composition.

2. The recombinant host cell of claim 1, further comprising one or more of:
(a) a gene encoding a polypeptide synthesizing geranylgeranyl pyrophosphate (GGPP) from farnesyl diphosphate (FPP) and isopentenyl diphosphate (IPP);
wherein the polypeptide synthesizing GGPP comprises a polypeptide having at least 70% sequence identity to the amino acid sequence set forth in any one of SEQ ID NOs:20, 22, 24, 26, 28, 30, 32, or 116;
(b) a gene encoding a polypeptide synthesizing *ent*-copalyl diphosphate from GGPP;
wherein the polypeptide synthesizing *ent*-copalyl diphosphate comprises a polypeptide having at least 70% sequence identity to the amino acid sequence set forth in any one of SEQ ID NOs:34, 36, 38, 40, or 42;
(c) a gene encoding an a polypeptide synthesizing *ent*-kaurene from *ent-*copalyl pyrophosphate;
wherein the polypeptide synthesizing *ent*-kaurene comprises a polypeptide having at least 70% sequence identity to the amino acid sequence set forth in any one of SEQ ID NOs:44, 46, 48, 50, or 52;
(d) a gene encoding a polypeptide synthesizing *ent*-kaurenoic acid from *ent-*kaurene;
wherein the polypeptide synthesizing *ent*-kaurenoic acid comprises a polypeptide having at least 70% sequence identity to the amino acid sequence set forth in any one of SEQ ID NOs:60, 62, 66, 68, 70, 72, 74, 76, or 117;
(e) a gene encoding a polypeptide reducing cytochrome P450 complex;
wherein the polypeptide reducing cytochrome P450 complex comprises a polypeptide having at least 70% sequence identity to the amino acid sequence set forth in any one of SEQ ID NOs:78, 80, 82, 84, 86, 88, 90, or 92;
(f) a gene encoding a polypeptide synthesizing steviol from *ent*-kaurenoic acid;
wherein the polypeptide catalyzing synthesis of steviol comprises a polypeptide having at least 70% sequence identity to the amino acid sequence set forth in any one of SEQ ID NOs:94, 97, 100-104, 106, 108, 110, 112, or 114;
(g) a gene encoding a polypeptide catalyzing glycosylation of steviol or a steviol glycoside at its C-13 hydroxyl group;
wherein the polypeptide catalyzing glycosylation of the steviol or the steviol glycoside at its C-13 hydroxyl group thereof comprises a polypeptide having at least 55% sequence identity to the amino acid sequence set forth in SEQ ID NO:7;
(h) a gene encoding a polypeptide beta 1,3 glycosylating the C3' of the 13-0-glucose, 19-*O*-glucose, or both 13-*O*-glucose and 19-*O*-glucose of a steviol glycoside;
wherein the polypeptide beta 1,3 glycosylating the C3' of the 13-*O-*glucose, 19-*O*-glucose, or both 13-*O*-glucose and 19-*O*-glucose of the steviol glycoside comprises a polypeptide having at least 50% sequence identity to the amino acid sequence set forth in SEQ ID NO:9;
(i) a gene encoding a second polypeptide catalyzing glycosylation of steviol or a steviol glycoside at its C-19 carboxyl group;
wherein the second polypeptide catalyzing glycosylation of the steviol or the steviol glycoside at its C-19 carboxyl group thereof comprises a polypeptide having at least 55% sequence identity to the amino acid sequence set forth in SEQ ID NO:4;
(j) a gene encoding a polypeptide beta 1,2 glycosylating the C2' of the 13-*O-*glucose, 19-*O*-glucose, or both 13-*O*-glucose and 19-*O*-glucose of a steviol glycoside;
wherein the polypeptide beta 1,2 glycosylating the C2' of the 13-0-glucose, 19-*O*-glucose, or both 13-*O*-glucose and 19-*O*-glucose of the steviol glycoside comprises a polypeptide having at least 50% sequence identity to the amino acid sequence set forth in SEQ ID NO:11 or 13; or a polypeptide having at least 65% sequence identity to the amino acid sequence set forth in SEQ ID NO:16; and/or
(k) a gene encoding a bifunctional polypeptide synthesizing *ent*-copalyl diphosphate from GGPP and synthesizing *ent*-kaurene from *ent*-copalyl pyrophosphate;
wherein the bifunctional polypeptide synthesizing *ent*-copalyl diphosphate from GGPP and synthesizing *ent*-kaurene from *ent*-copalyl pyrophosphate comprises a polypeptide having at least 50% sequence identity to the amino acid sequence set forth in any one of SEQ ID NOs:54, 56, or 58;
wherein at least one of the genes is a recombinant gene.

3. The recombinant host cell of claim 1 or 2, wherein the recombinant host cell comprises a plant cell, a fungal cell, an algal cell, an archeal cell, or a bacterial cell;
wherein the bacterial cell comprises *Escherichia* cells, *Lactobacillus* cells, *Lactococcus* cells, *Cornebacterium* cells, *Acetobacter* cells, *Acinetobacter* cells, or *Pseudomonas* cells;
wherein the fungal cell comprises a yeast cell;
wherein the yeast cell is a cell from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous,* or *Candida albicans* species; or
wherein the yeast cell is a *Saccharomycete;* or
wherein the yeast cell is a cell from the *Saccharomyces cerevisiae* species.

4. A method of producing a steviol glycoside or a steviol glycoside composition, comprising growing the recombinant host cell of any one of claims 1-3 in a cell culture medium, under conditions in which the genes are expressed, wherein the steviol glycoside or the steviol glycoside composition is produced by the recombinant host cell.

5. A method for producing a steviol glycoside or a steviol glycoside composition, comprising whole-cell bioconversion of a plant-derived or synthetic steviol and/or steviol glycosides in a cell culture medium using the recombinant polypeptide catalyzing glycosylation of steviol, steviol-1,2-bioside, steviol-1,3-bioside, 13-SMG, and/or RebB at its C-19 carboxyl group and having at least 80% sequence identity to the amino acid sequence set forth in SEQ ID NO:2 or 119 produced by the recombinant host cell of any one of claims 1-3, and synthesizing the steviol glycoside or the steviol glycoside composition thereby.

6. The method of claim 4 or 5, that further comprises isolating the steviol glycoside or the steviol glycoside composition;
wherein the isolating step comprises:
(a) providing the cell culture medium comprising the steviol glycoside or the steviol glycoside composition;
(b) separating a liquid phase of the cell culture medium from a solid phase of the cell culture to obtain a supernatant comprising the steviol glycoside or the steviol glycoside composition; and further comprising:
(c) providing one or more adsorbent resins, comprising providing the adsorbent resins in a packed column; and
(d) contacting the supernatant of step (b) with the one or more adsorbent resins in order to obtain at least a portion of the steviol glycoside or the steviol glycoside composition, thereby isolating the steviol glycoside or the steviol glycoside composition; or
(e) providing one or more ion exchange or ion exchange or reversed-phase chromatography columns; and
(f) contacting the supernatant of step (b) with the one or more ion exchange or ion exchange or reversed-phase chromatography columns in order to obtain at least a portion of the steviol glycoside or the steviol glycoside composition; or
(g) crystallizing or extracting the steviol glycoside, thereby isolating the steviol glycoside or the steviol glycoside composition.

7. The method of claim 6, that further comprises recovering the steviol glycoside alone or as a composition comprising the steviol glycoside;
wherein the recovered composition is enriched for the steviol glycoside, relative to a glycoside composition from a Stevia plant and has a reduced level of Stevia plant-derived components relative to a plant-derived Stevia extract; and
wherein the recovered composition has a reduced level of Stevia plant-derived components relative to a plant-derived Stevia extract.

8. The method of any one of claims 4-7, wherein the cell culture medium comprises:
(a) the steviol glycoside or the steviol glycoside composition produced by the recombinant host cell of any one of claims 1-3 or whole-cell bioconversion of a plant-derived or synthetic steviol and/or steviol glycosides,
(b) glucose, fructose, and/or sucrose; and
(c) supplemental nutrients comprising trace metals, vitamins, salts, yeast nitrogen base (YNB), and/or amino acids.

9. The method of any one of claims 4-8, wherein the steviol glycoside or the steviol glycoside composition is produced in a permeabilized recombinant host cell which has been transformed with the gene encoding the polypeptide catalyzing glycosylation of steviol, steviol-1,2-bioside, steviol-1,3-bioside, 13-SMG, and/or RebB at its C-19 carboxyl group and having at least 80% sequence identity to the amino acid sequence set forth in SEQ ID NO:2 or 119.

10. An *in vitro* method for producing steviol-19-*O*-glucoside (19-SMG), comprising adding a recombinant polypeptide catalyzing glycosylation of steviol at its C-19 carboxyl group and having at least 80% sequence identity to the amino acid sequence set forth in SEQ ID NO:2 or 119 and a plant-derived or synthetic steviol to a reaction mixture; and synthesizing 19-SMG thereby.

11. The method of claim 10, wherein the reaction mixture comprises:
(a) 19-SMG;
(b) the recombinant polypeptide;
(c) glucose, fructose, and/or sucrose, uridine diphosphate (UDP)-glucose, UDP-rhamnose, UDP-xylose, and/or N-acetyl-glucosamine; and
(d) reaction buffer and/or salts.

12. The method of any one of claims 4-9, wherein the steviol glycoside is or the steviol glycoside composition comprises 13-SMG, steviol-1,2-bioside, steviol-1,3-bioside, 19-SMG, stevioside, 1,3-stevioside, rubusoside, Rebaudioside A (RebA), RebB, Rebaudioside C (RebC), Rebaudioside D (RebD), Rebaudioside E (RebE), Rebaudioside F (RebF), Rebaudioside M (RebM), Rebaudioside Q (RebQ), Rebaudioside I (Rebl), dulcoside A, or isomers thereof.

13. A cell culture medium, comprising:
(a) the recombinant host cell of any one of claims 1-3; and
(b) one or more steviol glycosides produced by the recombinant host cell of any one of claims 1-3,
wherein one or more steviol glycosides is present at a concentration of at least 0.1 mg/liter of the cell culture medium and the cell culture medium further comprises glucose, sucrose, UDP-glucose, UDP-rhamnose, UDP-xylose, N-acetyl-glucosamine, and/or YNB; and
wherein the cell culture medium is enriched for 19-SMG relative to a steviol glycoside extract from Stevia plant and has a reduced level of Stevia plant-derived components relative to a plant-derived Stevia extract.

14. A method for transferring a sugar moiety to a C-19 carboxyl group of steviol or a steviol glycoside, comprising contacting steviol or the steviol glycoside with a recombinant polypeptide catalyzing glycosylation of the steviol or the steviol glycoside at its C-19 carboxyl group and having at least 80% sequence identity to the amino acid sequence set forth in SEQ ID NO:2 or 119 and a UDP-sugar under suitable conditions for the transfer of the sugar moiety to the steviol glycoside;
wherein the steviol glycoside comprises 13-SMG, steviol-1,2-bioside, steviol-1,3-bioside, and/or RebB; and
wherein 19-SMG, rubusoside, stevioside, 1,3-stevioside (RebG), RebA and/or a steviol glycoside composition thereof is produced upon transfer of the sugar moiety.

## Patentansprüche

1. Rekombinante Wirtszelle, die ein rekombinantes Gen umfasst, das ein Polypeptid kodiert, das die Glycosylierung von Steviol, Steviol-1,2-biosid, Steviol-1,3-biosid, Steviol-13-*O*-glycosid (13-SMG) und/oder Rebaudiosid B (RebB) an dessen C-19-Carboxylgruppe katalysiert und mindestens 80 % Sequenzidentität mit der Aminosäuresequenz aufweist, die in SEQ.-ID Nr. 2 oder 119 dargelegt ist, wobei die rekombinante Wirtszelle ein Steviolglycosid oder eine Steviolglycosidzusammensetzung produziert.

2. Rekombinante Wirtszelle nach Anspruch 1, die weiter eines oder mehrere von Folgenden umfasst:
(a) ein Gen, das ein Polypeptid kodiert, das Geranylgeranylpyrophosphat (GGPP) aus Farnesyldiphosphat (FPP) und Isopentenyldiphosphat (IPP) synthetisiert;
wobei das Polypeptid, das GGPP synthetisiert, ein Polypeptid umfasst, das mindestens 70 % Sequenzidentität mit der Aminosäuresequenz aufweist, die in einer der SEQ.-ID Nr. 20, 22, 24, 26, 28, 30, 32 oder 116 dargelegt ist;
(b) ein Gen, das ein Polypeptid kodiert, das *ent*-Copalyldiphosphat aus GGPP synthetisiert;
wobei das Polypeptid, das *ent*-Copalyldiphosphat synthetisiert, ein Polypeptid umfasst, das mindestens 70 % Sequenzidentität mit der Aminosäuresequenz aufweist, die in einer der SEQ.-ID Nr. 34, 36, 38, 40 oder 42 dargelegt ist;
(c) ein Gen, das ein Polypeptid kodiert, das *ent*-Kauren aus *ent-*Copalylpyrophosphat synthetisiert;
wobei das Polypeptid, das *ent*-Kauren synthetisiert, ein Polypeptid umfasst, das mindestens 70 % Sequenzidentität mit der Aminosäuresequenz aufweist, die in einer der SEQ.-ID Nr. 44, 46, 48, 50 oder 52 dargelegt ist;
(d) ein Gen, das ein Polypeptid kodiert, das *ent*-Kaurensäure aus *ent*-Kauren synthetisiert;
wobei das Polypeptid, das *ent*-Kaurensäure synthetisiert, ein Polypeptid umfasst, das mindestens 70 % Sequenzidentität mit der Aminosäuresequenz aufweist, die in einer der SEQ.-ID Nr. 60, 62, 66, 68, 70, 72, 74, 76 oder 117 dargelegt ist;
(e) ein Gen, das ein Polypeptid kodiert, das einen Cytochrom-P450-Komplex reduziert;
wobei das Polypeptid, das einen Cytochrom-P450-Komplex reduziert, ein Polypeptid umfasst, das mindestens 70 % Sequenzidentität mit der Aminosäuresequenz aufweist, die in einer der SEQ.-ID Nr. 78, 80, 82, 84, 86, 88, 90 oder 92 dargelegt ist;
(f) ein Gen, das ein Polypeptid kodiert, das Steviol aus *ent*-Kaurensäure synthetisiert;
wobei das Polypeptid, das die Synthese von Steviol katalysiert, ein Polypeptid umfasst, das mindestens 70 % Sequenzidentität mit der Aminosäuresequenz aufweist, die in einer der SEQ.-ID Nr. 94, 97, 100-104, 106, 108, 110, 112 oder 114 dargelegt ist;
(g) ein Gen, das ein Polypeptid kodiert, das die Glycosylierung von Steviol oder eines Steviolglycosids an dessen C-13-Hydroxylgruppe katalysiert;
wobei das Polypeptid, das die Glycosylierung des Steviols oder des Steviolglycosids an dessen C-13-Hydroxylgruppe davon katalysiert, ein Polypeptid umfasst, das mindestens 55 % Sequenzidentität mit der Aminosäuresequenz aufweist, die in SEQ.-ID Nr. 7 dargelegt ist;
(h) ein Gen, das ein Polypeptid kodiert, das die beta-1,3-Glycosylierung des C3' der 13-*O*-Glucose, 19-*O*-Glucose oder von sowohl 13-*O*-Glucose als auch 19-*O*-Glucose eines Steviolglycosids bewirkt;
wobei das Polypeptid, das die beta-1,3-Glycosylierung des C3' der 13-*O*-Glucose, 19-*O*-Glucose oder von sowohl 13-*O*-Glucose als auch 19-O-Glucose des Steviolglycosids bewirkt, ein Polypeptid umfasst, das mindestens 50 % Sequenzidentität mit der Aminosäuresequenz aufweist, die in SEQ.-ID Nr. 9 dargelegt ist;
(i) ein Gen, das ein zweites Polypeptid kodiert, das die Glycosylierung von Steviol oder eines Steviolglycosids an dessen C-19-Carboxylgruppe katalysiert;
wobei das zweite Polypeptid, das die Glycosylierung des Steviols oder des Steviolglycosids an dessen C-19-Carboxylgruppe davon katalysiert, ein Polypeptid umfasst, das mindestens 55 % Sequenzidentität mit der Aminosäuresequenz aufweist, die in SEQ.-ID Nr. 4 dargelegt ist;
(j) ein Gen, das ein Polypeptid kodiert, das die beta-1,2-Glycosylierung des C2' der 13-*O*-Glucose, 19-*O*-Glucose oder von sowohl 13-*O*-Glucose als auch 19-*O*-Glucose eines Steviolglycosids bewirkt;
wobei das Polypeptid, das die beta-1,2-Glycosylierung des C2' der 13-*O*-Glucose, 19-*O*-Glucose oder von sowohl 13-*O*-Glucose als auch 19-O-Glucose des Steviolglycosids bewirkt, Folgendes umfasst: ein Polypeptid, das mindestens 50 % Sequenzidentität mit der Aminosäuresequenz aufweist, die in SEQ.-ID Nr. 11 oder 13 dargelegt ist, oder ein Polypeptid, das mindestens 65 % Sequenzidentität mit der Aminosäuresequenz aufweist, die in SEQ.-ID Nr. 16 dargelegt ist; und/oder
(k) ein Gen, das ein bifunktionelles Polypeptid kodiert, das *ent-*Copalyldiphosphat aus GGPP synthetisiert und *ent*-Kauren aus *ent-*Copalylpyrophosphat synthetisiert;
wobei das bifunktionelle Polypeptid, das *ent*-Copalyldiphosphat aus GGPP synthetisiert und *ent*-Kauren aus *ent*-Copalylpyrophosphat synthetisiert, ein Polypeptid umfasst, das mindestens 50 % Sequenzidentität mit der Aminosäuresequenz aufweist, die in einer der SEQ.-ID Nr. 54, 56 oder 58 dargelegt ist;
wobei mindestens eines der Gene ein rekombinantes Gen ist.

3. Rekombinante Wirtszelle nach Anspruch 1 oder 2, wobei die rekombinante Wirtszelle eine Pflanzenzelle, eine Pilzzelle, eine Algenzelle, eine Archaeazelle oder eine Bakterienzelle umfasst;
wobei die Bakterienzelle *Escherichia-Zellen, Lactobacillus-Zellen, Lactococcus-Zellen, Corynebacterium-Zellen, Acetobacter-Zellen, Acinetobacter-*Zellen oder *Pseudomonas-Zellen* umfasst;
wobei die Pilzzelle eine Hefezelle umfasst;
wobei die Hefezelle eine Zelle von Folgenden ist: *Saccharomyces-cerevisiae-, Schizosaccharomyces-pombe-, Yarrowia-lipolytica-, Candida-glabrata-, Ashbyagossypii-, Cyberlindnera-jadinii-, Pichia-pastoris-, Kluyveromyces-lactis-, Hansenulapolymorpha-, Candida-boidinii-, Arxula-adeninivorans-, Xanthophyllomycesdendrorhous-* oder *Candida-albicans-*Spezies; oder
wobei die Hefezelle eine *Saccharomycete* ist; oder
wobei die Hefezelle eine Zelle von der *Saccharomyces-cerevisiae-Spezies* ist.

4. Verfahren zur Produktion eines Steviolglycosids oder einer Steviolglycosidzusammensetzung, das das Züchten der rekombinanten Wirtszelle nach einem der Ansprüche 1-3 in einem Zellkulturmedium unter Bedingungen umfasst, unter denen die Gene exprimiert werden, wobei das Steviolglycosid oder die Steviolglycosidzusammensetzung durch die rekombinante Wirtszelle produziert wird.

5. Verfahren zur Produktion eines Steviolglycosids oder einer Steviolglycosidzusammensetzung, das Folgendes umfasst: Ganzzell-Biokonversion eines pflanzlichen oder synthetischen Steviols und/oder von Steviolglycosiden in einem Zellkulturmedium unter Verwendung des rekombinanten Polypeptids, das die Glycosylierung von Steviol, Steviol-1,2-biosid, Steviol-1,3-biosid, 13-SMG und/oder RebB an dessen C-19-Carboxylgruppe katalysiert und mindestens 80 % Sequenzidentität mit der Aminosäuresequenz aufweist, die in SEQ.-ID Nr. 2 oder 119 dargelegt ist, das von der rekombinanten Wirtszelle nach einem der Ansprüche 1-3 produziert wird, und Synthetisieren des Steviolglycosids oder der Steviolglycosidzusammensetzung dadurch.

6. Verfahren nach Anspruch 4 oder 5, das weiter das Isolieren des Steviolglycosids oder der Steviolglycosidzusammensetzung umfasst;
wobei der Isolierungsschritt Folgendes umfasst:
(a) Bereitstellen des Zellkulturmediums, das das Steviolglycosid oder die Steviolglycosidzusammensetzung umfasst;
(b) Abtrennen einer flüssigen Phase des Zellkulturmediums von einer festen Phase der Zellkultur, um einen Überstand zu erhalten, der das Steviolglycosid oder die Steviolglycosidzusammensetzung umfasst; und weiter Folgendes umfasst:
(c) Bereitstellen von einem oder mehreren Adsorberharzen, das das Bereitstellen der Adsorberharze in einer gepackten Säule umfasst; und
(d) Inkontaktbringen des Überstands von Schritt (b) mit dem einen oder den mehreren Adsorberharzen, um zumindest einen Teil des Steviolglycosids oder der Steviolglycosidzusammensetzung zu erhalten, wodurch das Steviolglycosid oder die Steviolglycosidzusammensetzung isoliert wird; oder
(e) Bereitstellen von einer oder mehreren Ionenaustausch- oder Ionenaustausch-oder Umkehrphasen-Chromatographie-Säulen; und
(f) Inkontaktbringen des Überstands von Schritt (b) mit der einen oder den mehreren Ionenaustausch- oder Ionenaustausch- oder Umkehrphasen-Chromatographie-Säulen, um zumindest einen Teil des Steviolglycosids oder der Steviolglycosidzusammensetzung zu erhalten; oder
(g) Kristallisieren oder Extrahieren des Steviolglycosids, wodurch das Steviolglycosid oder die Steviolglycosidzusammensetzung isoliert wird.

7. Verfahren nach Anspruch 6, das weiter das Gewinnen des Steviolglycosids allein oder als eine Zusammensetzung, die das Steviolglycosid umfasst, umfasst;
wobei die gewonnene Zusammensetzung bezüglich des Steviolglycosids angereichert ist, im Vergleich mit einer Glycosidzusammensetzung von einer Stevia-Pflanze, und eine verringerte Höhe von Stevia-pflanzlichen Komponenten, im Vergleich mit einem pflanzlichen Stevia-Extrakt, aufweist; und
wobei die gewonnene Zusammensetzung eine verringerte Höhe von Stevia-pflanzlichen Komponenten, im Vergleich mit einem pflanzlichen Stevia-Extrakt, aufweist.

8. Verfahren nach einem der Ansprüche 4-7, wobei das Zellkulturmedium Folgendes umfasst:
(a) das Steviolglycosid oder die Steviolglycosidzusammensetzung, die durch die rekombinante Wirtszelle nach einem der Ansprüche 1-3 oder Ganzzell-Biokonversion eines pflanzlichen oder synthetischen Steviols und/oder von Steviolglycosiden produziert werden,
(b) Glucose, Fructose und/oder Saccharose; und
(c) ergänzende Nährstoffe, die Spurenmetalle, Vitamine, Salze, Hefe-Stickstoff-Basismedium (YNB) und/oder Aminosäuren umfassen.

9. Verfahren nach einem der Ansprüche 4-8, wobei das Steviolglycosid oder die Steviolglycosidzusammensetzung in einer permeabilisierten rekombinanten Wirtszelle produziert wird, die mit dem Gen transformiert wurde, das das Polypeptid kodiert, das die Glycosylierung von Steviol, Steviol-1,2-biosid, Steviol-1,3-biosid, 13-SMG und/oder RebB an dessen C-19-Carboxylgruppe katalysiert und mindestens 80 % Sequenzidentität mit der Aminosäuresequenz aufweist, die in SEQ.-ID Nr. 2 oder 119 dargelegt ist.

10. *In-vitro-*Verfahren zur Produktion von Steviol-19-*O*-glycosid (19-SMG), das Folgendes umfasst: Zugeben eines rekombinante Polypeptids, das die Glycosylierung von Steviol an dessen C-19-Carboxylgruppe katalysiert und mindestens 80 % Sequenzidentität mit der Aminosäuresequenz aufweist, die in SEQ.-ID Nr. 2 oder 119 dargelegt ist, und eines pflanzlichen oder synthetischen Steviols zu einer Reaktionsmischung; und Synthetisieren von 19-SMG dadurch.

11. Verfahren nach Anspruch 10, wobei die Reaktionsmischung Folgendes umfasst:
(a) 19-SMG;
(b) das rekombinante Polypeptid;
(c) Glucose, Fructose und/oder Saccharose, Uridindiphosphat (UDP)-Glucose, UDP-Rhamnose, UDP-Xylose und/oder N-Acetylglucosamin; und
(d) Reaktionspuffer und/oder Salze.

12. Verfahren nach einem der Ansprüche 4-9, wobei das Steviolglycosid Folgendes ist oder die Steviolglycosidzusammensetzung Folgendes umfasst: 13-SMG, Steviol-1,2-biosid, Steviol-1,3-biosid, 19-SMG, Steviosid, 1,3-Steviosid, Rubusosid, Rebaudiosid A (RebA), RebB, Rebaudiosid C (RebC), Rebaudiosid D (RebD), Rebaudiosid E (RebE), Rebaudiosid F (RebF), Rebaudiosid M (RebM), Rebaudiosid Q (RebQ), Rebaudiosid I (RebI), Dulcosid A oder Isomere davon.

13. Zellkulturmedium, das Folgendes umfasst:
(a) die rekombinante Wirtszelle nach einem der Ansprüche 1-3; und
(b) ein oder mehrere Steviolglycoside, die durch die rekombinante Wirtszelle nach einem der Ansprüche 1-3 produziert werden,
wobei ein oder mehrere Steviolglycoside in einer Konzentration von mindestens 0,1 mg/Liter des Zellkulturmediums vorliegen und das Zellkulturmedium weiter Glucose, Saccharose, UDP-Glucose, UDP-Rhamnose, UDP-Xylose, N-Acetylglucosamin und/oder YNB umfasst; und
wobei das Zellkulturmedium bezüglich 19-SMG angereichert ist, im Vergleich mit einem Steviolglycosidextrakt von einer Stevia-Pflanze, und eine verringerte Höhe von Stevia-pflanzlichen Komponenten, im Vergleich mit einem pflanzlichen Stevia-Extrakt, aufweist.

14. Verfahren zum Übertragen einer Zuckereinheit auf eine C-19-Carboxylgruppe von Steviol oder eines Steviolglycosids, das Folgendes umfasst: Inkontaktbringen von Steviol oder dem Steviolglycosid mit einem rekombinanten Polypeptid, das die Glycosylierung des Steviols oder des Steviolglycosids an dessen C-19-Carboxylgruppe katalysiert und mindestens 80 % Sequenzidentität mit der Aminosäuresequenz aufweist, die in SEQ.-ID Nr. 2 oder 119 dargelegt ist, und einem UDP-Zucker unter geeigneten Bedingungen für die Übertragung der Zuckereinheit auf das Steviolglycosid;
wobei das Steviolglycosid 13-SMG, Steviol-1,2-biosid, Steviol-1,3-biosid, und/oder RebB umfasst; und
wobei 19-SMG, Rubusosid, Steviosid, 1,3-Steviosid (RebG), RebA und/oder eine Steviolglycosidzusammensetzung davon bei der Übertragung der Zuckereinheit produziert werden.

## Revendications

1. Cellule hôte recombinée, comprenant un gène recombiné codant pour un polypeptide catalysant la glycosylation de stéviol, de stéviol-1,2-bioside, de stévol-1,3-bioside, de stéviol-13-O-glucoside (13-SMG), et/ou de Rébaudioside B (RebB) au niveau de son groupement C-19 carboxyle et ayant au moins 80% d'identité de séquence avec la séquence d'acides aminés exposée dans SEQ ID n°2 ou 119, la cellule hôte recombinée produisant un stéviol glycoside ou une composition de stéviol glycoside.

2. Cellule hôte recombinée selon la revendication 1, comprenant en outre un ou plusieurs parmi :
(a) un gène codant pour un polypeptide synthétisant le géranylgéranyl pyrophosphate (GGPP) à partir du farnésyl diphosphate (FPP) et de l'isopentényl diphosphate (IPP) ;
où le polypeptide synthétisant le GGPP comprend un polypeptide ayant au moins 70% d'identité de séquence avec la séquence d'acides aminés exposée dans l'une quelconque parmi SEQ ID n° 20, 22, 24, 26, 28, 30, 32, ou 116 ;
(b) un gène codant pour un polypeptide synthétisant l'ent-copalyl diphosphate à partir du GGPP ;
où le polypeptide synthétisant l'ent-copalyl diphosphate comprend un polypeptide ayant au moins 70% d'identité de séquence avec la séquence d'acides aminés exposée dans l'une quelconque parmi SEQ ID n° 34, 36, 38, 40 ou 42 ;
(c) un gène codant pour un polypeptide synthétisant l'ent-kaurène à partir de l'ent-copalyl pyrophosphate ;
où le polypeptide synthétisant l'ent-kaurène comprend un polypeptide ayant au moins 70% d'identité de séquence avec la séquence d'acides aminés exposée dans l'une quelconque parmi SEQ ID n° 44, 46, 48, 50 ou 52 ;
(d) un gène codant pour un polypeptide synthétisant l'acide ent-kaurénoïque à partir de l'ent-kaurène ;
où le polypeptide synthétisant l'acide ent-kaurénoïque comprend un polypeptide ayant au moins 70% d'identité de séquence avec la séquence d'acides aminés exposée dans l'une quelconque parmi SEQ ID n° 60, 62, 66, 68, 70, 72, 74, 76 ou 117 ;
(e) un gène codant pour un polypeptide réduisant le complexe de cytochrome P450 ;
où le polypeptide réduisant le complexe de cytochrome P450 comprend un polypeptide ayant au moins 70% d'identité de séquence avec la séquence d'acides aminés exposée dans l'une quelconque parmi SEQ ID n° 78, 80, 82, 84, 86, 88, 90 ou 92 ;
(f) un gène codant pour un polypeptide synthétisant le stéviol à partir de l'acide ent-kaurénoïque ;
où le polypeptide catalysant la synthèse de stéviol comprend un polypeptide ayant au moins 70% d'identité de séquence avec la séquence d'acides aminés exposée dans l'une quelconque parmi SEQ ID n° 94, 97, 100-104, 106, 108, 110, 112 ou 114 ;
(g) un gène codant pour un polypeptide catalysant la glycosylation du stéviol ou d'un stéviol glycoside au niveau de son groupement C-13 hydroxyle ;
où le polypeptide catalysant la glycosylation du stéviol ou du stéviol glycoside au niveau de son groupement C-13 hydroxyle comprend un polypeptide ayant au moins 55% d'identité de séquence avec la séquence d'acides aminés exposée dans SEQ ID n° 7 ;
(h) un gène codant pour un polypeptide effectuant la bêta-1,3 glycosylation du C3' du 13-O-glucose, du 19-O-glucose, ou du 13-O-glucose ainsi que du 19-O-glucose d'un stéviol glycoside ;
où le polypeptide effectuant la bêta-1,3 glycosylation du C3' du 13-O-glucose, du 19-O-glucose, ou du 13-O-glucose ainsi que du 19-O-glucose du stéviol glycoside comprend un polypeptide ayant au moins 50% d'identité de séquence avec la séquence d'acides aminés exposée dans SEQ ID n° 9 ;
(i) un gène codant pour un deuxième polypeptide catalysant la glycosylation du stéviol ou d'un stéviol glycoside au niveau de son groupement C-19 carboxyle ;
où le deuxième polypeptide catalysant la glycosylation du stéviol ou du stéviol glycoside au niveau de son groupement C-19 carboxyle comprend un polypeptide ayant au moins 55% d'identité de séquence avec la séquence d'acides aminés exposée dans SEQ ID n° 4 ;
(j) un gène codant pour un polypeptide effectuant la bêta-1,2 glycosylation du C2' du 13-O-glucose, du 19-O-glucose, ou du 13-O-glucose ainsi que du 19-O-glucose d'un stéviol glycoside ;
où le polypeptide effectuant la bêta-1,2 glycosylation du C2' du 13-O-glucose, du 19-O-glucose, ou du 13-O-glucose ainsi que du 19-O-glucose du stéviol glycoside comprend un polypeptide ayant au moins 50% d'identité de séquence avec la séquence d'acides aminés exposée dans SEQ ID n° 11 ou 13 ; ou un polypeptide ayant au moins 65% d'identité de séquence avec la séquence d'acides aminés exposée dans SEQ ID n° 16 ; et/ou
(k) un gène codant pour un polypeptide bifonctionnel synthétisant l'ent-copalyl diphosphate à partir du GGPP et synthétisant l'ent-kaurène à partir de l'ent-copalyl pyrophosphate ;
où le polypeptide bifonctionnel synthétisant l'ent-copalyl diphosphate à partir du GGPP et synthétisant l'ent-kaurène à partir de l'ent-copalyl pyrophosphate comprend un polypeptide ayant au moins 50% d'identité de séquence avec la séquence d'acides aminés exposée dans l'une quelconque parmi SEQ ID n° 54, 56 ou 58 ;
dans laquelle au moins l'un des gènes est un gène recombiné.

3. Cellule hôte recombinée selon la revendication 1 ou 2, la cellule hôte recombinée comprenant une cellule végétale, une cellule fongique, une cellule algale, une cellule archéale, ou une cellule bactérienne ;
la cellule bactérienne comprenant des cellules d'*Escherichia,* des cellules de *Lactobacillus,* des cellules de *Lactococcus,* des cellules de *Corynebacterium,* des cellules d'*Acetobacter,* des cellules *d'Acinetobacter,* ou des cellules de *Pseudomonas ;*
la cellule fongique comprenant une cellule de levure ;
la cellule de levure étant une cellule de l'espèce *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous,* ou *Candida albicans* ; ou
la cellule de levure étant un Saccharomycète ; ou
la cellule de levure étant une cellule de l'espèce *Saccharomyces cerevisiae.*

4. Méthode de production d'un stéviol glycoside ou d'une composition de stéviol glycoside, comprenant la culture de la cellule hôte recombinée selon l'une quelconque des revendications 1-3 dans un milieu de culture cellulaire, dans des conditions dans lesquelles les gènes sont exprimés, où le stéviol glycoside ou la composition de stéviol glycoside est produit(e) par la cellule hôte recombinée.

5. Méthode de production d'un stéviol glycoside ou d'une composition de stéviol glycoside, comprenant la bioconversion à cellules entières d'un stéviol et/ou de stéviol glycosides d'origine végétale ou synthétique dans un milieu de culture cellulaire en utilisant le polypeptide recombiné catalysant la glycosylation de stéviol, de stéviol-1,2-bioside, de stévol-1,3-bioside, de 13-SMG, et/ou de RebB au niveau de son groupement C-19 carboxyle et ayant au moins 80% d'identité de séquence avec la séquence d'acides aminés exposée dans SEQ ID n°2 ou 119, produit par la cellule hôte recombinée selon l'une quelconque des revendications 1-3, et synthétisant ainsi le stéviol glycoside ou la composition de stéviol glycoside.

6. Méthode selon la revendication 4 ou 5, comprenant en outre l'isolement du stéviol glycoside ou de la composition de stéviol glycoside ;
dans laquelle l'étape d'isolement comprend :
(a) la mise à disposition du milieu de culture cellulaire comprenant le stéviol glycoside ou la composition de stéviol glycoside ;
(b) la séparation d'une phase liquide du milieu de culture cellulaire et d'une phase solide de la culture cellulaire afin d'obtenir un surnageant comprenant le stéviol glycoside ou la composition de stéviol glycoside ; et comprenant en outre
(c) la mise à disposition d'une ou plusieurs résines adsorbantes, comprenant la mise à disposition des résines adsorbantes dans une colonne garnie ; et
(d) la mise en contact du surnageant de l'étape (b) avec les une ou plusieurs résines adsorbantes afin d'obtenir au moins une portion du stéviol glycoside ou de la composition de stéviol glycoside, isolant ainsi le stéviol glycoside ou la composition de stéviol glycoside ; ou
(e) la mise à disposition d'une ou plusieurs colonnes de chromatographie d'échange d'ions ou d'échange d'ions ou en phase inverse ; et
(f) la mise en contact du surnageant de l'étape (b) avec les une ou plusieurs colonnes de chromatographie d'échange d'ions ou d'échange d'ions ou en phase inverse afin d'obtenir au moins une portion du stéviol glycoside ou de la composition de stéviol glycoside ; ou
(g) la cristallisation ou l'extraction du stéviol glycoside, isolant ainsi le stéviol glycoside ou la composition de stéviol glycoside.

7. Méthode selon la revendication 6, comprenant en outre la récupération du stéviol glycoside seul ou sous forme d'une composition comprenant le stéviol glycoside ;
dans laquelle la composition récupérée est enrichie en stéviol glycoside, par rapport à une composition de glycoside issue d'une plante de Stevia et présente une teneur réduite en composants dérivés de plante de Stevia par rapport à un extrait de plante de Stevia ; et
dans laquelle la composition récupérée présente une teneur réduite en composants dérivés de plante de Stevia par rapport à un extrait de plante de Stevia.

8. Méthode selon l'une quelconque des revendications 4-7, dans laquelle le milieu de culture cellulaire comprend :
(a) le stéviol glycoside ou la composition de stéviol glycoside produit(e) par la cellule hôte recombinée selon l'une quelconque des revendications 1-3 ou la bioconversion à cellules entières d'un stéviol et/ou de stéviol glycosides d'origine végétale ou synthétique,
(b) du glucose, du fructose, et/ou du saccharose ; et
(c) un complément nutritionnel comprenant des métaux à l'état de traces, des vitamines, des sels, des bases azotées de levure (YNB), et/ou des acides aminés.

9. Méthode selon l'une quelconque des revendications 4-8, dans laquelle le stéviol glycoside ou la composition de stéviol glycoside est produit(e) dans une cellule hôte recombinée perméabilisée ayant été transformée par le gène codant pour le polypeptide catalysant la glycosylation de stéviol, de stéviol-1,2-bioside, de stévol-1,3-bioside, de 13-SMG, et/ou de RebB au niveau de son groupement C-19 carboxyle et ayant au moins 80% d'identité de séquence avec la séquence d'acides aminés exposée dans SEQ ID n°2 ou 119.

10. Méthode *in vitro* de production de stéviol-19-O-glucoside (19-SMG), comprenant l'addition d'un polypeptide recombiné catalysant la glycosylation de stéviol au niveau de son groupement C-19 carboxyle et ayant au moins 80% d'identité de séquence avec la séquence d'acides aminés exposée dans SEQ ID n°2 ou 119 et d'un stéviol d'origine végétale ou synthétique à un mélange réactionnel, et synthétiser ainsi le 19-SMG.

11. Méthode selon la revendication 10, dans laquelle le mélange réactionnel comprend :
(a) du 19-SMG ;
(b) le polypeptide recombiné ;
(c) du glucose, du fructose, et/ou du saccharose, de l'uridine diphosphate (UDP)-glucose, de l'UDP-rhamnose, de l'UDP-xylose, et/ou de la N-acétylglucosamine ; et
(d) un tampon de réaction et/ou des sels.

12. Méthode selon l'une quelconque des revendications 4-9, dans laquelle le stéviol glycoside ou la composition de stéviol glycoside comprend du 13-SMG, du stéviol-1,2-bioside, du stéviol-1,3-bioside, du 19-SMG, du stévioside, du 1,3-stévioside, du rubusoside, du Rébaudioside A (RebA), du RebB, du Rébaudioside C (RebC), du Rébaudioside D (RebD), du Rébaudioside E (RebE), du Rébaudioside F (RebF), du Rébaudioside M (RebM), du Rébaudioside Q (RebQ), du Rébaudioside I (Rebl), du dulcoside A, ou des isomères de ceux-ci.

13. Milieu de culture cellulaire, comprenant :
(a) la cellule hôte recombinée selon l'une quelconque des revendications 1-3 ; et
(b) un ou plusieurs stéviol glycosides produits par la cellule hôte recombinée selon l'une quelconque des revendications 1-3 ;
dans lequel les un ou plusieurs stéviol glycosides sont présents selon une concentration d'au moins 0,1 mg/litre du milieu de culture cellulaire, et le milieu de culture cellulaire comprend en outre du glucose, du saccharose, de l'UDP-glucose, de l'UDP-rhamnose, de l'UDP-xylose, de la N-acétylglucosamine, et/ou des YNB ; et
le milieu de culture cellulaire étant enrichi en 19-SMG par rapport à un extrait de stéviol glycoside issu d'une plante de Stevia et présentant un taux réduit en composants dérivés de plante de Stevia par rapport à un extrait de plante de Stevia.

14. Méthode de transfert d'un motif de sucre sur un groupement C-19 carboxyle de stéviol ou d'un stéviol glycoside, comprenant la mise en contact du stéviol ou du stéviol glycoside avec un polypeptide recombiné catalysant la glycosylation du stéviol ou du stéviol glycoside au niveau de son groupement C-19 carboxyle et ayant au moins 80% d'identité de séquence avec la séquence d'acides aminés exposée dans SEQ ID n°2 ou 119 et une UDP-sucre dans des conditions convenables pour le transfert du motif de sucre sur le stéviol glycoside ;
dans laquelle le stéviol glycoside comprend le 13-SMG, le stéviol-1,2-bioside, le stéviol-1,3-bioside, et/ou le RebB ; et
dans laquelle du 19-SMG, du rubusoside, du stévioside, du 1,3-stévioside (RebG), du RebA, et/ou une composition de stéviol glycoside de ceux-ci est produit(e) lors du transfert du motif de sucre.


### SEQUENCE LISTING

<110> Evolva SA
<120> 15-1924-WO
<130> PRODUCTION OF STEVIOL GLYCOSIDES IN RECOMBINANT HOSTS
<160> 119
<170> PatentIn version 3.5
<210> 1
   <211> 1410
   <212> DNA
   <213> Rubus suavissimus
<400> 1
   atggcttgca cttccaccgg aagcacaaag cctcatgttg tttgtactaa tgttgcttct      60
   caaagccaca ttaaggcaat gcttaaatta gccaaaatcc ttcaccatag aggctttcac     120
   gtaacctttg ttaacacaga atccatccac aagcgttttc ttgaatctca aggacccaat     180
   tccctcaacg gcttagctga ttttcgcttc gaaacccttc cagattcaga cgaaaatgcc     240
   cccagaaacc atctgttggc tccatttcgt gacctattga tgaaactcaa cgatactagt     300
   cctccggtga cttgcattgt ttcaaatggt ttcatgtcca cattcacaat cactgctgca     360
   gaagaactaa gtgtccctat tgcattgttc tacagttttt ctgcttgcag ctttatggga     420
   ttaaagcaat tccgcacttt acgagaaaaa ggccttacac cactcaaaga tgagagctgt     480
   ttgacaaatg gatttttgga taaagttata gattgggttc caggaatgaa gggtatctgt     540
   ttaaagcatc tcccaacctt ctttagaact acaaatcctg atgataaatt gttcaacatc     600
   agcatggaaa caacagaagc agtagacaaa gcttcagcag ttgttcttct cacctttgat     660
   gctttggaaa aagatgtttt ggcagctctc tccgcttcta tttctccacc tgtttataca     720
   attggtcccc tccaattact tctcaaccaa ataccagaag accctctgga ggctatggga     780
   tacagtattt tgaaagaaga atcagaatgt ctccaatggc tgaactccaa acctccaaat     840
   tcagttgttt atgtcaactt tggcagcata gcggtcttga cagcagaaca gcttctggaa     900
   tttggatggg gacttgcaaa tactaagctt cccttcttct gggttattag gcctgatttg     960
   gttgttggca agtcggcggt tttgcctcca gagttcgaag ctgaaaccaa agaccggggt    1020
   ctaatcgcaa gttggtgccc ccaagaacaa gtcctaaacc acccatcagt tggagggttt    1080
   ctgacacata gtggttggaa ttcaaccatt gaaagcgtga cagctggagt gccgatgctg    1140
   tgttggccat tttttgcaga ccagccaaca aacagttact acacttgcaa taaatgggga    1200
   attggcatgg agatcaacaa tgatgtcaag agaaatgatg tagaaaagct tgtaaaggag    1260
   ttaatggagg gagagaaggg taagaaaatg aaaagcaagg tcttggagtg gaagaaactt    1320
   gcagaagaag caactgttcc acatggttct tcatcgatta atttagataa tctagtgaat    1380
   caagttctac acagtgtaag acaaagctag                                     1410
<210> 2
   <211> 469
   <212> PRT
   <213> Rubus suavissimus
<400> 2
   Met Ala Cys Thr Ser Thr Gly Ser Thr Lys Pro His Val Val Cys Thr
   1               5                   10                  15
   Asn Val Ala Ser Gln Ser His Ile Lys Ala Met Leu Lys Leu Ala Lys
               20                  25                  30
   Ile Leu His His Arg Gly Phe His Val Thr Phe Val Asn Thr Glu Ser
           35                  40                  45
   Ile His Lys Arg Phe Leu Glu Ser Gln Gly Pro Asn Ser Leu Asn Gly
       50                  55                  60
   Leu Ala Asp Phe Arg Phe Glu Thr Leu Pro Asp Ser Asp Glu Asn Ala
   65                  70                  75                  80
   Pro Arg Asn His Leu Leu Ala Pro Phe Arg Asp Leu Leu Met Lys Leu
                   85                  90                  95
   Asn Asp Thr Ser Pro Pro Val Thr Cys Ile Val Ser Asn Gly Phe Met
               100                 105                 110
   Ser Thr Phe Thr Ile Thr Ala Ala Glu Glu Leu Ser Val Pro Ile Ala
           115                 120                 125
   Leu Phe Tyr Ser Phe Ser Ala Cys Ser Phe Met Gly Leu Lys Gln Phe
       130                 135                 140
   Arg Thr Leu Arg Glu Lys Gly Leu Thr Pro Leu Lys Asp Glu Ser Cys
   145                 150                 155                 160
   Leu Thr Asn Gly Phe Leu Asp Lys Val Ile Asp Trp Val Pro Gly Met
                   165                 170                 175
   Lys Gly Ile Cys Leu Lys His Leu Pro Thr Phe Phe Arg Thr Thr Asn
               180                 185                 190
   Pro Asp Asp Lys Leu Phe Asn Ile Ser Met Glu Thr Thr Glu Ala Val
           195                 200                 205
   Asp Lys Ala Ser Ala Val Val Leu Leu Thr Phe Asp Ala Leu Glu Lys
       210                 215                 220
   Asp Val Leu Ala Ala Leu Ser Ala Ser Ile Ser Pro Pro Val Tyr Thr
   225                 230                 235                 240
   Ile Gly Pro Leu Gln Leu Leu Leu Asn Gln Ile Pro Glu Asp Pro Leu
                   245                 250                 255
   Glu Ala Met Gly Tyr Ser Ile Leu Lys Glu Glu Ser Glu Cys Leu Gln
               260                 265                 270
   Trp Leu Asn Ser Lys Pro Pro Asn Ser Val Val Tyr Val Asn Phe Gly
           275                 280                 285
   Ser Ile Ala Val Leu Thr Ala Glu Gln Leu Leu Glu Phe Gly Trp Gly
       290                 295                 300
   Leu Ala Asn Thr Lys Leu Pro Phe Phe Trp Val Ile Arg Pro Asp Leu
   305                 310                 315                 320
   Val Val Gly Lys Ser Ala Val Leu Pro Pro Glu Phe Glu Ala Glu Thr
                   325                 330                 335
   Lys Asp Arg Gly Leu Ile Ala Ser Trp Cys Pro Gln Glu Gln Val Leu
               340                 345                 350
   Asn His Pro Ser Val Gly Gly Phe Leu Thr His Ser Gly Trp Asn Ser
           355                 360                 365
   Thr Ile Glu Ser Val Thr Ala Gly Val Pro Met Leu Cys Trp Pro Phe
       370                 375                 380
   Phe Ala Asp Gln Pro Thr Asn Ser Tyr Tyr Thr Cys Asn Lys Trp Gly
   385                 390                 395                 400
   Ile Gly Met Glu Ile Asn Asn Asp Val Lys Arg Asn Asp Val Glu Lys
                   405                 410                 415
   Leu Val Lys Glu Leu Met Glu Gly Glu Lys Gly Lys Lys Met Lys Ser
               420                 425                 430
   Lys Val Leu Glu Trp Lys Lys Leu Ala Glu Glu Ala Thr Val Pro His
           435                 440                 445
   Gly Ser Ser Ser Ile Asn Leu Asp Asn Leu Val Asn Gln Val Leu His
       450                 455                 460
   Ser Val Arg Gln Ser
   465
<210> 3
   <211> 1383
   <212> DNA
   <213> Stevia rebaudiana
<400> 3
   atggcagagc aacaaaagat caaaaagtca cctcacgtct tacttattcc atttcctctg 60
   caaggacata tcaacccatt catacaattt gggaaaagat tgattagtaa gggtgtaaag 120
   acaacactgg taaccactat ccacactttg aattctactc tgaaccactc aaatactact 180
   actacaagta tagaaattca agctatatca gacggatgcg atgagggtgg ctttatgtct 240
   gccggtgaat cttacttgga aacattcaag caagtgggat ccaagtctct ggccgatcta 300
   atcaaaaagt tacagagtga aggcaccaca attgacgcca taatctacga ttctatgaca 360
   gagtgggttt tagacgttgc tatcgaattt ggtattgatg gaggttcctt tttcacacaa 420
   gcatgtgttg tgaattctct atactaccat gtgcataaag ggttaatctc tttaccattg 480
   ggtgaaactg tttcagttcc aggttttcca gtgttacaac gttgggaaac cccattgatc 540
   ttacaaaatc atgaacaaat acaatcacct tggtcccaga tgttgtttgg tcaattcgct 600
   aacatcgatc aagcaagatg ggtctttact aattcattct ataagttaga ggaagaggta 660
   attgaatgga ctaggaagat ctggaatttg aaagtcattg gtccaacatt gccatcaatg 720
   tatttggaca aaagacttga tgatgataaa gataatggtt tcaatttgta caaggctaat 780
   catcacgaat gtatgaattg gctggatgac aaaccaaagg aatcagttgt atatgttgct 840
   ttcggctctc ttgttaaaca tggtccagaa caagttgagg agattacaag agcacttata 900
   gactctgacg taaacttttt gtgggtcatt aagcacaaag aggaggggaa actgccagaa 960
   aacctttctg aagtgataaa gaccggaaaa ggtctaatcg ttgcttggtg taaacaattg 1020
   gatgttttag ctcatgaatc tgtaggctgt tttgtaacac attgcggatt caactctaca 1080
   ctagaagcca tttccttagg cgtacctgtc gttgcaatgc ctcagttctc cgatcagaca 1140
   accaacgcta aacttttgga cgaaatacta ggggtgggtg tcagagttaa agcagacgag 1200
   aatggtatcg tcagaagagg gaacctagct tcatgtatca aaatgatcat ggaagaggaa 1260
   agaggagtta tcataaggaa aaacgcagtt aagtggaagg atcttgcaaa ggttgccgtc 1320
   catgaaggcg gctcttcaga taatgatatt gttgaatttg tgtccgaact aatcaaagcc 1380
   taa 1383
<210> 4
   <211> 460
   <212> PRT
   <213> Stevia rebaudiana
<400> 4
   Met Ala Glu Gln Gln Lys Ile Lys Lys Ser Pro His Val Leu Leu Ile
   1               5                   10                  15
   Pro Phe Pro Leu Gln Gly His Ile Asn Pro Phe Ile Gln Phe Gly Lys
               20                  25                  30
   Arg Leu Ile Ser Lys Gly Val Lys Thr Thr Leu Val Thr Thr Ile His
           35                  40                  45
   Thr Leu Asn Ser Thr Leu Asn His Ser Asn Thr Thr Thr Thr Ser Ile
       50                  55                  60
   Glu Ile Gln Ala Ile Ser Asp Gly Cys Asp Glu Gly Gly Phe Met Ser
   65                  70                  75                  80
   Ala Gly Glu Ser Tyr Leu Glu Thr Phe Lys Gln Val Gly Ser Lys Ser
                   85                  90                  95
   Leu Ala Asp Leu Ile Lys Lys Leu Gln Ser Glu Gly Thr Thr Ile Asp
               100                 105                 110
   Ala Ile Ile Tyr Asp Ser Met Thr Glu Trp Val Leu Asp Val Ala Ile
           115                 120                 125
   Glu Phe Gly Ile Asp Gly Gly Ser Phe Phe Thr Gln Ala Cys Val Val
       130                 135                 140
   Asn Ser Leu Tyr Tyr His Val His Lys Gly Leu Ile Ser Leu Pro Leu
   145                 150                 155                 160
   Gly Glu Thr Val Ser Val Pro Gly Phe Pro Val Leu Gln Arg Trp Glu
                   165                 170                 175
   Thr Pro Leu Ile Leu Gln Asn His Glu Gln Ile Gln Ser Pro Trp Ser
               180                 185                 190
   Gln Met Leu Phe Gly Gln Phe Ala Asn Ile Asp Gln Ala Arg Trp Val
           195                 200                 205
   Phe Thr Asn Ser Phe Tyr Lys Leu Glu Glu Glu Val Ile Glu Trp Thr
       210                 215                 220
   Arg Lys Ile Trp Asn Leu Lys Val Ile Gly Pro Thr Leu Pro Ser Met
   225                 230                 235                 240
   Tyr Leu Asp Lys Arg Leu Asp Asp Asp Lys Asp Asn Gly Phe Asn Leu
                   245                 250                 255
   Tyr Lys Ala Asn His His Glu Cys Met Asn Trp Leu Asp Asp Lys Pro
               260                 265                 270
   Lys Glu Ser Val Val Tyr Val Ala Phe Gly Ser Leu Val Lys His Gly
           275                 280                 285
   Pro Glu Gln Val Glu Glu Ile Thr Arg Ala Leu Ile Asp Ser Asp Val
       290                 295                 300
   Asn Phe Leu Trp Val Ile Lys His Lys Glu Glu Gly Lys Leu Pro Glu
   305                 310                 315                 320
   Asn Leu Ser Glu Val Ile Lys Thr Gly Lys Gly Leu Ile Val Ala Trp
                   325                 330                 335
   Cys Lys Gln Leu Asp Val Leu Ala His Glu Ser Val Gly Cys Phe Val
               340                 345                 350
   Thr His Cys Gly Phe Asn Ser Thr Leu Glu Ala Ile Ser Leu Gly Val
           355                 360                 365
   Pro Val Val Ala Met Pro Gln Phe Ser Asp Gln Thr Thr Asn Ala Lys
       370                 375                 380
   Leu Leu Asp Glu Ile Leu Gly Val Gly Val Arg Val Lys Ala Asp Glu
   385                 390                 395                 400
   Asn Gly Ile Val Arg Arg Gly Asn Leu Ala Ser Cys Ile Lys Met Ile
                   405                 410                 415
   Met Glu Glu Glu Arg Gly Val Ile Ile Arg Lys Asn Ala Val Lys Trp
               420                 425                 430
   Lys Asp Leu Ala Lys Val Ala Val His Glu Gly Gly Ser Ser Asp Asn
           435                 440                 445
   Asp Ile Val Glu Phe Val Ser Glu Leu Ile Lys Ala
       450                 455                 460
<210> 5
   <211> 1586
   <212> DNA
   <213> Stevia rebaudiana
<400> 5
   atggatgcaa tggctacaac tgagaagaaa ccacacgtca tcttcatacc atttccagca 60
   caaagccaca ttaaagccat gctcaaacta gcacaacttc tccaccacaa aggactccag 120
   ataaccttcg tcaacaccga cttcatccac aaccagtttc ttgaatcatc gggcccacat 180
   tgtctagacg gtgcaccggg tttccggttc gaaaccattc cggatggtgt ttctcacagt 240
   ccggaagcga gcatcccaat cagagaatca ctcttgagat ccattgaaac caacttcttg 300
   gatcgtttca ttgatcttgt aaccaaactt ccggatcctc cgacttgtat tatctcagat 360
   gggttcttgt cggttttcac aattgacgct gcaaaaaagc ttggaattcc ggtcatgatg 420
   tattggacac ttgctgcctg tgggttcatg ggtttttacc atattcattc tctcattgag 480
   aaaggatttg caccacttaa agatgcaagt tacttgacaa atgggtattt ggacaccgtc 540
   attgattggg ttccgggaat ggaaggcatc cgtctcaagg atttcccgct ggactggagc 600
   actgacctca atgacaaagt tttgatgttc actacggaag ctcctcaaag gtcacacaag 660
   gtttcacatc atattttcca cacgttcgat gagttggagc ctagtattat aaaaactttg 720
   tcattgaggt ataatcacat ttacaccatc ggcccactgc aattacttct tgatcaaata 780
   cccgaagaga aaaagcaaac tggaattacg agtctccatg gatacagttt agtaaaagaa 840
   gaaccagagt gtttccagtg gcttcagtct aaagaaccaa attccgtcgt ttatgtaaat 900
   tttggaagta ctacagtaat gtctttagaa gacatgacgg aatttggttg gggacttgct 960
   aatagcaacc attatttcct ttggatcatc cgatcaaact tggtgatagg ggaaaatgca 1020
   gttttgcccc ctgaacttga ggaacatata aagaaaagag gctttattgc tagctggtgt 1080
   tcacaagaaa aggtcttgaa gcacccttcg gttggagggt tcttgactca ttgtgggtgg 1140
   ggatcgacca tcgagagctt gtctgctggg gtgccaatga tatgctggcc ttattcgtgg 1200
   gaccagctga ccaactgtag gtatatatgc aaagaatggg aggttgggct cgagatggga 1260
   accaaagtga aacgagatga agtcaagagg cttgtacaag agttgatggg agaaggaggt 1320
   cacaaaatga ggaacaaggc taaagattgg aaagaaaagg ctcgcattgc aatagctcct 1380
   aacggttcat cttctttgaa catagacaaa atggtcaagg aaatcaccgt gctagcaaga 1440
   aactagttac aaagttgttt cacattgtgc tttctattta agatgtaact ttgttctaat 1500
   ttaatattgt ctagatgtat tgaaccataa gtttagttgg tctcaggaat tgatttttaa 1560
   tgaaataatg gtcattaggg gtgagt 1586
<210> 6
   <211> 1446
   <212> DNA
   <213> Artificial Sequence
<220>
   <223> Codon-optimized UGT85C2
<400> 6
   atggatgcaa tggcaactac tgagaaaaag cctcatgtga tcttcattcc atttcctgca      60
   caatctcaca taaaggcaat gctaaagtta gcacaactat tacaccataa gggattacag     120
   ataactttcg tgaataccga cttcatccat aatcaatttc tggaatctag tggccctcat     180
   tgtttggacg gagccccagg gtttagattc gaaacaattc ctgacggtgt ttcacattcc     240
   ccagaggcct ccatcccaat aagagagagt ttactgaggt caatagaaac caactttttg     300
   gatcgtttca ttgacttggt cacaaaactt ccagacccac caacttgcat aatctctgat     360
   ggctttctgt cagtgtttac tatcgacgct gccaaaaagt tgggtatccc agttatgatg     420
   tactggactc ttgctgcatg cggtttcatg ggtttctatc acatccattc tcttatcgaa     480
   aagggttttg ctccactgaa agatgcatca tacttaacca acggctacct ggatactgtt     540
   attgactggg taccaggtat ggaaggtata agacttaaag attttccttt ggattggtct     600
   acagacctta atgataaagt attgatgttt actacagaag ctccacaaag atctcataag     660
   gtttcacatc atatctttca cacctttgat gaattggaac catcaatcat caaaaccttg     720
   tctctaagat acaatcatat ctacactatt ggtccattac aattacttct agatcaaatt     780
   cctgaagaga aaaagcaaac tggtattaca tccttacacg gctactcttt agtgaaagag     840
   gaaccagaat gttttcaatg gctacaaagt aaagagccta attctgtggt ctacgtcaac     900
   ttcggaagta caacagtcat gtccttggaa gatatgactg aatttggttg gggccttgct     960
   aattcaaatc attactttct atggattatc aggtccaatt tggtaatagg ggaaaacgcc    1020
   gtattacctc cagaattgga ggaacacatc aaaaagagag gtttcattgc ttcctggtgt    1080
   tctcaggaaa aggtattgaa acatccttct gttggtggtt tccttactca ttgcggttgg    1140
   ggctctacaa tcgaatcact aagtgcagga gttccaatga tttgttggcc atattcatgg    1200
   gaccaactta caaattgtag gtatatctgt aaagagtggg aagttggatt agaaatggga    1260
   acaaaggtta aacgtgatga agtgaaaaga ttggttcagg agttgatggg ggaaggtggc    1320
   cacaagatga gaaacaaggc caaagattgg aaggaaaaag ccagaattgc tattgctcct    1380
   aacgggtcat cctctctaaa cattgataag atggtcaaag agattacagt cttagccaga    1440
   aactaa                                                               1446
<210> 7
   <211> 481
   <212> PRT
   <213> Stevia rebaudiana
<400> 7
   Met Asp Ala Met Ala Thr Thr Glu Lys Lys Pro His Val Ile Phe Ile
   1               5                   10                  15
   Pro Phe Pro Ala Gln Ser His Ile Lys Ala Met Leu Lys Leu Ala Gln
               20                  25                  30
   Leu Leu His His Lys Gly Leu Gln Ile Thr Phe Val Asn Thr Asp Phe
           35                  40                  45
   Ile His Asn Gln Phe Leu Glu Ser Ser Gly Pro His Cys Leu Asp Gly
       50                  55                  60
   Ala Pro Gly Phe Arg Phe Glu Thr Ile Pro Asp Gly Val Ser His Ser
   65                  70                  75                  80
   Pro Glu Ala Ser Ile Pro Ile Arg Glu Ser Leu Leu Arg Ser Ile Glu
                   85                  90                  95
   Thr Asn Phe Leu Asp Arg Phe Ile Asp Leu Val Thr Lys Leu Pro Asp
               100                 105                 110
   Pro Pro Thr Cys Ile Ile Ser Asp Gly Phe Leu Ser Val Phe Thr Ile
           115                 120                 125
   Asp Ala Ala Lys Lys Leu Gly Ile Pro Val Met Met Tyr Trp Thr Leu
       130                 135                 140
   Ala Ala Cys Gly Phe Met Gly Phe Tyr His Ile His Ser Leu Ile Glu
   145                 150                 155                 160
   Lys Gly Phe Ala Pro Leu Lys Asp Ala Ser Tyr Leu Thr Asn Gly Tyr
                   165                 170                 175
   Leu Asp Thr Val Ile Asp Trp Val Pro Gly Met Glu Gly Ile Arg Leu
               180                 185                 190
   Lys Asp Phe Pro Leu Asp Trp Ser Thr Asp Leu Asn Asp Lys Val Leu
           195                 200                 205
   Met Phe Thr Thr Glu Ala Pro Gln Arg Ser His Lys Val Ser His His
       210                 215                 220
   Ile Phe His Thr Phe Asp Glu Leu Glu Pro Ser Ile Ile Lys Thr Leu
   225                 230                 235                 240
   Ser Leu Arg Tyr Asn His Ile Tyr Thr Ile Gly Pro Leu Gln Leu Leu
                   245                 250                 255
   Leu Asp Gln Ile Pro Glu Glu Lys Lys Gln Thr Gly Ile Thr Ser Leu
               260                 265                 270
   His Gly Tyr Ser Leu Val Lys Glu Glu Pro Glu Cys Phe Gln Trp Leu
   275 280 285
   Gln Ser Lys Glu Pro Asn Ser Val Val Tyr Val Asn Phe Gly Ser Thr
   290 295 300
   Thr Val Met Ser Leu Glu Asp Met Thr Glu Phe Gly Trp Gly Leu Ala
   305 310 315 320
   Asn Ser Asn His Tyr Phe Leu Trp Ile Ile Arg Ser Asn Leu Val Ile
   325 330 335
   Gly Glu Asn Ala Val Leu Pro Pro Glu Leu Glu Glu His Ile Lys Lys
   340 345 350
   Arg Gly Phe Ile Ala Ser Trp Cys Ser Gln Glu Lys Val Leu Lys His
   355 360 365
   Pro Ser Val Gly Gly Phe Leu Thr His Cys Gly Trp Gly Ser Thr Ile
   370 375 380
   Glu Ser Leu Ser Ala Gly Val Pro Met Ile Cys Trp Pro Tyr Ser Trp
   385 390 395 400
   Asp Gln Leu Thr Asn Cys Arg Tyr Ile Cys Lys Glu Trp Glu Val Gly
   405 410 415
   Leu Glu Met Gly Thr Lys Val Lys Arg Asp Glu Val Lys Arg Leu Val
   420 425 430
   Gln Glu Leu Met Gly Glu Gly Gly His Lys Met Arg Asn Lys Ala Lys
   435 440 445
   Asp Trp Lys Glu Lys Ala Arg Ile Ala Ile Ala Pro Asn Gly Ser Ser
   450 455 460
   Ser Leu Asn Ile Asp Lys Met Val Lys Glu Ile Thr Val Leu Ala Arg
   465 470 475 480
   Asn
<210> 8
   <211> 1377
   <212> DNA <213> Artificial Sequence
<220>
<210> 9
   <211> 458
   <212> PRT
   <213> Stevia rebaudiana
<400> 9
   Met Glu Asn Lys Thr Glu Thr Thr Val Arg Arg Arg Arg Arg Ile Ile
   1               5                   10                  15
   Leu Phe Pro Val Pro Phe Gln Gly His Ile Asn Pro Ile Leu Gln Leu
               20                  25                  30
   Ala Asn Val Leu Tyr Ser Lys Gly Phe Ser Ile Thr Ile Phe His Thr
           35                  40                  45
   Asn Phe Asn Lys Pro Lys Thr Ser Asn Tyr Pro His Phe Thr Phe Arg
       50                  55                  60
   Phe Ile Leu Asp Asn Asp Pro Gln Asp Glu Arg Ile Ser Asn Leu Pro
   65                  70                  75                  80
   Thr His Gly Pro Leu Ala Gly Met Arg Ile Pro Ile Ile Asn Glu His
                   85                  90                  95
   Gly Ala Asp Glu Leu Arg Arg Glu Leu Glu Leu Leu Met Leu Ala Ser
               100                 105                 110
   Glu Glu Asp Glu Glu Val Ser Cys Leu Ile Thr Asp Ala Leu Trp Tyr
           115                 120                 125
   Phe Ala Gln Ser Val Ala Asp Ser Leu Asn Leu Arg Arg Leu Val Leu
       130                 135                 140
   Met Thr Ser Ser Leu Phe Asn Phe His Ala His Val Ser Leu Pro Gln
   145                 150                 155                 160
   Phe Asp Glu Leu Gly Tyr Leu Asp Pro Asp Asp Lys Thr Arg Leu Glu
                   165                 170                 175
   Glu Gln Ala Ser Gly Phe Pro Met Leu Lys Val Lys Asp Ile Lys Ser
               180                 185                 190
   Ala Tyr Ser Asn Trp Gln Ile Leu Lys Glu Ile Leu Gly Lys Met Ile
           195                 200                 205
   Lys Gln Thr Lys Ala Ser Ser Gly Val Ile Trp Asn Ser Phe Lys Glu
       210                 215                 220
   Leu Glu Glu Ser Glu Leu Glu Thr Val Ile Arg Glu Ile Pro Ala Pro
   225                 230                 235                 240
   Ser Phe Leu Ile Pro Leu Pro Lys His Leu Thr Ala Ser Ser Ser Ser
                   245                 250                 255
   Leu Leu Asp His Asp Arg Thr Val Phe Gln Trp Leu Asp Gln Gln Pro
               260                 265                 270
   Pro Ser Ser Val Leu Tyr Val Ser Phe Gly Ser Thr Ser Glu Val Asp
           275                 280                 285
   Glu Lys Asp Phe Leu Glu Ile Ala Arg Gly Leu Val Asp Ser Lys Gln
       290                 295                 300
   Ser Phe Leu Trp Val Val Arg Pro Gly Phe Val Lys Gly Ser Thr Trp
   305                 310                 315                 320
   Val Glu Pro Leu Pro Asp Gly Phe Leu Gly Glu Arg Gly Arg Ile Val
                   325                 330                 335
   Lys Trp Val Pro Gln Gln Glu Val Leu Ala His Gly Ala Ile Gly Ala
               340                 345                 350
   Phe Trp Thr His Ser Gly Trp Asn Ser Thr Leu Glu Ser Val Cys Glu
           355                 360                 365
   Gly Val Pro Met Ile Phe Ser Asp Phe Gly Leu Asp Gln Pro Leu Asn
       370                 375                 380
   Ala Arg Tyr Met Ser Asp Val Leu Lys Val Gly Val Tyr Leu Glu Asn
   385                 390                 395                 400
   Gly Trp Glu Arg Gly Glu Ile Ala Asn Ala Ile Arg Arg Val Met Val
                   405                 410                 415
   Asp Glu Glu Gly Glu Tyr Ile Arg Gln Asn Ala Arg Val Leu Lys Gln
               420                 425                 430
   Lys Ala Asp Val Ser Leu Met Lys Gly Gly Ser Ser Tyr Glu Ser Leu
           435                 440                 445
   Glu Ser Leu Val Ser Tyr Ile Ser Ser Leu
       450                 455
<210> 10
   <211> 1422
   <212> DNA
   <213> Artificial Sequence
<220>
   <223> Codon-optimized UGT91D2e
<400> 10
   atggctacat ctgattctat tgttgatgac aggaagcagt tgcatgtggc tactttccct 60
   tggcttgctt tcggtcatat actgccttac ctacaactat caaaactgat agctgaaaaa 120
   ggacataaag tgtcattcct ttcaacaact agaaacattc aaagattatc ttcccacata 180
   tcaccattga ttaacgtcgt tcaattgaca cttccaagag tacaggaatt accagaagat 240
   gctgaagcta caacagatgt gcatcctgaa gatatccctt acttgaaaaa ggcatccgat 300
   ggattacagc ctgaggtcac tagattcctt gagcaacaca gtccagattg gatcatatac 360
   gactacactc actattggtt gccttcaatt gcagcatcac taggcatttc tagggcacat 420
   ttcagtgtaa ccacaccttg ggccattgct tacatgggtc catccgctga tgctatgatt 480
   aacggcagtg atggtagaac taccgttgaa gatttgacaa ccccaccaaa gtggtttcca 540
   tttccaacta aagtctgttg gagaaaacac gacttagcaa gactggttcc atacaaggca 600
   ccaggaatct cagacggcta tagaatgggt ttagtcctta aaggtctgga ctgcctattg 660
   tctaagtgtt accatgagtt tgggacacaa tggctaccac ttttggaaac attacaccaa 720
   gttcctgtcg taccagttgg tctattacct ccagaaatcc ctggtgatga gaaggacgag 780
   acttgggttt caatcaaaaa gtggttagac gggaagcaaa aaggctcagt ggtatatgtg 840
   gcactgggtt ccgaagtttt agtatctcaa acagaagttg tggaacttgc cttaggtttg 900
   gaactatctg gattgccatt tgtctgggcc tacagaaaac caaaaggccc tgcaaagtcc 960
   gattcagttg aattgccaga cggctttgtc gagagaacta gagatagagg gttggtatgg 1020
   acttcatggg ctccacaatt gagaatcctg agtcacgaat ctgtgtgcgg tttcctaaca 1080
   cattgtggtt ctggttctat agttgaagga ctgatgtttg gtcatccact tatcatgttg 1140
   ccaatctttg gtgaccagcc tttgaatgca cgtctgttag aagataaaca agttggaatt 1200
   gaaatcccac gtaatgagga agatggatgt ttaaccaagg agtctgtggc cagatcatta 1260
   cgttccgttg tcgttgaaaa ggaaggcgaa atctacaagg ccaatgcccg tgaactttca 1320
   aagatctaca atgacacaaa agtagagaag gaatatgttt ctcaatttgt agattaccta 1380
   gagaaaaacg ctagagccgt agctattgat catgaatcct aa 1422
<210> 11
   <211> 473
   <212> PRT
   <213> Stevia rebaudiana
<400> 11
   Met Ala Thr Ser Asp Ser Ile Val Asp Asp Arg Lys Gln Leu His Val
   1               5                   10                  15
   Ala Thr Phe Pro Trp Leu Ala Phe Gly His Ile Leu Pro Tyr Leu Gln
               20                  25                  30
   Leu Ser Lys Leu Ile Ala Glu Lys Gly His Lys Val Ser Phe Leu Ser
           35                  40                  45
   Thr Thr Arg Asn Ile Gln Arg Leu Ser Ser His Ile Ser Pro Leu Ile
       50                  55                  60
   Asn Val Val Gln Leu Thr Leu Pro Arg Val Gln Glu Leu Pro Glu Asp
   65                  70                  75                  80
   Ala Glu Ala Thr Thr Asp Val His Pro Glu Asp Ile Pro Tyr Leu Lys
                   85                  90                  95
   Lys Ala Ser Asp Gly Leu Gln Pro Glu Val Thr Arg Phe Leu Glu Gln
               100                 105                 110
   His Ser Pro Asp Trp Ile Ile Tyr Asp Tyr Thr His Tyr Trp Leu Pro
           115                 120                 125
   Ser Ile Ala Ala Ser Leu Gly Ile Ser Arg Ala His Phe Ser Val Thr
       130                 135                 140
   Thr Pro Trp Ala Ile Ala Tyr Met Gly Pro Ser Ala Asp Ala Met Ile
   145                 150                 155                 160
   Asn Gly Ser Asp Gly Arg Thr Thr Val Glu Asp Leu Thr Thr Pro Pro
                   165                 170                 175
   Lys Trp Phe Pro Phe Pro Thr Lys Val Cys Trp Arg Lys His Asp Leu
               180                 185                 190
   Ala Arg Leu Val Pro Tyr Lys Ala Pro Gly Ile Ser Asp Gly Tyr Arg
           195                 200                 205
   Met Gly Leu Val Leu Lys Gly Ser Asp Cys Leu Leu Ser Lys Cys Tyr
       210                 215                 220
   His Glu Phe Gly Thr Gln Trp Leu Pro Leu Leu Glu Thr Leu His Gln
   225                 230                 235                 240
   Val Pro Val Val Pro Val Gly Leu Leu Pro Pro Glu Ile Pro Gly Asp
                   245                 250                 255
   Glu Lys Asp Glu Thr Trp Val Ser Ile Lys Lys Trp Leu Asp Gly Lys
               260                 265                 270
   Gln Lys Gly Ser Val Val Tyr Val Ala Leu Gly Ser Glu Val Leu Val
           275                 280                 285
   Ser Gln Thr Glu Val Val Glu Leu Ala Leu Gly Leu Glu Leu Ser Gly
       290                 295                 300
   Leu Pro Phe Val Trp Ala Tyr Arg Lys Pro Lys Gly Pro Ala Lys Ser
   305                 310                 315                 320
   Asp Ser Val Glu Leu Pro Asp Gly Phe Val Glu Arg Thr Arg Asp Arg
                   325                 330                 335
   Gly Leu Val Trp Thr Ser Trp Ala Pro Gln Leu Arg Ile Leu Ser His
               340                 345                 350
   Glu Ser Val Cys Gly Phe Leu Thr His Cys Gly Ser Gly Ser Ile Val
           355                 360                 365
   Glu Gly Leu Met Phe Gly His Pro Leu Ile Met Leu Pro Ile Phe Gly
       370                 375                 380
   Asp Gln Pro Leu Asn Ala Arg Leu Leu Glu Asp Lys Gln Val Gly Ile
   385                 390                 395                 400
   Glu Ile Pro Arg Asn Glu Glu Asp Gly Cys Leu Thr Lys Glu Ser Val
                   405                 410                 415
   Ala Arg Ser Leu Arg Ser Val Val Val Glu Lys Glu Gly Glu Ile Tyr
               420                 425                 430
   Lys Ala Asn Ala Arg Glu Leu Ser Lys Ile Tyr Asn Asp Thr Lys Val
           435                 440                 445
   Glu Lys Glu Tyr Val Ser Gln Phe Val Asp Tyr Leu Glu Lys Asn Ala
       450                 455                 460
   Arg Ala Val Ala Ile Asp His Glu Ser
   465                 470
<210> 12
   <211> 1422
   <212> DNA
   <213> Artificial Sequence
<220>
   <223> Codon-optimized UGT91D2e-b
<400> 12
   atggctactt ctgattccat cgttgacgat agaaagcaat tgcatgttgc tacttttcca 60
   tggttggctt tcggtcatat tttgccatac ttgcaattgt ccaagttgat tgctgaaaag 120
   ggtcacaagg tttcattctt gtctaccacc agaaacatcc aaagattgtc ctctcatatc 180
   tccccattga tcaacgttgt tcaattgact ttgccaagag tccaagaatt gccagaagat 240
   gctgaagcta ctactgatgt tcatccagaa gatatccctt acttgaaaaa ggcttccgat 300
   ggtttacaac cagaagttac tagattcttg gaacaacatt ccccagattg gatcatctac 360
   gattatactc attactggtt gccatccatt gctgcttcat tgggtatttc tagagcccat 420
   ttctctgtta ctactccatg ggctattgct tatatgggtc catctgctga tgctatgatt 480
   aacggttctg atggtagaac taccgttgaa gatttgacta ctccaccaaa gtggtttcca 540
   tttccaacaa aagtctgttg gagaaaacac gatttggcta gattggttcc atacaaagct 600
   ccaggtattt ctgatggtta cagaatgggt atggttttga aaggttccga ttgcttgttg 660
   tctaagtgct atcatgaatt cggtactcaa tggttgcctt tgttggaaac attgcatcaa 720
   gttccagttg ttccagtagg tttgttgcca ccagaaattc caggtgacga aaaagacgaa 780
   acttgggttt ccatcaaaaa gtggttggat ggtaagcaaa agggttctgt tgtttatgtt 840
   gctttgggtt ccgaagcttt ggtttctcaa accgaagttg ttgaattggc tttgggtttg 900
   gaattgtctg gtttgccatt tgtttgggct tacagaaaac ctaaaggtcc agctaagtct 960
   gattctgttg aattgccaga tggtttcgtt gaaagaacta gagatagagg tttggtttgg 1020
   acttcttggg ctccacaatt gagaattttg tctcatgaat ccgtctgtgg tttcttgact 1080
   cattgtggtt ctggttctat cgttgaaggt ttgatgtttg gtcacccatt gattatgttg 1140
   ccaatctttg gtgaccaacc attgaacgct agattattgg aagataagca agtcggtatc 1200
   gaaatcccaa gaaatgaaga agatggttgc ttgaccaaag aatctgttgc tagatctttg 1260
   agatccgttg tcgttgaaaa agaaggtgaa atctacaagg ctaacgctag agaattgtcc 1320
   aagatctaca acgataccaa ggtcgaaaaa gaatacgttt cccaattcgt tgactacttg 1380
   gaaaagaatg ctagagctgt tgccattgat catgaatctt ga 1422
<210> 13
   <211> 473
   <212> PRT
   <213> Artificial Sequence
<220>
   <223> UGT91D2e-b
<400> 13
   Met Ala Thr Ser Asp Ser Ile Val Asp Asp Arg Lys Gln Leu His Val
   1               5                   10                  15
   Ala Thr Phe Pro Trp Leu Ala Phe Gly His Ile Leu Pro Tyr Leu Gln
               20                  25                  30
   Leu Ser Lys Leu Ile Ala Glu Lys Gly His Lys Val Ser Phe Leu Ser
           35                  40                  45
   Thr Thr Arg Asn Ile Gln Arg Leu Ser Ser His Ile Ser Pro Leu Ile
       50                  55                  60
   Asn Val Val Gln Leu Thr Leu Pro Arg Val Gln Glu Leu Pro Glu Asp
   65                  70                  75                  80
   Ala Glu Ala Thr Thr Asp Val His Pro Glu Asp Ile Pro Tyr Leu Lys
                   85                  90                  95
   Lys Ala Ser Asp Gly Leu Gln Pro Glu Val Thr Arg Phe Leu Glu Gln
               100                 105                 110
   His Ser Pro Asp Trp Ile Ile Tyr Asp Tyr Thr His Tyr Trp Leu Pro
           115                 120                 125
   Ser Ile Ala Ala Ser Leu Gly Ile Ser Arg Ala His Phe Ser Val Thr
       130                 135                 140
   Thr Pro Trp Ala Ile Ala Tyr Met Gly Pro Ser Ala Asp Ala Met Ile
   145                 150                 155                 160
   Asn Gly Ser Asp Gly Arg Thr Thr Val Glu Asp Leu Thr Thr Pro Pro
                   165                 170                 175
   Lys Trp Phe Pro Phe Pro Thr Lys Val Cys Trp Arg Lys His Asp Leu
               180                 185                 190
   Ala Arg Leu Val Pro Tyr Lys Ala Pro Gly Ile Ser Asp Gly Tyr Arg
           195                 200                 205
   Met Gly Met Val Leu Lys Gly Ser Asp Cys Leu Leu Ser Lys Cys Tyr
       210                 215                 220
   His Glu Phe Gly Thr Gln Trp Leu Pro Leu Leu Glu Thr Leu His Gln
   225                 230                 235                 240
   Val Pro Val Val Pro Val Gly Leu Leu Pro Pro Glu Ile Pro Gly Asp
                   245                 250                 255
   Glu Lys Asp Glu Thr Trp Val Ser Ile Lys Lys Trp Leu Asp Gly Lys
               260                 265                 270
   Gln Lys Gly Ser Val Val Tyr Val Ala Leu Gly Ser Glu Ala Leu Val
           275                 280                 285
   Ser Gln Thr Glu Val Val Glu Leu Ala Leu Gly Leu Glu Leu Ser Gly
       290                 295                 300
   Leu Pro Phe Val Trp Ala Tyr Arg Lys Pro Lys Gly Pro Ala Lys Ser
   305                 310                 315                 320
   Asp Ser Val Glu Leu Pro Asp Gly Phe Val Glu Arg Thr Arg Asp Arg
                   325                 330                 335
   Gly Leu Val Trp Thr Ser Trp Ala Pro Gln Leu Arg Ile Leu Ser His
               340                 345                 350
   Glu Ser Val Cys Gly Phe Leu Thr His Cys Gly Ser Gly Ser Ile Val
           355                 360                 365
   Glu Gly Leu Met Phe Gly His Pro Leu Ile Met Leu Pro Ile Phe Gly
       370                 375                 380
   Asp Gln Pro Leu Asn Ala Arg Leu Leu Glu Asp Lys Gln Val Gly Ile
   385                 390                 395                 400
   Glu Ile Pro Arg Asn Glu Glu Asp Gly Cys Leu Thr Lys Glu Ser Val
                   405                 410                 415
   Ala Arg Ser Leu Arg Ser Val Val Val Glu Lys Glu Gly Glu Ile Tyr
               420                 425                 430
   Lys Ala Asn Ala Arg Glu Leu Ser Lys Ile Tyr Asn Asp Thr Lys Val
           435                 440                 445
   Glu Lys Glu Tyr Val Ser Gln Phe Val Asp Tyr Leu Glu Lys Asn Ala
       450                 455                 460
   Arg Ala Val Ala Ile Asp His Glu Ser
   465                 470
<210> 14
   <211> 1389
   <212> DNA
   <213> Oryza sativa
<400> 14
   atggactccg gctactcctc ctcctacgcc gccgccgccg ggatgcacgt cgtgatctgc 60
   ccgtggctcg ccttcggcca cctgctcccg tgcctcgacc tcgcccagcg cctcgcgtcg 120
   cggggccacc gcgtgtcgtt cgtctccacg ccgcggaaca tatcccgcct cccgccggtg 180
   cgccccgcgc tcgcgccgct cgtcgccttc gtggcgctgc cgctcccgcg cgtcgagggg 240
   ctccccgacg gcgccgagtc caccaacgac gtcccccacg acaggccgga catggtcgag 300
   ctccaccgga gggccttcga cgggctcgcc gcgcccttct cggagttctt gggcaccgcg 360
   tgcgccgact gggtcatcgt cgacgtcttc caccactggg ccgcagccgc cgctctcgag 420
   cacaaggtgc catgtgcaat gatgttgttg ggctctgcac atatgatcgc ttccatagca 480
   gacagacggc tcgagcgcgc ggagacagag tcgcctgcgg ctgccgggca gggacgccca 540
   gcggcggcgc caacgttcga ggtggcgagg atgaagttga tacgaaccaa aggctcatcg 600
   ggaatgtccc tcgccgagcg cttctccttg acgctctcga ggagcagcct cgtcgtcggg 660
   cggagctgcg tggagttcga gccggagacc gtcccgctcc tgtcgacgct ccgcggtaag 720
   cctattacct tccttggcct tatgccgccg ttgcatgaag gccgccgcga ggacggcgag 780
   gatgccaccg tccgctggct cgacgcgcag ccggccaagt ccgtcgtgta cgtcgcgcta 840
   ggcagcgagg tgccactggg agtggagaag gtccacgagc tcgcgctcgg gctggagctc 900
   gccgggacgc gcttcctctg ggctcttagg aagcccactg gcgtctccga cgccgacctc 960
   ctccccgccg gcttcgagga gcgcacgcgc ggccgcggcg tcgtggcgac gagatgggtt 1020
   cctcagatga gcatactggc gcacgccgcc gtgggcgcgt tcctgaccca ctgcggctgg 1080
   aactcgacca tcgaggggct catgttcggc cacccgctta tcatgctgcc gatcttcggc 1140
   gaccagggac cgaacgcgcg gctaatcgag gcgaagaacg ccggattgca ggtggcaaga 1200
   aacgacggcg atggatcgtt cgaccgagaa ggcgtcgcgg cggcgattcg tgcagtcgcg 1260
   gtggaggaag aaagcagcaa agtgtttcaa gccaaagcca agaagctgca ggagatcgtc 1320
   gcggacatgg cctgccatga gaggtacatc gacggattca ttcagcaatt gagatcttac 1380
   aaggattga 1389
<210> 15
   <211> 1389
   <212> DNA
   <213> Artificial Sequence
<220>
   <223> Codon-optimized EUGT11
<400> 15
   atggatagtg gctactcctc atcttatgct gctgccgctg gtatgcacgt tgtgatctgc 60
   ccttggttgg cctttggtca cctgttacca tgtctggatt tagcccaaag actggcctca 120
   agaggccata gagtatcatt tgtgtctact cctagaaata tctctcgttt accaccagtc 180
   agacctgctc tagctcctct agttgcattc gttgctcttc cacttccaag agtagaagga 240
   ttgccagacg gcgctgaatc tactaatgac gtaccacatg atagacctga catggtcgaa 300
   ttgcatagaa gagcctttga tggattggca gctccatttt ctgagttcct gggcacagca 360
   tgtgcagact gggttatagt cgatgtattt catcactggg ctgctgcagc cgcattggaa 420
   cataaggtgc cttgtgctat gatgttgtta gggtcagcac acatgatcgc atccatagct 480
   gatagaagat tggaaagagc tgaaacagaa tccccagccg cagcaggaca aggtaggcca 540
   gctgccgccc caacctttga agtggctaga atgaaattga ttcgtactaa aggtagttca 600
   gggatgagtc ttgctgaaag gttttctctg acattatcta gatcatcatt agttgtaggt 660
   agatcctgcg tcgagttcga acctgaaaca gtacctttac tatctacttt gagaggcaaa 720
   cctattactt tccttggtct aatgcctcca ttacatgaag gaaggagaga agatggtgaa 780
   gatgctactg ttaggtggtt agatgcccaa cctgctaagt ctgttgttta cgttgcattg 840
   ggttctgagg taccactagg ggtggaaaag gtgcatgaat tagcattagg acttgagctg 900
   gccggaacaa gattcctttg ggctttgaga aaaccaaccg gtgtttctga cgccgacttg 960
   ctaccagctg ggttcgaaga gagaacaaga ggccgtggtg tcgttgctac tagatgggtc 1020
   ccacaaatga gtattctagc tcatgcagct gtaggggcct ttctaaccca ttgcggttgg 1080
   aactcaacaa tagaaggact gatgtttggt catccactta ttatgttacc aatctttggc 1140
   gatcagggac ctaacgcaag attgattgag gcaaagaacg caggtctgca ggttgcacgt 1200
   aatgatggtg atggttcctt tgatagagaa ggcgttgcag ctgccatcag agcagtcgcc 1260
   gttgaggaag agtcatctaa agttttccaa gctaaggcca aaaaattaca agagattgtg 1320
   gctgacatgg cttgtcacga aagatacatc gatggtttca tccaacaatt gagaagttat 1380
   aaagactaa 1389
<210> 16
   <211> 462
   <212> PRT
   <213> Oryza sativa
<400> 16
   Met Asp Ser Gly Tyr Ser Ser Ser Tyr Ala Ala Ala Ala Gly Met His
   1               5                   10                  15
   Val Val Ile Cys Pro Trp Leu Ala Phe Gly His Leu Leu Pro Cys Leu
               20                  25                  30
   Asp Leu Ala Gln Arg Leu Ala Ser Arg Gly His Arg Val Ser Phe Val
           35                  40                  45
   Ser Thr Pro Arg Asn Ile Ser Arg Leu Pro Pro Val Arg Pro Ala Leu
       50                  55                  60
   Ala Pro Leu Val Ala Phe Val Ala Leu Pro Leu Pro Arg Val Glu Gly
   65                  70                  75                  80
   Leu Pro Asp Gly Ala Glu Ser Thr Asn Asp Val Pro His Asp Arg Pro
                   85                  90                  95
   Asp Met Val Glu Leu His Arg Arg Ala Phe Asp Gly Leu Ala Ala Pro
               100                 105                 110
   Phe Ser Glu Phe Leu Gly Thr Ala Cys Ala Asp Trp Val Ile Val Asp
   115 120 125
   Val Phe His His Trp Ala Ala Ala Ala Ala Leu Glu His Lys Val Pro
   130 135 140
   Cys Ala Met Met Leu Leu Gly Ser Ala His Met Ile Ala Ser Ile Ala
   145 150 155 160
   Asp Arg Arg Leu Glu Arg Ala Glu Thr Glu Ser Pro Ala Ala Ala Gly
   165 170 175
   Gln Gly Arg Pro Ala Ala Ala Pro Thr Phe Glu Val Ala Arg Met Lys
   180 185 190
   Leu Ile Arg Thr Lys Gly Ser Ser Gly Met Ser Leu Ala Glu Arg Phe
   195 200 205
   Ser Leu Thr Leu Ser Arg Ser Ser Leu Val Val Gly Arg Ser Cys Val
   210 215 220
   Glu Phe Glu Pro Glu Thr Val Pro Leu Leu Ser Thr Leu Arg Gly Lys
   225 230 235 240
   Pro Ile Thr Phe Leu Gly Leu Met Pro Pro Leu His Glu Gly Arg Arg
   245 250 255
   Glu Asp Gly Glu Asp Ala Thr Val Arg Trp Leu Asp Ala Gln Pro Ala
   260 265 270
   Lys Ser Val Val Tyr Val Ala Leu Gly Ser Glu Val Pro Leu Gly Val
   275 280 285
   Glu Lys Val His Glu Leu Ala Leu Gly Leu Glu Leu Ala Gly Thr Arg
   290 295 300
   Phe Leu Trp Ala Leu Arg Lys Pro Thr Gly Val Ser Asp Ala Asp Leu
   305 310 315 320
   Leu Pro Ala Gly Phe Glu Glu Arg Thr Arg Gly Arg Gly Val Val Ala
   325 330 335
   Thr Arg Trp Val Pro Gln Met Ser Ile Leu Ala His Ala Ala Val Gly
   340 345 350
   Ala Phe Leu Thr His Cys Gly Trp Asn Ser Thr Ile Glu Gly Leu Met
   355 360 365
   Phe Gly His Pro Leu Ile Met Leu Pro Ile Phe Gly Asp Gln Gly Pro
   370 375 380
   Asn Ala Arg Leu Ile Glu Ala Lys Asn Ala Gly Leu Gln Val Ala Arg
   385 390 395 400
   Asn Asp Gly Asp Gly Ser Phe Asp Arg Glu Gly Val Ala Ala Ala Ile
   405 410 415
   Arg Ala Val Ala Val Glu Glu Glu Ser Ser Lys Val Phe Gln Ala Lys
   420 425 430
   Ala Lys Lys Leu Gln Glu Ile Val Ala Asp Met Ala Cys His Glu Arg
   435 440 445
   Tyr Ile Asp Gly Phe Ile Gln Gln Leu Arg Ser Tyr Lys Asp
   450 455 460
<210> 17
   <211> 465
   <212> PRT
   <213> Artificial Sequence
<220>
   <223> UGT91D2e-b-EUGT11 chimera 3
<400> 17
   Met Asp Ser Gly Tyr Ser Ser Ser Tyr Ala Ala Ala Ala Gly Met His
   1 5 10 15
   Val Val Ile Cys Pro Trp Leu Ala Phe Gly His Leu Leu Pro Cys Leu
   20 25 30
   Asp Leu Ala Gln Arg Leu Ala Ser Arg Gly His Arg Val Ser Phe Val
   35 40 45
   Ser Thr Pro Arg Asn Ile Ser Arg Leu Pro Pro Val Arg Pro Ala Leu
   50 55 60
   Ala Pro Leu Val Ala Phe Val Ala Leu Pro Leu Pro Arg Val Glu Gly
   65 70 75 80
   Leu Pro Asp Gly Ala Glu Ser Thr Asn Asp Val Pro His Asp Arg Pro
   85 90 95
   Asp Met Val Glu Leu His Arg Arg Ala Phe Asp Gly Leu Ala Ala Pro
   100 105 110
   Phe Ser Glu Phe Leu Gly Thr Ala Cys Ala Asp Trp Val Ile Val Asp
           115                 120                 125
   Val Phe His His Trp Ala Ala Ala Ala Ala Leu Glu His Lys Val Pro
       130                 135                 140
   Cys Ala Met Met Leu Leu Gly Ser Ala His Met Ile Ala Ser Ile Ala
   145                 150                 155                 160
   Asp Arg Arg Leu Glu Arg Ala Glu Thr Glu Ser Pro Ala Ala Ala Gly
                   165                 170                 175
   Gln Gly Arg Pro Ala Ala Ala Pro Thr Phe Glu Val Ala Arg Met Lys
               180                 185                 190
   Leu Ile Arg Thr Lys Gly Ser Ser Gly Met Ser Leu Ala Glu Arg Phe
           195                 200                 205
   Ser Leu Thr Leu Ser Arg Ser Ser Leu Val Val Gly Arg Ser Cys Val
       210                 215                 220
   Glu Phe Glu Pro Glu Thr Val Pro Leu Leu Ser Thr Leu Arg Gly Lys
   225                 230                 235                 240
   Pro Ile Thr Phe Leu Gly Leu Leu Pro Pro Glu Ile Pro Gly Asp Glu
                   245                 250                 255
   Lys Asp Glu Thr Trp Val Ser Ile Lys Lys Trp Leu Asp Gly Lys Gln
               260                 265                 270
   Lys Gly Ser Val Val Tyr Val Ala Leu Gly Ser Glu Ala Leu Val Ser
           275                 280                 285
   Gln Thr Glu Val Val Glu Leu Ala Leu Gly Leu Glu Leu Ser Gly Leu
       290                 295                 300
   Pro Phe Val Trp Ala Tyr Arg Lys Pro Lys Gly Pro Ala Lys Ser Asp
   305                 310                 315                 320
   Ser Val Glu Leu Pro Asp Gly Phe Val Glu Arg Thr Arg Asp Arg Gly
                   325                 330                 335
   Leu Val Trp Thr Ser Trp Ala Pro Gln Leu Arg Ile Leu Ser His Glu
               340                 345                 350
   Ser Val Cys Gly Phe Leu Thr His Cys Gly Ser Gly Ser Ile Val Glu
           355                 360                 365
   Gly Leu Met Phe Gly His Pro Leu Ile Met Leu Pro Ile Phe Gly Asp
   370 375 380
   Gln Pro Leu Asn Ala Arg Leu Leu Glu Asp Lys Gln Val Gly Ile Glu
   385 390 395 400
   Ile Ala Arg Asn Asp Gly Asp Gly Ser Phe Asp Arg Glu Gly Val Ala
   405 410 415
   Ala Ala Ile Arg Ala Val Ala Val Glu Glu Glu Ser Ser Lys Val Phe
   420 425 430
   Gln Ala Lys Ala Lys Lys Leu Gln Glu Ile Val Ala Asp Met Ala Cys
   435 440 445
   His Glu Arg Tyr Ile Asp Gly Phe Ile Gln Gln Leu Arg Ser Tyr Lys
   450 455 460
   Asp
   465
<210> 18
   <211> 470
   <212> PRT
   <213> Artificial Sequence
<220>
   <223> UGT91D2e-b-EUGT11 chimera 7
<400> 18
   Met Ala Thr Ser Asp Ser Ile Val Asp Asp Arg Lys Gln Leu His Val
   1 5 10 15
   Ala Thr Phe Pro Trp Leu Ala Phe Gly His Ile Leu Pro Tyr Leu Gln
   20 25 30
   Leu Ser Lys Leu Ile Ala Glu Lys Gly His Lys Val Ser Phe Leu Ser
   35 40 45
   Thr Thr Arg Asn Ile Gln Arg Leu Ser Ser His Ile Ser Pro Leu Ile
   50 55 60
   Asn Val Val Gln Leu Thr Leu Pro Arg Val Gln Glu Leu Pro Glu Asp
   65 70 75 80
   Ala Glu Ala Thr Thr Asp Val His Pro Glu Asp Ile Pro Tyr Leu Lys
   85 90 95
   Lys Ala Ser Asp Gly Leu Gln Pro Glu Val Thr Arg Phe Leu Glu Gln
   100 105 110
   His Ser Pro Asp Trp Ile Ile Tyr Asp Tyr Thr His Tyr Trp Leu Pro
   115 120 125
   Ser Ile Ala Ala Ser Leu Gly Ile Ser Arg Ala His Phe Ser Val Thr
   130 135 140
   Thr Pro Trp Ala Ile Ala Tyr Met Gly Pro Ser Ala Asp Ala Met Ile
   145 150 155 160
   Asn Gly Ser Asp Gly Arg Thr Thr Val Glu Asp Leu Thr Thr Pro Pro
   165 170 175
   Lys Trp Phe Pro Phe Pro Thr Lys Val Cys Trp Arg Lys His Asp Leu
   180 185 190
   Ala Arg Leu Val Pro Tyr Lys Ala Pro Gly Ile Ser Asp Gly Tyr Arg
   195 200 205
   Met Gly Met Val Leu Lys Gly Ser Asp Cys Leu Leu Ser Lys Cys Tyr
   210 215 220
   His Glu Phe Gly Thr Gln Trp Leu Pro Leu Leu Glu Thr Leu His Gln
   225 230 235 240
   Val Pro Val Val Pro Val Gly Leu Met Pro Pro Leu His Glu Gly Arg
   245 250 255
   Arg Glu Asp Gly Glu Asp Ala Thr Val Arg Trp Leu Asp Ala Gln Pro
   260 265 270
   Ala Lys Ser Val Val Tyr Val Ala Leu Gly Ser Glu Val Pro Leu Gly
   275 280 285
   Val Glu Lys Val His Glu Leu Ala Leu Gly Leu Glu Leu Ala Gly Thr
   290 295 300
   Arg Phe Leu Trp Ala Leu Arg Lys Pro Thr Gly Val Ser Asp Ala Asp
   305 310 315 320
   Leu Leu Pro Ala Gly Phe Glu Glu Arg Thr Arg Gly Arg Gly Val Val
   325 330 335
   Ala Thr Arg Trp Val Pro Gln Met Ser Ile Leu Ala His Ala Ala Val
   340 345 350
   Gly Ala Phe Leu Thr His Cys Gly Trp Asn Ser Thr Ile Glu Gly Leu
             355                 360                 365
   Met Phe Gly His Pro Leu Ile Met Leu Pro Ile Phe Gly Asp Gln Gly
       370                 375                 380
   Pro Asn Ala Arg Leu Ile Glu Ala Lys Asn Ala Gly Leu Gln Val Pro
   385                 390                 395                 400
   Arg Asn Glu Glu Asp Gly Cys Leu Thr Lys Glu Ser Val Ala Arg Ser
                   405                 410                 415
   Leu Arg Ser Val Val Val Glu Lys Glu Gly Glu Ile Tyr Lys Ala Asn
               420                 425                 430
   Ala Arg Glu Leu Ser Lys Ile Tyr Asn Asp Thr Lys Val Glu Lys Glu
           435                 440                 445
   Tyr Val Ser Gln Phe Val Asp Tyr Leu Glu Lys Asn Ala Arg Ala Val
       450                 455                 460
   Ala Ile Asp His Glu Ser
   465                 470
<210> 19
   <211> 1086
   <212> DNA
   <213> Artificial Sequence
<220>
   <223> Codon-optimized GGPPS
<400> 19
   atggctttgg taaacccaac cgctcttttc tatggtacct ctatcagaac aagacctaca      60
   aacttactaa atccaactca aaagctaaga ccagtttcat catcttcctt accttctttc     120
   tcatcagtta gtgcgattct tactgaaaaa catcaatcta atccttctga gaacaacaat     180
   ttgcaaactc atctagaaac tcctttcaac tttgatagtt atatgttgga aaaagtcaac     240
   atggttaacg aggcgcttga tgcatctgtc ccactaaaag acccaatcaa aatccatgaa     300
   tccatgagat actctttatt ggcaggcggt aagagaatca gaccaatgat gtgtattgca     360
   gcctgcgaaa tagtcggagg taatatcctt aacgccatgc cagccgcatg tgccgtggaa     420
   atgattcata ctatgtcttt ggtgcatgac gatcttccat gtatggataa tgatgacttc     480
   agaagaggta aacctatttc acacaaggtc tacggggagg aaatggcagt attgaccggc     540
   gatgctttac taagtttatc tttcgaacat atagctactg ctacaaaggg tgtatcaaag     600
   gatagaatcg tcagagctat aggggagttg gcccgttcag ttggctccga aggtttagtg      660
   gctggacaag ttgtagatat cttgtcagag ggtgctgatg ttggattaga tcacctagaa      720
   tacattcaca tccacaaaac agcaatgttg cttgagtcct cagtagttat tggcgctatc      780
   atgggaggag gatctgatca gcagatcgaa aagttgagaa aattcgctag atctattggt      840
   ctactattcc aagttgtgga tgacattttg gatgttacaa aatctaccga agagttgggg      900
   aaaacagctg gtaaggattt gttgacagat aagacaactt acccaaagtt gttaggtata      960
   gaaaagtcca gagaatttgc cgaaaaactt aacaaggaag cacaagagca attaagtggc     1020
   tttgatagac gtaaggcagc tcctttgatc gcgttagcca actacaatgc gtaccgtcaa     1080
   aattga                                                                1086
<210> 20
   <211> 361
   <212> PRT
   <213> Stevia rebaudiana
<400> 20
   Met Ala Leu Val Asn Pro Thr Ala Leu Phe Tyr Gly Thr Ser Ile Arg
   1               5                   10                  15
   Thr Arg Pro Thr Asn Leu Leu Asn Pro Thr Gln Lys Leu Arg Pro Val
               20                  25                  30
   Ser Ser Ser Ser Leu Pro Ser Phe Ser Ser Val Ser Ala Ile Leu Thr
           35                  40                  45
   Glu Lys His Gln Ser Asn Pro Ser Glu Asn Asn Asn Leu Gln Thr His
       50                  55                  60
   Leu Glu Thr Pro Phe Asn Phe Asp Ser Tyr Met Leu Glu Lys Val Asn
   65                  70                  75                  80
   Met Val Asn Glu Ala Leu Asp Ala Ser Val Pro Leu Lys Asp Pro Ile
                   85                  90                  95
   Lys Ile His Glu Ser Met Arg Tyr Ser Leu Leu Ala Gly Gly Lys Arg
               100                 105                 110
   Ile Arg Pro Met Met Cys Ile Ala Ala Cys Glu Ile Val Gly Gly Asn
           115                 120                 125
   Ile Leu Asn Ala Met Pro Ala Ala Cys Ala Val Glu Met Ile His Thr
       130                 135                 140
   Met Ser Leu Val His Asp Asp Leu Pro Cys Met Asp Asn Asp Asp Phe
   145 150 155 160
   Arg Arg Gly Lys Pro Ile Ser His Lys Val Tyr Gly Glu Glu Met Ala
   165 170 175
   Val Leu Thr Gly Asp Ala Leu Leu Ser Leu Ser Phe Glu His Ile Ala
   180 185 190
   Thr Ala Thr Lys Gly Val Ser Lys Asp Arg Ile Val Arg Ala Ile Gly
   195 200 205
   Glu Leu Ala Arg Ser Val Gly Ser Glu Gly Leu Val Ala Gly Gln Val
   210 215 220
   Val Asp Ile Leu Ser Glu Gly Ala Asp Val Gly Leu Asp His Leu Glu
   225 230 235 240
   Tyr Ile His Ile His Lys Thr Ala Met Leu Leu Glu Ser Ser Val Val
   245 250 255
   Ile Gly Ala Ile Met Gly Gly Gly Ser Asp Gln Gln Ile Glu Lys Leu
   260 265 270
   Arg Lys Phe Ala Arg Ser Ile Gly Leu Leu Phe Gln Val Val Asp Asp
   275 280 285
   Ile Leu Asp Val Thr Lys Ser Thr Glu Glu Leu Gly Lys Thr Ala Gly
   290 295 300
   Lys Asp Leu Leu Thr Asp Lys Thr Thr Tyr Pro Lys Leu Leu Gly Ile
   305 310 315 320
   Glu Lys Ser Arg Glu Phe Ala Glu Lys Leu Asn Lys Glu Ala Gln Glu
   325 330 335
   Gln Leu Ser Gly Phe Asp Arg Arg Lys Ala Ala Pro Leu Ile Ala Leu
   340 345 350
   Ala Asn Tyr Asn Ala Tyr Arg Gln Asn
   355 360
<210> 21
   <211> 1029
   <212> DNA
   <213> Artificial Sequence
<220>
   <223> Codon-optimized GGPPS
<400> 21
   atggctgagc aacaaatatc taacttgctg tctatgtttg atgcttcaca tgctagtcag      60
   aaattagaaa ttactgtcca aatgatggac acataccatt acagagaaac gcctccagat     120
   tcctcatctt ctgaaggcgg ttcattgtct agatacgacg agagaagagt ctctttgcct     180
   ctcagtcata atgctgcctc tccagatatt gtatcacaac tatgtttttc cactgcaatg     240
   tcttcagagt tgaatcacag atggaaatct caaagattaa aggtggccga ttctccttac     300
   aactatatcc taacattacc atcaaaagga attagaggtg cctttatcga ttccctgaac     360
   gtatggttgg aggttccaga ggatgaaaca tcagtcatca aggaagttat tggtatgctc     420
   cacaactctt cattaatcat tgatgacttc caagataatt ctccacttag aagaggaaag     480
   ccatctaccc atacagtctt cggccctgcc caggctatca atactgctac ttacgttata     540
   gttaaagcaa tcgaaaagat acaagacata gtgggacacg atgcattggc agatgttacg     600
   ggtactatta caactatttt ccaaggtcag gccatggact tgtggtggac agcaaatgca     660
   atcgttccat caatacagga atacttactt atggtaaacg ataaaaccgg tgctctcttt     720
   agactgagtt tggagttgtt agctctgaat tccgaagcca gtatttctga ctctgcttta     780
   gaaagtttat ctagtgctgt ttccttgcta ggtcaatact tccaaatcag agacgactat     840
   atgaacttga tcgataacaa gtatacagat cagaaaggct tctgcgaaga tcttgatgaa     900
   ggcaagtact cactaacact tattcatgcc ctccaaactg attcatccga tctactgacc     960
   aacatccttt caatgagaag agtgcaagga aagttaacgg cacaaaagag atgttggttc    1020
   tggaaatga                                                            1029
<210> 22
   <211> 342
   <212> PRT
   <213> Gibberella fujikuroi
<400> 22
   Met Ala Glu Gln Gln Ile Ser Asn Leu Leu Ser Met Phe Asp Ala Ser
   1               5                   10                  15
   His Ala Ser Gln Lys Leu Glu Ile Thr Val Gln Met Met Asp Thr Tyr
               20                  25                  30
   His Tyr Arg Glu Thr Pro Pro Asp Ser Ser Ser Ser Glu Gly Gly Ser
           35                  40                  45
   Leu Ser Arg Tyr Asp Glu Arg Arg Val Ser Leu Pro Leu Ser His Asn
       50                  55                  60
   Ala Ala Ser Pro Asp Ile Val Ser Gln Leu Cys Phe Ser Thr Ala Met
   65                  70                  75                  80
   Ser Ser Glu Leu Asn His Arg Trp Lys Ser Gln Arg Leu Lys Val Ala
   85 90 95
   Asp Ser Pro Tyr Asn Tyr Ile Leu Thr Leu Pro Ser Lys Gly Ile Arg
   100 105 110
   Gly Ala Phe Ile Asp Ser Leu Asn Val Trp Leu Glu Val Pro Glu Asp
   115 120 125
   Glu Thr Ser Val Ile Lys Glu Val Ile Gly Met Leu His Asn Ser Ser
   130 135 140
   Leu Ile Ile Asp Asp Phe Gln Asp Asn Ser Pro Leu Arg Arg Gly Lys
   145 150 155 160
   Pro Ser Thr His Thr Val Phe Gly Pro Ala Gln Ala Ile Asn Thr Ala
   165 170 175
   Thr Tyr Val Ile Val Lys Ala Ile Glu Lys Ile Gln Asp Ile Val Gly
   180 185 190
   His Asp Ala Leu Ala Asp Val Thr Gly Thr Ile Thr Thr Ile Phe Gln
   195 200 205
   Gly Gln Ala Met Asp Leu Trp Trp Thr Ala Asn Ala Ile Val Pro Ser
   210 215 220
   Ile Gln Glu Tyr Leu Leu Met Val Asn Asp Lys Thr Gly Ala Leu Phe
   225 230 235 240
   Arg Leu Ser Leu Glu Leu Leu Ala Leu Asn Ser Glu Ala Ser Ile Ser
   245 250 255
   Asp Ser Ala Leu Glu Ser Leu Ser Ser Ala Val Ser Leu Leu Gly Gln
   260 265 270
   Tyr Phe Gln Ile Arg Asp Asp Tyr Met Asn Leu Ile Asp Asn Lys Tyr
   275 280 285
   Thr Asp Gln Lys Gly Phe Cys Glu Asp Leu Asp Glu Gly Lys Tyr Ser
   290 295 300
   Leu Thr Leu Ile His Ala Leu Gln Thr Asp Ser Ser Asp Leu Leu Thr
   305 310 315 320
   Asn Ile Leu Ser Met Arg Arg Val Gln Gly Lys Leu Thr Ala Gln Lys
   325 330 335
   Arg Cys Trp Phe Trp Lys
   340
<210> 23
   <211> 903
   <212> DNA
   <213> Artificial Sequence
<220>
   <223> Codon-optimized GGPPS
<400> 23
   atggaaaaga ctaaggagaa agcagaacgt atcttgctgg agccatacag atacttatta 60
   caactaccag gaaagcaagt ccgttctaaa ctatcacaag cgttcaatca ctggttaaaa 120
   gttcctgaag ataagttaca aatcattatt gaagtcacag aaatgctaca caatgcttct 180
   ttactgatcg atgatataga ggattcttcc aaactgagaa gaggttttcc tgtcgctcat 240
   tccatatacg gggtaccaag tgtaatcaac tcagctaatt acgtctactt cttgggattg 300
   gaaaaagtat tgacattaga tcatccagac gctgtaaagc tattcaccag acaacttctt 360
   gaattgcatc aaggtcaagg tttggatatc tattggagag acacttatac ttgcccaaca 420
   gaagaggagt acaaagcaat ggttctacaa aagactggcg gtttgttcgg acttgccgtt 480
   ggtctgatgc aacttttctc tgattacaag gaggacttaa agcctctgtt ggataccttg 540
   ggcttgtttt tccagattag agatgactac gctaacttac attcaaagga atattcagaa 600
   aacaaatcat tctgtgaaga tttgactgaa gggaagttta gttttccaac aatccacgcc 660
   atttggtcaa gaccagaatc tactcaagtg caaaacattc tgcgtcagag aacagagaat 720
   attgacatca aaaagtattg tgttcagtac ttggaagatg ttggttcttt tgcttacaca 780
   agacatacac ttagagaatt agaggcaaaa gcatacaagc aaatagaagc ctgtggaggc 840
   aatccttctc tagtggcatt ggttaaacat ttgtccaaaa tgttcaccga ggaaaacaag 900
   taa 903
<210> 24
   <211> 300
   <212> PRT
   <213> Mus musculus
<400> 24
   Met Glu Lys Thr Lys Glu Lys Ala Glu Arg Ile Leu Leu Glu Pro Tyr
   1               5                   10                  15
   Arg Tyr Leu Leu Gln Leu Pro Gly Lys Gln Val Arg Ser Lys Leu Ser
               20                  25                  30
   Gln Ala Phe Asn His Trp Leu Lys Val Pro Glu Asp Lys Leu Gln Ile
           35                  40                  45
   Ile Ile Glu Val Thr Glu Met Leu His Asn Ala Ser Leu Leu Ile Asp
       50                  55                  60
   Asp Ile Glu Asp Ser Ser Lys Leu Arg Arg Gly Phe Pro Val Ala His
   65                  70                  75                  80
   Ser Ile Tyr Gly Val Pro Ser Val Ile Asn Ser Ala Asn Tyr Val Tyr
                   85                  90                  95
   Phe Leu Gly Leu Glu Lys Val Leu Thr Leu Asp His Pro Asp Ala Val
               100                 105                 110
   Lys Leu Phe Thr Arg Gln Leu Leu Glu Leu His Gln Gly Gln Gly Leu
           115                 120                 125
   Asp Ile Tyr Trp Arg Asp Thr Tyr Thr Cys Pro Thr Glu Glu Glu Tyr
       130                 135                 140
   Lys Ala Met Val Leu Gln Lys Thr Gly Gly Leu Phe Gly Leu Ala Val
   145                 150                 155                 160
   Gly Leu Met Gln Leu Phe Ser Asp Tyr Lys Glu Asp Leu Lys Pro Leu
                   165                 170                 175
   Leu Asp Thr Leu Gly Leu Phe Phe Gln Ile Arg Asp Asp Tyr Ala Asn
               180                 185                 190
   Leu His Ser Lys Glu Tyr Ser Glu Asn Lys Ser Phe Cys Glu Asp Leu
           195                 200                 205
   Thr Glu Gly Lys Phe Ser Phe Pro Thr Ile His Ala Ile Trp Ser Arg
       210                 215                 220
   Pro Glu Ser Thr Gln Val Gln Asn Ile Leu Arg Gln Arg Thr Glu Asn
   225                 230                 235                 240
   Ile Asp Ile Lys Lys Tyr Cys Val Gln Tyr Leu Glu Asp Val Gly Ser
                   245                 250                 255
   Phe Ala Tyr Thr Arg His Thr Leu Arg Glu Leu Glu Ala Lys Ala Tyr
               260                 265                 270
   Lys Gln Ile Glu Ala Cys Gly Gly Asn Pro Ser Leu Val Ala Leu Val
           275                 280                 285
   Lys His Leu Ser Lys Met Phe Thr Glu Glu Asn Lys
   290 295 300
<210> 25
   <211> 1020
   <212> DNA
   <213> Artificial Sequence
<220>
   <223> Codon-optimized GGPPS
<400> 25
   atggcaagat tctattttct taacgcacta ttgatggtta tctcattaca atcaactaca 60
   gccttcactc cagctaaact tgcttatcca acaacaacaa cagctctaaa tgtcgcctcc 120
   gccgaaactt ctttcagtct agatgaatac ttggcctcta agataggacc tatagagtct 180
   gccttggaag catcagtcaa atccagaatt ccacagaccg ataagatctg cgaatctatg 240
   gcctactctt tgatggcagg aggcaagaga attagaccag tgttgtgtat cgctgcatgt 300
   gagatgttcg gtggatccca agatgtcgct atgcctactg ctgtggcatt agaaatgata 360
   cacacaatgt ctttgattca tgatgatttg ccatccatgg ataacgatga cttgagaaga 420
   ggtaaaccaa caaaccatgt cgttttcggc gaagatgtag ctattcttgc aggtgactct 480
   ttattgtcaa cttccttcga gcacgtcgct agagaaacaa aaggagtgtc agcagaaaag 540
   atcgtggatg ttatcgctag attaggcaaa tctgttggtg ccgagggcct tgctggcggt 600
   caagttatgg acttagaatg tgaagctaaa ccaggtacca cattagacga cttgaaatgg 660
   attcatatcc ataaaaccgc tacattgtta caagttgctg tagcttctgg tgcagttcta 720
   ggtggtgcaa ctcctgaaga ggttgctgca tgcgagttgt ttgctatgaa tataggtctt 780
   gcctttcaag ttgccgacga tatccttgat gtaaccgctt catcagaaga tttgggtaaa 840
   actgcaggca aagatgaagc tactgataag acaacttacc caaagttatt aggattagaa 900
   gagagtaagg catacgcaag acaactaatc gatgaagcca aggaaagttt ggctcctttt 960
   ggagatagag ctgccccttt attggccatt gcagatttca ttattgatag aaagaattga 1020
<210> 26
   <211> 339
   <212> PRT
   <213> Thalassiosira pseudonana
<400> 26
   Met Ala Arg Phe Tyr Phe Leu Asn Ala Leu Leu Met Val Ile Ser Leu
   1               5                   10                  15
   Gln Ser Thr Thr Ala Phe Thr Pro Ala Lys Leu Ala Tyr Pro Thr Thr
               20                  25                  30
   Thr Thr Ala Leu Asn Val Ala Ser Ala Glu Thr Ser Phe Ser Leu Asp
           35                  40                  45
   Glu Tyr Leu Ala Ser Lys Ile Gly Pro Ile Glu Ser Ala Leu Glu Ala
       50                  55                  60
   Ser Val Lys Ser Arg Ile Pro Gln Thr Asp Lys Ile Cys Glu Ser Met
   65                  70                  75                  80
   Ala Tyr Ser Leu Met Ala Gly Gly Lys Arg Ile Arg Pro Val Leu Cys
                   85                  90                  95
   Ile Ala Ala Cys Glu Met Phe Gly Gly Ser Gln Asp Val Ala Met Pro
               100                 105                 110
   Thr Ala Val Ala Leu Glu Met Ile His Thr Met Ser Leu Ile His Asp
           115                 120                 125
   Asp Leu Pro Ser Met Asp Asn Asp Asp Leu Arg Arg Gly Lys Pro Thr
       130                 135                 140
   Asn His Val Val Phe Gly Glu Asp Val Ala Ile Leu Ala Gly Asp Ser
   145                 150                 155                 160
   Leu Leu Ser Thr Ser Phe Glu His Val Ala Arg Glu Thr Lys Gly Val
                   165                 170                 175
   Ser Ala Glu Lys Ile Val Asp Val Ile Ala Arg Leu Gly Lys Ser Val
               180                 185                 190
   Gly Ala Glu Gly Leu Ala Gly Gly Gln Val Met Asp Leu Glu Cys Glu
           195                 200                 205
   Ala Lys Pro Gly Thr Thr Leu Asp Asp Leu Lys Trp Ile His Ile His
       210                 215                 220
   Lys Thr Ala Thr Leu Leu Gln Val Ala Val Ala Ser Gly Ala Val Leu
   225                 230                 235                 240
   Gly Gly Ala Thr Pro Glu Glu Val Ala Ala Cys Glu Leu Phe Ala Met
                   245                 250                 255
   Asn Ile Gly Leu Ala Phe Gln Val Ala Asp Asp Ile Leu Asp Val Thr
               260                 265                 270
   Ala Ser Ser Glu Asp Leu Gly Lys Thr Ala Gly Lys Asp Glu Ala Thr
   275 280 285
   Asp Lys Thr Thr Tyr Pro Lys Leu Leu Gly Leu Glu Glu Ser Lys Ala
   290 295 300
   Tyr Ala Arg Gln Leu Ile Asp Glu Ala Lys Glu Ser Leu Ala Pro Phe
   305 310 315 320
   Gly Asp Arg Ala Ala Pro Leu Leu Ala Ile Ala Asp Phe Ile Ile Asp
   325 330 335
   Arg Lys Asn
<210> 27
   <211> 1068
   <212> DNA
   <213> Artificial Sequence
<220>
   <223> Codon-optimized GGPPS
<400> 27
   atgcacttag caccacgtag agtccctaga ggtagaagat caccacctga cagagttcct      60
   gaaagacaag gtgccttggg tagaagacgt ggagctggct ctactggctg tgcccgtgct     120
   gctgctggtg ttcaccgtag aagaggagga ggcgaggctg atccatcagc tgctgtgcat     180
   agaggctggc aagccggtgg tggcaccggt ttgcctgatg aggtggtgtc taccgcagcc     240
   gccttagaaa tgtttcatgc ttttgcttta atccatgatg atatcatgga tgatagtgca     300
   actagaagag gctccccaac tgttcacaga gccctagctg atcgtttagg cgctgctctg     360
   gacccagatc aggccggtca actaggagtt tctactgcta tcttggttgg agatctggct     420
   ttgacatggt ccgatgaatt gttatacgct ccattgactc cacatagact ggcagcagta     480
   ctaccattgg taacagctat gagagctgaa accgttcatg gccaatatct tgatataact     540
   agtgctagaa gacctgggac cgatacttct cttgcattga gaatagccag atataagaca     600
   gcagcttaca caatggaacg tccactgcac attggtgcag ccctggctgg ggcaagacca     660
   gaactattag cagggctttc agcatacgcc ttgccagctg gagaagcctt ccaattggca     720
   gatgacctgc taggcgtctt cggtgatcca agacgtacag ggaaacctga cctagatgat     780
   cttagaggtg gaaagcatac tgtcttagtc gccttggcaa gagaacatgc cactccagaa     840
   cagagacaca cattggatac attattgggt acaccaggtc ttgatagaca aggcgcttca     900
   agactaagat gcgtattggt agcaactggt gcaagagccg aagccgaaag acttattaca     960
   gagagaagag atcaagcatt aactgcattg aacgcattaa cactgccacc tcctttagct    1020
   gaggcattag caagattgac attagggtct acagctcatc ctgcctaa                 1068
<210> 28
   <211> 355
   <212> PRT
   <213> Streptomyces clavuligerus
<400> 28
   Met His Leu Ala Pro Arg Arg Val Pro Arg Gly Arg Arg Ser Pro Pro
   1               5                   10                  15
   Asp Arg Val Pro Glu Arg Gln Gly Ala Leu Gly Arg Arg Arg Gly Ala
               20                  25                  30
   Gly Ser Thr Gly Cys Ala Arg Ala Ala Ala Gly Val His Arg Arg Arg
           35                  40                  45
   Gly Gly Gly Glu Ala Asp Pro Ser Ala Ala Val His Arg Gly Trp Gln
       50                  55                  60
   Ala Gly Gly Gly Thr Gly Leu Pro Asp Glu Val Val Ser Thr Ala Ala
   65                  70                  75                  80
   Ala Leu Glu Met Phe His Ala Phe Ala Leu Ile His Asp Asp Ile Met
                   85                  90                  95
   Asp Asp Ser Ala Thr Arg Arg Gly Ser Pro Thr Val His Arg Ala Leu
               100                 105                 110
   Ala Asp Arg Leu Gly Ala Ala Leu Asp Pro Asp Gln Ala Gly Gln Leu
           115                 120                 125
   Gly Val Ser Thr Ala Ile Leu Val Gly Asp Leu Ala Leu Thr Trp Ser
       130                 135                 140
   Asp Glu Leu Leu Tyr Ala Pro Leu Thr Pro His Arg Leu Ala Ala Val
   145                 150                 155                 160
   Leu Pro Leu Val Thr Ala Met Arg Ala Glu Thr Val His Gly Gln Tyr
                   165                 170                 175
   Leu Asp Ile Thr Ser Ala Arg Arg Pro Gly Thr Asp Thr Ser Leu Ala
               180                 185                 190
   Leu Arg Ile Ala Arg Tyr Lys Thr Ala Ala Tyr Thr Met Glu Arg Pro
           195                 200                 205
   Leu His Ile Gly Ala Ala Leu Ala Gly Ala Arg Pro Glu Leu Leu Ala
       210                 215                 220
   Gly Leu Ser Ala Tyr Ala Leu Pro Ala Gly Glu Ala Phe Gln Leu Ala
   225                 230                 235                 240
   Asp Asp Leu Leu Gly Val Phe Gly Asp Pro Arg Arg Thr Gly Lys Pro
                   245                 250                 255
   Asp Leu Asp Asp Leu Arg Gly Gly Lys His Thr Val Leu Val Ala Leu
               260                 265                 270
   Ala Arg Glu His Ala Thr Pro Glu Gln Arg His Thr Leu Asp Thr Leu
           275                 280                 285
   Leu Gly Thr Pro Gly Leu Asp Arg Gln Gly Ala Ser Arg Leu Arg Cys
       290                 295                 300
   Val Leu Val Ala Thr Gly Ala Arg Ala Glu Ala Glu Arg Leu Ile Thr
   305                 310                 315                 320
   Glu Arg Arg Asp Gln Ala Leu Thr Ala Leu Asn Ala Leu Thr Leu Pro
                   325                 330                 335
   Pro Pro Leu Ala Glu Ala Leu Ala Arg Leu Thr Leu Gly Ser Thr Ala
               340                 345                 350
   His Pro Ala
           355
<210> 29
   <211> 993
   <212> DNA
   <213> Artificial Sequence
<220>
   <223> Codon-optimized GGPPS
<400> 29
   atgtcatatt tcgataacta cttcaatgag atagttaatt ccgtgaacga catcattaag      60
   tcttacatct ctggcgacgt accaaaacta tacgaagcct cctaccattt gtttacatca     120
   ggaggaaaga gactaagacc attgatcctt acaatttctt ctgatctttt cggtggacag     180
   agagaaagag catactatgc tggcgcagca atcgaagttt tgcacacatt cactttggtt     240
   cacgatgata tcatggatca agataacatt cgtagaggtc ttcctactgt acatgtcaag     300
   tatggcctac ctttggccat tttagctggt gacttattgc atgcaaaagc ctttcaattg     360
   ttgactcagg cattgagagg tctaccatct gaaactatca tcaaggcgtt tgatatcttt     420
   acaagatcta tcattatcat atcagaaggt caagctgtcg atatggaatt cgaagataga     480
   attgatatca aggaacaaga gtatttggat atgatatctc gtaaaaccgc tgccttattc      540
   tcagcttctt cttccattgg ggcgttgata gctggagcta atgataacga tgtgagatta      600
   atgtccgatt tcggtacaaa tcttgggatc gcatttcaaa ttgtagatga tatacttggt      660
   ttaacagctg atgaaaaaga gctaggaaaa cctgttttca gtgatatcag agaaggtaaa      720
   aagaccatat tagtcattaa gactttagaa ttgtgtaagg aagacgagaa aaagattgtg      780
   ttaaaagcgc taggcaacaa gtcagcatca aaggaagagt tgatgagttc tgctgacata      840
   atcaaaaagt actcattgga ttacgcctac aacttagctg agaaatacta caaaaacgcc      900
   atcgattctc taaatcaagt ttcaagtaaa agtgatattc cagggaaggc attgaaatat      960
   cttgctgaat tcaccatcag aagacgtaag taa                                   993
<210> 30
   <211> 330
   <212> PRT
   <213> Sulfolobus acidocaldarius
<400> 30
   Met Ser Tyr Phe Asp Asn Tyr Phe Asn Glu Ile Val Asn Ser Val Asn
   1               5                   10                  15
   Asp Ile Ile Lys Ser Tyr Ile Ser Gly Asp Val Pro Lys Leu Tyr Glu
               20                  25                  30
   Ala Ser Tyr His Leu Phe Thr Ser Gly Gly Lys Arg Leu Arg Pro Leu
           35                  40                  45
   Ile Leu Thr Ile Ser Ser Asp Leu Phe Gly Gly Gln Arg Glu Arg Ala
       50                  55                  60
   Tyr Tyr Ala Gly Ala Ala Ile Glu Val Leu His Thr Phe Thr Leu Val
   65                  70                  75                  80
   His Asp Asp Ile Met Asp Gln Asp Asn Ile Arg Arg Gly Leu Pro Thr
                   85                  90                  95
   Val His Val Lys Tyr Gly Leu Pro Leu Ala Ile Leu Ala Gly Asp Leu
               100                 105                 110
   Leu His Ala Lys Ala Phe Gln Leu Leu Thr Gln Ala Leu Arg Gly Leu
           115                 120                 125
   Pro Ser Glu Thr Ile Ile Lys Ala Phe Asp Ile Phe Thr Arg Ser Ile
       130                 135                 140
   Ile Ile Ile Ser Glu Gly Gln Ala Val Asp Met Glu Phe Glu Asp Arg
   145 150 155 160
   Ile Asp Ile Lys Glu Gln Glu Tyr Leu Asp Met Ile Ser Arg Lys Thr
   165 170 175
   Ala Ala Leu Phe Ser Ala Ser Ser Ser Ile Gly Ala Leu Ile Ala Gly
   180 185 190
   Ala Asn Asp Asn Asp Val Arg Leu Met Ser Asp Phe Gly Thr Asn Leu
   195 200 205
   Gly Ile Ala Phe Gln Ile Val Asp Asp Ile Leu Gly Leu Thr Ala Asp
   210 215 220
   Glu Lys Glu Leu Gly Lys Pro Val Phe Ser Asp Ile Arg Glu Gly Lys
   225 230 235 240
   Lys Thr Ile Leu Val Ile Lys Thr Leu Glu Leu Cys Lys Glu Asp Glu
   245 250 255
   Lys Lys Ile Val Leu Lys Ala Leu Gly Asn Lys Ser Ala Ser Lys Glu
   260 265 270
   Glu Leu Met Ser Ser Ala Asp Ile Ile Lys Lys Tyr Ser Leu Asp Tyr
   275 280 285
   Ala Tyr Asn Leu Ala Glu Lys Tyr Tyr Lys Asn Ala Ile Asp Ser Leu
   290 295 300
   Asn Gln Val Ser Ser Lys Ser Asp Ile Pro Gly Lys Ala Leu Lys Tyr
   305 310 315 320
   Leu Ala Glu Phe Thr Ile Arg Arg Arg Lys
   325 330
<210> 31
   <211> 894
   <212> DNA
   <213> Artificial Sequence
<220>
   <223> Codon-optimized GGPPS
<400> 31
   atggtcgcac aaactttcaa cctggatacc tacttatccc aaagacaaca acaagttgaa       60
   gaggccctaa gtgctgctct tgtgccagct tatcctgaga gaatatacga agctatgaga      120
   tactccctcc tggcaggtgg caaaagatta agacctatct tatgtttagc tgcttgcgaa      180
   ttggcaggtg gttctgttga acaagccatg ccaactgcgt gtgcacttga aatgatccat      240
   acaatgtcac taattcatga tgacctgcca gccatggata acgatgattt cagaagagga      300
   aagccaacta atcacaaggt gttcggggaa gatatagcca tcttagcggg tgatgcgctt      360
   ttagcttacg cttttgaaca tattgcttct caaacaagag gagtaccacc tcaattggtg      420
   ctacaagtta ttgctagaat cggacacgcc gttgctgcaa caggcctcgt tggaggccaa      480
   gtcgtagacc ttgaatctga aggtaaagct atttccttag aaacattgga gtatattcac      540
   tcacataaga ctggagcctt gctggaagca tcagttgtct caggcggtat tctcgcaggg      600
   gcagatgaag agcttttggc cagattgtct cattacgcta gagatatagg cttggctttt      660
   caaatcgtcg atgatatcct ggatgttact gctacatctg aacagttggg gaaaaccgct      720
   ggtaaagacc aggcagccgc aaaggcaact tatccaagtc tattgggttt agaagcctct      780
   agacagaaag cggaagagtt gattcaatct gctaaggaag ccttaagacc ttacggttca      840
   caagcagagc cactcctagc gctggcagac ttcatcacac gtcgtcagca ttaa            894
<210> 32
   <211> 297
   <212> PRT
   <213> Synechococcus sp.
<400> 32
   Met Val Ala Gln Thr Phe Asn Leu Asp Thr Tyr Leu Ser Gln Arg Gln
   1               5                   10                  15
   Gln Gln Val Glu Glu Ala Leu Ser Ala Ala Leu Val Pro Ala Tyr Pro
               20                  25                  30
   Glu Arg Ile Tyr Glu Ala Met Arg Tyr Ser Leu Leu Ala Gly Gly Lys
           35                  40                  45
   Arg Leu Arg Pro Ile Leu Cys Leu Ala Ala Cys Glu Leu Ala Gly Gly
       50                  55                  60
   Ser Val Glu Gln Ala Met Pro Thr Ala Cys Ala Leu Glu Met Ile His
   65                  70                  75                  80
   Thr Met Ser Leu Ile His Asp Asp Leu Pro Ala Met Asp Asn Asp Asp
                   85                  90                  95
   Phe Arg Arg Gly Lys Pro Thr Asn His Lys Val Phe Gly Glu Asp Ile
               100                 105                 110
   Ala Ile Leu Ala Gly Asp Ala Leu Leu Ala Tyr Ala Phe Glu His Ile
           115                 120                 125
   Ala Ser Gln Thr Arg Gly Val Pro Pro Gln Leu Val Leu Gln Val Ile
       130                 135                 140
   Ala Arg Ile Gly His Ala Val Ala Ala Thr Gly Leu Val Gly Gly Gln
   145                 150                 155                 160
   Val Val Asp Leu Glu Ser Glu Gly Lys Ala Ile Ser Leu Glu Thr Leu
                   165                 170                 175
   Glu Tyr Ile His Ser His Lys Thr Gly Ala Leu Leu Glu Ala Ser Val
               180                 185                 190
   Val Ser Gly Gly Ile Leu Ala Gly Ala Asp Glu Glu Leu Leu Ala Arg
           195                 200                 205
   Leu Ser His Tyr Ala Arg Asp Ile Gly Leu Ala Phe Gln Ile Val Asp
       210                 215                 220
   Asp Ile Leu Asp Val Thr Ala Thr Ser Glu Gln Leu Gly Lys Thr Ala
   225                 230                 235                 240
   Gly Lys Asp Gln Ala Ala Ala Lys Ala Thr Tyr Pro Ser Leu Leu Gly
                   245                 250                 255
   Leu Glu Ala Ser Arg Gln Lys Ala Glu Glu Leu Ile Gln Ser Ala Lys
               260                 265                 270
   Glu Ala Leu Arg Pro Tyr Gly Ser Gln Ala Glu Pro Leu Leu Ala Leu
           275                 280                 285
   Ala Asp Phe Ile Thr Arg Arg Gln His
       290                 295
<210> 33
   <211> 1680
   <212> DNA
   <213> Artificial Sequence
<220>
   <223> Codon-optimized CDPS
<400> 33
   atgaaaaccg ggtttatctc accagcaaca gtatttcatc acagaatctc accagcgacc      60
   actttcagac atcacttatc acctgctact acaaactcta caggcattgt cgccttaaga     120
   gacatcaact tcagatgtaa agcagtttct aaagagtact ctgatctgtt gcagaaagat     180
   gaggcttctt tcacaaaatg ggacgatgac aaggtgaaag atcatcttga taccaacaaa     240
   aacttatacc caaatgatga gattaaggaa tttgttgaat cagtaaaggc tatgttcggt     300
   agtatgaatg acggggagat aaacgtctct gcatacgata ctgcatgggt tgctttggtt     360
   caagatgtcg atggatcagg tagtcctcag ttcccttctt ctttagaatg gattgccaac     420
   aatcaattgt cagatggatc atggggagat catttgctgt tctcagctca cgatagaatc     480
   atcaacacat tagcatgcgt tattgcactt acaagttgga atgttcatcc ttctaagtgt     540
   gaaaaaggtt tgaattttct gagagaaaac atttgcaaat tagaagatga aaacgcagaa     600
   catatgccaa ttggttttga agtaacattc ccatcactaa ttgatatcgc gaaaaagttg     660
   aacattgaag tacctgagga tactccagca cttaaagaga tctacgcacg tagagatatc     720
   aagttaacta agatcccaat ggaagttctt cacaaggtac ctactacttt gttacattct     780
   ttggaaggaa tgcctgattt ggagtgggaa aaactgttaa agctacaatg taaagatggt     840
   agtttcttgt tttccccatc tagtaccgca ttcgccctaa tgcaaacaaa agatgagaaa     900
   tgcttacagt atctaacaaa tatcgtcact aagttcaacg gtggcgtgcc taatgtgtac     960
   ccagtcgatt tgtttgaaca tatttgggtt gttgatagac tgcagagatt ggggattgcc    1020
   agatacttca aatcagagat aaaagattgt gtagagtata tcaataagta ctggaccaaa    1080
   aatggaattt gttgggctag aaatactcac gttcaagata tcgatgatac agccatggga    1140
   ttcagagtgt tgagagcgca cggttatgac gtcactccag atgtttttag acaatttgaa    1200
   aaagatggta aattcgtttg ctttgcaggg caatcaacac aagccgtgac aggaatgttt    1260
   aacgtttaca gagcctctca aatgttgttc ccaggggaga gaattttgga agatgccaaa    1320
   aagttctctt acaattactt aaaggaaaag caaagtacca acgaattgct ggataaatgg    1380
   ataatcgcta aagatctacc tggtgaagtt ggttatgctc tggatatccc atggtatgct    1440
   tccttaccaa gattggaaac tcgttattac cttgaacaat acggcggtga agatgatgtc    1500
   tggataggca agacattata cagaatgggt tacgtgtcca ataacacata tctagaaatg    1560
   gcaaagctgg attacaataa ctatgttgca gtccttcaat tagaatggta cacaatacaa    1620
   caatggtacg tcgatattgg tatagagaag ttcgaatctg acaacatcaa gtcagtcctg    1680
<210> 34
   <211> 787
   <212> PRT
   <213> Stevia rebaudiana
<400> 34
   Met Lys Thr Gly Phe Ile Ser Pro Ala Thr Val Phe His His Arg Ile
   1               5                   10                  15
   Ser Pro Ala Thr Thr Phe Arg His His Leu Ser Pro Ala Thr Thr Asn
               20                  25                  30
   Ser Thr Gly Ile Val Ala Leu Arg Asp Ile Asn Phe Arg Cys Lys Ala
   35 40 45
   Val Ser Lys Glu Tyr Ser Asp Leu Leu Gln Lys Asp Glu Ala Ser Phe
   50 55 60
   Thr Lys Trp Asp Asp Asp Lys Val Lys Asp His Leu Asp Thr Asn Lys
   65 70 75 80
   Asn Leu Tyr Pro Asn Asp Glu Ile Lys Glu Phe Val Glu Ser Val Lys
   85 90 95
   Ala Met Phe Gly Ser Met Asn Asp Gly Glu Ile Asn Val Ser Ala Tyr
   100 105 110
   Asp Thr Ala Trp Val Ala Leu Val Gln Asp Val Asp Gly Ser Gly Ser
   115 120 125
   Pro Gln Phe Pro Ser Ser Leu Glu Trp Ile Ala Asn Asn Gln Leu Ser
   130 135 140
   Asp Gly Ser Trp Gly Asp His Leu Leu Phe Ser Ala His Asp Arg Ile
   145 150 155 160
   Ile Asn Thr Leu Ala Cys Val Ile Ala Leu Thr Ser Trp Asn Val His
   165 170 175
   Pro Ser Lys Cys Glu Lys Gly Leu Asn Phe Leu Arg Glu Asn Ile Cys
   180 185 190
   Lys Leu Glu Asp Glu Asn Ala Glu His Met Pro Ile Gly Phe Glu Val
   195 200 205
   Thr Phe Pro Ser Leu Ile Asp Ile Ala Lys Lys Leu Asn Ile Glu Val
   210 215 220
   Pro Glu Asp Thr Pro Ala Leu Lys Glu Ile Tyr Ala Arg Arg Asp Ile
   225 230 235 240
   Lys Leu Thr Lys Ile Pro Met Glu Val Leu His Lys Val Pro Thr Thr
   245 250 255
   Leu Leu His Ser Leu Glu Gly Met Pro Asp Leu Glu Trp Glu Lys Leu
   260 265 270
   Leu Lys Leu Gln Cys Lys Asp Gly Ser Phe Leu Phe Ser Pro Ser Ser
   275 280 285
   Thr Ala Phe Ala Leu Met Gln Thr Lys Asp Glu Lys Cys Leu Gln Tyr
       290                 295                 300
   Leu Thr Asn Ile Val Thr Lys Phe Asn Gly Gly Val Pro Asn Val Tyr
   305                 310                 315                 320
   Pro Val Asp Leu Phe Glu His Ile Trp Val Val Asp Arg Leu Gln Arg
                   325                 330                 335
   Leu Gly Ile Ala Arg Tyr Phe Lys Ser Glu Ile Lys Asp Cys Val Glu
               340                 345                 350
   Tyr Ile Asn Lys Tyr Trp Thr Lys Asn Gly Ile Cys Trp Ala Arg Asn
           355                 360                 365
   Thr His Val Gln Asp Ile Asp Asp Thr Ala Met Gly Phe Arg Val Leu
       370                 375                 380
   Arg Ala His Gly Tyr Asp Val Thr Pro Asp Val Phe Arg Gln Phe Glu
   385                 390                 395                 400
   Lys Asp Gly Lys Phe Val Cys Phe Ala Gly Gln Ser Thr Gln Ala Val
                   405                 410                 415
   Thr Gly Met Phe Asn Val Tyr Arg Ala Ser Gln Met Leu Phe Pro Gly
               420                 425                 430
   Glu Arg Ile Leu Glu Asp Ala Lys Lys Phe Ser Tyr Asn Tyr Leu Lys
           435                 440                 445
   Glu Lys Gln Ser Thr Asn Glu Leu Leu Asp Lys Trp Ile Ile Ala Lys
       450                 455                 460
   Asp Leu Pro Gly Glu Val Gly Tyr Ala Leu Asp Ile Pro Trp Tyr Ala
   465                 470                 475                 480
   Ser Leu Pro Arg Leu Glu Thr Arg Tyr Tyr Leu Glu Gln Tyr Gly Gly
                   485                 490                 495
   Glu Asp Asp Val Trp Ile Gly Lys Thr Leu Tyr Arg Met Gly Tyr Val
               500                 505                 510
   Ser Asn Asn Thr Tyr Leu Glu Met Ala Lys Leu Asp Tyr Asn Asn Tyr
           515                 520                 525
   Val Ala Val Leu Gln Leu Glu Trp Tyr Thr Ile Gln Gln Trp Tyr Val
       530                 535                 540
   Asp Ile Gly Ile Glu Lys Phe Glu Ser Asp Asn Ile Lys Ser Val Leu
   545                 550                 555                 560
   Val Ser Tyr Tyr Leu Ala Ala Ala Ser Ile Phe Glu Pro Glu Arg Ser
                   565                 570                 575
   Lys Glu Arg Ile Ala Trp Ala Lys Thr Thr Ile Leu Val Asp Lys Ile
               580                 585                 590
   Thr Ser Ile Phe Asp Ser Ser Gln Ser Ser Lys Glu Asp Ile Thr Ala
           595                 600                 605
   Phe Ile Asp Lys Phe Arg Asn Lys Ser Ser Ser Lys Lys His Ser Ile
       610                 615                 620
   Asn Gly Glu Pro Trp His Glu Val Met Val Ala Leu Lys Lys Thr Leu
   625                 630                 635                 640
   His Gly Phe Ala Leu Asp Ala Leu Met Thr His Ser Gln Asp Ile His
                   645                 650                 655
   Pro Gln Leu His Gln Ala Trp Glu Met Trp Leu Thr Lys Leu Gln Asp
               660                 665                 670
   Gly Val Asp Val Thr Ala Glu Leu Met Val Gln Met Ile Asn Met Thr
           675                 680                 685
   Ala Gly Arg Trp Val Ser Lys Glu Leu Leu Thr His Pro Gln Tyr Gln
       690                 695                 700
   Arg Leu Ser Thr Val Thr Asn Ser Val Cys His Asp Ile Thr Lys Leu
   705                 710                 715                 720
   His Asn Phe Lys Glu Asn Ser Thr Thr Val Asp Ser Lys Val Gln Glu
                   725                 730                 735
   Leu Val Gln Leu Val Phe Ser Asp Thr Pro Asp Asp Leu Asp Gln Asp
               740                 745                 750
   Met Lys Gln Thr Phe Leu Thr Val Met Lys Thr Phe Tyr Tyr Lys Ala
           755                 760                 765
   Trp Cys Asp Pro Asn Thr Ile Asn Asp His Ile Ser Lys Val Phe Glu
       770                 775                 780
   Ile Val Ile
   785
<210> 35
   <211> 1584
   <212> DNA
   <213> Artificial Sequence
<220>
   <223> Codon-optimized CDPS
<400> 35
   atgcctgatg cacacgatgc tccacctcca caaataagac agagaacact agtagatgag      60
   gctacccaac tgctaactga gtccgcagaa gatgcatggg gtgaagtcag tgtgtcagaa     120
   tacgaaacag caaggctagt tgcccatgct acatggttag gtggacacgc cacaagagtg     180
   gccttccttc tggagagaca acacgaagac gggtcatggg gtccaccagg tggatatagg     240
   ttagtcccta cattatctgc tgttcacgca ttattgacat gtcttgcctc tcctgctcag     300
   gatcatggcg ttccacatga tagactttta agagctgttg acgcaggctt gactgccttg     360
   agaagattgg ggacatctga ctccccacct gatactatag cagttgagct ggttatccca     420
   tctttgctag agggcattca acacttactg gaccctgctc atcctcatag tagaccagcc     480
   ttctctcaac atagaggctc tcttgtttgt cctggtggac tagatgggag aactctagga     540
   gctttgagat cacacgccgc agcaggtaca ccagtaccag gaaaagtctg gcacgcttcc     600
   gagactttgg gcttgagtac cgaagctgct tctcacttgc aaccagccca aggtataatc     660
   ggtggctctg ctgctgccac agcaacatgg ctaaccaggg ttgcaccatc tcaacagtca     720
   gattctgcca gaagatacct tgaggaatta caacacagat actctggccc agttccttcc     780
   attaccccta tcacatactt cgaaagagca tggttattga acaattttgc agcagccggt     840
   gttccttgtg aggctccagc tgctttgttg gattccttag aagcagcact tacaccacaa     900
   ggtgctcctg ctggagcagg attgcctcca gatgctgatg atacagccgc tgtgttgctt     960
   gcattggcaa cacatgggag aggtagaaga ccagaagtac tgatggatta caggactgac    1020
   gggtatttcc aatgctttat tggggaaagg actccatcaa tttcaacaaa cgctcacgta    1080
   ttggaaacat tagggcatca tgtggcccaa catccacaag atagagccag atacggatca    1140
   gccatggata ccgcatcagc ttggctgctg gcagctcaaa agcaagatgg ctcttggtta    1200
   gataaatggc atgcctcacc atactacgct actgtttgtt gcacacaagc cctagccgct    1260
   catgcaagtc ctgcaactgc accagctaga cagagagctg tcagatgggt tttagccaca    1320
   caaagatccg atggcggttg gggtctatgg cattcaactg ttgaagagac tgcttatgcc    1380
   ttacagatct tggccccacc ttctggtggt ggcaatatcc cagtccaaca agcacttact    1440
   agaggcagag caagattgtg tggagccttg ccactgactc ctttatggca tgataaggat    1500
   ttgtatactc cagtaagagt agtcagagct gccagagctg ctgctctgta cactaccaga    1560
   gatctattgt taccaccatt gtaa 1584
<210> 36
   <211> 527
   <212> PRT
   <213> Streptomyces clavuligerus
<400> 36
   Met Pro Asp Ala His Asp Ala Pro Pro Pro Gln Ile Arg Gln Arg Thr
   1               5                   10                  15
   Leu Val Asp Glu Ala Thr Gln Leu Leu Thr Glu Ser Ala Glu Asp Ala
               20                  25                  30
   Trp Gly Glu Val Ser Val Ser Glu Tyr Glu Thr Ala Arg Leu Val Ala
           35                  40                  45
   His Ala Thr Trp Leu Gly Gly His Ala Thr Arg Val Ala Phe Leu Leu
       50                  55                  60
   Glu Arg Gln His Glu Asp Gly Ser Trp Gly Pro Pro Gly Gly Tyr Arg
   65                  70                  75                  80
   Leu Val Pro Thr Leu Ser Ala Val His Ala Leu Leu Thr Cys Leu Ala
                   85                  90                  95
   Ser Pro Ala Gln Asp His Gly Val Pro His Asp Arg Leu Leu Arg Ala
               100                 105                 110
   Val Asp Ala Gly Leu Thr Ala Leu Arg Arg Leu Gly Thr Ser Asp Ser
           115                 120                 125
   Pro Pro Asp Thr Ile Ala Val Glu Leu Val Ile Pro Ser Leu Leu Glu
       130                 135                 140
   Gly Ile Gln His Leu Leu Asp Pro Ala His Pro His Ser Arg Pro Ala
   145                 150                 155                 160
   Phe Ser Gln His Arg Gly Ser Leu Val Cys Pro Gly Gly Leu Asp Gly
                   165                 170                 175
   Arg Thr Leu Gly Ala Leu Arg Ser His Ala Ala Ala Gly Thr Pro Val
               180                 185                 190
   Pro Gly Lys Val Trp His Ala Ser Glu Thr Leu Gly Leu Ser Thr Glu
           195                 200                 205
   Ala Ala Ser His Leu Gln Pro Ala Gln Gly Ile Ile Gly Gly Ser Ala
   210 215 220
   Ala Ala Thr Ala Thr Trp Leu Thr Arg Val Ala Pro Ser Gln Gln Ser
   225 230 235 240
   Asp Ser Ala Arg Arg Tyr Leu Glu Glu Leu Gln His Arg Tyr Ser Gly
   245 250 255
   Pro Val Pro Ser Ile Thr Pro Ile Thr Tyr Phe Glu Arg Ala Trp Leu
   260 265 270
   Leu Asn Asn Phe Ala Ala Ala Gly Val Pro Cys Glu Ala Pro Ala Ala
   275 280 285
   Leu Leu Asp Ser Leu Glu Ala Ala Leu Thr Pro Gln Gly Ala Pro Ala
   290 295 300
   Gly Ala Gly Leu Pro Pro Asp Ala Asp Asp Thr Ala Ala Val Leu Leu
   305 310 315 320
   Ala Leu Ala Thr His Gly Arg Gly Arg Arg Pro Glu Val Leu Met Asp
   325 330 335
   Tyr Arg Thr Asp Gly Tyr Phe Gln Cys Phe Ile Gly Glu Arg Thr Pro
   340 345 350
   Ser Ile Ser Thr Asn Ala His Val Leu Glu Thr Leu Gly His His Val
   355 360 365
   Ala Gln His Pro Gln Asp Arg Ala Arg Tyr Gly Ser Ala Met Asp Thr
   370 375 380
   Ala Ser Ala Trp Leu Leu Ala Ala Gln Lys Gln Asp Gly Ser Trp Leu
   385 390 395 400
   Asp Lys Trp His Ala Ser Pro Tyr Tyr Ala Thr Val Cys Cys Thr Gln
   405 410 415
   Ala Leu Ala Ala His Ala Ser Pro Ala Thr Ala Pro Ala Arg Gln Arg
   420 425 430
   Ala Val Arg Trp Val Leu Ala Thr Gln Arg Ser Asp Gly Gly Trp Gly
   435 440 445
   Leu Trp His Ser Thr Val Glu Glu Thr Ala Tyr Ala Leu Gln Ile Leu
   450 455 460
   Ala Pro Pro Ser Gly Gly Gly Asn Ile Pro Val Gln Gln Ala Leu Thr
   465                 470                 475                 480
   Arg Gly Arg Ala Arg Leu Cys Gly Ala Leu Pro Leu Thr Pro Leu Trp
                   485                 490                 495
   His Asp Lys Asp Leu Tyr Thr Pro Val Arg Val Val Arg Ala Ala Arg
               500                 505                 510
   Ala Ala Ala Leu Tyr Thr Thr Arg Asp Leu Leu Leu Pro Pro Leu
           515                 520                 525
<210> 37
   <211> 1551
   <212> DNA
   <213> Artificial Sequence
<220>
   <223> Codon-optimized CDPS
<400> 37
   atgaacgccc tatccgaaca cattttgtct gaattgagaa gattattgtc tgaaatgagt 60
   gatggcggat ctgttggtcc atctgtgtat gatacggccc aggccctaag attccacggt 120
   aacgtaacag gtagacaaga tgcatatgct tggttgatcg cccagcaaca agcagatgga 180
   ggttggggct ctgccgactt tccactcttt agacatgctc caacatgggc tgcacttctc 240
   gcattacaaa gagctgatcc acttcctggc gcagcagacg cagttcagac cgcaacaaga 300
   ttcttgcaaa gacaaccaga tccatacgct catgccgttc ctgaggatgc ccctattggt 360
   gctgaactga tcttgcctca gttttgtgga gaggctgctt ggttgttggg aggtgtggcc 420
   ttccctagac acccagccct attaccatta agacaggctt gtttagtcaa actgggtgca 480
   gtcgccatgt tgccttcagg acacccattg ctccactcct gggaggcatg gggtacttct 540
   ccaacaacag cctgtccaga cgatgatggt tctataggta tctcaccagc agctacagcc 600
   gcctggagag cccaggctgt gaccagaggc tcaactcctc aagtgggcag agctgacgca 660
   tacttacaaa tggcttcaag agcaacgaga tcaggcatag aaggagtctt ccctaatgtt 720
   tggcctataa acgtattcga accatgctgg tcactgtaca ctctccatct tgccggtctg 780
   ttcgcccatc cagcactggc tgaggctgta agagttatcg ttgctcaact tgaagcaaga 840
   ttgggagtgc atggcctcgg accagcttta cattttgctg ccgacgctga tgatactgca 900
   gttgccttat gcgttctgca tttggctggc agagatcctg cagttgacgc attgagacat 960
   tttgaaattg gtgagctctt tgttacattc ccaggagaga gaaatgctag tgtctctacg 1020
   aacattcacg ctcttcatgc tttgagattg ttaggtaaac cagctgccgg agcaagtgca 1080
   tacgtcgaag caaatagaaa tccacatggt ttgtgggaca acgaaaaatg gcacgtttca 1140
   tggctttatc caactgcaca cgccgttgca gctctagctc aaggcaagcc tcaatggaga 1200
   gatgaaagag cactagccgc tctactacaa gctcaaagag atgatggtgg ttggggagct 1260
   ggtagaggat ccactttcga ggaaaccgcc tacgctcttt tcgctttaca cgttatggac 1320
   ggatctgagg aagccacagg cagaagaaga atcgctcaag tcgtcgcaag agccttagaa 1380
   tggatgctag ctagacatgc cgcacatgga ttaccacaaa caccactctg gattggtaag 1440
   gaattgtact gtcctactag agtcgtaaga gtagctgagc tagctggcct gtggttagca 1500
   ttaagatggg gtagaagagt attagctgaa ggtgctggtg ctgcacctta a 1551
<210> 38
   <211> 516
   <212> PRT
   <213> Bradyrhizobium japonicum
<400> 38
   Met Asn Ala Leu Ser Glu His Ile Leu Ser Glu Leu Arg Arg Leu Leu
   1               5                   10                  15
   Ser Glu Met Ser Asp Gly Gly Ser Val Gly Pro Ser Val Tyr Asp Thr
               20                  25                  30
   Ala Gln Ala Leu Arg Phe His Gly Asn Val Thr Gly Arg Gln Asp Ala
           35                  40                  45
   Tyr Ala Trp Leu Ile Ala Gln Gln Gln Ala Asp Gly Gly Trp Gly Ser
       50                  55                  60
   Ala Asp Phe Pro Leu Phe Arg His Ala Pro Thr Trp Ala Ala Leu Leu
   65                  70                  75                  80
   Ala Leu Gln Arg Ala Asp Pro Leu Pro Gly Ala Ala Asp Ala Val Gln
                   85                  90                  95
   Thr Ala Thr Arg Phe Leu Gln Arg Gln Pro Asp Pro Tyr Ala His Ala
               100                 105                 110
   Val Pro Glu Asp Ala Pro Ile Gly Ala Glu Leu Ile Leu Pro Gln Phe
           115                 120                 125
   Cys Gly Glu Ala Ala Trp Leu Leu Gly Gly Val Ala Phe Pro Arg His
       130                 135                 140
   Pro Ala Leu Leu Pro Leu Arg Gln Ala Cys Leu Val Lys Leu Gly Ala
   145                 150                 155                 160
   Val Ala Met Leu Pro Ser Gly His Pro Leu Leu His Ser Trp Glu Ala
   165 170 175
   Trp Gly Thr Ser Pro Thr Thr Ala Cys Pro Asp Asp Asp Gly Ser Ile
   180 185 190
   Gly Ile Ser Pro Ala Ala Thr Ala Ala Trp Arg Ala Gln Ala Val Thr
   195 200 205
   Arg Gly Ser Thr Pro Gln Val Gly Arg Ala Asp Ala Tyr Leu Gln Met
   210 215 220
   Ala Ser Arg Ala Thr Arg Ser Gly Ile Glu Gly Val Phe Pro Asn Val
   225 230 235 240
   Trp Pro Ile Asn Val Phe Glu Pro Cys Trp Ser Leu Tyr Thr Leu His
   245 250 255
   Leu Ala Gly Leu Phe Ala His Pro Ala Leu Ala Glu Ala Val Arg Val
   260 265 270
   Ile Val Ala Gln Leu Glu Ala Arg Leu Gly Val His Gly Leu Gly Pro
   275 280 285
   Ala Leu His Phe Ala Ala Asp Ala Asp Asp Thr Ala Val Ala Leu Cys
   290 295 300
   Val Leu His Leu Ala Gly Arg Asp Pro Ala Val Asp Ala Leu Arg His
   305 310 315 320
   Phe Glu Ile Gly Glu Leu Phe Val Thr Phe Pro Gly Glu Arg Asn Ala
   325 330 335
   Ser Val Ser Thr Asn Ile His Ala Leu His Ala Leu Arg Leu Leu Gly
   340 345 350
   Lys Pro Ala Ala Gly Ala Ser Ala Tyr Val Glu Ala Asn Arg Asn Pro
   355 360 365
   His Gly Leu Trp Asp Asn Glu Lys Trp His Val Ser Trp Leu Tyr Pro
   370 375 380
   Thr Ala His Ala Val Ala Ala Leu Ala Gln Gly Lys Pro Gln Trp Arg
   385 390 395 400
   Asp Glu Arg Ala Leu Ala Ala Leu Leu Gln Ala Gln Arg Asp Asp Gly
   405 410 415
   Gly Trp Gly Ala Gly Arg Gly Ser Thr Phe Glu Glu Thr Ala Tyr Ala
               420                 425                 430
   Leu Phe Ala Leu His Val Met Asp Gly Ser Glu Glu Ala Thr Gly Arg
           435                 440                 445
   Arg Arg Ile Ala Gln Val Val Ala Arg Ala Leu Glu Trp Met Leu Ala
       450                 455                 460
   Arg His Ala Ala His Gly Leu Pro Gln Thr Pro Leu Trp Ile Gly Lys
   465                 470                 475                 480
   Glu Leu Tyr Cys Pro Thr Arg Val Val Arg Val Ala Glu Leu Ala Gly
                   485                 490                 495
   Leu Trp Leu Ala Leu Arg Trp Gly Arg Arg Val Leu Ala Glu Gly Ala
               500                 505                 510
   Gly Ala Ala Pro
           515
<210> 39
   <211> 2490
   <212> DNA
   <213> Artificial Sequence
<220>
   <223> Codon-optimized CDPS
<400> 39
   atggttttgt cttcttcttg tactacagta ccacacttat cttcattagc tgtcgtgcaa      60
   cttggtcctt ggagcagtag gattaaaaag aaaaccgata ctgttgcagt accagccgct     120
   gcaggaaggt ggagaagggc cttggctaga gcacagcaca catcagaatc cgcagctgtc     180
   gcaaagggca gcagtttgac ccctatagtg agaactgacg ctgagtcaag gagaacaaga     240
   tggccaaccg atgacgatga cgccgaacct ttagtggatg agatcagggc aatgcttact     300
   tccatgtctg atggtgacat ttccgtgagc gcatacgata cagcctgggt cggattggtt     360
   ccaagattag acggcggtga aggtcctcaa tttccagcag ctgtgagatg gataagaaat     420
   aaccagttgc ctgacggaag ttggggcgat gccgcattat tctctgccta tgacaggctt     480
   atcaataccc ttgcctgcgt tgtaactttg acaaggtggt ccctagaacc agagatgaga     540
   ggtagaggac tatctttttt gggtaggaac atgtggaaat tagcaactga agatgaagag     600
   tcaatgccta ttggcttcga attagcattt ccatctttga tagagcttgc taagagccta     660
   ggtgtccatg acttccctta tgatcaccag gccctacaag gaatctactc ttcaagagag     720
   atcaaaatga agaggattcc aaaagaagtg atgcataccg ttccaacatc aatattgcac     780
   agtttggagg gtatgcctgg cctagattgg gctaaactac ttaaactaca gagcagcgac 840
   ggaagttttt tgttctcacc agctgccact gcatatgctt taatgaatac cggagatgac 900
   aggtgtttta gctacatcga tagaacagta aagaaattca acggcggcgt ccctaatgtt 960
   tatccagtgg atctatttga acatatttgg gccgttgata gacttgaaag attaggaatc 1020
   tccaggtact tccaaaagga gatcgaacaa tgcatggatt atgtaaacag gcattggact 1080
   gaggacggta tttgttgggc aaggaactct gatgtcaaag aggtggacga cacagctatg 1140
   gcctttagac ttcttaggtt gcacggctac agcgtcagtc ctgatgtgtt taaaaacttc 1200
   gaaaaggacg gtgaattttt cgcatttgtc ggacagtcta atcaagctgt taccggtatg 1260
   tacaacttaa acagagcaag ccagatatcc ttcccaggcg aggatgtgct tcatagagct 1320
   ggtgccttct catatgagtt cttgaggaga aaagaagcag agggagcttt gagggacaag 1380
   tggatcattt ctaaagatct acctggtgaa gttgtgtata ctttggattt tccatggtac 1440
   ggcaacttac ctagagtcga ggccagagac tacctagagc aatacggagg tggtgatgac 1500
   gtttggattg gcaagacatt gtataggatg ccacttgtaa acaatgatgt atatttggaa 1560
   ttggcaagaa tggatttcaa ccactgccag gctttgcatc agttagagtg gcaaggacta 1620
   aaaagatggt atactgaaaa taggttgatg gactttggtg tcgcccaaga agatgccctt 1680
   agagcttatt ttcttgcagc cgcatctgtt tacgagcctt gtagagctgc cgagaggctt 1740
   gcatgggcta gagccgcaat actagctaac gccgtgagca cccacttaag aaatagccca 1800
   tcattcagag aaaggttaga gcattctctt aggtgtagac ctagtgaaga gacagatggc 1860
   tcctggttta actcctcaag tggctctgat gcagttttag taaaggctgt cttaagactt 1920
   actgattcat tagccaggga agcacagcca atccatggag gtgacccaga agatattata 1980
   cacaagttgt taagatctgc ttgggccgag tgggttaggg aaaaggcaga cgctgccgat 2040
   agcgtgtgca atggtagttc tgcagtagaa caagagggat caagaatggt ccatgataaa 2100
   cagacctgtc tattattggc tagaatgatc gaaatttctg ccggtagggc agctggtgaa 2160
   gcagccagtg aggacggcga tagaagaata attcaattaa caggctccat ctgcgacagt 2220
   cttaagcaaa aaatgctagt ttcacaggac cctgaaaaaa atgaagagat gatgtctcac 2280
   gtggatgacg aattgaagtt gaggattaga gagttcgttc aatatttgct tagactaggt 2340
   gaaaaaaaga ctggatctag cgaaaccagg caaacatttt taagtatagt gaaatcatgt 2400
   tactatgctg ctcattgccc acctcatgtc gttgatagac acattagtag agtgattttc 2460
   gagccagtaa gtgccgcaaa gtaaccgcgg 2490
<210> 40
   <211> 827
   <212> PRT
   <213> Zea mays
<400> 40
   Met Val Leu Ser Ser Ser Cys Thr Thr Val Pro His Leu Ser Ser Leu
   1               5                   10                  15
   Ala Val Val Gln Leu Gly Pro Trp Ser Ser Arg Ile Lys Lys Lys Thr
               20                  25                  30
   Asp Thr Val Ala Val Pro Ala Ala Ala Gly Arg Trp Arg Arg Ala Leu
           35                  40                  45
   Ala Arg Ala Gln His Thr Ser Glu Ser Ala Ala Val Ala Lys Gly Ser
       50                  55                  60
   Ser Leu Thr Pro Ile Val Arg Thr Asp Ala Glu Ser Arg Arg Thr Arg
   65                  70                  75                  80
   Trp Pro Thr Asp Asp Asp Asp Ala Glu Pro Leu Val Asp Glu Ile Arg
                   85                  90                  95
   Ala Met Leu Thr Ser Met Ser Asp Gly Asp Ile Ser Val Ser Ala Tyr
               100                 105                 110
   Asp Thr Ala Trp Val Gly Leu Val Pro Arg Leu Asp Gly Gly Glu Gly
           115                 120                 125
   Pro Gln Phe Pro Ala Ala Val Arg Trp Ile Arg Asn Asn Gln Leu Pro
       130                 135                 140
   Asp Gly Ser Trp Gly Asp Ala Ala Leu Phe Ser Ala Tyr Asp Arg Leu
   145                 150                 155                 160
   Ile Asn Thr Leu Ala Cys Val Val Thr Leu Thr Arg Trp Ser Leu Glu
                   165                 170                 175
   Pro Glu Met Arg Gly Arg Gly Leu Ser Phe Leu Gly Arg Asn Met Trp
               180                 185                 190
   Lys Leu Ala Thr Glu Asp Glu Glu Ser Met Pro Ile Gly Phe Glu Leu
           195                 200                 205
   Ala Phe Pro Ser Leu Ile Glu Leu Ala Lys Ser Leu Gly Val His Asp
       210                 215                 220
   Phe Pro Tyr Asp His Gln Ala Leu Gln Gly Ile Tyr Ser Ser Arg Glu
   225                 230                 235                 240
   Ile Lys Met Lys Arg Ile Pro Lys Glu Val Met His Thr Val Pro Thr
   245 250 255
   Ser Ile Leu His Ser Leu Glu Gly Met Pro Gly Leu Asp Trp Ala Lys
   260 265 270
   Leu Leu Lys Leu Gln Ser Ser Asp Gly Ser Phe Leu Phe Ser Pro Ala
   275 280 285
   Ala Thr Ala Tyr Ala Leu Met Asn Thr Gly Asp Asp Arg Cys Phe Ser
   290 295 300
   Tyr Ile Asp Arg Thr Val Lys Lys Phe Asn Gly Gly Val Pro Asn Val
   305 310 315 320
   Tyr Pro Val Asp Leu Phe Glu His Ile Trp Ala Val Asp Arg Leu Glu
   325 330 335
   Arg Leu Gly Ile Ser Arg Tyr Phe Gln Lys Glu Ile Glu Gln Cys Met
   340 345 350
   Asp Tyr Val Asn Arg His Trp Thr Glu Asp Gly Ile Cys Trp Ala Arg
   355 360 365
   Asn Ser Asp Val Lys Glu Val Asp Asp Thr Ala Met Ala Phe Arg Leu
   370 375 380
   Leu Arg Leu His Gly Tyr Ser Val Ser Pro Asp Val Phe Lys Asn Phe
   385 390 395 400
   Glu Lys Asp Gly Glu Phe Phe Ala Phe Val Gly Gln Ser Asn Gln Ala
   405 410 415
   Val Thr Gly Met Tyr Asn Leu Asn Arg Ala Ser Gln Ile Ser Phe Pro
   420 425 430
   Gly Glu Asp Val Leu His Arg Ala Gly Ala Phe Ser Tyr Glu Phe Leu
   435 440 445
   Arg Arg Lys Glu Ala Glu Gly Ala Leu Arg Asp Lys Trp Ile Ile Ser
   450 455 460
   Lys Asp Leu Pro Gly Glu Val Val Tyr Thr Leu Asp Phe Pro Trp Tyr
   465 470 475 480
   Gly Asn Leu Pro Arg Val Glu Ala Arg Asp Tyr Leu Glu Gln Tyr Gly
   485 490 495
   Gly Gly Asp Asp Val Trp Ile Gly Lys Thr Leu Tyr Arg Met Pro Leu
   500 505 510
   Val Asn Asn Asp Val Tyr Leu Glu Leu Ala Arg Met Asp Phe Asn His
   515 520 525
   Cys Gln Ala Leu His Gln Leu Glu Trp Gln Gly Leu Lys Arg Trp Tyr
   530 535 540
   Thr Glu Asn Arg Leu Met Asp Phe Gly Val Ala Gln Glu Asp Ala Leu
   545 550 555 560
   Arg Ala Tyr Phe Leu Ala Ala Ala Ser Val Tyr Glu Pro Cys Arg Ala
   565 570 575
   Ala Glu Arg Leu Ala Trp Ala Arg Ala Ala Ile Leu Ala Asn Ala Val
   580 585 590
   Ser Thr His Leu Arg Asn Ser Pro Ser Phe Arg Glu Arg Leu Glu His
   595 600 605
   Ser Leu Arg Cys Arg Pro Ser Glu Glu Thr Asp Gly Ser Trp Phe Asn
   610 615 620
   Ser Ser Ser Gly Ser Asp Ala Val Leu Val Lys Ala Val Leu Arg Leu
   625 630 635 640
   Thr Asp Ser Leu Ala Arg Glu Ala Gln Pro Ile His Gly Gly Asp Pro
   645 650 655
   Glu Asp Ile Ile His Lys Leu Leu Arg Ser Ala Trp Ala Glu Trp Val
   660 665 670
   Arg Glu Lys Ala Asp Ala Ala Asp Ser Val Cys Asn Gly Ser Ser Ala
   675 680 685
   Val Glu Gln Glu Gly Ser Arg Met Val His Asp Lys Gln Thr Cys Leu
   690 695 700
   Leu Leu Ala Arg Met Ile Glu Ile Ser Ala Gly Arg Ala Ala Gly Glu
   705 710 715 720
   Ala Ala Ser Glu Asp Gly Asp Arg Arg Ile Ile Gln Leu Thr Gly Ser
   725 730 735
   Ile Cys Asp Ser Leu Lys Gln Lys Met Leu Val Ser Gln Asp Pro Glu
   740 745 750
   Lys Asn Glu Glu Met Met Ser His Val Asp Asp Glu Leu Lys Leu Arg
           755                 760                 765
   Ile Arg Glu Phe Val Gln Tyr Leu Leu Arg Leu Gly Glu Lys Lys Thr
       770                 775                 780
   Gly Ser Ser Glu Thr Arg Gln Thr Phe Leu Ser Ile Val Lys Ser Cys
   785                 790                 795                 800
   Tyr Tyr Ala Ala His Cys Pro Pro His Val Val Asp Arg His Ile Ser
                   805                 810                 815
   Arg Val Ile Phe Glu Pro Val Ser Ala Ala Lys
               820                 825
<210> 41
   <211> 2570
   <212> DNA
   <213> Artificial Sequence
<220>
   <223> Codon-optimized CDPS
<400> 41
   cttcttcact aaatacttag acagagaaaa cagagctttt taaagccatg tctcttcagt      60
   atcatgttct aaactccatt ccaagtacaa cctttctcag ttctactaaa acaacaatat     120
   cttcttcttt ccttaccatc tcaggatctc ctctcaatgt cgctagagac aaatccagaa     180
   gcggttccat acattgttca aagcttcgaa ctcaagaata cattaattct caagaggttc     240
   aacatgattt gcctctaata catgagtggc aacagcttca aggagaagat gctcctcaga     300
   ttagtgttgg aagtaatagt aatgcattca aagaagcagt gaagagtgtg aaaacgatct     360
   tgagaaacct aacggacggg gaaattacga tatcggctta cgatacagct tgggttgcat     420
   tgatcgatgc cggagataaa actccggcgt ttccctccgc cgtgaaatgg atcgccgaga     480
   accaactttc cgatggttct tggggagatg cgtatctctt ctcttatcat gatcgtctca     540
   tcaataccct tgcatgcgtc gttgctctaa gatcatggaa tctctttcct catcaatgca     600
   acaaaggaat cacgtttttc cgggaaaata ttgggaagct agaagacgaa aatgatgagc     660
   atatgccaat cggattcgaa gtagcattcc catcgttgct tgagatagct cgaggaataa     720
   acattgatgt accgtacgat tctccggtct taaaagatat atacgccaag aaagagctaa     780
   agcttacaag gataccaaaa gagataatgc acaagatacc aacaacattg ttgcatagtt     840
   tggaggggat gcgtgattta gattgggaaa agctcttgaa acttcaatct caagacggat     900
   ctttcctctt ctctccttcc tctaccgctt ttgcattcat gcagacccga gacagtaact     960
   gcctcgagta tttgcgaaat gccgtcaaac gtttcaatgg aggagttccc aatgtctttc 1020
   ccgtggatct tttcgagcac atatggatag tggatcggtt acaacgttta gggatatcga 1080
   gatactttga agaagagatt aaagagtgtc ttgactatgt ccacagatat tggaccgaca 1140
   atggcatatg ttgggctaga tgttcccatg tccaagacat cgatgataca gccatggcat 1200
   ttaggctctt aagacaacat ggataccaag tgtccgcaga tgtattcaag aactttgaga 1260
   aagagggaga gtttttctgc tttgtggggc aatcaaacca agcagtaacc ggtatgttca 1320
   acctataccg ggcatcacaa ttggcgtttc caagggaaga gatattgaaa aacgccaaag 1380
   agttttctta taattatctg ctagaaaaac gggagagaga ggagttgatt gataagtgga 1440
   ttataatgaa agacttacct ggcgagattg ggtttgcgtt agagattcca tggtacgcaa 1500
   gcttgcctcg agtagagacg agattctata ttgatcaata tggtggagaa aacgacgttt 1560
   ggattggcaa gactctttat aggatgccat acgtgaacaa taatggatat ctggaattag 1620
   caaaacaaga ttacaacaat tgccaagctc agcatcagct cgaatgggac atattccaaa 1680
   agtggtatga agaaaatagg ttaagtgagt ggggtgtgcg cagaagtgag cttctcgagt 1740
   gttactactt agcggctgca actatatttg aatcagaaag gtcacatgag agaatggttt 1800
   gggctaagtc aagtgtattg gttaaagcca tttcttcttc ttttggggaa tcctctgact 1860
   ccagaagaag cttctccgat cagtttcatg aatacattgc caatgctcga cgaagtgatc 1920
   atcactttaa tgacaggaac atgagattgg accgaccagg atcggttcag gccagtcggc 1980
   ttgccggagt gttaatcggg actttgaatc aaatgtcttt tgaccttttc atgtctcatg 2040
   gccgtgacgt taacaatctc ctctatctat cgtggggaga ttggatggaa aaatggaaac 2100
   tatatggaga tgaaggagaa ggagagctca tggtgaagat gataattcta atgaagaaca 2160
   atgacctaac taacttcttc acccacactc acttcgttcg tctcgcggaa atcatcaatc 2220
   gaatctgtct tcctcgccaa tacttaaagg caaggagaaa cgatgagaag gagaagacaa 2280
   taaagagtat ggagaaggag atggggaaaa tggttgagtt agcattgtcg gagagtgaca 2340
   catttcgtga cgtcagcatc acgtttcttg atgtagcaaa agcattttac tactttgctt 2400
   tatgtggcga tcatctccaa actcacatct ccaaagtctt gtttcaaaaa gtctagtaac 2460
   ctcatcatca tcatcgatcc attaacaatc agtggatcga tgtatccata gatgcgtgaa 2520
   taatatttca tgtagagaag gagaacaaat tagatcatgt agggttatca 2570
<210> 42
   <211> 802
   <212> PRT
   <213> Arabidopsis thaliana
<400> 42
   Met Ser Leu Gln Tyr His Val Leu Asn Ser Ile Pro Ser Thr Thr Phe
   1 5 10 15
   Leu Ser Ser Thr Lys Thr Thr Ile Ser Ser Ser Phe Leu Thr Ile Ser
   20 25 30
   Gly Ser Pro Leu Asn Val Ala Arg Asp Lys Ser Arg Ser Gly Ser Ile
   35 40 45
   His Cys Ser Lys Leu Arg Thr Gln Glu Tyr Ile Asn Ser Gln Glu Val
   50 55 60
   Gln His Asp Leu Pro Leu Ile His Glu Trp Gln Gln Leu Gln Gly Glu
   65 70 75 80
   Asp Ala Pro Gln Ile Ser Val Gly Ser Asn Ser Asn Ala Phe Lys Glu
   85 90 95
   Ala Val Lys Ser Val Lys Thr Ile Leu Arg Asn Leu Thr Asp Gly Glu
   100 105 110
   Ile Thr Ile Ser Ala Tyr Asp Thr Ala Trp Val Ala Leu Ile Asp Ala
   115 120 125
   Gly Asp Lys Thr Pro Ala Phe Pro Ser Ala Val Lys Trp Ile Ala Glu
   130 135 140
   Asn Gln Leu Ser Asp Gly Ser Trp Gly Asp Ala Tyr Leu Phe Ser Tyr
   145 150 155 160
   His Asp Arg Leu Ile Asn Thr Leu Ala Cys Val Val Ala Leu Arg Ser
   165 170 175
   Trp Asn Leu Phe Pro His Gln Cys Asn Lys Gly Ile Thr Phe Phe Arg
   180 185 190
   Glu Asn Ile Gly Lys Leu Glu Asp Glu Asn Asp Glu His Met Pro Ile
   195 200 205
   Gly Phe Glu Val Ala Phe Pro Ser Leu Leu Glu Ile Ala Arg Gly Ile
   210 215 220
   Asn Ile Asp Val Pro Tyr Asp Ser Pro Val Leu Lys Asp Ile Tyr Ala
   225 230 235 240
   Lys Lys Glu Leu Lys Leu Thr Arg Ile Pro Lys Glu Ile Met His Lys
   245 250 255
   Ile Pro Thr Thr Leu Leu His Ser Leu Glu Gly Met Arg Asp Leu Asp
               260                 265                 270
   Trp Glu Lys Leu Leu Lys Leu Gln Ser Gln Asp Gly Ser Phe Leu Phe
           275                 280                 285
   Ser Pro Ser Ser Thr Ala Phe Ala Phe Met Gln Thr Arg Asp Ser Asn
       290                 295                 300
   Cys Leu Glu Tyr Leu Arg Asn Ala Val Lys Arg Phe Asn Gly Gly Val
   305                 310                 315                 320
   Pro Asn Val Phe Pro Val Asp Leu Phe Glu His Ile Trp Ile Val Asp
                   325                 330                 335
   Arg Leu Gln Arg Leu Gly Ile Ser Arg Tyr Phe Glu Glu Glu Ile Lys
               340                 345                 350
   Glu Cys Leu Asp Tyr Val His Arg Tyr Trp Thr Asp Asn Gly Ile Cys
           355                 360                 365
   Trp Ala Arg Cys Ser His Val Gln Asp Ile Asp Asp Thr Ala Met Ala
       370                 375                 380
   Phe Arg Leu Leu Arg Gln His Gly Tyr Gln Val Ser Ala Asp Val Phe
   385                 390                 395                 400
   Lys Asn Phe Glu Lys Glu Gly Glu Phe Phe Cys Phe Val Gly Gln Ser
                   405                 410                 415
   Asn Gln Ala Val Thr Gly Met Phe Asn Leu Tyr Arg Ala Ser Gln Leu
               420                 425                 430
   Ala Phe Pro Arg Glu Glu Ile Leu Lys Asn Ala Lys Glu Phe Ser Tyr
           435                 440                 445
   Asn Tyr Leu Leu Glu Lys Arg Glu Arg Glu Glu Leu Ile Asp Lys Trp
       450                 455                 460
   Ile Ile Met Lys Asp Leu Pro Gly Glu Ile Gly Phe Ala Leu Glu Ile
   465                 470                 475                 480
   Pro Trp Tyr Ala Ser Leu Pro Arg Val Glu Thr Arg Phe Tyr Ile Asp
                   485                 490                 495
   Gln Tyr Gly Gly Glu Asn Asp Val Trp Ile Gly Lys Thr Leu Tyr Arg
               500                 505                 510
   Met Pro Tyr Val Asn Asn Asn Gly Tyr Leu Glu Leu Ala Lys Gln Asp
           515                 520                 525
   Tyr Asn Asn Cys Gln Ala Gln His Gln Leu Glu Trp Asp Ile Phe Gln
       530                 535                 540
   Lys Trp Tyr Glu Glu Asn Arg Leu Ser Glu Trp Gly Val Arg Arg Ser
   545                 550                 555                 560
   Glu Leu Leu Glu Cys Tyr Tyr Leu Ala Ala Ala Thr Ile Phe Glu Ser
                   565                 570                 575
   Glu Arg Ser His Glu Arg Met Val Trp Ala Lys Ser Ser Val Leu Val
               580                 585                 590
   Lys Ala Ile Ser Ser Ser Phe Gly Glu Ser Ser Asp Ser Arg Arg Ser
           595                 600                 605
   Phe Ser Asp Gln Phe His Glu Tyr Ile Ala Asn Ala Arg Arg Ser Asp
       610                 615                 620
   His His Phe Asn Asp Arg Asn Met Arg Leu Asp Arg Pro Gly Ser Val
   625                 630                 635                 640
   Gln Ala Ser Arg Leu Ala Gly Val Leu Ile Gly Thr Leu Asn Gln Met
                   645                 650                 655
   Ser Phe Asp Leu Phe Met Ser His Gly Arg Asp Val Asn Asn Leu Leu
               660                 665                 670
   Tyr Leu Ser Trp Gly Asp Trp Met Glu Lys Trp Lys Leu Tyr Gly Asp
           675                 680                 685
   Glu Gly Glu Gly Glu Leu Met Val Lys Met Ile Ile Leu Met Lys Asn
       690                 695                 700
   Asn Asp Leu Thr Asn Phe Phe Thr His Thr His Phe Val Arg Leu Ala
   705                 710                 715                 720
   Glu Ile Ile Asn Arg Ile Cys Leu Pro Arg Gln Tyr Leu Lys Ala Arg
                   725                 730                 735
   Arg Asn Asp Glu Lys Glu Lys Thr Ile Lys Ser Met Glu Lys Glu Met
               740                 745                 750
   Gly Lys Met Val Glu Leu Ala Leu Ser Glu Ser Asp Thr Phe Arg Asp
           755                 760                 765
   Val Ser Ile Thr Phe Leu Asp Val Ala Lys Ala Phe Tyr Tyr Phe Ala
       770                 775                 780
   Leu Cys Gly Asp His Leu Gln Thr His Ile Ser Lys Val Leu Phe Gln
   785                 790                 795                 800
   Lys Val
<210> 43
   <211> 2355
   <212> DNA
   <213> Artificial Sequence
<220>
   <223> Codon-optimized KS
<400> 43
   atgaatttga gtttgtgtat agcatctcca ctattgacca aatctaatag accagctgct      60
   ttatcagcaa ttcatacagc tagtacatcc catggtggcc aaaccaaccc tacgaatctg     120
   ataatcgata cgaccaagga gagaatacaa aaacaattca aaaatgttga aatttcagtt     180
   tcttcttatg atactgcgtg ggttgccatg gttccatcac ctaattctcc aaagtctcca     240
   tgtttcccag aatgtttgaa ttggctgatt aacaaccagt tgaatgatgg atcttggggt     300
   ttagtcaatc acacgcacaa tcacaaccat ccacttttga aagattcttt atcctcaact     360
   ttggcttgca tcgtggccct aaagagatgg aacgtaggtg aggatcagat taacaagggg     420
   cttagtttca ttgaatctaa cttggcttcc gcgactgaaa aatctcaacc atctccaata     480
   ggattcgata tcatctttcc aggtctgtta gagtacgcca aaaatctaga tatcaactta     540
   ctgtctaagc aaactgattt ctcactaatg ttacacaaga gagaattaga acaaaagaga     600
   tgtcattcaa acgaaatgga tggttaccta gcttatatct ctgaaggtct tggtaatctt     660
   tacgattgga atatggtgaa aaagtaccag atgaaaaatg gctcagtttt caattcccct     720
   tctgcaactg cggcagcatt cattaaccat caaaatccag gatgcctgaa ctatttgaat     780
   tcactactag acaaattcgg caacgcagtt ccaactgtat accctcacga tttgtttatc     840
   agattgagta tggtggatac aattgaaaga cttggtatat cccaccactt tagagtcgag     900
   atcaaaaatg ttttggatga gacataccgt tgttgggtgg agagagatga acaaatcttt     960
   atggatgttg tgacgtgcgc gttggccttt agattgttgc gtattaacgg ttacgaagtt    1020
   agtccagatc cacttgccga aattacaaac gaattagctt taaaggatga atacgccgct    1080
   cttgaaacat atcatgcgtc acatatcctt taccaagagg acttatcatc tggaaaacaa    1140
   attcttaaat ctgctgattt cctgaaggaa atcatatcca ctgatagtaa tagactgtcc    1200
   aaactgatcc ataaagaggt tgaaaatgca cttaagttcc ctattaacac cggcttagaa 1260
   cgtattaaca caagacgtaa catccagctt tacaacgtag acaatactag aatcttgaaa 1320
   accacttacc attcttccaa catatcaaac actgattacc taagattagc tgttgaagat 1380
   ttctacacat gtcagtctat ctatagagaa gagctgaaag gattagagag atgggtcgtt 1440
   gagaataagc tagatcaatt gaaatttgcc agacaaaaga cagcttattg ttacttctca 1500
   gttgccgcca ctttatcaag tccagaattg tcagatgcac gtatttcttg ggctaaaaac 1560
   ggaattttga caactgttgt tgatgatttc tttgatattg gcgggacaat cgacgaattg 1620
   acaaacctga ttcaatgcgt tgaaaagtgg aatgtcgatg tcgataaaga ctgttgctca 1680
   gaacatgtta gaatactgtt cttggctctg aaagatgcta tctgttggat cggggatgag 1740
   gctttcaaat ggcaagctag agatgtgacg tctcacgtca ttcaaacctg gctagaactg 1800
   atgaactcta tgttgagaga agcaatttgg actagagatg catacgttcc tacattaaac 1860
   gagtatatgg aaaacgctta tgtctccttt gctttgggtc ctatcgttaa gcctgccata 1920
   tactttgtag gaccaaagct atccgaggaa atcgtcgaat catcagaata ccataacttg 1980
   ttcaagttaa tgtccacaca aggcagatta cttaatgata ttcattcttt caaaagagag 2040
   tttaaggaag gaaagttaaa tgctgttgct ctgcatcttt ctaatggcga aagtggtaaa 2100
   gtcgaagagg aagtagttga ggaaatgatg atgatgatca aaaacaagag aaaggagttg 2160
   atgaaactaa tcttcgaaga gaacggttca attgttccta gagcatgtaa ggatgcattt 2220
   tggaacatgt gtcatgtgct aaactttttc tacgcaaacg acgatggttt tactgggaac 2280
   acaatactag atacagtaaa agacatcata tacaaccctt tggtcttagt aaacgaaaac 2340
   gaggagcaaa gataa                                                  2355
<210> 44
   <211> 784
   <212> PRT
   <213> Stevia rebaudiana
<400> 44
   Met Asn Leu Ser Leu Cys Ile Ala Ser Pro Leu Leu Thr Lys Ser Asn
   1               5                   10                  15
   Arg Pro Ala Ala Leu Ser Ala Ile His Thr Ala Ser Thr Ser His Gly
               20                  25                  30
   Gly Gln Thr Asn Pro Thr Asn Leu Ile Ile Asp Thr Thr Lys Glu Arg
           35                  40                  45
   Ile Gln Lys Gln Phe Lys Asn Val Glu Ile Ser Val Ser Ser Tyr Asp
       50                  55                  60
   Thr Ala Trp Val Ala Met Val Pro Ser Pro Asn Ser Pro Lys Ser Pro
   65 70 75 80
   Cys Phe Pro Glu Cys Leu Asn Trp Leu Ile Asn Asn Gln Leu Asn Asp
   85 90 95
   Gly Ser Trp Gly Leu Val Asn His Thr His Asn His Asn His Pro Leu
   100 105 110
   Leu Lys Asp Ser Leu Ser Ser Thr Leu Ala Cys Ile Val Ala Leu Lys
   115 120 125
   Arg Trp Asn Val Gly Glu Asp Gln Ile Asn Lys Gly Leu Ser Phe Ile
   130 135 140
   Glu Ser Asn Leu Ala Ser Ala Thr Glu Lys Ser Gln Pro Ser Pro Ile
   145 150 155 160
   Gly Phe Asp Ile Ile Phe Pro Gly Leu Leu Glu Tyr Ala Lys Asn Leu
   165 170 175
   Asp Ile Asn Leu Leu Ser Lys Gln Thr Asp Phe Ser Leu Met Leu His
   180 185 190
   Lys Arg Glu Leu Glu Gln Lys Arg Cys His Ser Asn Glu Met Asp Gly
   195 200 205
   Tyr Leu Ala Tyr Ile Ser Glu Gly Leu Gly Asn Leu Tyr Asp Trp Asn
   210 215 220
   Met Val Lys Lys Tyr Gln Met Lys Asn Gly Ser Val Phe Asn Ser Pro
   225 230 235 240
   Ser Ala Thr Ala Ala Ala Phe Ile Asn His Gln Asn Pro Gly Cys Leu
   245 250 255
   Asn Tyr Leu Asn Ser Leu Leu Asp Lys Phe Gly Asn Ala Val Pro Thr
   260 265 270
   Val Tyr Pro His Asp Leu Phe Ile Arg Leu Ser Met Val Asp Thr Ile
   275 280 285
   Glu Arg Leu Gly Ile Ser His His Phe Arg Val Glu Ile Lys Asn Val
   290 295 300
   Leu Asp Glu Thr Tyr Arg Cys Trp Val Glu Arg Asp Glu Gln Ile Phe
   305 310 315 320
   Met Asp Val Val Thr Cys Ala Leu Ala Phe Arg Leu Leu Arg Ile Asn
   325 330 335
   Gly Tyr Glu Val Ser Pro Asp Pro Leu Ala Glu Ile Thr Asn Glu Leu
   340 345 350
   Ala Leu Lys Asp Glu Tyr Ala Ala Leu Glu Thr Tyr His Ala Ser His
   355 360 365
   Ile Leu Tyr Gln Glu Asp Leu Ser Ser Gly Lys Gln Ile Leu Lys Ser
   370 375 380
   Ala Asp Phe Leu Lys Glu Ile Ile Ser Thr Asp Ser Asn Arg Leu Ser
   385 390 395 400
   Lys Leu Ile His Lys Glu Val Glu Asn Ala Leu Lys Phe Pro Ile Asn
   405 410 415
   Thr Gly Leu Glu Arg Ile Asn Thr Arg Arg Asn Ile Gln Leu Tyr Asn
   420 425 430
   Val Asp Asn Thr Arg Ile Leu Lys Thr Thr Tyr His Ser Ser Asn Ile
   435 440 445
   Ser Asn Thr Asp Tyr Leu Arg Leu Ala Val Glu Asp Phe Tyr Thr Cys
   450 455 460
   Gln Ser Ile Tyr Arg Glu Glu Leu Lys Gly Leu Glu Arg Trp Val Val
   465 470 475 480
   Glu Asn Lys Leu Asp Gln Leu Lys Phe Ala Arg Gln Lys Thr Ala Tyr
   485 490 495
   Cys Tyr Phe Ser Val Ala Ala Thr Leu Ser Ser Pro Glu Leu Ser Asp
   500 505 510
   Ala Arg Ile Ser Trp Ala Lys Asn Gly Ile Leu Thr Thr Val Val Asp
   515 520 525
   Asp Phe Phe Asp Ile Gly Gly Thr Ile Asp Glu Leu Thr Asn Leu Ile
   530 535 540
   Gln Cys Val Glu Lys Trp Asn Val Asp Val Asp Lys Asp Cys Cys Ser
   545 550 555 560
   Glu His Val Arg Ile Leu Phe Leu Ala Leu Lys Asp Ala Ile Cys Trp
   565 570 575
   Ile Gly Asp Glu Ala Phe Lys Trp Gln Ala Arg Asp Val Thr Ser His
   580 585 590
   Val Ile Gln Thr Trp Leu Glu Leu Met Asn Ser Met Leu Arg Glu Ala
   595 600 605
   Ile Trp Thr Arg Asp Ala Tyr Val Pro Thr Leu Asn Glu Tyr Met Glu
   610 615 620
   Asn Ala Tyr Val Ser Phe Ala Leu Gly Pro Ile Val Lys Pro Ala Ile
   625 630 635 640
   Tyr Phe Val Gly Pro Lys Leu Ser Glu Glu Ile Val Glu Ser Ser Glu
   645 650 655
   Tyr His Asn Leu Phe Lys Leu Met Ser Thr Gln Gly Arg Leu Leu Asn
   660 665 670
   Asp Ile His Ser Phe Lys Arg Glu Phe Lys Glu Gly Lys Leu Asn Ala
   675 680 685
   Val Ala Leu His Leu Ser Asn Gly Glu Ser Gly Lys Val Glu Glu Glu
   690 695 700
   Val Val Glu Glu Met Met Met Met Ile Lys Asn Lys Arg Lys Glu Leu
   705 710 715 720
   Met Lys Leu Ile Phe Glu Glu Asn Gly Ser Ile Val Pro Arg Ala Cys
   725 730 735
   Lys Asp Ala Phe Trp Asn Met Cys His Val Leu Asn Phe Phe Tyr Ala
   740 745 750
   Asn Asp Asp Gly Phe Thr Gly Asn Thr Ile Leu Asp Thr Val Lys Asp
   755 760 765
   Ile Ile Tyr Asn Pro Leu Val Leu Val Asn Glu Asn Glu Glu Gln Arg
   770 775 780
<210> 45
   <211> 2355
   <212> DNA
   <213> Artificial Sequence
<220>
   <223> Codon-optimized KS
<400> 45
   atgaatctgt ccctttgtat agctagtcca ctgttgacaa aatcttctag accaactgct 60
   ctttctgcaa ttcatactgc cagtactagt catggaggtc aaacaaaccc aacaaatttg 120
   ataatcgata ctactaagga gagaatccaa aagctattca aaaatgttga aatctcagta 180
   tcatcttatg acaccgcatg ggttgcaatg gtgccatcac ctaattcccc aaaaagtcca 240
   tgttttccag agtgcttgaa ttggttaatc aataatcagt taaacgatgg ttcttggggt 300
   ttagtcaacc acactcataa ccacaatcat ccattattga aggactcttt atcatcaaca 360
   ttagcctgta ttgttgcatt gaaaagatgg aatgtaggtg aagatcaaat caacaagggt 420
   ttatcattca tagaatccaa tctagcttct gctaccgaca aatcacaacc atctccaatc 480
   gggttcgaca taatcttccc tggtttgctg gagtatgcca aaaaccttga tatcaactta 540
   ctgtctaaac aaacagattt ctctttgatg ctacacaaaa gagagttaga gcagaaaaga 600
   tgccattcta acgaaattga cgggtactta gcatatatct cagaaggttt gggtaatttg 660
   tatgactgga acatggtcaa aaagtatcag atgaaaaatg gatccgtatt caattctcct 720
   tctgcaactg ccgcagcatt cattaatcat caaaaccctg ggtgtcttaa ctacttgaac 780
   tcactattag ataagtttgg aaatgcagtt ccaacagtct atcctttgga cttgtacatc 840
   agattatcta tggttgacac tatagagaga ttaggtattt ctcatcattt cagagttgag 900
   atcaaaaatg ttttggacga gacatacaga tgttgggtcg aaagagatga gcaaatcttt 960
   atggatgtcg tgacctgcgc tctggctttt agattgctaa ggatacacgg atacaaagta 1020
   tctcctgatc aactggctga gattacaaac gaactggctt tcaaagacga atacgccgca 1080
   ttagaaacat accatgcatc ccaaatactt taccaggaag acctaagttc aggaaaacaa 1140
   atcttgaagt ctgcagattt cctgaaaggc attctgtcta cagatagtaa taggttgtct 1200
   aaattgatac acaaggaagt agaaaacgca ctaaagtttc ctattaacac tggtttagag 1260
   agaatcaata ctaggagaaa cattcagctg tacaacgtag ataatacaag gattcttaag 1320
   accacctacc atagttcaaa catttccaac acctattact taagattagc tgtcgaagac 1380
   ttttacactt gtcaatcaat ctacagagag gagttaaagg gcctagaaag atgggtagtt 1440
   caaaacaagt tggatcaact gaagtttgct agacagaaga cagcatactg ttatttctct 1500
   gttgctgcta ccctttcatc cccagaattg tctgatgcca gaataagttg ggccaaaaat 1560
   ggtattctta caactgtagt cgatgatttc tttgatattg gaggtactat tgatgaactg 1620
   acaaatctta ttcaatgtgt tgaaaagtgg aacgtggatg tagataagga ttgctgcagt 1680
   gaacatgtga gaatactttt cctggctcta aaagatgcaa tatgttggat tggcgacgag 1740
   gccttcaagt ggcaagctag agatgttaca tctcatgtca tccaaacttg gcttgaactg 1800
   atgaactcaa tgctaagaga agcaatctgg acaagagatg catacgttcc aacattgaac 1860
   gaatacatgg aaaacgctta cgtctcattt gccttgggtc ctattgttaa gccagccata 1920
   tactttgttg ggccaaagtt atccgaagag attgttgagt cttccgaata tcataaccta 1980
   ttcaagttaa tgtcaacaca aggcagactt ctgaacgata tccactcctt caaaagagaa 2040
   ttcaaggaag gtaagctaaa cgctgttgct ttgcacttgt ctaatggtga atctggcaaa 2100
   gtggaagagg aagtcgttga ggaaatgatg atgatgatca aaaacaagag aaaggaattg 2160
   atgaaattga ttttcgagga aaatggttca atcgtaccta gagcttgtaa agatgctttt 2220
   tggaatatgt gccatgttct taacttcttt tacgctaatg atgatggctt cactggaaat 2280
   acaatattgg atacagttaa agatatcatc tacaacccac ttgttttggt caatgagaac 2340
   gaggaacaaa gataa 2355
<210> 46
   <211> 784
   <212> PRT
   <213> Stevia rebaudiana
<400> 46
   Met Asn Leu Ser Leu Cys Ile Ala Ser Pro Leu Leu Thr Lys Ser Ser
   1               5                   10                  15
   Arg Pro Thr Ala Leu Ser Ala Ile His Thr Ala Ser Thr Ser His Gly
               20                  25                  30
   Gly Gln Thr Asn Pro Thr Asn Leu Ile Ile Asp Thr Thr Lys Glu Arg
           35                  40                  45
   Ile Gln Lys Leu Phe Lys Asn Val Glu Ile Ser Val Ser Ser Tyr Asp
       50                  55                  60
   Thr Ala Trp Val Ala Met Val Pro Ser Pro Asn Ser Pro Lys Ser Pro
   65                  70                  75                  80
   Cys Phe Pro Glu Cys Leu Asn Trp Leu Ile Asn Asn Gln Leu Asn Asp
                   85                  90                  95
   Gly Ser Trp Gly Leu Val Asn His Thr His Asn His Asn His Pro Leu
               100                 105                 110
   Leu Lys Asp Ser Leu Ser Ser Thr Leu Ala Cys Ile Val Ala Leu Lys
           115                 120                 125
   Arg Trp Asn Val Gly Glu Asp Gln Ile Asn Lys Gly Leu Ser Phe Ile
       130                 135                 140
   Glu Ser Asn Leu Ala Ser Ala Thr Asp Lys Ser Gln Pro Ser Pro Ile
   145 150 155 160
   Gly Phe Asp Ile Ile Phe Pro Gly Leu Leu Glu Tyr Ala Lys Asn Leu
   165 170 175
   Asp Ile Asn Leu Leu Ser Lys Gln Thr Asp Phe Ser Leu Met Leu His
   180 185 190
   Lys Arg Glu Leu Glu Gln Lys Arg Cys His Ser Asn Glu Ile Asp Gly
   195 200 205
   Tyr Leu Ala Tyr Ile Ser Glu Gly Leu Gly Asn Leu Tyr Asp Trp Asn
   210 215 220
   Met Val Lys Lys Tyr Gln Met Lys Asn Gly Ser Val Phe Asn Ser Pro
   225 230 235 240
   Ser Ala Thr Ala Ala Ala Phe Ile Asn His Gln Asn Pro Gly Cys Leu
   245 250 255
   Asn Tyr Leu Asn Ser Leu Leu Asp Lys Phe Gly Asn Ala Val Pro Thr
   260 265 270
   Val Tyr Pro Leu Asp Leu Tyr Ile Arg Leu Ser Met Val Asp Thr Ile
   275 280 285
   Glu Arg Leu Gly Ile Ser His His Phe Arg Val Glu Ile Lys Asn Val
   290 295 300
   Leu Asp Glu Thr Tyr Arg Cys Trp Val Glu Arg Asp Glu Gln Ile Phe
   305 310 315 320
   Met Asp Val Val Thr Cys Ala Leu Ala Phe Arg Leu Leu Arg Ile His
   325 330 335
   Gly Tyr Lys Val Ser Pro Asp Gln Leu Ala Glu Ile Thr Asn Glu Leu
   340 345 350
   Ala Phe Lys Asp Glu Tyr Ala Ala Leu Glu Thr Tyr His Ala Ser Gln
   355 360 365
   Ile Leu Tyr Gln Glu Asp Leu Ser Ser Gly Lys Gln Ile Leu Lys Ser
   370 375 380
   Ala Asp Phe Leu Lys Gly Ile Leu Ser Thr Asp Ser Asn Arg Leu Ser
   385 390 395 400
   Lys Leu Ile His Lys Glu Val Glu Asn Ala Leu Lys Phe Pro Ile Asn
                   405                 410                 415
   Thr Gly Leu Glu Arg Ile Asn Thr Arg Arg Asn Ile Gln Leu Tyr Asn
               420                 425                 430
   Val Asp Asn Thr Arg Ile Leu Lys Thr Thr Tyr His Ser Ser Asn Ile
           435                 440                 445
   Ser Asn Thr Tyr Tyr Leu Arg Leu Ala Val Glu Asp Phe Tyr Thr Cys
       450                 455                 460
   Gln Ser Ile Tyr Arg Glu Glu Leu Lys Gly Leu Glu Arg Trp Val Val
   465                 470                 475                 480
   Gln Asn Lys Leu Asp Gln Leu Lys Phe Ala Arg Gln Lys Thr Ala Tyr
                   485                 490                 495
   Cys Tyr Phe Ser Val Ala Ala Thr Leu Ser Ser Pro Glu Leu Ser Asp
               500                 505                 510
   Ala Arg Ile Ser Trp Ala Lys Asn Gly Ile Leu Thr Thr Val Val Asp
           515                 520                 525
   Asp Phe Phe Asp Ile Gly Gly Thr Ile Asp Glu Leu Thr Asn Leu Ile
       530                 535                 540
   Gln Cys Val Glu Lys Trp Asn Val Asp Val Asp Lys Asp Cys Cys Ser
   545                 550                 555                 560
   Glu His Val Arg Ile Leu Phe Leu Ala Leu Lys Asp Ala Ile Cys Trp
                   565                 570                 575
   Ile Gly Asp Glu Ala Phe Lys Trp Gln Ala Arg Asp Val Thr Ser His
               580                 585                 590
   Val Ile Gln Thr Trp Leu Glu Leu Met Asn Ser Met Leu Arg Glu Ala
           595                 600                 605
   Ile Trp Thr Arg Asp Ala Tyr Val Pro Thr Leu Asn Glu Tyr Met Glu
       610                 615                 620
   Asn Ala Tyr Val Ser Phe Ala Leu Gly Pro Ile Val Lys Pro Ala Ile
   625                 630                 635                 640
   Tyr Phe Val Gly Pro Lys Leu Ser Glu Glu Ile Val Glu Ser Ser Glu
                   645                 650                 655
   Tyr His Asn Leu Phe Lys Leu Met Ser Thr Gln Gly Arg Leu Leu Asn
   660 665 670
   Asp Ile His Ser Phe Lys Arg Glu Phe Lys Glu Gly Lys Leu Asn Ala
   675 680 685
   Val Ala Leu His Leu Ser Asn Gly Glu Ser Gly Lys Val Glu Glu Glu
   690 695 700
   Val Val Glu Glu Met Met Met Met Ile Lys Asn Lys Arg Lys Glu Leu
   705 710 715 720
   Met Lys Leu Ile Phe Glu Glu Asn Gly Ser Ile Val Pro Arg Ala Cys
   725 730 735
   Lys Asp Ala Phe Trp Asn Met Cys His Val Leu Asn Phe Phe Tyr Ala
   740 745 750
   Asn Asp Asp Gly Phe Thr Gly Asn Thr Ile Leu Asp Thr Val Lys Asp
   755 760 765
   Ile Ile Tyr Asn Pro Leu Val Leu Val Asn Glu Asn Glu Glu Gln Arg
   770 775 780
<210> 47
   <211> 1773
   <212> DNA
   <213> Artificial Sequence
<220>
   <223> Codon-optimized KS
<400> 47
   atggctatgc cagtgaagct aacacctgcg tcattatcct taaaagctgt gtgctgcaga      60
   ttctcatccg gtggccatgc tttgagattc gggagtagtc tgccatgttg gagaaggacc     120
   cctacccaaa gatctacttc ttcctctact actagaccag ctgccgaagt gtcatcaggt     180
   aagagtaaac aacatgatca ggaagctagt gaagcgacta tcagacaaca attacaactt     240
   gtggatgtcc tggagaatat gggaatatcc agacattttg ctgcagagat aaagtgcata     300
   ctagacagaa cttacagatc ttggttacaa agacacgagg aaatcatgct ggacactatg     360
   acatgtgcta tggcttttag aatcctaaga ttgaacggat acaacgtttc atcagatgaa     420
   ctataccacg ttgtagaggc atctggtctg cataattctt tgggtgggta tcttaacgat     480
   accagaacac tacttgaatt acacaaggct tcaacagtta gtatctctga ggatgaatct     540
   atcttagatt caattggctc tagatccaga acattgctta gagaacaatt ggagtctggt     600
   ggcgcactga gaaagccttc tttattcaaa gaggttgaac atgcactgga tggacctttt     660
   tacaccacac ttgatagact tcatcatagg tggaatattg aaaacttcaa cattattgag     720
   caacacatgt tggagactcc atacttatct aaccagcata catcaaggga tatcctagca     780
   ttgtcaatta gagatttttc ctcctcacaa ttcacttatc aacaagagct acagcatctg     840
   gagagttggg ttaaggaatg tagattagat caactacagt tcgcaagaca gaaattagcg     900
   tacttttacc tatcagccgc aggcaccatg ttttctcctg agctttctga tgcgagaaca     960
   ttatgggcca aaaacggggt gttgacaact attgttgatg atttctttga tgttgccggt    1020
   tctaaagagg aattggaaaa cttagtcatg ctggtcgaaa tgtgggatga acatcacaaa    1080
   gttgaattct attctgagca ggtcgaaatc atcttctctt ccatctacga ttctgtcaac    1140
   caattgggtg agaaggcctc tttggttcaa gacagatcaa ttacaaaaca ccttgttgaa    1200
   atatggttag acttgttaaa gtccatgatg acggaagttg aatggagact gtcaaaatac    1260
   gtgcctacag aaaaggaata catgattaat gcctctctta tcttcggcct aggtccaatc    1320
   gttttaccag ctttgtattt cgttggtcca aagatttcag aaagtatagt aaaggaccca    1380
   gaatatgatg aattgttcaa actaatgtca acatgtggta gattgttgaa tgacgtgcaa    1440
   acgttcgaaa gagaatacaa tgagggtaaa ctgaattctg tcagtctatt ggttcttcac    1500
   ggaggcccaa tgtctatttc agacgcaaag aggaaattac aaaagcctat tgatacgtgt    1560
   agaagagatc ttctttcttt ggtccttaga gaagagtctg tagtaccaag accatgtaag    1620
   gaactattct ggaaaatgtg taaagtgtgc tatttctttt actcaacaac tgatgggttt    1680
   tctagtcaag tcgaaagagc aaaagaggta gacgctgtca taaatgagcc actgaagttg    1740
   caaggttctc atacactggt atctgatgtt taa                                 1773
<210> 48
   <211> 590
   <212> PRT
   <213> Zea mays
<400> 48
   Met Ala Met Pro Val Lys Leu Thr Pro Ala Ser Leu Ser Leu Lys Ala
   1               5                   10                  15
   Val Cys Cys Arg Phe Ser Ser Gly Gly His Ala Leu Arg Phe Gly Ser
               20                  25                  30
   Ser Leu Pro Cys Trp Arg Arg Thr Pro Thr Gln Arg Ser Thr Ser Ser
           35                  40                  45
   Ser Thr Thr Arg Pro Ala Ala Glu Val Ser Ser Gly Lys Ser Lys Gln
       50                  55                  60
   His Asp Gln Glu Ala Ser Glu Ala Thr Ile Arg Gln Gln Leu Gln Leu
   65 70 75 80
   Val Asp Val Leu Glu Asn Met Gly Ile Ser Arg His Phe Ala Ala Glu
   85 90 95
   Ile Lys Cys Ile Leu Asp Arg Thr Tyr Arg Ser Trp Leu Gln Arg His
   100 105 110
   Glu Glu Ile Met Leu Asp Thr Met Thr Cys Ala Met Ala Phe Arg Ile
   115 120 125
   Leu Arg Leu Asn Gly Tyr Asn Val Ser Ser Asp Glu Leu Tyr His Val
   130 135 140
   Val Glu Ala Ser Gly Leu His Asn Ser Leu Gly Gly Tyr Leu Asn Asp
   145 150 155 160
   Thr Arg Thr Leu Leu Glu Leu His Lys Ala Ser Thr Val Ser Ile Ser
   165 170 175
   Glu Asp Glu Ser Ile Leu Asp Ser Ile Gly Ser Arg Ser Arg Thr Leu
   180 185 190
   Leu Arg Glu Gln Leu Glu Ser Gly Gly Ala Leu Arg Lys Pro Ser Leu
   195 200 205
   Phe Lys Glu Val Glu His Ala Leu Asp Gly Pro Phe Tyr Thr Thr Leu
   210 215 220
   Asp Arg Leu His His Arg Trp Asn Ile Glu Asn Phe Asn Ile Ile Glu
   225 230 235 240
   Gln His Met Leu Glu Thr Pro Tyr Leu Ser Asn Gln His Thr Ser Arg
   245 250 255
   Asp Ile Leu Ala Leu Ser Ile Arg Asp Phe Ser Ser Ser Gln Phe Thr
   260 265 270
   Tyr Gln Gln Glu Leu Gln His Leu Glu Ser Trp Val Lys Glu Cys Arg
   275 280 285
   Leu Asp Gln Leu Gln Phe Ala Arg Gln Lys Leu Ala Tyr Phe Tyr Leu
   290 295 300
   Ser Ala Ala Gly Thr Met Phe Ser Pro Glu Leu Ser Asp Ala Arg Thr
   305 310 315 320
   Leu Trp Ala Lys Asn Gly Val Leu Thr Thr Ile Val Asp Asp Phe Phe
   325 330 335
   Asp Val Ala Gly Ser Lys Glu Glu Leu Glu Asn Leu Val Met Leu Val
   340 345 350
   Glu Met Trp Asp Glu His His Lys Val Glu Phe Tyr Ser Glu Gln Val
   355 360 365
   Glu Ile Ile Phe Ser Ser Ile Tyr Asp Ser Val Asn Gln Leu Gly Glu
   370 375 380
   Lys Ala Ser Leu Val Gln Asp Arg Ser Ile Thr Lys His Leu Val Glu
   385 390 395 400
   Ile Trp Leu Asp Leu Leu Lys Ser Met Met Thr Glu Val Glu Trp Arg
   405 410 415
   Leu Ser Lys Tyr Val Pro Thr Glu Lys Glu Tyr Met Ile Asn Ala Ser
   420 425 430
   Leu Ile Phe Gly Leu Gly Pro Ile Val Leu Pro Ala Leu Tyr Phe Val
   435 440 445
   Gly Pro Lys Ile Ser Glu Ser Ile Val Lys Asp Pro Glu Tyr Asp Glu
   450 455 460
   Leu Phe Lys Leu Met Ser Thr Cys Gly Arg Leu Leu Asn Asp Val Gln
   465 470 475 480
   Thr Phe Glu Arg Glu Tyr Asn Glu Gly Lys Leu Asn Ser Val Ser Leu
   485 490 495
   Leu Val Leu His Gly Gly Pro Met Ser Ile Ser Asp Ala Lys Arg Lys
   500 505 510
   Leu Gln Lys Pro Ile Asp Thr Cys Arg Arg Asp Leu Leu Ser Leu Val
   515 520 525
   Leu Arg Glu Glu Ser Val Val Pro Arg Pro Cys Lys Glu Leu Phe Trp
   530 535 540
   Lys Met Cys Lys Val Cys Tyr Phe Phe Tyr Ser Thr Thr Asp Gly Phe
   545 550 555 560
   Ser Ser Gln Val Glu Arg Ala Lys Glu Val Asp Ala Val Ile Asn Glu
   565 570 575
   Pro Leu Lys Leu Gln Gly Ser His Thr Leu Val Ser Asp Val
   580 585 590
<210> 49
   <211> 2232
   <212> DNA
   <213> Artificial Sequence
<220>
   <223> Codon-optimized KS
<400> 49
   atgcagaact tccatggtac aaaggaaagg atcaaaaaga tgtttgacaa gattgaattg 60
   tccgtttctt cttatgatac agcctgggtt gcaatggtcc catcccctga ttgcccagaa 120
   acaccttgtt ttccagaatg tactaaatgg atcctagaaa atcagttggg tgatggtagt 180
   tggtcacttc ctcatggcaa tccacttcta gttaaagatg cattatcttc cactcttgct 240
   tgtattctgg ctcttaaaag atggggaatc ggtgaggaac agattaacaa aggactgaga 300
   ttcatagaac tcaactctgc tagtgtaacc gataacgaac aacacaaacc aattggattt 360
   gacattatct ttccaggtat gattgaatac gctatagact tagacctgaa tctaccacta 420
   aaaccaactg acattaactc catgttgcat cgtagagccc ttgaattgac atcaggtgga 480
   ggcaaaaatc tagaaggtag aagagcttac ttggcctacg tctctgaagg aatcggtaag 540
   ctgcaagatt gggaaatggc tatgaaatac caacgtaaaa acggatctct gttcaatagt 600
   ccatcaacaa ctgcagctgc attcatccat atacaagatg ctgaatgcct ccactatatt 660
   cgttctcttc tccagaaatt tggaaacgca gtccctacaa tataccctct cgatatctat 720
   gccagacttt caatggtaga tgccctggaa cgtcttggta ttgatagaca tttcagaaag 780
   gagagaaagt tcgttctgga tgaaacatac agattttggt tgcaaggaga agaggagatt 840
   ttctccgata acgcaacctg tgctttggcc ttcagaatat tgagacttaa tggttacgat 900
   gtctctcttg aagatcactt ctctaactct ctgggcggtt acttaaagga ctcaggagca 960
   gctttagaac tgtacagagc cctccaattg tcttacccag acgagtccct cctggaaaag 1020
   caaaattcta gaacttctta cttcttaaaa caaggtttat ccaatgtctc cctctgtggt 1080
   gacagattgc gtaaaaacat aattggagag gtgcatgatg ctttaaactt ttccgaccac 1140
   gctaacttac aaagattagc tattcgtaga aggattaagc attacgctac tgacgataca 1200
   aggattctaa aaacttccta cagatgctca acaatcggta accaagattt tctaaaactt 1260
   gcagtggaag atttcaatat ctgtcaatca atacaaagag aggaattcaa gcatattgaa 1320
   agatgggtcg ttgaaagacg tctagacaag ttaaagttcg ctagacaaaa agaggcctat 1380
   tgctatttct cagccgcagc aacattgttt gcccctgaat tgtctgatgc tagaatgtct 1440
   tgggccaaaa atggtgtatt gacaactgtg gttgatgatt tcttcgatgt cggaggctct      1500
   gaagaggaat tagttaactt gatagaattg atcgagcgtt gggatgtgaa tggcagtgca      1560
   gatttttgta gtgaggaagt tgagattatc tattctgcta tccactcaac tatctctgaa      1620
   ataggtgata agtcatttgg ctggcaaggt agagatgtaa agtctcaagt tatcaagatc      1680
   tggctggact tattgaaatc aatgttaact gaagctcaat ggtcttcaaa caagtctgtt      1740
   cctaccctag atgagtatat gacaaccgcc catgtttcat tcgcacttgg tccaattgta      1800
   cttccagcct tatacttcgt tggcccaaag ttgtcagaag aggttgcagg tcatcctgaa      1860
   ctactaaacc tctacaaagt cacatctact tgtggcagac tactgaatga ttggagaagt      1920
   tttaagagag aatccgagga aggtaagctc aacgctatta gtttatacat gatccactcc      1980
   ggtggtgctt ctacagaaga ggaaacaatc gaacatttca aaggtttgat tgattctcag      2040
   agaaggcaac tgttacaatt ggtgttgcaa gagaaggata gtatcatacc tagaccatgt      2100
   aaagatctat tttggaatat gattaagtta ttacacactt tctacatgaa agatgatggc      2160
   ttcacctcaa atgagatgag gaatgtagtt aaggcaatca ttaacgaacc aatctcactg      2220
   gatgaattat ga                                                          2232
<210> 50
   <211> 775
   <212> PRT
   <213> Populus trichocarpa
<400> 50
   Met Ser Cys Ile Arg Pro Trp Phe Cys Pro Ser Ser Ile Ser Ala Thr
   1               5                   10                  15
   Leu Thr Asp Pro Ala Ser Lys Leu Val Thr Gly Glu Phe Lys Thr Thr
               20                  25                  30
   Ser Leu Asn Phe His Gly Thr Lys Glu Arg Ile Lys Lys Met Phe Asp
           35                  40                  45
   Lys Ile Glu Leu Ser Val Ser Ser Tyr Asp Thr Ala Trp Val Ala Met
       50                  55                  60
   Val Pro Ser Pro Asp Cys Pro Glu Thr Pro Cys Phe Pro Glu Cys Thr
   65                  70                  75                  80
   Lys Trp Ile Leu Glu Asn Gln Leu Gly Asp Gly Ser Trp Ser Leu Pro
                   85                  90                  95
   His Gly Asn Pro Leu Leu Val Lys Asp Ala Leu Ser Ser Thr Leu Ala
               100                 105                 110
   Cys Ile Leu Ala Leu Lys Arg Trp Gly Ile Gly Glu Glu Gln Ile Asn
   115 120 125
   Lys Gly Leu Arg Phe Ile Glu Leu Asn Ser Ala Ser Val Thr Asp Asn
   130 135 140
   Glu Gln His Lys Pro Ile Gly Phe Asp Ile Ile Phe Pro Gly Met Ile
   145 150 155 160
   Glu Tyr Ala Lys Asp Leu Asp Leu Asn Leu Pro Leu Lys Pro Thr Asp
   165 170 175
   Ile Asn Ser Met Leu His Arg Arg Ala Leu Glu Leu Thr Ser Gly Gly
   180 185 190
   Gly Lys Asn Leu Glu Gly Arg Arg Ala Tyr Leu Ala Tyr Val Ser Glu
   195 200 205
   Gly Ile Gly Lys Leu Gln Asp Trp Glu Met Ala Met Lys Tyr Gln Arg
   210 215 220
   Lys Asn Gly Ser Leu Phe Asn Ser Pro Ser Thr Thr Ala Ala Ala Phe
   225 230 235 240
   Ile His Ile Gln Asp Ala Glu Cys Leu His Tyr Ile Arg Ser Leu Leu
   245 250 255
   Gln Lys Phe Gly Asn Ala Val Pro Thr Ile Tyr Pro Leu Asp Ile Tyr
   260 265 270
   Ala Arg Leu Ser Met Val Asp Ala Leu Glu Arg Leu Gly Ile Asp Arg
   275 280 285
   His Phe Arg Lys Glu Arg Lys Phe Val Leu Asp Glu Thr Tyr Arg Phe
   290 295 300
   Trp Leu Gln Gly Glu Glu Glu Ile Phe Ser Asp Asn Ala Thr Cys Ala
   305 310 315 320
   Leu Ala Phe Arg Ile Leu Arg Leu Asn Gly Tyr Asp Val Ser Leu Glu
   325 330 335
   Asp His Phe Ser Asn Ser Leu Gly Gly Tyr Leu Lys Asp Ser Gly Ala
   340 345 350
   Ala Leu Glu Leu Tyr Arg Ala Leu Gln Leu Ser Tyr Pro Asp Glu Ser
   355 360 365
   Leu Leu Glu Lys Gln Asn Ser Arg Thr Ser Tyr Phe Leu Lys Gln Gly
       370                 375                 380
   Leu Ser Asn Val Ser Leu Cys Gly Asp Arg Leu Arg Lys Asn Ile Ile
   385                 390                 395                 400
   Gly Glu Val His Asp Ala Leu Asn Phe Pro Asp His Ala Asn Leu Gln
                   405                 410                 415
   Arg Leu Ala Ile Arg Arg Arg Ile Lys His Tyr Ala Thr Asp Asp Thr
               420                 425                 430
   Arg Ile Leu Lys Thr Ser Tyr Arg Cys Ser Thr Ile Gly Asn Gln Asp
           435                 440                 445
   Phe Leu Lys Leu Ala Val Glu Asp Phe Asn Ile Cys Gln Ser Ile Gln
       450                 455                 460
   Arg Glu Glu Phe Lys His Ile Glu Arg Trp Val Val Glu Arg Arg Leu
   465                 470                 475                 480
   Asp Lys Leu Lys Phe Ala Arg Gln Lys Glu Ala Tyr Cys Tyr Phe Ser
                   485                 490                 495
   Ala Ala Ala Thr Leu Phe Ala Pro Glu Leu Ser Asp Ala Arg Met Ser
               500                 505                 510
   Trp Ala Lys Asn Gly Val Leu Thr Thr Val Val Asp Asp Phe Phe Asp
           515                 520                 525
   Val Gly Gly Ser Glu Glu Glu Leu Val Asn Leu Ile Glu Leu Ile Glu
       530                 535                 540
   Arg Trp Asp Val Asn Gly Ser Ala Asp Phe Cys Ser Glu Glu Val Glu
   545                 550                 555                 560
   Ile Ile Tyr Ser Ala Ile His Ser Thr Ile Ser Glu Ile Gly Asp Lys
                   565                 570                 575
   Ser Phe Gly Trp Gln Gly Arg Asp Val Lys Ser His Val Ile Lys Ile
               580                 585                 590
   Trp Leu Asp Leu Leu Lys Ser Met Leu Thr Glu Ala Gln Trp Ser Ser
           595                 600                 605
   Asn Lys Ser Val Pro Thr Leu Asp Glu Tyr Met Thr Thr Ala His Val
   610 615 620
   Ser Phe Ala Leu Gly Pro Ile Val Leu Pro Ala Leu Tyr Phe Val Gly
   625 630 635 640
   Pro Lys Leu Ser Glu Glu Val Ala Gly His Pro Glu Leu Leu Asn Leu
   645 650 655
   Tyr Lys Val Met Ser Thr Cys Gly Arg Leu Leu Asn Asp Trp Arg Ser
   660 665 670
   Phe Lys Arg Glu Ser Glu Glu Gly Lys Leu Asn Ala Ile Ser Leu Tyr
   675 680 685
   Met Ile His Ser Gly Gly Ala Ser Thr Glu Glu Glu Thr Ile Glu His
   690 695 700
   Phe Lys Gly Leu Ile Asp Ser Gln Arg Arg Gln Leu Leu Gln Leu Val
   705 710 715 720
   Leu Gln Glu Lys Asp Ser Ile Ile Pro Arg Pro Cys Lys Asp Leu Phe
   725 730 735
   Trp Asn Met Ile Lys Leu Leu His Thr Phe Tyr Met Lys Asp Asp Gly
   740 745 750
   Phe Thr Ser Asn Glu Met Arg Asn Val Val Lys Ala Ile Ile Asn Glu
   755 760 765
   Pro Ile Ser Leu Asp Glu Leu
   770 775
<210> 51
   <211> 2358
   <212> DNA
   <213> Artificial Sequence
<220>
   <223> Codon-optimized KS
<400> 51
   atgtctatca accttcgctc ctccggttgt tcgtctccga tctcagctac tttggaacga      60
   ggattggact cagaagtaca gacaagagct aacaatgtga gctttgagca aacaaaggag     120
   aagattagga agatgttgga gaaagtggag ctttctgttt cggcctacga tactagttgg     180
   gtagcaatgg ttccatcacc gagctcccaa aatgctccac ttttcccaca gtgtgtgaaa     240
   tggttattgg ataatcaaca tgaagatgga tcttggggac ttgataacca tgaccatcaa     300
   tctcttaaga aggatgtgtt atcatctaca ctggctagta tcctcgcgtt aaagaagtgg     360
   ggaattggtg aaagacaaat aaacaagggt ctccagttta ttgagctgaa ttctgcatta 420
   gtcactgatg aaaccataca gaaaccaaca gggtttgata ttatatttcc tgggatgatt 480
   aaatatgcta gagatttgaa tctgacgatt ccattgggct cagaagtggt ggatgacatg 540
   atacgaaaaa gagatctgga tcttaaatgt gatagtgaaa agttttcaaa gggaagagaa 600
   gcatatctgg cctatgtttt agaggggaca agaaacctaa aagattggga tttgatagtc 660
   aaatatcaaa ggaaaaatgg gtcactgttt gattctccag ccacaacagc agctgctttt 720
   actcagtttg ggaatgatgg ttgtctccgt tatctctgtt ctctccttca gaaattcgag 780
   gctgcagttc cttcagttta tccatttgat caatatgcac gccttagtat aattgtcact 840
   cttgaaagct taggaattga tagagatttc aaaaccgaaa tcaaaagcat attggatgaa 900
   acctatagat attggcttcg tggggatgaa gaaatatgtt tggacttggc cacttgtgct 960
   ttggctttcc gattattgct tgctcatggc tatgatgtgt cttacgatcc gctaaaacca 1020
   tttgcagaag aatctggttt ctctgatact ttggaaggat atgttaagaa tacgttttct 1080
   gtgttagaat tatttaaggc tgctcaaagt tatccacatg aatcagcttt gaagaagcag 1140
   tgttgttgga ctaaacaata tctggagatg gaattgtcca gctgggttaa gacctctgtt 1200
   cgagataaat acctcaagaa agaggtcgag gatgctcttg cttttccctc ctatgcaagc 1260
   ctagaaagat cagatcacag gagaaaaata ctcaatggtt ctgctgtgga aaacaccaga 1320
   gttacaaaaa cctcatatcg tttgcacaat atttgcacct ctgatatcct gaagttagct 1380
   gtggatgact tcaatttctg ccagtccata caccgtgaag aaatggaacg tcttgatagg 1440
   tggattgtgg agaatagatt gcaggaactg aaatttgcca gacagaagct ggcttactgt 1500
   tatttctctg gggctgcaac tttattttct ccagaactat ctgatgctcg tatatcgtgg 1560
   gccaaaggtg gagtacttac aacggttgta gacgacttct ttgatgttgg agggtccaaa 1620
   gaagaactgg aaaacctcat acacttggtc gaaaagtggg atttgaacgg tgttcctgag 1680
   tacagctcag aacatgttga gatcatattc tcagttctaa gggacaccat tctcgaaaca 1740
   ggagacaaag cattcaccta tcaaggacgc aatgtgacac accacattgt gaaaatttgg 1800
   ttggatctgc tcaagtctat gttgagagaa gccgagtggt ccagtgacaa gtcaacacca 1860
   agcttggagg attacatgga aaatgcgtac atatcatttg cattaggacc aattgtcctc 1920
   ccagctacct atctgatcgg acctccactt ccagagaaga cagtcgatag ccaccaatat 1980
   aatcagctct acaagctcgt gagcactatg ggtcgtcttc taaatgacat acaaggtttt 2040
   aagagagaaa gcgcggaagg gaagctgaat gcggtttcat tgcacatgaa acacgagaga 2100
   gacaatcgca gcaaagaagt gatcatagaa tcgatgaaag gtttagcaga gagaaagagg 2160
   gaagaattgc ataagctagt tttggaggag aaaggaagtg tggttccaag ggaatgcaaa 2220
   gaagcgttct tgaaaatgag caaagtgttg aacttatttt acaggaagga cgatggattc 2280
   acatcaaatg atctgatgag tcttgttaaa tcagtgatct acgagcctgt tagcttacag 2340
   aaagaatctt taacttga 2358
<210> 52
   <211> 785
   <212> PRT
   <213> Arabidopsis thaliana
<400> 52
   Met Ser Ile Asn Leu Arg Ser Ser Gly Cys Ser Ser Pro Ile Ser Ala
   1               5                   10                  15
   Thr Leu Glu Arg Gly Leu Asp Ser Glu Val Gln Thr Arg Ala Asn Asn
               20                  25                  30
   Val Ser Phe Glu Gln Thr Lys Glu Lys Ile Arg Lys Met Leu Glu Lys
           35                  40                  45
   Val Glu Leu Ser Val Ser Ala Tyr Asp Thr Ser Trp Val Ala Met Val
       50                  55                  60
   Pro Ser Pro Ser Ser Gln Asn Ala Pro Leu Phe Pro Gln Cys Val Lys
   65                  70                  75                  80
   Trp Leu Leu Asp Asn Gln His Glu Asp Gly Ser Trp Gly Leu Asp Asn
                   85                  90                  95
   His Asp His Gln Ser Leu Lys Lys Asp Val Leu Ser Ser Thr Leu Ala
               100                 105                 110
   Ser Ile Leu Ala Leu Lys Lys Trp Gly Ile Gly Glu Arg Gln Ile Asn
           115                 120                 125
   Lys Gly Leu Gln Phe Ile Glu Leu Asn Ser Ala Leu Val Thr Asp Glu
       130                 135                 140
   Thr Ile Gln Lys Pro Thr Gly Phe Asp Ile Ile Phe Pro Gly Met Ile
   145                 150                 155                 160
   Lys Tyr Ala Arg Asp Leu Asn Leu Thr Ile Pro Leu Gly Ser Glu Val
                   165                 170                 175
   Val Asp Asp Met Ile Arg Lys Arg Asp Leu Asp Leu Lys Cys Asp Ser
               180                 185                 190
   Glu Lys Phe Ser Lys Gly Arg Glu Ala Tyr Leu Ala Tyr Val Leu Glu
   195 200 205
   Gly Thr Arg Asn Leu Lys Asp Trp Asp Leu Ile Val Lys Tyr Gln Arg
   210 215 220
   Lys Asn Gly Ser Leu Phe Asp Ser Pro Ala Thr Thr Ala Ala Ala Phe
   225 230 235 240
   Thr Gln Phe Gly Asn Asp Gly Cys Leu Arg Tyr Leu Cys Ser Leu Leu
   245 250 255
   Gln Lys Phe Glu Ala Ala Val Pro Ser Val Tyr Pro Phe Asp Gln Tyr
   260 265 270
   Ala Arg Leu Ser Ile Ile Val Thr Leu Glu Ser Leu Gly Ile Asp Arg
   275 280 285
   Asp Phe Lys Thr Glu Ile Lys Ser Ile Leu Asp Glu Thr Tyr Arg Tyr
   290 295 300
   Trp Leu Arg Gly Asp Glu Glu Ile Cys Leu Asp Leu Ala Thr Cys Ala
   305 310 315 320
   Leu Ala Phe Arg Leu Leu Leu Ala His Gly Tyr Asp Val Ser Tyr Asp
   325 330 335
   Pro Leu Lys Pro Phe Ala Glu Glu Ser Gly Phe Ser Asp Thr Leu Glu
   340 345 350
   Gly Tyr Val Lys Asn Thr Phe Ser Val Leu Glu Leu Phe Lys Ala Ala
   355 360 365
   Gln Ser Tyr Pro His Glu Ser Ala Leu Lys Lys Gln Cys Cys Trp Thr
   370 375 380
   Lys Gln Tyr Leu Glu Met Glu Leu Ser Ser Trp Val Lys Thr Ser Val
   385 390 395 400
   Arg Asp Lys Tyr Leu Lys Lys Glu Val Glu Asp Ala Leu Ala Phe Pro
   405 410 415
   Ser Tyr Ala Ser Leu Glu Arg Ser Asp His Arg Arg Lys Ile Leu Asn
   420 425 430
   Gly Ser Ala Val Glu Asn Thr Arg Val Thr Lys Thr Ser Tyr Arg Leu
   435 440 445
   His Asn Ile Cys Thr Ser Asp Ile Leu Lys Leu Ala Val Asp Asp Phe
   450 455 460
   Asn Phe Cys Gln Ser Ile His Arg Glu Glu Met Glu Arg Leu Asp Arg
   465 470 475 480
   Trp Ile Val Glu Asn Arg Leu Gln Glu Leu Lys Phe Ala Arg Gln Lys
   485 490 495
   Leu Ala Tyr Cys Tyr Phe Ser Gly Ala Ala Thr Leu Phe Ser Pro Glu
   500 505 510
   Leu Ser Asp Ala Arg Ile Ser Trp Ala Lys Gly Gly Val Leu Thr Thr
   515 520 525
   Val Val Asp Asp Phe Phe Asp Val Gly Gly Ser Lys Glu Glu Leu Glu
   530 535 540
   Asn Leu Ile His Leu Val Glu Lys Trp Asp Leu Asn Gly Val Pro Glu
   545 550 555 560
   Tyr Ser Ser Glu His Val Glu Ile Ile Phe Ser Val Leu Arg Asp Thr
   565 570 575
   Ile Leu Glu Thr Gly Asp Lys Ala Phe Thr Tyr Gln Gly Arg Asn Val
   580 585 590
   Thr His His Ile Val Lys Ile Trp Leu Asp Leu Leu Lys Ser Met Leu
   595 600 605
   Arg Glu Ala Glu Trp Ser Ser Asp Lys Ser Thr Pro Ser Leu Glu Asp
   610 615 620
   Tyr Met Glu Asn Ala Tyr Ile Ser Phe Ala Leu Gly Pro Ile Val Leu
   625 630 635 640
   Pro Ala Thr Tyr Leu Ile Gly Pro Pro Leu Pro Glu Lys Thr Val Asp
   645 650 655
   Ser His Gln Tyr Asn Gln Leu Tyr Lys Leu Val Ser Thr Met Gly Arg
   660 665 670
   Leu Leu Asn Asp Ile Gln Gly Phe Lys Arg Glu Ser Ala Glu Gly Lys
   675 680 685
   Leu Asn Ala Val Ser Leu His Met Lys His Glu Arg Asp Asn Arg Ser
   690 695 700
   Lys Glu Val Ile Ile Glu Ser Met Lys Gly Leu Ala Glu Arg Lys Arg
   705 710 715 720
   Glu Glu Leu His Lys Leu Val Leu Glu Glu Lys Gly Ser Val Val Pro
   725 730 735
   Arg Glu Cys Lys Glu Ala Phe Leu Lys Met Ser Lys Val Leu Asn Leu
   740 745 750
   Phe Tyr Arg Lys Asp Asp Gly Phe Thr Ser Asn Asp Leu Met Ser Leu
   755 760 765
   Val Lys Ser Val Ile Tyr Glu Pro Val Ser Leu Gln Lys Glu Ser Leu
   770 775 780
   Thr
   785
<210> 53
   <211> 2952
   <212> DNA
   <213> Artificial Sequence
<220>
   <223> Codon-optimized CDPS-KS
<400> 53
   atggaatttg atgaaccatt ggttgacgaa gcaagatctt tagtgcagcg tactttacaa      60
   gattatgatg acagatacgg cttcggtact atgtcatgtg ctgcttatga tacagcctgg     120
   gtgtctttag ttacaaaaac agtcgatggg agaaaacaat ggcttttccc agagtgtttt     180
   gaatttctac tagaaacaca atctgatgcc ggaggatggg aaatcgggaa ttcagcacca     240
   atcgacggta tattgaatac agctgcatcc ttacttgctc taaaacgtca cgttcaaact     300
   gagcaaatca tccaacctca acatgaccat aaggatctag caggtagagc tgaacgtgcc     360
   gctgcatctt tgagagcaca attggctgca ttggatgtgt ctacaactga acacgtcggt     420
   tttgagataa ttgttcctgc aatgctagac ccattagaag ccgaagatcc atctctagtt     480
   ttcgattttc cagctaggaa acctttgatg aagattcatg atgctaagat gagtagattc     540
   aggccagaat acttgtatgg caaacaacca atgaccgcct tacattcatt agaggctttc     600
   ataggcaaaa tcgacttcga taaggtaaga caccaccgta cccatgggtc tatgatgggt     660
   tctccttcat ctaccgcagc ctacttaatg cacgcttcac aatgggatgg tgactcagag     720
   gcttacctta gacacgtgat taaacacgca gcagggcagg gaactggtgc tgtaccatct     780
   gctttcccat caacacattt tgagtcatct tggattctta ccacattgtt tagagctgga     840
   ttttcagctt ctcatcttgc ctgtgatgag ttgaacaagt tggtcgagat acttgagggc     900
   tcattcgaga aggaaggtgg ggcaatcggt tacgctccag ggtttcaagc agatgttgat 960
   gatactgcta aaacaataag tacattagca gtccttggaa gagatgctac accaagacaa 1020
   atgatcaagg tatttgaagc taatacacat tttagaacat accctggtga aagagatcct 1080
   tctttgacag ctaattgtaa tgctctatca gccttactac accaaccaga tgcagcaatg 1140
   tatggatctc aaattcaaaa gattaccaaa tttgtctgtg actattggtg gaagtctgat 1200
   ggtaagatta aagataagtg gaacacttgc tacttgtacc catctgtctt attagttgag 1260
   gttttggttg atcttgttag tttattggag cagggtaaat tgcctgatgt tttggatcaa 1320
   gagcttcaat acagagtcgc catcacattg ttccaagcat gtttaaggcc attactagac 1380
   caagatgccg aaggatcatg gaacaagtct atcgaagcca cagcctacgg catccttatc 1440
   ctaactgaag ctaggagagt ttgtttcttc gacagattgt ctgagccatt gaatgaggca 1500
   atccgtagag gtatcgcttt cgccgactct atgtctggaa ctgaagctca gttgaactac 1560
   atttggatcg aaaaggttag ttacgcacct gcattattga ctaaatccta tttgttagca 1620
   gcaagatggg ctgctaagtc tcctttaggc gcttccgtag gctcttcttt gtggactcca 1680
   ccaagagaag gattggataa gcatgtcaga ttattccatc aagctgagtt attcagatcc 1740
   cttccagaat gggaattaag agcctccatg attgaagcag ctttgttcac accacttcta 1800
   agagcacata gactagacgt tttccctaga caagatgtag gtgaagacaa atatcttgat 1860
   gtagttccat tcttttggac tgccgctaac aacagagata gaacttacgc ttccactcta 1920
   ttcctttacg atatgtgttt tatcgcaatg ttaaacttcc agttagacga attcatggag 1980
   gccacagccg gtatcttatt cagagatcat atggatgatt tgaggcaatt gattcatgat 2040
   cttttggcag agaaaacttc cccaaagagt tctggtagaa gtagtcaggg cacaaaagat 2100
   gctgactcag gtatagagga agacgtgtca atgtccgatt cagcttcaga ttcccaggat 2160
   agaagtccag aatacgactt ggttttcagt gcattgagta cctttacaaa acatgtcttg 2220
   caacacccat ctatacaaag tgcctctgta tgggatagaa aactacttgc tagagagatg 2280
   aaggcttact tacttgctca tatccaacaa gcagaagatt caactccatt gtctgaattg 2340
   aaagatgtgc ctcaaaagac tgatgtaaca agagtttcta catctactac taccttcttt 2400
   aactgggtta gaacaacttc cgcagaccat atatcctgcc catactcctt ccactttgta 2460
   gcatgccatc taggcgcagc attgtcacct aaagggtcta acggtgattg ctatccttca 2520
   gctggtgaga agttcttggc agctgcagtc tgcagacatt tggccaccat gtgtagaatg 2580
   tacaacgatc ttggatcagc tgaacgtgat tctgatgaag gtaatttgaa ctccttggac 2640
   ttccctgaat tcgccgattc cgcaggaaac ggagggatag aaattcagaa ggccgctcta 2700
   ttaaggttag ctgagtttga gagagattca tacttagagg ccttccgtcg tttacaagat 2760
   gaatccaata gagttcacgg tccagccggt ggtgatgaag ccagattgtc cagaaggaga 2820
   atggcaatcc ttgaattctt cgcccagcag gtagatttgt acggtcaagt atacgtcatt 2880
   agggatattt ccgctcgtat tcctaaaaac gaggttgaga aaaagagaaa attggatgat 2940
   gctttcaatt ga 2952
<210> 54
   <211> 983
   <212> PRT
   <213> Phomopsis amygdali
<400> 54
   Met Glu Phe Asp Glu Pro Leu Val Asp Glu Ala Arg Ser Leu Val Gln
   1 5 10 15
   Arg Thr Leu Gln Asp Tyr Asp Asp Arg Tyr Gly Phe Gly Thr Met Ser
   20 25 30
   Cys Ala Ala Tyr Asp Thr Ala Trp Val Ser Leu Val Thr Lys Thr Val
   35 40 45
   Asp Gly Arg Lys Gln Trp Leu Phe Pro Glu Cys Phe Glu Phe Leu Leu
   50 55 60
   Glu Thr Gln Ser Asp Ala Gly Gly Trp Glu Ile Gly Asn Ser Ala Pro
   65 70 75 80
   Ile Asp Gly Ile Leu Asn Thr Ala Ala Ser Leu Leu Ala Leu Lys Arg
   85 90 95
   His Val Gln Thr Glu Gln Ile Ile Gln Pro Gln His Asp His Lys Asp
   100 105 110
   Leu Ala Gly Arg Ala Glu Arg Ala Ala Ala Ser Leu Arg Ala Gln Leu
   115 120 125
   Ala Ala Leu Asp Val Ser Thr Thr Glu His Val Gly Phe Glu Ile Ile
   130 135 140
   Val Pro Ala Met Leu Asp Pro Leu Glu Ala Glu Asp Pro Ser Leu Val
   145 150 155 160
   Phe Asp Phe Pro Ala Arg Lys Pro Leu Met Lys Ile His Asp Ala Lys
   165 170 175
   Met Ser Arg Phe Arg Pro Glu Tyr Leu Tyr Gly Lys Gln Pro Met Thr
   180 185 190
   Ala Leu His Ser Leu Glu Ala Phe Ile Gly Lys Ile Asp Phe Asp Lys
           195                 200                 205
   Val Arg His His Arg Thr His Gly Ser Met Met Gly Ser Pro Ser Ser
       210                 215                 220
   Thr Ala Ala Tyr Leu Met His Ala Ser Gln Trp Asp Gly Asp Ser Glu
   225                 230                 235                 240
   Ala Tyr Leu Arg His Val Ile Lys His Ala Ala Gly Gln Gly Thr Gly
                   245                 250                 255
   Ala Val Pro Ser Ala Phe Pro Ser Thr His Phe Glu Ser Ser Trp Ile
               260                 265                 270
   Leu Thr Thr Leu Phe Arg Ala Gly Phe Ser Ala Ser His Leu Ala Cys
           275                 280                 285
   Asp Glu Leu Asn Lys Leu Val Glu Ile Leu Glu Gly Ser Phe Glu Lys
       290                 295                 300
   Glu Gly Gly Ala Ile Gly Tyr Ala Pro Gly Phe Gln Ala Asp Val Asp
   305                 310                 315                 320
   Asp Thr Ala Lys Thr Ile Ser Thr Leu Ala Val Leu Gly Arg Asp Ala
                   325                 330                 335
   Thr Pro Arg Gln Met Ile Lys Val Phe Glu Ala Asn Thr His Phe Arg
               340                 345                 350
   Thr Tyr Pro Gly Glu Arg Asp Pro Ser Leu Thr Ala Asn Cys Asn Ala
           355                 360                 365
   Leu Ser Ala Leu Leu His Gln Pro Asp Ala Ala Met Tyr Gly Ser Gln
       370                 375                 380
   Ile Gln Lys Ile Thr Lys Phe Val Cys Asp Tyr Trp Trp Lys Ser Asp
   385                 390                 395                 400
   Gly Lys Ile Lys Asp Lys Trp Asn Thr Cys Tyr Leu Tyr Pro Ser Val
                   405                 410                 415
   Leu Leu Val Glu Val Leu Val Asp Leu Val Ser Leu Leu Glu Gln Gly
               420                 425                 430
   Lys Leu Pro Asp Val Leu Asp Gln Glu Leu Gln Tyr Arg Val Ala Ile
           435                 440                 445
   Thr Leu Phe Gln Ala Cys Leu Arg Pro Leu Leu Asp Gln Asp Ala Glu
   450 455 460
   Gly Ser Trp Asn Lys Ser Ile Glu Ala Thr Ala Tyr Gly Ile Leu Ile
   465 470 475 480
   Leu Thr Glu Ala Arg Arg Val Cys Phe Phe Asp Arg Leu Ser Glu Pro
   485 490 495
   Leu Asn Glu Ala Ile Arg Arg Gly Ile Ala Phe Ala Asp Ser Met Ser
   500 505 510
   Gly Thr Glu Ala Gln Leu Asn Tyr Ile Trp Ile Glu Lys Val Ser Tyr
   515 520 525
   Ala Pro Ala Leu Leu Thr Lys Ser Tyr Leu Leu Ala Ala Arg Trp Ala
   530 535 540
   Ala Lys Ser Pro Leu Gly Ala Ser Val Gly Ser Ser Leu Trp Thr Pro
   545 550 555 560
   Pro Arg Glu Gly Leu Asp Lys His Val Arg Leu Phe His Gln Ala Glu
   565 570 575
   Leu Phe Arg Ser Leu Pro Glu Trp Glu Leu Arg Ala Ser Met Ile Glu
   580 585 590
   Ala Ala Leu Phe Thr Pro Leu Leu Arg Ala His Arg Leu Asp Val Phe
   595 600 605
   Pro Arg Gln Asp Val Gly Glu Asp Lys Tyr Leu Asp Val Val Pro Phe
   610 615 620
   Phe Trp Thr Ala Ala Asn Asn Arg Asp Arg Thr Tyr Ala Ser Thr Leu
   625 630 635 640
   Phe Leu Tyr Asp Met Cys Phe Ile Ala Met Leu Asn Phe Gln Leu Asp
   645 650 655
   Glu Phe Met Glu Ala Thr Ala Gly Ile Leu Phe Arg Asp His Met Asp
   660 665 670
   Asp Leu Arg Gln Leu Ile His Asp Leu Leu Ala Glu Lys Thr Ser Pro
   675 680 685
   Lys Ser Ser Gly Arg Ser Ser Gln Gly Thr Lys Asp Ala Asp Ser Gly
   690 695 700
   Ile Glu Glu Asp Val Ser Met Ser Asp Ser Ala Ser Asp Ser Gln Asp
   705 710 715 720
   Arg Ser Pro Glu Tyr Asp Leu Val Phe Ser Ala Leu Ser Thr Phe Thr
   725 730 735
   Lys His Val Leu Gln His Pro Ser Ile Gln Ser Ala Ser Val Trp Asp
   740 745 750
   Arg Lys Leu Leu Ala Arg Glu Met Lys Ala Tyr Leu Leu Ala His Ile
   755 760 765
   Gln Gln Ala Glu Asp Ser Thr Pro Leu Ser Glu Leu Lys Asp Val Pro
   770 775 780
   Gln Lys Thr Asp Val Thr Arg Val Ser Thr Ser Thr Thr Thr Phe Phe
   785 790 795 800
   Asn Trp Val Arg Thr Thr Ser Ala Asp His Ile Ser Cys Pro Tyr Ser
   805 810 815
   Phe His Phe Val Ala Cys His Leu Gly Ala Ala Leu Ser Pro Lys Gly
   820 825 830
   Ser Asn Gly Asp Cys Tyr Pro Ser Ala Gly Glu Lys Phe Leu Ala Ala
   835 840 845
   Ala Val Cys Arg His Leu Ala Thr Met Cys Arg Met Tyr Asn Asp Leu
   850 855 860
   Gly Ser Ala Glu Arg Asp Ser Asp Glu Gly Asn Leu Asn Ser Leu Asp
   865 870 875 880
   Phe Pro Glu Phe Ala Asp Ser Ala Gly Asn Gly Gly Ile Glu Ile Gln
   885 890 895
   Lys Ala Ala Leu Leu Arg Leu Ala Glu Phe Glu Arg Asp Ser Tyr Leu
   900 905 910
   Glu Ala Phe Arg Arg Leu Gln Asp Glu Ser Asn Arg Val His Gly Pro
   915 920 925
   Ala Gly Gly Asp Glu Ala Arg Leu Ser Arg Arg Arg Met Ala Ile Leu
   930 935 940
   Glu Phe Phe Ala Gln Gln Val Asp Leu Tyr Gly Gln Val Tyr Val Ile
   945 950 955 960
   Arg Asp Ile Ser Ala Arg Ile Pro Lys Asn Glu Val Glu Lys Lys Arg
   965 970 975
   Lys Leu Asp Asp Ala Phe Asn
   980
<210> 55
   <211> 2646
   <212> DNA
   <213> Artificial Sequence
<220>
   <223> Codon-optimized CDPS-KS
<400> 55
   atggcttcta gtacacttat ccaaaacaga tcatgtggcg tcacatcatc tatgtcaagt      60
   tttcaaatct tcagaggtca accactaaga tttcctggca ctagaacccc agctgcagtt     120
   caatgcttga aaaagaggag atgccttagg ccaaccgaat ccgtactaga atcatctcct     180
   ggctctggtt catatagaat agtaactggc ccttctggaa ttaaccctag ttctaacggg     240
   cacttgcaag agggttcctt gactcacagg ttaccaatac caatggaaaa atctatcgat     300
   aacttccaat ctactctata tgtgtcagat atttggtctg aaacactaca gagaactgaa     360
   tgtttgctac aagtaactga aaacgtccag atgaatgagt ggattgagga aattagaatg     420
   tactttagaa atatgacttt aggtgaaatt tccatgtccc cttacgacac tgcttgggtg     480
   gctagagttc cagcgttgga cggttctcat gggcctcaat tccacagatc tttgcaatgg     540
   attatcgaca accaattacc agatggggac tggggcgaac cttctctttt cttgggttac     600
   gatagagttt gtaatacttt agcctgtgtg attgcgttga aaacatgggg tgttggggca     660
   caaaacgttg aaagaggaat tcagttccta caatctaaca tatacaagat ggaggaagat     720
   gacgctaatc atatgccaat aggattcgaa atcgtattcc ctgctatgat ggaagatgcc     780
   aaagcattag gtttggattt gccatacgat gctactattt tgcaacagat ttcagccgaa     840
   agagagaaaa agatgaaaaa gatcccaatg gcaatggtgt acaaataccc aaccacttta     900
   cttcactcct tagaaggctt gcatagagaa gttgattgga ataagttgtt acaattacaa     960
   tctgaaaatg gtagttttct ttattcacct gcttcaaccg catgcgcctt aatgtacact    1020
   aaggacgtta aatgttttga ttacttaaac cagttgttga tcaagttcga ccacgcatgc    1080
   ccaaatgtat atccagtcga tctattcgaa agattatgga tggttgacag attgcagaga    1140
   ttagggatct ccagatactt tgaaagagag attagagatt gtttacaata cgtctacaga    1200
   tattggaaag attgtggaat cggatgggct tctaactctt ccgtacaaga tgttgatgat    1260
   acagccatgg cgtttagact tttaaggact catggtttcg acgtaaagga agattgcttt 1320
   agacagtttt tcaaggacgg agaattcttc tgcttcgcag gccaatcatc tcaagcagtt 1380
   acaggcatgt ttaatctttc aagagccagt caaacattgt ttccaggaga atctttattg 1440
   aaaaaggcta gaaccttctc tagaaacttc ttgagaacaa agcatgagaa caacgaatgt 1500
   ttcgataaat ggatcattac taaagatttg gctggtgaag tcgagtataa cttgaccttc 1560
   ccatggtatg cctctttgcc tagattagaa cataggacat acttagatca atatggaatc 1620
   gatgatatct ggataggcaa atctttatac aaaatgcctg ctgttaccaa cgaagttttc 1680
   ctaaagttgg caaaggcaga ctttaacatg tgtcaagctc tacacaaaaa ggaattggaa 1740
   caagtgataa agtggaacgc gtcctgtcaa ttcagagatc ttgaattcgc cagacaaaaa 1800
   tcagtagaat gctattttgc tggtgcagcc acaatgttcg aaccagaaat ggttcaagct 1860
   agattagtct gggcaagatg ttgtgtattg acaactgtct tagacgatta ctttgaccac 1920
   gggacacctg ttgaggaact tagagtgttt gttcaagctg tcagaacatg gaatccagag 1980
   ttgatcaacg gtttgccaga gcaagctaaa atcttgttta tgggcttata caaaacagtt 2040
   aacacaattg cagaggaagc attcatggca cagaaaagag acgtccatca tcatttgaaa 2100
   cactattggg acaagttgat aacaagtgcc ctaaaggagg ccgaatgggc agagtcaggt 2160
   tacgtcccaa catttgatga atacatggaa gtagctgaaa tttctgttgc tctagaacca 2220
   attgtctgta gtaccttgtt ctttgcgggt catagactag atgaggatgt tctagatagt 2280
   tacgattacc atctagttat gcatttggta aacagagtcg gtagaatctt gaatgatata 2340
   caaggcatga agagggaggc ttcacaaggt aagatctcat cagttcaaat ctacatggag 2400
   gaacatccat ctgttccatc tgaggccatg gcgatcgctc atcttcaaga gttagttgat 2460
   aattcaatgc agcaattgac atacgaagtt cttaggttca ctgcggttcc aaaaagttgt 2520
   aagagaatcc acttgaatat ggctaaaatc atgcatgcct tctacaagga tactgatgga 2580
   ttctcatccc ttactgcaat gacaggattc gtcaaaaagg ttcttttcga acctgtgcct 2640
   gagtaa 2646
<210> 56
   <211> 881
   <212> PRT
   <213> Physcomitrella patens
<400> 56
   Met Ala Ser Ser Thr Leu Ile Gln Asn Arg Ser Cys Gly Val Thr Ser
   1               5                   10                  15
   Ser Met Ser Ser Phe Gln Ile Phe Arg Gly Gln Pro Leu Arg Phe Pro
               20                  25                  30
   Gly Thr Arg Thr Pro Ala Ala Val Gln Cys Leu Lys Lys Arg Arg Cys
           35                  40                  45
   Leu Arg Pro Thr Glu Ser Val Leu Glu Ser Ser Pro Gly Ser Gly Ser
       50                  55                  60
   Tyr Arg Ile Val Thr Gly Pro Ser Gly Ile Asn Pro Ser Ser Asn Gly
   65                  70                  75                  80
   His Leu Gln Glu Gly Ser Leu Thr His Arg Leu Pro Ile Pro Met Glu
                   85                  90                  95
   Lys Ser Ile Asp Asn Phe Gln Ser Thr Leu Tyr Val Ser Asp Ile Trp
               100                 105                 110
   Ser Glu Thr Leu Gln Arg Thr Glu Cys Leu Leu Gln Val Thr Glu Asn
           115                 120                 125
   Val Gln Met Asn Glu Trp Ile Glu Glu Ile Arg Met Tyr Phe Arg Asn
       130                 135                 140
   Met Thr Leu Gly Glu Ile Ser Met Ser Pro Tyr Asp Thr Ala Trp Val
   145                 150                 155                 160
   Ala Arg Val Pro Ala Leu Asp Gly Ser His Gly Pro Gln Phe His Arg
                   165                 170                 175
   Ser Leu Gln Trp Ile Ile Asp Asn Gln Leu Pro Asp Gly Asp Trp Gly
               180                 185                 190
   Glu Pro Ser Leu Phe Leu Gly Tyr Asp Arg Val Cys Asn Thr Leu Ala
           195                 200                 205
   Cys Val Ile Ala Leu Lys Thr Trp Gly Val Gly Ala Gln Asn Val Glu
       210                 215                 220
   Arg Gly Ile Gln Phe Leu Gln Ser Asn Ile Tyr Lys Met Glu Glu Asp
   225                 230                 235                 240
   Asp Ala Asn His Met Pro Ile Gly Phe Glu Ile Val Phe Pro Ala Met
                   245                 250                 255
   Met Glu Asp Ala Lys Ala Leu Gly Leu Asp Leu Pro Tyr Asp Ala Thr
               260                 265                 270
   Ile Leu Gln Gln Ile Ser Ala Glu Arg Glu Lys Lys Met Lys Lys Ile
           275                 280                 285
   Pro Met Ala Met Val Tyr Lys Tyr Pro Thr Thr Leu Leu His Ser Leu
       290                 295                 300
   Glu Gly Leu His Arg Glu Val Asp Trp Asn Lys Leu Leu Gln Leu Gln
   305                 310                 315                 320
   Ser Glu Asn Gly Ser Phe Leu Tyr Ser Pro Ala Ser Thr Ala Cys Ala
                   325                 330                 335
   Leu Met Tyr Thr Lys Asp Val Lys Cys Phe Asp Tyr Leu Asn Gln Leu
               340                 345                 350
   Leu Ile Lys Phe Asp His Ala Cys Pro Asn Val Tyr Pro Val Asp Leu
           355                 360                 365
   Phe Glu Arg Leu Trp Met Val Asp Arg Leu Gln Arg Leu Gly Ile Ser
       370                 375                 380
   Arg Tyr Phe Glu Arg Glu Ile Arg Asp Cys Leu Gln Tyr Val Tyr Arg
   385                 390                 395                 400
   Tyr Trp Lys Asp Cys Gly Ile Gly Trp Ala Ser Asn Ser Ser Val Gln
                   405                 410                 415
   Asp Val Asp Asp Thr Ala Met Ala Phe Arg Leu Leu Arg Thr His Gly
               420                 425                 430
   Phe Asp Val Lys Glu Asp Cys Phe Arg Gln Phe Phe Lys Asp Gly Glu
           435                 440                 445
   Phe Phe Cys Phe Ala Gly Gln Ser Ser Gln Ala Val Thr Gly Met Phe
       450                 455                 460
   Asn Leu Ser Arg Ala Ser Gln Thr Leu Phe Pro Gly Glu Ser Leu Leu
   465                 470                 475                 480
   Lys Lys Ala Arg Thr Phe Ser Arg Asn Phe Leu Arg Thr Lys His Glu
                   485                 490                 495
   Asn Asn Glu Cys Phe Asp Lys Trp Ile Ile Thr Lys Asp Leu Ala Gly
               500                 505                 510
   Glu Val Glu Tyr Asn Leu Thr Phe Pro Trp Tyr Ala Ser Leu Pro Arg
           515                 520                 525
   Leu Glu His Arg Thr Tyr Leu Asp Gln Tyr Gly Ile Asp Asp Ile Trp
   530 535 540
   Ile Gly Lys Ser Leu Tyr Lys Met Pro Ala Val Thr Asn Glu Val Phe
   545 550 555 560
   Leu Lys Leu Ala Lys Ala Asp Phe Asn Met Cys Gln Ala Leu His Lys
   565 570 575
   Lys Glu Leu Glu Gln Val Ile Lys Trp Asn Ala Ser Cys Gln Phe Arg
   580 585 590
   Asp Leu Glu Phe Ala Arg Gln Lys Ser Val Glu Cys Tyr Phe Ala Gly
   595 600 605
   Ala Ala Thr Met Phe Glu Pro Glu Met Val Gln Ala Arg Leu Val Trp
   610 615 620
   Ala Arg Cys Cys Val Leu Thr Thr Val Leu Asp Asp Tyr Phe Asp His
   625 630 635 640
   Gly Thr Pro Val Glu Glu Leu Arg Val Phe Val Gln Ala Val Arg Thr
   645 650 655
   Trp Asn Pro Glu Leu Ile Asn Gly Leu Pro Glu Gln Ala Lys Ile Leu
   660 665 670
   Phe Met Gly Leu Tyr Lys Thr Val Asn Thr Ile Ala Glu Glu Ala Phe
   675 680 685
   Met Ala Gln Lys Arg Asp Val His His His Leu Lys His Tyr Trp Asp
   690 695 700
   Lys Leu Ile Thr Ser Ala Leu Lys Glu Ala Glu Trp Ala Glu Ser Gly
   705 710 715 720
   Tyr Val Pro Thr Phe Asp Glu Tyr Met Glu Val Ala Glu Ile Ser Val
   725 730 735
   Ala Leu Glu Pro Ile Val Cys Ser Thr Leu Phe Phe Ala Gly His Arg
   740 745 750
   Leu Asp Glu Asp Val Leu Asp Ser Tyr Asp Tyr His Leu Val Met His
   755 760 765
   Leu Val Asn Arg Val Gly Arg Ile Leu Asn Asp Ile Gln Gly Met Lys
   770 775 780
   Arg Glu Ala Ser Gln Gly Lys Ile Ser Ser Val Gln Ile Tyr Met Glu
   785                 790                 795                 800
   Glu His Pro Ser Val Pro Ser Glu Ala Met Ala Ile Ala His Leu Gln
                   805                 810                 815
   Glu Leu Val Asp Asn Ser Met Gln Gln Leu Thr Tyr Glu Val Leu Arg
               820                 825                 830
   Phe Thr Ala Val Pro Lys Ser Cys Lys Arg Ile His Leu Asn Met Ala
           835                 840                 845
   Lys Ile Met His Ala Phe Tyr Lys Asp Thr Asp Gly Phe Ser Ser Leu
       850                 855                 860
   Thr Ala Met Thr Gly Phe Val Lys Lys Val Leu Phe Glu Pro Val Pro
   865                 870                 875                 880
   Glu
<210> 57
   <211> 2859
   <212> DNA
   <213> Artificial Sequence
<220>
   <223> Codon-optimized CDPS-KS
<400> 57
   atgcctggta aaattgaaaa tggtacccca aaggacctca agactggaaa tgattttgtt      60
   tctgctgcta agagtttact agatcgagct ttcaaaagtc atcattccta ctacggatta     120
   tgctcaactt catgtcaagt ttatgataca gcttgggttg caatgattcc aaaaacaaga     180
   gataatgtaa aacagtggtt gtttccagaa tgtttccatt acctcttaaa aacacaagcc     240
   gcagatggct catggggttc attgcctaca acacagacag cgggtatcct agatacagcc     300
   tcagctgtgc tggcattatt gtgccacgca caagagcctt tacaaatatt ggatgtatct     360
   ccagatgaaa tggggttgag aatagaacac ggtgtcacat ccttgaaacg tcaattagca     420
   gtttggaatg atgtggagga caccaaccat attggcgtcg agtttatcat accagcctta     480
   ctttccatgc tagaaaagga attagatgtt ccatcttttg aatttccatg taggtccatc     540
   ttagagagaa tgcacgggga gaaattaggt catttcgacc tggaacaagt ttacggcaag     600
   ccaagctcat tgttgcactc attggaagca tttctcggta agctagattt tgatcgacta     660
   tcacatcacc tataccacgg cagtatgatg gcatctccat cttcaacggc tgcttatctt     720
   attggggcta caaaatggga tgacgaagcc gaagattacc taagacatgt aatgcgtaat     780
   ggtgcaggac atgggaatgg aggtatttct ggtacatttc caactactca tttcgaatgt      840
   agctggatta tagcaacgtt gttaaaggtt ggctttactt tgaagcaaat tgacggcgat      900
   ggcttaagag gtttatcaac catcttactt gaggcgcttc gtgatgagaa tggtgtcata      960
   ggctttgccc ctagaacagc agatgtagat gacacagcca aagctctatt ggccttgtca     1020
   ttggtaaacc agccagtgtc acctgatatc atgattaagg tctttgaggg caaagaccat     1080
   tttaccactt ttggttcaga aagagatcca tcattgactt ccaacctgca cgtcctttta     1140
   tctttactta aacaatctaa cttgtctcaa taccatcctc aaatcctcaa aacaacatta     1200
   ttcacttgta gatggtggtg gggttccgat cattgtgtca aagacaaatg gaatttgagt     1260
   cacctatatc caactatgtt gttggttgaa gccttcactg aagtgctcca tctcattgac     1320
   ggtggtgaat tgtctagtct gtttgatgaa tcctttaagt gtaagattgg tcttagcatc     1380
   tttcaagcgg tacttagaat aatcctcacc caagacaacg acggctcttg gagaggatac     1440
   agagaacaga cgtgttacgc aatattggct ttagttcaag cgagacatgt atgctttttc     1500
   actcacatgg ttgacagact gcaatcatgt gttgatcgag gtttctcatg gttgaaatct     1560
   tgctcttttc attctcaaga cctgacttgg acctctaaaa cagcttatga agtgggtttc     1620
   gtagctgaag catataaact agctgcttta caatctgctt ccctggaggt tcctgctgcc     1680
   accattggac attctgtcac gtctgccgtt ccatcaagtg atcttgaaaa atacatgaga     1740
   ttggtgagaa aaactgcgtt attctctcca ctggatgagt ggggtctaat ggcttctatc     1800
   atcgaatctt catttttcgt accattactg caggcacaaa gagttgaaat ataccctaga     1860
   gataatatca aggtggacga agataagtac ttgtctatta tcccattcac atgggtcgga     1920
   tgcaataata ggtctagaac tttcgcaagt aacagatggc tatacgatat gatgtacctt     1980
   tcattactcg gctatcaaac cgacgagtac atggaagctg tagctgggcc agtgtttggg     2040
   gatgtttcct tgttacatca aacaattgat aaggtgattg ataatacaat gggtaacctt     2100
   gcgagagcca atggaacagt acacagtggt aatggacatc agcacgaatc tcctaatata     2160
   ggtcaagtcg aggacacctt gactcgtttc acaaattcag tcttgaatca caaagacgtc     2220
   cttaactcta gctcatctga tcaagatact ttgagaagag agtttagaac attcatgcac     2280
   gctcatataa cacaaatcga agataactca cgattcagta agcaagcctc atccgatgcg     2340
   ttttcctctc ctgaacaatc ttactttcaa tgggtgaact caactggtgg ctcacatgtc     2400
   gcttgcgcct attcatttgc cttctctaat tgcctcatgt ctgcaaattt gttgcagggt     2460
   aaagacgcat ttccaagcgg aacgcaaaag tacttaatct cctctgttat gagacatgcc     2520
   acaaacatgt gtagaatgta taacgacttt ggctctattg ccagagacaa cgctgagaga     2580
   aatgttaata gtattcattt tcctgagttt actctctgta acggaacttc tcaaaaccta     2640
   gatgaaagga aggaaagact tctgaaaatc gcaacttacg aacaagggta tttggataga     2700
   gcactagagg ccttggaaag acagagtaga gatgatgccg gagacagagc tggatctaaa 2760
   gatatgagaa agttgaaaat cgttaagtta ttctgtgatg ttacggactt atacgatcag 2820
   ctctacgtta tcaaagattt gtcatcctct atgaagtaa 2859
<210> 58
   <211> 952
   <212> PRT
   <213> Gibberella fujikuroi
<400> 58
   Met Pro Gly Lys Ile Glu Asn Gly Thr Pro Lys Asp Leu Lys Thr Gly
   1 5 10 15
   Asn Asp Phe Val Ser Ala Ala Lys Ser Leu Leu Asp Arg Ala Phe Lys
   20 25 30
   Ser His His Ser Tyr Tyr Gly Leu Cys Ser Thr Ser Cys Gln Val Tyr
   35 40 45
   Asp Thr Ala Trp Val Ala Met Ile Pro Lys Thr Arg Asp Asn Val Lys
   50 55 60
   Gln Trp Leu Phe Pro Glu Cys Phe His Tyr Leu Leu Lys Thr Gln Ala
   65 70 75 80
   Ala Asp Gly Ser Trp Gly Ser Leu Pro Thr Thr Gln Thr Ala Gly Ile
   85 90 95
   Leu Asp Thr Ala Ser Ala Val Leu Ala Leu Leu Cys His Ala Gln Glu
   100 105 110
   Pro Leu Gln Ile Leu Asp Val Ser Pro Asp Glu Met Gly Leu Arg Ile
   115 120 125
   Glu His Gly Val Thr Ser Leu Lys Arg Gln Leu Ala Val Trp Asn Asp
   130 135 140
   Val Glu Asp Thr Asn His Ile Gly Val Glu Phe Ile Ile Pro Ala Leu
   145 150 155 160
   Leu Ser Met Leu Glu Lys Glu Leu Asp Val Pro Ser Phe Glu Phe Pro
   165 170 175
   Cys Arg Ser Ile Leu Glu Arg Met His Gly Glu Lys Leu Gly His Phe
   180 185 190
   Asp Leu Glu Gln Val Tyr Gly Lys Pro Ser Ser Leu Leu His Ser Leu
   195 200 205
   Glu Ala Phe Leu Gly Lys Leu Asp Phe Asp Arg Leu Ser His His Leu
   210 215 220
   Tyr His Gly Ser Met Met Ala Ser Pro Ser Ser Thr Ala Ala Tyr Leu
   225 230 235 240
   Ile Gly Ala Thr Lys Trp Asp Asp Glu Ala Glu Asp Tyr Leu Arg His
   245 250 255
   Val Met Arg Asn Gly Ala Gly His Gly Asn Gly Gly Ile Ser Gly Thr
   260 265 270
   Phe Pro Thr Thr His Phe Glu Cys Ser Trp Ile Ile Ala Thr Leu Leu
   275 280 285
   Lys Val Gly Phe Thr Leu Lys Gln Ile Asp Gly Asp Gly Leu Arg Gly
   290 295 300
   Leu Ser Thr Ile Leu Leu Glu Ala Leu Arg Asp Glu Asn Gly Val Ile
   305 310 315 320
   Gly Phe Ala Pro Arg Thr Ala Asp Val Asp Asp Thr Ala Lys Ala Leu
   325 330 335
   Leu Ala Leu Ser Leu Val Asn Gln Pro Val Ser Pro Asp Ile Met Ile
   340 345 350
   Lys Val Phe Glu Gly Lys Asp His Phe Thr Thr Phe Gly Ser Glu Arg
   355 360 365
   Asp Pro Ser Leu Thr Ser Asn Leu His Val Leu Leu Ser Leu Leu Lys
   370 375 380
   Gln Ser Asn Leu Ser Gln Tyr His Pro Gln Ile Leu Lys Thr Thr Leu
   385 390 395 400
   Phe Thr Cys Arg Trp Trp Trp Gly Ser Asp His Cys Val Lys Asp Lys
   405 410 415
   Trp Asn Leu Ser His Leu Tyr Pro Thr Met Leu Leu Val Glu Ala Phe
   420 425 430
   Thr Glu Val Leu His Leu Ile Asp Gly Gly Glu Leu Ser Ser Leu Phe
   435 440 445
   Asp Glu Ser Phe Lys Cys Lys Ile Gly Leu Ser Ile Phe Gln Ala Val
       450                 455                 460
   Leu Arg Ile Ile Leu Thr Gln Asp Asn Asp Gly Ser Trp Arg Gly Tyr
   465                 470                 475                 480
   Arg Glu Gln Thr Cys Tyr Ala Ile Leu Ala Leu Val Gln Ala Arg His
                   485                 490                 495
   Val Cys Phe Phe Thr His Met Val Asp Arg Leu Gln Ser Cys Val Asp
               500                 505                 510
   Arg Gly Phe Ser Trp Leu Lys Ser Cys Ser Phe His Ser Gln Asp Leu
           515                 520                 525
   Thr Trp Thr Ser Lys Thr Ala Tyr Glu Val Gly Phe Val Ala Glu Ala
       530                 535                 540
   Tyr Lys Leu Ala Ala Leu Gln Ser Ala Ser Leu Glu Val Pro Ala Ala
   545                 550                 555                 560
   Thr Ile Gly His Ser Val Thr Ser Ala Val Pro Ser Ser Asp Leu Glu
                   565                 570                 575
   Lys Tyr Met Arg Leu Val Arg Lys Thr Ala Leu Phe Ser Pro Leu Asp
               580                 585                 590
   Glu Trp Gly Leu Met Ala Ser Ile Ile Glu Ser Ser Phe Phe Val Pro
           595                 600                 605
   Leu Leu Gln Ala Gln Arg Val Glu Ile Tyr Pro Arg Asp Asn Ile Lys
       610                 615                 620
   Val Asp Glu Asp Lys Tyr Leu Ser Ile Ile Pro Phe Thr Trp Val Gly
   625                 630                 635                 640
   Cys Asn Asn Arg Ser Arg Thr Phe Ala Ser Asn Arg Trp Leu Tyr Asp
                   645                 650                 655
   Met Met Tyr Leu Ser Leu Leu Gly Tyr Gln Thr Asp Glu Tyr Met Glu
               660                 665                 670
   Ala Val Ala Gly Pro Val Phe Gly Asp Val Ser Leu Leu His Gln Thr
           675                 680                 685
   Ile Asp Lys Val Ile Asp Asn Thr Met Gly Asn Leu Ala Arg Ala Asn
       690                 695                 700
   Gly Thr Val His Ser Gly Asn Gly His Gln His Glu Ser Pro Asn Ile
   705                 710                 715                 720
   Gly Gln Val Glu Asp Thr Leu Thr Arg Phe Thr Asn Ser Val Leu Asn
                   725                 730                 735
   His Lys Asp Val Leu Asn Ser Ser Ser Ser Asp Gln Asp Thr Leu Arg
               740                 745                 750
   Arg Glu Phe Arg Thr Phe Met His Ala His Ile Thr Gln Ile Glu Asp
           755                 760                 765
   Asn Ser Arg Phe Ser Lys Gln Ala Ser Ser Asp Ala Phe Ser Ser Pro
       770                 775                 780
   Glu Gln Ser Tyr Phe Gln Trp Val Asn Ser Thr Gly Gly Ser His Val
   785                 790                 795                 800
   Ala Cys Ala Tyr Ser Phe Ala Phe Ser Asn Cys Leu Met Ser Ala Asn
                   805                 810                 815
   Leu Leu Gln Gly Lys Asp Ala Phe Pro Ser Gly Thr Gln Lys Tyr Leu
               820                 825                 830
   Ile Ser Ser Val Met Arg His Ala Thr Asn Met Cys Arg Met Tyr Asn
           835                 840                 845
   Asp Phe Gly Ser Ile Ala Arg Asp Asn Ala Glu Arg Asn Val Asn Ser
       850                 855                 860
   Ile His Phe Pro Glu Phe Thr Leu Cys Asn Gly Thr Ser Gln Asn Leu
   865                 870                 875                 880
   Asp Glu Arg Lys Glu Arg Leu Leu Lys Ile Ala Thr Tyr Glu Gln Gly
                   885                 890                 895
   Tyr Leu Asp Arg Ala Leu Glu Ala Leu Glu Arg Gln Ser Arg Asp Asp
               900                 905                 910
   Ala Gly Asp Arg Ala Gly Ser Lys Asp Met Arg Lys Leu Lys Ile Val
           915                 920                 925
   Lys Leu Phe Cys Asp Val Thr Asp Leu Tyr Asp Gln Leu Tyr Val Ile
       930                 935                 940
   Lys Asp Leu Ser Ser Ser Met Lys
   945 950
<210> 59
   <211> 1542
   <212> DNA
   <213> Artificial Sequence
<220>
   <223> Codon-optimized KO
<400> 59
   atggatgctg tgacgggttt gttaactgtc ccagcaaccg ctataactat tggtggaact      60
   gctgtagcat tggcggtagc gctaatcttt tggtacctga aatcctacac atcagctaga     120
   agatcccaat caaatcatct tccaagagtg cctgaagtcc caggtgttcc attgttagga     180
   aatctgttac aattgaagga gaaaaagcca tacatgactt ttacgagatg ggcagcgaca     240
   tatggaccta tctatagtat caaaactggg gctacaagta tggttgtggt atcatctaat     300
   gagatagcca aggaggcatt ggtgaccaga ttccaatcca tatctacaag gaacttatct     360
   aaagccctga aagtacttac agcagataag acaatggtcg caatgtcaga ttatgatgat     420
   tatcataaaa cagttaagag acacatactg accgccgtct tgggtcctaa tgcacagaaa     480
   aagcatagaa ttcacagaga tatcatgatg gataacatat ctactcaact tcatgaattc     540
   gtgaaaaaca acccagaaca ggaagaggta gaccttagaa aaatctttca atctgagtta     600
   ttcggcttag ctatgagaca agccttagga aaggatgttg aaagtttgta cgttgaagac     660
   ctgaaaatca ctatgaatag agacgaaatc tttcaagtcc ttgttgttga tccaatgatg     720
   ggagcaatcg atgttgattg gagagacttc tttccatacc taaagtgggt cccaaacaaa     780
   aagttcgaaa atactattca acaaatgtac atcagaagag aagctgttat gaaatcttta     840
   atcaaagagc acaaaaagag aatagcgtca ggcgaaaagc taaatagtta tatcgattac     900
   cttttatctg aagctcaaac tttaaccgat cagcaactat tgatgtcctt gtgggaacca     960
   atcattgaat cttcagatac aacaatggtc acaacagaat gggcaatgta cgaattagct    1020
   aaaaacccta aattgcaaga taggttgtac agagacatta agtccgtctg tggatctgaa    1080
   aagataaccg aagagcatct atcacagctg ccttacatta cagctatttt ccacgaaaca    1140
   ctgagaagac actcaccagt tcctatcatt cctctaagac atgtacatga agataccgtt    1200
   ctaggcggct accatgttcc tgctggcaca gaacttgccg ttaacatcta cggttgcaac    1260
   atggacaaaa acgtttggga aaatccagag gaatggaacc cagaaagatt catgaaagag    1320
   aatgagacaa ttgattttca aaagacgatg gccttcggtg gtggtaagag agtttgtgct    1380
   ggttccttgc aagccctttt aactgcatct attgggattg ggagaatggt tcaagagttc    1440
   gaatggaaac tgaaggatat gactcaagag gaagtgaaca cgataggcct aactacacaa    1500
   atgttaagac cattgagagc tattatcaaa cctaggatct aa                       1542
<210> 60
   <211> 513
   <212> PRT
   <213> Stevia rebaudiana
<400> 60
   Met Asp Ala Val Thr Gly Leu Leu Thr Val Pro Ala Thr Ala Ile Thr
   1               5                   10                  15
   Ile Gly Gly Thr Ala Val Ala Leu Ala Val Ala Leu Ile Phe Trp Tyr
               20                  25                  30
   Leu Lys Ser Tyr Thr Ser Ala Arg Arg Ser Gln Ser Asn His Leu Pro
           35                  40                  45
   Arg Val Pro Glu Val Pro Gly Val Pro Leu Leu Gly Asn Leu Leu Gln
       50                  55                  60
   Leu Lys Glu Lys Lys Pro Tyr Met Thr Phe Thr Arg Trp Ala Ala Thr
   65                  70                  75                  80
   Tyr Gly Pro Ile Tyr Ser Ile Lys Thr Gly Ala Thr Ser Met Val Val
                   85                  90                  95
   Val Ser Ser Asn Glu Ile Ala Lys Glu Ala Leu Val Thr Arg Phe Gln
               100                 105                 110
   Ser Ile Ser Thr Arg Asn Leu Ser Lys Ala Leu Lys Val Leu Thr Ala
           115                 120                 125
   Asp Lys Thr Met Val Ala Met Ser Asp Tyr Asp Asp Tyr His Lys Thr
       130                 135                 140
   Val Lys Arg His Ile Leu Thr Ala Val Leu Gly Pro Asn Ala Gln Lys
   145                 150                 155                 160
   Lys His Arg Ile His Arg Asp Ile Met Met Asp Asn Ile Ser Thr Gln
                   165                 170                 175
   Leu His Glu Phe Val Lys Asn Asn Pro Glu Gln Glu Glu Val Asp Leu
               180                 185                 190
   Arg Lys Ile Phe Gln Ser Glu Leu Phe Gly Leu Ala Met Arg Gln Ala
           195                 200                 205
   Leu Gly Lys Asp Val Glu Ser Leu Tyr Val Glu Asp Leu Lys Ile Thr
       210                 215                 220
   Met Asn Arg Asp Glu Ile Phe Gln Val Leu Val Val Asp Pro Met Met
   225 230 235 240
   Gly Ala Ile Asp Val Asp Trp Arg Asp Phe Phe Pro Tyr Leu Lys Trp
   245 250 255
   Val Pro Asn Lys Lys Phe Glu Asn Thr Ile Gln Gln Met Tyr Ile Arg
   260 265 270
   Arg Glu Ala Val Met Lys Ser Leu Ile Lys Glu His Lys Lys Arg Ile
   275 280 285
   Ala Ser Gly Glu Lys Leu Asn Ser Tyr Ile Asp Tyr Leu Leu Ser Glu
   290 295 300
   Ala Gln Thr Leu Thr Asp Gln Gln Leu Leu Met Ser Leu Trp Glu Pro
   305 310 315 320
   Ile Ile Glu Ser Ser Asp Thr Thr Met Val Thr Thr Glu Trp Ala Met
   325 330 335
   Tyr Glu Leu Ala Lys Asn Pro Lys Leu Gln Asp Arg Leu Tyr Arg Asp
   340 345 350
   Ile Lys Ser Val Cys Gly Ser Glu Lys Ile Thr Glu Glu His Leu Ser
   355 360 365
   Gln Leu Pro Tyr Ile Thr Ala Ile Phe His Glu Thr Leu Arg Arg His
   370 375 380
   Ser Pro Val Pro Ile Ile Pro Leu Arg His Val His Glu Asp Thr Val
   385 390 395 400
   Leu Gly Gly Tyr His Val Pro Ala Gly Thr Glu Leu Ala Val Asn Ile
   405 410 415
   Tyr Gly Cys Asn Met Asp Lys Asn Val Trp Glu Asn Pro Glu Glu Trp
   420 425 430
   Asn Pro Glu Arg Phe Met Lys Glu Asn Glu Thr Ile Asp Phe Gln Lys
   435 440 445
   Thr Met Ala Phe Gly Gly Gly Lys Arg Val Cys Ala Gly Ser Leu Gln
   450 455 460
   Ala Leu Leu Thr Ala Ser Ile Gly Ile Gly Arg Met Val Gln Glu Phe
   465 470 475 480
   Glu Trp Lys Leu Lys Asp Met Thr Gln Glu Glu Val Asn Thr Ile Gly
   485 490 495
   Leu Thr Thr Gln Met Leu Arg Pro Leu Arg Ala Ile Ile Lys Pro Arg
   500 505 510
   Ile
<210> 61
   <211> 1566
   <212> DNA
   <213> Artificial Sequence
<220>
   <223> Codon-optimized KO
<400> 61
   aagcttacta gtaaaatgga cggtgtcatc gatatgcaaa ccattccatt gagaaccgct      60
   attgctattg gtggtactgc tgttgctttg gttgttgcat tatacttttg gttcttgaga      120
   tcctacgctt ccccatctca tcattctaat catttgccac cagtacctga agttccaggt     180
   gttccagttt tgggtaattt gttgcaattg aaagaaaaaa agccttacat gaccttcacc      240
   aagtgggctg aaatgtatgg tccaatctac tctattagaa ctggtgctac ttccatggtt     300
   gttgtctctt ctaacgaaat cgccaaagaa gttgttgtta ccagattccc atctatctct     360
   accagaaaat tgtcttacgc cttgaaggtt ttgaccgaag ataagtctat ggttgccatg     420
   tctgattatc acgattacca taagaccgtc aagagacata ttttgactgc tgttttgggt     480
   ccaaacgccc aaaaaaagtt tagagcacat agagacacca tgatggaaaa cgtttccaat     540
   gaattgcatg ccttcttcga aaagaaccca aatcaagaag tcaacttgag aaagatcttc     600
   caatcccaat tattcggttt ggctatgaag caagccttgg gtaaagatgt tgaatccatc     660
   tacgttaagg atttggaaac caccatgaag agagaagaaa tcttcgaagt tttggttgtc     720
   gatccaatga tgggtgctat tgaagttgat tggagagact ttttcccata cttgaaatgg      780
   gttccaaaca agtccttcga aaacatcatc catagaatgt acactagaag agaagctgtt     840
   atgaaggcct tgatccaaga acacaagaaa agaattgcct ccggtgaaaa cttgaactcc     900
   tacattgatt acttgttgtc tgaagcccaa accttgaccg ataagcaatt attgatgtct     960
   ttgtgggaac ctattatcga atcttctgat accactatgg ttactactga atgggctatg    1020
   tacgaattgg ctaagaatcc aaacatgcaa gacagattat acgaagaaat ccaatccgtt    1080
   tgcggttccg aaaagattac tgaagaaaac ttgtcccaat tgccatactt gtacgctgtt    1140
   ttccaagaaa ctttgagaaa gcactgtcca gttcctatta tgccattgag atatgttcac    1200
   gaaaacaccg ttttgggtgg ttatcatgtt ccagctggta ctgaagttgc tattaacatc    1260
   tacggttgca acatggataa gaaggtctgg gaaaatccag aagaatggaa tccagaaaga    1320
   ttcttgtccg aaaaagaatc catggacttg tacaaaacta tggcttttgg tggtggtaaa    1380
   agagtttgcg ctggttcttt acaagccatg gttatttctt gcattggtat cggtagattg    1440
   gtccaagatt ttgaatggaa gttgaaggat gatgccgaag aagatgttaa cactttgggt    1500
   ttgactaccc aaaagttgca tccattattg gccttgatta acccaagaaa gtaactcgag    1560
   ccgcgg                                                               1566
<210> 62
   <211> 512
   <212> PRT
   <213> Lactuca sativa
<400> 62
   Met Asp Gly Val Ile Asp Met Gln Thr Ile Pro Leu Arg Thr Ala Ile
   1               5                   10                  15
   Ala Ile Gly Gly Thr Ala Val Ala Leu Val Val Ala Leu Tyr Phe Trp
               20                  25                  30
   Phe Leu Arg Ser Tyr Ala Ser Pro Ser His His Ser Asn His Leu Pro
           35                  40                  45
   Pro Val Pro Glu Val Pro Gly Val Pro Val Leu Gly Asn Leu Leu Gln
       50                  55                  60
   Leu Lys Glu Lys Lys Pro Tyr Met Thr Phe Thr Lys Trp Ala Glu Met
   65                  70                  75                  80
   Tyr Gly Pro Ile Tyr Ser Ile Arg Thr Gly Ala Thr Ser Met Val Val
                   85                  90                  95
   Val Ser Ser Asn Glu Ile Ala Lys Glu Val Val Val Thr Arg Phe Pro
               100                 105                 110
   Ser Ile Ser Thr Arg Lys Leu Ser Tyr Ala Leu Lys Val Leu Thr Glu
           115                 120                 125
   Asp Lys Ser Met Val Ala Met Ser Asp Tyr His Asp Tyr His Lys Thr
       130                 135                 140
   Val Lys Arg His Ile Leu Thr Ala Val Leu Gly Pro Asn Ala Gln Lys
   145                 150                 155                 160
   Lys Phe Arg Ala His Arg Asp Thr Met Met Glu Asn Val Ser Asn Glu
                   165                 170                 175
   Leu His Ala Phe Phe Glu Lys Asn Pro Asn Gln Glu Val Asn Leu Arg
               180                 185                 190
   Lys Ile Phe Gln Ser Gln Leu Phe Gly Leu Ala Met Lys Gln Ala Leu
           195                 200                 205
   Gly Lys Asp Val Glu Ser Ile Tyr Val Lys Asp Leu Glu Thr Thr Met
       210                 215                 220
   Lys Arg Glu Glu Ile Phe Glu Val Leu Val Val Asp Pro Met Met Gly
   225                 230                 235                 240
   Ala Ile Glu Val Asp Trp Arg Asp Phe Phe Pro Tyr Leu Lys Trp Val
                   245                 250                 255
   Pro Asn Lys Ser Phe Glu Asn Ile Ile His Arg Met Tyr Thr Arg Arg
               260                 265                 270
   Glu Ala Val Met Lys Ala Leu Ile Gln Glu His Lys Lys Arg Ile Ala
           275                 280                 285
   Ser Gly Glu Asn Leu Asn Ser Tyr Ile Asp Tyr Leu Leu Ser Glu Ala
       290                 295                 300
   Gln Thr Leu Thr Asp Lys Gln Leu Leu Met Ser Leu Trp Glu Pro Ile
   305                 310                 315                 320
   Ile Glu Ser Ser Asp Thr Thr Met Val Thr Thr Glu Trp Ala Met Tyr
                   325                 330                 335
   Glu Leu Ala Lys Asn Pro Asn Met Gln Asp Arg Leu Tyr Glu Glu Ile
               340                 345                 350
   Gln Ser Val Cys Gly Ser Glu Lys Ile Thr Glu Glu Asn Leu Ser Gln
           355                 360                 365
   Leu Pro Tyr Leu Tyr Ala Val Phe Gln Glu Thr Leu Arg Lys His Cys
       370                 375                 380
   Pro Val Pro Ile Met Pro Leu Arg Tyr Val His Glu Asn Thr Val Leu
   385                 390                 395                 400
   Gly Gly Tyr His Val Pro Ala Gly Thr Glu Val Ala Ile Asn Ile Tyr
                   405                 410                 415
   Gly Cys Asn Met Asp Lys Lys Val Trp Glu Asn Pro Glu Glu Trp Asn
   420 425 430
   Pro Glu Arg Phe Leu Ser Glu Lys Glu Ser Met Asp Leu Tyr Lys Thr
   435 440 445
   Met Ala Phe Gly Gly Gly Lys Arg Val Cys Ala Gly Ser Leu Gln Ala
   450 455 460
   Met Val Ile Ser Cys Ile Gly Ile Gly Arg Leu Val Gln Asp Phe Glu
   465 470 475 480
   Trp Lys Leu Lys Asp Asp Ala Glu Glu Asp Val Asn Thr Leu Gly Leu
   485 490 495
   Thr Thr Gln Lys Leu His Pro Leu Leu Ala Leu Ile Asn Pro Arg Lys
   500 505 510
<210> 63
   <211> 1535
   <212> DNA
   <213> Rubus suavissimus
<400> 63
   atggccaccc tccttgagca tttccaagct atgccctttg ccatccctat tgcactggct 60
   gctctgtctt ggctgttcct cttttacatc aaagtttcat tcttttccaa caagagtgct 120
   caggctaagc tccctcctgt gccagtggtt cctgggctgc cggtgattgg gaatttactg 180
   caactcaagg agaagaaacc ctaccagact tttacaaggt gggctgagga gtatggacca 240
   atctattcta tcaggactgg tgcttccacc atggtcgttc tcaataccac ccaagttgca 300
   aaagaggcca tggtgaccag atatttatcc atctcaacca gaaagctatc aaacgcacta 360
   aagattctta ctgctgataa atgtatggtt gcaataagtg actacaacga ttttcacaag 420
   atgataaagc gatacatact ctcaaatgtt cttggaccta gtgctcagaa gcgtcaccgg 480
   agcaacagag ataccttgag agctaatgtc tgcagccgat tgcattctca agtaaagaac 540
   tctcctcgag aagctgtgaa tttcagaaga gtttttgagt gggaactctt tggaattgca 600
   ttgaagcaag cctttggaaa ggacatagaa aagcccattt atgtggagga acttggcact 660
   acactgtcaa gagatgagat ctttaaggtt ctagtgcttg acataatgga gggtgcaatt 720
   gaggttgatt ggagagattt cttcccttac ctgagatgga ttccgaatac gcgcatggaa 780
   acaaaaattc agcgactcta tttccgcagg aaagcagtga tgactgccct gatcaacgag 840
   cagaagaagc gaattgcttc aggagaggaa atcaactgtt atatcgactt cttgcttaag 900
   gaagggaaga cactgacaat ggaccaaata agtatgttgc tttgggagac ggttattgaa 960
   acagcagata ctacaatggt aacgacagaa tgggctatgt atgaagttgc taaagactca 1020
   aagcgtcagg atcgtctcta tcaggaaatc caaaaggttt gtggatcgga gatggttaca 1080
   gaggaatact tgtcccaact gccgtacctg aatgcagttt tccatgaaac gctaaggaag 1140
   cacagtccgg ctgcgttagt tcctttaaga tatgcacatg aagataccca actaggaggt 1200
   tactacattc cagctggaac tgagattgct ataaacatat acgggtgtaa catggacaag 1260
   catcaatggg aaagccctga ggaatggaaa ccggagagat ttttggaccc gaaatttgat 1320
   cctatggatt tgtacaagac catggctttt ggggctggaa agagggtatg tgctggttct 1380
   cttcaggcaa tgttaatagc gtgcccgacg attggtaggc tggtgcagga gtttgagtgg 1440
   aagctgagag atggagaaga agaaaatgta gatactgttg ggctcaccac tcacaaacgc 1500
   tatccaatgc atgcaatcct gaagccaaga agtta 1535
<210> 64
   <211> 1536
   <212> DNA
   <213> Artificial Sequence
<220>
   <223> Codon-optimized KO
<400> 64
   atggctacct tgttggaaca ttttcaagct atgccattcg ctattccaat tgctttggct      60
   gctttgtctt ggttgttttt gttctacatc aaggtttctt tcttctccaa caaatccgct     120
   caagctaaat tgccaccagt tccagttgtt ccaggtttgc cagttattgg taatttgttg     180
   caattgaaag aaaagaagcc ataccaaacc ttcactagat gggctgaaga atatggtcca     240
   atctactcta ttagaactgg tgcttctact atggttgtct tgaacactac tcaagttgcc     300
   aaagaagcta tggttaccag atacttgtct atctctacca gaaagttgtc caacgccttg     360
   aaaattttga ccgctgataa gtgcatggtt gccatttctg attacaacga tttccacaag     420
   atgatcaaga gatatatctt gtctaacgtt ttgggtccat ctgcccaaaa aagacataga     480
   tctaacagag ataccttgag agccaacgtt tgttctagat tgcattccca agttaagaac     540
   tctccaagag aagctgtcaa ctttagaaga gttttcgaat gggaattatt cggtatcgct     600
   ttgaaacaag ccttcggtaa ggatattgaa aagccaatct acgtcgaaga attgggtact     660
   actttgtcca gagatgaaat cttcaaggtt ttggtcttgg acattatgga aggtgccatt     720
   gaagttgatt ggagagattt tttcccatac ttgcgttgga ttccaaacac cagaatggaa     780
   actaagatcc aaagattata ctttagaaga aaggccgtta tgaccgcctt gattaacgaa     840
   caaaagaaaa gaattgcctc cggtgaagaa atcaactgct acatcgattt cttgttgaaa     900
   gaaggtaaga ccttgaccat ggaccaaatc tctatgttgt tgtgggaaac cgttattgaa     960
   actgctgata ccacaatggt tactactgaa tgggctatgt acgaagttgc taaggattct    1020
   aaaagacaag acagattata ccaagaaatc caaaaggtct gcggttctga aatggttaca    1080
   gaagaatact tgtcccaatt gccatacttg aatgctgttt tccacgaaac tttgagaaaa    1140
   cattctccag ctgctttggt tccattgaga tatgctcatg aagatactca attgggtggt    1200
   tattacattc cagccggtac tgaaattgcc attaacatct acggttgcaa catggacaaa    1260
   caccaatggg aatctccaga agaatggaag ccagaaagat ttttggatcc taagtttgac    1320
   ccaatggact tgtacaaaac tatggctttt ggtgctggta aaagagtttg cgctggttct    1380
   ttacaagcta tgttgattgc ttgtccaacc atcggtagat tggttcaaga atttgaatgg    1440
   aagttgagag atggtgaaga agaaaacgtt gatactgttg gtttgaccac ccataagaga    1500
   tatccaatgc atgctatttt gaagccaaga tcttaa                              1536
<210> 65
   <211> 1572
   <212> DNA
   <213> Artificial Sequence
<220>
   <223> Codon-optimized KO
<400> 65
   aagcttacta gtaaaatggc ctccatcacc catttcttac aagattttca agctactcca      60
   ttcgctactg cttttgctgt tggtggtgtt tctttgttga tattcttctt cttcatccgt     120
   ggtttccact ctactaagaa aaacgaatat tacaagttgc caccagttcc agttgttcca     180
   ggtttgccag ttgttggtaa tttgttgcaa ttgaaagaaa agaagccata caagactttc     240
   ttgagatggg ctgaaattca tggtccaatc tactctatta gaactggtgc ttctaccatg     300
   gttgttgtta actctactca tgttgccaaa gaagctatgg ttaccagatt ctcttcaatc     360
   tctaccagaa agttgtccaa ggctttggaa ttattgacct ccaacaaatc tatggttgcc     420
   acctctgatt acaacgaatt tcacaagatg gtcaagaagt acatcttggc cgaattattg     480
   ggtgctaatg ctcaaaagag acacagaatt catagagaca ccttgatcga aaacgtcttg     540
   aacaaattgc atgcccatac caagaattct ccattgcaag ctgttaactt cagaaagatc     600
   ttcgaatctg aattattcgg tttggctatg aagcaagcct tgggttatga tgttgattcc     660
   ttgttcgttg aagaattggg tactaccttg tccagagaag aaatctacaa cgttttggtc     720
   agtgacatgt tgaagggtgc tattgaagtt gattggagag actttttccc atacttgaaa     780
   tggatcccaa acaagtcctt cgaaatgaag attcaaagat tggcctctag aagacaagcc     840
   gttatgaact ctattgtcaa agaacaaaag aagtccattg cctctggtaa gggtgaaaac     900
   tgttacttga attacttgtt gtccgaagct aagactttga ccgaaaagca aatttccatt     960
   ttggcctggg aaaccattat tgaaactgct gatacaactg ttgttaccac tgaatgggct    1020
   atgtacgaat tggctaaaaa cccaaagcaa caagacagat tatacaacga aatccaaaac    1080
   gtctgcggta ctgataagat taccgaagaa catttgtcca agttgcctta cttgtctgct    1140
   gtttttcacg aaaccttgag aaagtattct ccatctccat tggttccatt gagatacgct    1200
   catgaagata ctcaattggg tggttattat gttccagccg gtactgaaat tgctgttaat    1260
   atctacggtt gcaacatgga caagaatcaa tgggaaactc cagaagaatg gaagccagaa    1320
   agatttttgg acgaaaagta cgatccaatg gacatgtaca agactatgtc ttttggttcc    1380
   ggtaaaagag tttgcgctgg ttctttacaa gctagtttga ttgcttgtac ctccatcggt    1440
   agattggttc aagaatttga atggagattg aaagacggtg aagttgaaaa cgttgatacc    1500
   ttgggtttga ctacccataa gttgtatcca atgcaagcta tcttgcaacc tagaaactga    1560
   ctcgagccgc gg                                                        1572
<210> 66
   <211> 514
   <212> PRT
   <213> Castanea mollissima
<400> 66
   Met Ala Ser Ile Thr His Phe Leu Gln Asp Phe Gln Ala Thr Pro Phe
   1               5                   10                  15
   Ala Thr Ala Phe Ala Val Gly Gly Val Ser Leu Leu Ile Phe Phe Phe
               20                  25                  30
   Phe Ile Arg Gly Phe His Ser Thr Lys Lys Asn Glu Tyr Tyr Lys Leu
           35                  40                  45
   Pro Pro Val Pro Val Val Pro Gly Leu Pro Val Val Gly Asn Leu Leu
       50                  55                  60
   Gln Leu Lys Glu Lys Lys Pro Tyr Lys Thr Phe Leu Arg Trp Ala Glu
   65                  70                  75                  80
   Ile His Gly Pro Ile Tyr Ser Ile Arg Thr Gly Ala Ser Thr Met Val
                   85                  90                  95
   Val Val Asn Ser Thr His Val Ala Lys Glu Ala Met Val Thr Arg Phe
               100                 105                 110
   Ser Ser Ile Ser Thr Arg Lys Leu Ser Lys Ala Leu Glu Leu Leu Thr
           115                 120                 125
   Ser Asn Lys Ser Met Val Ala Thr Ser Asp Tyr Asn Glu Phe His Lys
       130                 135                 140
   Met Val Lys Lys Tyr Ile Leu Ala Glu Leu Leu Gly Ala Asn Ala Gln
   145 150 155 160
   Lys Arg His Arg Ile His Arg Asp Thr Leu Ile Glu Asn Val Leu Asn
   165 170 175
   Lys Leu His Ala His Thr Lys Asn Ser Pro Leu Gln Ala Val Asn Phe
   180 185 190
   Arg Lys Ile Phe Glu Ser Glu Leu Phe Gly Leu Ala Met Lys Gln Ala
   195 200 205
   Leu Gly Tyr Asp Val Asp Ser Leu Phe Val Glu Glu Leu Gly Thr Thr
   210 215 220
   Leu Ser Arg Glu Glu Ile Tyr Asn Val Leu Val Ser Asp Met Leu Lys
   225 230 235 240
   Gly Ala Ile Glu Val Asp Trp Arg Asp Phe Phe Pro Tyr Leu Lys Trp
   245 250 255
   Ile Pro Asn Lys Ser Phe Glu Met Lys Ile Gln Arg Leu Ala Ser Arg
   260 265 270
   Arg Gln Ala Val Met Asn Ser Ile Val Lys Glu Gln Lys Lys Ser Ile
   275 280 285
   Ala Ser Gly Lys Gly Glu Asn Cys Tyr Leu Asn Tyr Leu Leu Ser Glu
   290 295 300
   Ala Lys Thr Leu Thr Glu Lys Gln Ile Ser Ile Leu Ala Trp Glu Thr
   305 310 315 320
   Ile Ile Glu Thr Ala Asp Thr Thr Val Val Thr Thr Glu Trp Ala Met
   325 330 335
   Tyr Glu Leu Ala Lys Asn Pro Lys Gln Gln Asp Arg Leu Tyr Asn Glu
   340 345 350
   Ile Gln Asn Val Cys Gly Thr Asp Lys Ile Thr Glu Glu His Leu Ser
   355 360 365
   Lys Leu Pro Tyr Leu Ser Ala Val Phe His Glu Thr Leu Arg Lys Tyr
   370 375 380
   Ser Pro Ser Pro Leu Val Pro Leu Arg Tyr Ala His Glu Asp Thr Gln
   385 390 395 400
   Leu Gly Gly Tyr Tyr Val Pro Ala Gly Thr Glu Ile Ala Val Asn Ile
   405 410 415
   Tyr Gly Cys Asn Met Asp Lys Asn Gln Trp Glu Thr Pro Glu Glu Trp
   420 425 430
   Lys Pro Glu Arg Phe Leu Asp Glu Lys Tyr Asp Pro Met Asp Met Tyr
   435 440 445
   Lys Thr Met Ser Phe Gly Ser Gly Lys Arg Val Cys Ala Gly Ser Leu
   450 455 460
   Gln Ala Ser Leu Ile Ala Cys Thr Ser Ile Gly Arg Leu Val Gln Glu
   465 470 475 480
   Phe Glu Trp Arg Leu Lys Asp Gly Glu Val Glu Asn Val Asp Thr Leu
   485 490 495
   Gly Leu Thr Thr His Lys Leu Tyr Pro Met Gln Ala Ile Leu Gln Pro
   500 505 510
   Arg Asn
<210> 67
   <211> 1512
   <212> DNA
   <213> Artificial Sequence
<220>
   <223> Codon-optimized KO
<400> 67
   atgatttcct tgttgttggg ttttgttgtc tcctccttct tgtttatctt cttcttgaaa 60
   aaattgttgt tcttcttcag tcgtcacaaa atgtccgaag tttctagatt gccatctgtt 120
   ccagttccag gttttccatt gattggtaac ttgttgcaat tgaaagaaaa gaagccacac 180
   aagactttca ccaagtggtc tgaattatat ggtccaatct actctatcaa gatgggttcc 240
   tcttctttga tcgtcttgaa ctctattgaa accgccaaag aagctatggt cagtagattc 300
   tcttcaatct ctaccagaaa gttgtctaac gctttgactg ttttgacctg caacaaatct 360
   atggttgcta cctctgatta cgatgacttt cataagttcg tcaagagatg cttgttgaac 420
   ggtttgttgg gtgctaatgc tcaagaaaga aaaagacatt acagagatgc cttgatcgaa 480
   aacgttacct ctaaattgca tgcccatacc agaaatcatc cacaagaacc agttaacttc 540
   agagccattt tcgaacacga attattcggt gttgctttga aacaagcctt cggtaaagat 600
   gtcgaatcca tctatgtaaa agaattgggt gtcaccttgt ccagagatga aattttcaag 660
   gttttggtcc acgacatgat ggaaggtgct attgatgttg attggagaga tttcttccca 720
   tacttgaaat ggatcccaaa caactctttc gaagccagaa ttcaacaaaa gcacaagaga 780
   agattggctg ttatgaacgc cttgatccaa gacagattga atcaaaacga ttccgaatcc 840
   gatgatgact gctacttgaa tttcttgatg tctgaagcta agaccttgac catggaacaa 900
   attgctattt tggtttggga aaccattatc gaaactgctg ataccacttt ggttactact 960
   gaatgggcta tgtacgaatt ggccaaacat caatctgttc aagatagatt attcaaagaa 1020
   atccaatccg tctgcggtgg tgaaaagatc aaagaagaac aattgccaag attgccttac 1080
   gtcaatggtg tttttcacga aaccttgaga aagtattctc cagctccatt ggttccaatt 1140
   agatacgctc atgaagatac ccaaattggt ggttatcata ttccagccgg ttctgaaatt 1200
   gccattaaca tctacggttg caacatggat aagaagagat gggaaagacc tgaagaatgg 1260
   tggccagaaa gatttttgga agatagatac gaatcctccg acttgcataa gactatggct 1320
   tttggtgctg gtaaaagagt ttgtgctggt gctttacaag ctagtttgat ggctggtatt 1380
   gctatcggta gattggttca agaattcgaa tggaagttga gagatggtga agaagaaaac 1440
   gttgatactt acggtttgac ctcccaaaag ttgtatccat tgatggccat tatcaaccca 1500
   agaagatctt aa 1512
<210> 68
   <211> 506
   <212> PRT
   <213> Thellungiella halophila
<400> 68
   Met Ala Ser Met Ile Ser Leu Leu Leu Gly Phe Val Val Ser Ser Phe
   1               5                   10                  15
   Leu Phe Ile Phe Phe Leu Lys Lys Leu Leu Phe Phe Phe Ser Arg His
               20                  25                  30
   Lys Met Ser Glu Val Ser Arg Leu Pro Ser Val Pro Val Pro Gly Phe
           35                  40                  45
   Pro Leu Ile Gly Asn Leu Leu Gln Leu Lys Glu Lys Lys Pro His Lys
       50                  55                  60
   Thr Phe Thr Lys Trp Ser Glu Leu Tyr Gly Pro Ile Tyr Ser Ile Lys
   65                  70                  75                  80
   Met Gly Ser Ser Ser Leu Ile Val Leu Asn Ser Ile Glu Thr Ala Lys
                   85                  90                  95
   Glu Ala Met Val Ser Arg Phe Ser Ser Ile Ser Thr Arg Lys Leu Ser
               100                 105                 110
   Asn Ala Leu Thr Val Leu Thr Cys Asn Lys Ser Met Val Ala Thr Ser
           115                 120                 125
   Asp Tyr Asp Asp Phe His Lys Phe Val Lys Arg Cys Leu Leu Asn Gly
       130                 135                 140
   Leu Leu Gly Ala Asn Ala Gln Glu Arg Lys Arg His Tyr Arg Asp Ala
   145                 150                 155                 160
   Leu Ile Glu Asn Val Thr Ser Lys Leu His Ala His Thr Arg Asn His
                   165                 170                 175
   Pro Gln Glu Pro Val Asn Phe Arg Ala Ile Phe Glu His Glu Leu Phe
               180                 185                 190
   Gly Val Ala Leu Lys Gln Ala Phe Gly Lys Asp Val Glu Ser Ile Tyr
           195                 200                 205
   Val Lys Glu Leu Gly Val Thr Leu Ser Arg Asp Glu Ile Phe Lys Val
       210                 215                 220
   Leu Val His Asp Met Met Glu Gly Ala Ile Asp Val Asp Trp Arg Asp
   225                 230                 235                 240
   Phe Phe Pro Tyr Leu Lys Trp Ile Pro Asn Asn Ser Phe Glu Ala Arg
                   245                 250                 255
   Ile Gln Gln Lys His Lys Arg Arg Leu Ala Val Met Asn Ala Leu Ile
               260                 265                 270
   Gln Asp Arg Leu Asn Gln Asn Asp Ser Glu Ser Asp Asp Asp Cys Tyr
           275                 280                 285
   Leu Asn Phe Leu Met Ser Glu Ala Lys Thr Leu Thr Met Glu Gln Ile
       290                 295                 300
   Ala Ile Leu Val Trp Glu Thr Ile Ile Glu Thr Ala Asp Thr Thr Leu
   305                 310                 315                 320
   Val Thr Thr Glu Trp Ala Met Tyr Glu Leu Ala Lys His Gln Ser Val
                   325                 330                 335
   Gln Asp Arg Leu Phe Lys Glu Ile Gln Ser Val Cys Gly Gly Glu Lys
               340                 345                 350
   Ile Lys Glu Glu Gln Leu Pro Arg Leu Pro Tyr Val Asn Gly Val Phe
           355                 360                 365
   His Glu Thr Leu Arg Lys Tyr Ser Pro Ala Pro Leu Val Pro Ile Arg
       370                 375                 380
   Tyr Ala His Glu Asp Thr Gln Ile Gly Gly Tyr His Ile Pro Ala Gly
   385                 390                 395                 400
   Ser Glu Ile Ala Ile Asn Ile Tyr Gly Cys Asn Met Asp Lys Lys Arg
                   405                 410                 415
   Trp Glu Arg Pro Glu Glu Trp Trp Pro Glu Arg Phe Leu Glu Asp Arg
               420                 425                 430
   Tyr Glu Ser Ser Asp Leu His Lys Thr Met Ala Phe Gly Ala Gly Lys
           435                 440                 445
   Arg Val Cys Ala Gly Ala Leu Gln Ala Ser Leu Met Ala Gly Ile Ala
       450                 455                 460
   Ile Gly Arg Leu Val Gln Glu Phe Glu Trp Lys Leu Arg Asp Gly Glu
   465                 470                 475                 480
   Glu Glu Asn Val Asp Thr Tyr Gly Leu Thr Ser Gln Lys Leu Tyr Pro
                   485                 490                 495
   Leu Met Ala Ile Ile Asn Pro Arg Arg Ser
               500                 505
<210> 69
   <211> 1554
   <212> DNA
   <213> Artificial Sequence
<220>
   <223> Codon-optimized KO
<400> 69
   aagcttacta gtaaaatgga catgatgggt attgaagctg ttccatttgc tactgctgtt      60
   gttttgggtg gtatttcctt ggttgttttg atcttcatca gaagattcgt ttccaacaga     120
   aagagatccg ttgaaggttt gccaccagtt ccagatattc caggtttacc attgattggt     180
   aacttgttgc aattgaaaga aaagaagcca cataagacct ttgctagatg ggctgaaact     240
   tacggtccaa ttttctctat tagaactggt gcttctacca tgatcgtctt gaattcttct     300
   gaagttgcca aagaagctat ggtcactaga ttctcttcaa tctctaccag aaagttgtcc     360
   aacgccttga agattttgac cttcgataag tgtatggttg ccacctctga ttacaacgat      420
   tttcacaaaa tggtcaaggg tttcatcttg agaaacgttt taggtgctcc agcccaaaaa      480
   agacatagat gtcatagaga taccttgatc gaaaacatct ctaagtactt gcatgcccat      540
   gttaagactt ctccattgga accagttgtc ttgaagaaga ttttcgaatc cgaaattttc      600
   ggtttggctt tgaaacaagc cttgggtaag gatatcgaat ccatctatgt tgaagaattg      660
   ggtactacct tgtccagaga agaaattttt gccgttttgg ttgttgatcc aatggctggt      720
   gctattgaag ttgattggag agattttttc ccatacttgt cctggattcc aaacaagtct       780
   atggaaatga agatccaaag aatggatttt agaagaggtg ctttgatgaa ggccttgatt      840
   ggtgaacaaa agaaaagaat cggttccggt gaagaaaaga actcctacat tgatttcttg      900
   ttgtctgaag ctaccacttt gaccgaaaag caaattgcta tgttgatctg ggaaaccatc      960
   atcgaaattt ccgatacaac tttggttacc tctgaatggg ctatgtacga attggctaaa     1020
   gacccaaata gacaagaaat cttgtacaga gaaatccaca aggtttgcgg ttctaacaag     1080
   ttgactgaag aaaacttgtc caagttgcca tacttgaact ctgttttcca cgaaaccttg     1140
   agaaagtatt ctccagctcc aatggttcca gttagatatg ctcatgaaga tactcaattg     1200
   ggtggttacc atattccagc tggttctcaa attgccatta acatctacgg ttgcaacatg     1260
   aacaaaaagc aatgggaaaa tcctgaagaa tggaagccag aaagattctt ggacgaaaag     1320
   tatgacttga tggacttgca taagactatg gcttttggtg gtggtaaaag agtttgtgct     1380
   ggtgctttac aagcaatgtt gattgcttgc acttccatcg gtagattcgt tcaagaattt     1440
   gaatggaagt tgatgggtgg tgaagaagaa aacgttgata ctgttgcttt gacctcccaa     1500
   aaattgcatc caatgcaagc cattattaag gccagagaat gactcgagcc gcgg           1554
<210> 70
   <211> 508
   <212> PRT
   <213> Vitis vinifera
<400> 70
   Met Asp Met Met Gly Ile Glu Ala Val Pro Phe Ala Thr Ala Val Val
   1               5                   10                  15
   Leu Gly Gly Ile Ser Leu Val Val Leu Ile Phe Ile Arg Arg Phe Val
               20                  25                  30
   Ser Asn Arg Lys Arg Ser Val Glu Gly Leu Pro Pro Val Pro Asp Ile
           35                  40                  45
   Pro Gly Leu Pro Leu Ile Gly Asn Leu Leu Gln Leu Lys Glu Lys Lys
       50                  55                  60
   Pro His Lys Thr Phe Ala Arg Trp Ala Glu Thr Tyr Gly Pro Ile Phe
   65                  70                  75                  80
   Ser Ile Arg Thr Gly Ala Ser Thr Met Ile Val Leu Asn Ser Ser Glu
                   85                  90                  95
   Val Ala Lys Glu Ala Met Val Thr Arg Phe Ser Ser Ile Ser Thr Arg
               100                 105                 110
   Lys Leu Ser Asn Ala Leu Lys Ile Leu Thr Phe Asp Lys Cys Met Val
           115                 120                 125
   Ala Thr Ser Asp Tyr Asn Asp Phe His Lys Met Val Lys Gly Phe Ile
       130                 135                 140
   Leu Arg Asn Val Leu Gly Ala Pro Ala Gln Lys Arg His Arg Cys His
   145                 150                 155                 160
   Arg Asp Thr Leu Ile Glu Asn Ile Ser Lys Tyr Leu His Ala His Val
                   165                 170                 175
   Lys Thr Ser Pro Leu Glu Pro Val Val Leu Lys Lys Ile Phe Glu Ser
               180                 185                 190
   Glu Ile Phe Gly Leu Ala Leu Lys Gln Ala Leu Gly Lys Asp Ile Glu
           195                 200                 205
   Ser Ile Tyr Val Glu Glu Leu Gly Thr Thr Leu Ser Arg Glu Glu Ile
       210                 215                 220
   Phe Ala Val Leu Val Val Asp Pro Met Ala Gly Ala Ile Glu Val Asp
   225                 230                 235                 240
   Trp Arg Asp Phe Phe Pro Tyr Leu Ser Trp Ile Pro Asn Lys Ser Met
                   245                 250                 255
   Glu Met Lys Ile Gln Arg Met Asp Phe Arg Arg Gly Ala Leu Met Lys
               260                 265                 270
   Ala Leu Ile Gly Glu Gln Lys Lys Arg Ile Gly Ser Gly Glu Glu Lys
           275                 280                 285
   Asn Ser Tyr Ile Asp Phe Leu Leu Ser Glu Ala Thr Thr Leu Thr Glu
       290                 295                 300
   Lys Gln Ile Ala Met Leu Ile Trp Glu Thr Ile Ile Glu Ile Ser Asp
   305                 310                 315                 320
   Thr Thr Leu Val Thr Ser Glu Trp Ala Met Tyr Glu Leu Ala Lys Asp
   325 330 335
   Pro Asn Arg Gln Glu Ile Leu Tyr Arg Glu Ile His Lys Val Cys Gly
   340 345 350
   Ser Asn Lys Leu Thr Glu Glu Asn Leu Ser Lys Leu Pro Tyr Leu Asn
   355 360 365
   Ser Val Phe His Glu Thr Leu Arg Lys Tyr Ser Pro Ala Pro Met Val
   370 375 380
   Pro Val Arg Tyr Ala His Glu Asp Thr Gln Leu Gly Gly Tyr His Ile
   385 390 395 400
   Pro Ala Gly Ser Gln Ile Ala Ile Asn Ile Tyr Gly Cys Asn Met Asn
   405 410 415
   Lys Lys Gln Trp Glu Asn Pro Glu Glu Trp Lys Pro Glu Arg Phe Leu
   420 425 430
   Asp Glu Lys Tyr Asp Leu Met Asp Leu His Lys Thr Met Ala Phe Gly
   435 440 445
   Gly Gly Lys Arg Val Cys Ala Gly Ala Leu Gln Ala Met Leu Ile Ala
   450 455 460
   Cys Thr Ser Ile Gly Arg Phe Val Gln Glu Phe Glu Trp Lys Leu Met
   465 470 475 480
   Gly Gly Glu Glu Glu Asn Val Asp Thr Val Ala Leu Thr Ser Gln Lys
   485 490 495
   Leu His Pro Met Gln Ala Ile Ile Lys Ala Arg Glu
   500 505
<210> 71
   <211> 1593
   <212> DNA
   <213> Artificial Sequence
<220>
   <223> Codon-optimized KO
<400> 71
   aagcttaaaa tgagtaagtc taatagtatg aattctacat cacacgaaac cctttttcaa 60
   caattggtct tgggtttgga ccgtatgcca ttgatggatg ttcactggtt gatctacgtt 120
   gctttcggcg catggttatg ttcttatgtg atacatgttt tatcatcttc ctctacagta 180
   aaagtgccag ttgttggata caggtctgta ttcgaaccta catggttgct tagacttaga 240
   ttcgtctggg aaggtggctc tatcataggt caagggtaca ataagtttaa agactctatt 300
   ttccaagtta ggaaattggg aactgatatt gtcattatac cacctaacta tattgatgaa 360
   gtgagaaaat tgtcacagga caagactaga tcagttgaac ctttcattaa tgattttgca 420
   ggtcaataca caagaggcat ggttttcttg caatctgact tacaaaaccg tgttatacaa 480
   caaagactaa ctccaaaatt ggtttccttg accaaggtca tgaaggaaga gttggattat 540
   gctttaacaa aagagatgcc tgatatgaaa aatgacgaat gggtagaagt agatatcagt 600
   agtataatgg tgagattgat ttccaggatc tccgccagag tctttctagg gcctgaacac 660
   tgtcgtaacc aggaatggtt gactactaca gcagaatatt cagaatcact tttcattaca 720
   gggtttatct taagagttgt acctcatatc ttaagaccat tcatcgcccc tctattacct 780
   tcatacagga ctctacttag aaacgtttca agtggtagaa gagtcatcgg tgacatcata 840
   agatctcagc aaggggatgg taacgaagat atactttcct ggatgagaga tgctgccaca 900
   ggagaggaaa agcaaatcga taacattgct cagagaatgt taattctttc tttagcatca 960
   atccacacta ctgcgatgac catgacacat gccatgtacg atctatgtgc ttgccctgag 1020
   tacattgaac cattaagaga tgaagttaaa tctgttgttg gggcttctgg ctgggacaag 1080
   acagcgttaa acagatttca taagttggac tccttcctaa aagagtcaca aagattcaac 1140
   ccagtattct tattgacatt caatagaatc taccatcaat ctatgacctt atcagatggc 1200
   actaacattc catctggaac acgtattgct gttccatcac acgcaatgtt gcaagattct 1260
   gcacatgtcc caggtccaac cccacctact gaatttgatg gattcagata tagtaagata 1320
   cgttctgata gtaactacgc acaaaagtac ctattctcca tgaccgattc ttcaaacatg 1380
   gctttcggat acggcaagta tgcttgtcca ggtagatttt acgcgtctaa tgagatgaaa 1440
   ctaacattag ccattttgtt gctacaattt gagttcaaac taccagatgg taaaggtcgt 1500
   cctagaaata tcactatcga ttctgatatg attccagacc caagagctag actttgcgtc 1560
   agaaaaagat cacttagaga tgaatgaccg cgg 1593
<210> 72
   <211> 525
   <212> PRT
   <213> Gibberella fujikuroi
<400> 72
   Met Ser Lys Ser Asn Ser Met Asn Ser Thr Ser His Glu Thr Leu Phe
   1 5 10 15
   Gln Gln Leu Val Leu Gly Leu Asp Arg Met Pro Leu Met Asp Val His
   20 25 30
   Trp Leu Ile Tyr Val Ala Phe Gly Ala Trp Leu Cys Ser Tyr Val Ile
   35 40 45
   His Val Leu Ser Ser Ser Ser Thr Val Lys Val Pro Val Val Gly Tyr
   50 55 60
   Arg Ser Val Phe Glu Pro Thr Trp Leu Leu Arg Leu Arg Phe Val Trp
   65 70 75 80
   Glu Gly Gly Ser Ile Ile Gly Gln Gly Tyr Asn Lys Phe Lys Asp Ser
   85 90 95
   Ile Phe Gln Val Arg Lys Leu Gly Thr Asp Ile Val Ile Ile Pro Pro
   100 105 110
   Asn Tyr Ile Asp Glu Val Arg Lys Leu Ser Gln Asp Lys Thr Arg Ser
   115 120 125
   Val Glu Pro Phe Ile Asn Asp Phe Ala Gly Gln Tyr Thr Arg Gly Met
   130 135 140
   Val Phe Leu Gln Ser Asp Leu Gln Asn Arg Val Ile Gln Gln Arg Leu
   145 150 155 160
   Thr Pro Lys Leu Val Ser Leu Thr Lys Val Met Lys Glu Glu Leu Asp
   165 170 175
   Tyr Ala Leu Thr Lys Glu Met Pro Asp Met Lys Asn Asp Glu Trp Val
   180 185 190
   Glu Val Asp Ile Ser Ser Ile Met Val Arg Leu Ile Ser Arg Ile Ser
   195 200 205
   Ala Arg Val Phe Leu Gly Pro Glu His Cys Arg Asn Gln Glu Trp Leu
   210 215 220
   Thr Thr Thr Ala Glu Tyr Ser Glu Ser Leu Phe Ile Thr Gly Phe Ile
   225 230 235 240
   Leu Arg Val Val Pro His Ile Leu Arg Pro Phe Ile Ala Pro Leu Leu
   245 250 255
   Pro Ser Tyr Arg Thr Leu Leu Arg Asn Val Ser Ser Gly Arg Arg Val
   260 265 270
   Ile Gly Asp Ile Ile Arg Ser Gln Gln Gly Asp Gly Asn Glu Asp Ile
           275                 280                 285
   Leu Ser Trp Met Arg Asp Ala Ala Thr Gly Glu Glu Lys Gln Ile Asp
       290                 295                 300
   Asn Ile Ala Gln Arg Met Leu Ile Leu Ser Leu Ala Ser Ile His Thr
   305                 310                 315                 320
   Thr Ala Met Thr Met Thr His Ala Met Tyr Asp Leu Cys Ala Cys Pro
                   325                 330                 335
   Glu Tyr Ile Glu Pro Leu Arg Asp Glu Val Lys Ser Val Val Gly Ala
               340                 345                 350
   Ser Gly Trp Asp Lys Thr Ala Leu Asn Arg Phe His Lys Leu Asp Ser
           355                 360                 365
   Phe Leu Lys Glu Ser Gln Arg Phe Asn Pro Val Phe Leu Leu Thr Phe
       370                 375                 380
   Asn Arg Ile Tyr His Gln Ser Met Thr Leu Ser Asp Gly Thr Asn Ile
   385                 390                 395                 400
   Pro Ser Gly Thr Arg Ile Ala Val Pro Ser His Ala Met Leu Gln Asp
                   405                 410                 415
   Ser Ala His Val Pro Gly Pro Thr Pro Pro Thr Glu Phe Asp Gly Phe
               420                 425                 430
   Arg Tyr Ser Lys Ile Arg Ser Asp Ser Asn Tyr Ala Gln Lys Tyr Leu
           435                 440                 445
   Phe Ser Met Thr Asp Ser Ser Asn Met Ala Phe Gly Tyr Gly Lys Tyr
       450                 455                 460
   Ala Cys Pro Gly Arg Phe Tyr Ala Ser Asn Glu Met Lys Leu Thr Leu
   465                 470                 475                 480
   Ala Ile Leu Leu Leu Gln Phe Glu Phe Lys Leu Pro Asp Gly Lys Gly
                   485                 490                 495
   Arg Pro Arg Asn Ile Thr Ile Asp Ser Asp Met Ile Pro Asp Pro Arg
               500                 505                 510
   Ala Arg Leu Cys Val Arg Lys Arg Ser Leu Arg Asp Glu
           515                 520                 525
<210> 73
   <211> 1515
   <212> DNA
   <213> Artificial Sequence
<220>
   <223> Codon-optimized KO
<400> 73
   aagcttaaaa tggaagatcc tactgtctta tatgcttgtc ttgccattgc agttgcaact      60
   ttcgttgtta gatggtacag agatccattg agatccatcc caacagttgg tggttccgat     120
   ttgcctattc tatcttacat cggcgcacta agatggacaa gacgtggcag agagatactt     180
   caagagggat atgatggcta cagaggatct acattcaaaa tcgcgatgtt agaccgttgg     240
   atcgtgatcg caaatggtcc taaactagct gatgaagtca gacgtagacc agatgaagag     300
   ttaaacttta tggacggatt aggagcattc gtccaaacta agtacacctt aggtgaagct     360
   attcataacg atccatacca tgtcgatatc ataagagaaa aactaacaag aggccttcca     420
   gccgtgcttc ctgatgtcat tgaagagttg acacttgcgg ttagacagta cattccaaca     480
   gaaggtgatg aatgggtgtc cgtaaactgt tcaaaggccg caagagatat tgttgctaga     540
   gcttctaata gagtctttgt aggtttgcct gcttgcagaa accaaggtta cttagatttg     600
   gcaatagact ttacattgtc tgttgtcaag gatagagcca tcatcaatat gtttccagaa     660
   ttgttgaagc caatagttgg cagagttgta ggtaacgcca ccagaaatgt tcgtagagct     720
   gttccttttg ttgctccatt ggtggaggaa agacgtagac ttatggaaga gtacggtgaa     780
   gactggtctg aaaaacctaa tgatatgtta cagtggataa tggatgaagc tgcatccaga     840
   gatagttcag tgaaggcaat cgcagagaga ttgttaatgg tgaacttcgc ggctattcat     900
   acctcatcaa acactatcac tcatgctttg taccaccttg ccgaaatgcc tgaaactttg     960
   caaccactta gagaagagat cgaaccatta gtcaaagagg agggctggac caaggctgct    1020
   atgggaaaaa tgtggtggtt agattcattt ctaagagaat ctcaaagata caatggcatt    1080
   aacatcgtat ctttaactag aatggctgac aaagatatta cattgagtga tggcacattt    1140
   ttgccaaaag gtactctagt ggccgttcca gcgtattcta ctcatagaga tgatgctgtc    1200
   tacgctgatg ccttagtatt cgatcctttc agattctcac gtatgagagc gagagaaggt    1260
   gaaggtacaa agcaccagtt cgttaatact tcagtcgagt acgttccatt tggtcacgga    1320
   aagcatgctt gtccaggaag attcttcgcc gcaaacgaat tgaaagcaat gttggcttac    1380
   attgttctaa actatgatgt aaagttgcct ggtgacggta aacgtccatt gaacatgtat    1440
   tggggtccaa cagttttgcc tgcaccagca ggccaagtat tgttcagaaa gagacaagtt    1500
   agtctataac cgcgg                                                     1515
<210> 74
   <211> 499
   <212> PRT
   <213> Trametes versicolor
<400> 74
   Met Glu Asp Pro Thr Val Leu Tyr Ala Cys Leu Ala Ile Ala Val Ala
   1               5                   10                  15
   Thr Phe Val Val Arg Trp Tyr Arg Asp Pro Leu Arg Ser Ile Pro Thr
               20                  25                  30
   Val Gly Gly Ser Asp Leu Pro Ile Leu Ser Tyr Ile Gly Ala Leu Arg
           35                  40                  45
   Trp Thr Arg Arg Gly Arg Glu Ile Leu Gln Glu Gly Tyr Asp Gly Tyr
       50                  55                  60
   Arg Gly Ser Thr Phe Lys Ile Ala Met Leu Asp Arg Trp Ile Val Ile
   65                  70                  75                  80
   Ala Asn Gly Pro Lys Leu Ala Asp Glu Val Arg Arg Arg Pro Asp Glu
                   85                  90                  95
   Glu Leu Asn Phe Met Asp Gly Leu Gly Ala Phe Val Gln Thr Lys Tyr
               100                 105                 110
   Thr Leu Gly Glu Ala Ile His Asn Asp Pro Tyr His Val Asp Ile Ile
           115                 120                 125
   Arg Glu Lys Leu Thr Arg Gly Leu Pro Ala Val Leu Pro Asp Val Ile
       130                 135                 140
   Glu Glu Leu Thr Leu Ala Val Arg Gln Tyr Ile Pro Thr Glu Gly Asp
   145                 150                 155                 160
   Glu Trp Val Ser Val Asn Cys Ser Lys Ala Ala Arg Asp Ile Val Ala
                   165                 170                 175
   Arg Ala Ser Asn Arg Val Phe Val Gly Leu Pro Ala Cys Arg Asn Gln
               180                 185                 190
   Gly Tyr Leu Asp Leu Ala Ile Asp Phe Thr Leu Ser Val Val Lys Asp
           195                 200                 205
   Arg Ala Ile Ile Asn Met Phe Pro Glu Leu Leu Lys Pro Ile Val Gly
       210                 215                 220
   Arg Val Val Gly Asn Ala Thr Arg Asn Val Arg Arg Ala Val Pro Phe
   225 230 235 240
   Val Ala Pro Leu Val Glu Glu Arg Arg Arg Leu Met Glu Glu Tyr Gly
   245 250 255
   Glu Asp Trp Ser Glu Lys Pro Asn Asp Met Leu Gln Trp Ile Met Asp
   260 265 270
   Glu Ala Ala Ser Arg Asp Ser Ser Val Lys Ala Ile Ala Glu Arg Leu
   275 280 285
   Leu Met Val Asn Phe Ala Ala Ile His Thr Ser Ser Asn Thr Ile Thr
   290 295 300
   His Ala Leu Tyr His Leu Ala Glu Met Pro Glu Thr Leu Gln Pro Leu
   305 310 315 320
   Arg Glu Glu Ile Glu Pro Leu Val Lys Glu Glu Gly Trp Thr Lys Ala
   325 330 335
   Ala Met Gly Lys Met Trp Trp Leu Asp Ser Phe Leu Arg Glu Ser Gln
   340 345 350
   Arg Tyr Asn Gly Ile Asn Ile Val Ser Leu Thr Arg Met Ala Asp Lys
   355 360 365
   Asp Ile Thr Leu Ser Asp Gly Thr Phe Leu Pro Lys Gly Thr Leu Val
   370 375 380
   Ala Val Pro Ala Tyr Ser Thr His Arg Asp Asp Ala Val Tyr Ala Asp
   385 390 395 400
   Ala Leu Val Phe Asp Pro Phe Arg Phe Ser Arg Met Arg Ala Arg Glu
   405 410 415
   Gly Glu Gly Thr Lys His Gln Phe Val Asn Thr Ser Val Glu Tyr Val
   420 425 430
   Pro Phe Gly His Gly Lys His Ala Cys Pro Gly Arg Phe Phe Ala Ala
   435 440 445
   Asn Glu Leu Lys Ala Met Leu Ala Tyr Ile Val Leu Asn Tyr Asp Val
   450 455 460
   Lys Leu Pro Gly Asp Gly Lys Arg Pro Leu Asn Met Tyr Trp Gly Pro
   465 470 475 480
   Thr Val Leu Pro Ala Pro Ala Gly Gln Val Leu Phe Arg Lys Arg Gln
   485 490 495
   Val Ser Leu
<210> 75
   <211> 1530
   <212> DNA
   <213> Artificial Sequence
<220>
   <223> Codon-optimized KO
<400> 75
   atggcatttt tctctatgat ttcaattttg ttgggatttg ttatttcttc tttcatcttc      60
   atctttttct tcaaaaagtt acttagtttt agtaggaaaa acatgtcaga agtttctact     120
   ttgccaagtg ttccagtagt gcctggtttt ccagttattg ggaatttgtt gcaactaaag     180
   gagaaaaagc ctcataaaac tttcactaga tggtcagaga tatatggacc tatctactct     240
   ataaagatgg gttcttcatc tcttattgta ttgaacagta cagaaactgc taaggaagca     300
   atggtcacta gattttcatc aatatctacc agaaaattgt caaacgccct aacagttcta     360
   acctgcgata agtctatggt cgccacttct gattatgatg acttccacaa attagttaag     420
   agatgtttgc taaatggact tcttggtgct aatgctcaaa agagaaaaag acactacaga     480
   gatgctttga ttgaaaatgt gagttccaag ctacatgcac acgctagaga tcatccacaa     540
   gagccagtta actttagagc aattttcgaa cacgaattgt ttggtgtagc attaaagcaa     600
   gccttcggta aagacgtaga atccatatac gtcaaggagt taggcgtaac attatcaaaa     660
   gatgaaatct ttaaggtgct tgtacatgat atgatggagg gtgcaattga tgtagattgg     720
   agagatttct tcccatattt gaaatggatc cctaataagt cttttgaagc taggatacaa     780
   caaaagcaca agagaagact agctgttatg aacgcactta tacaggacag attgaagcaa     840
   aatgggtctg aatcagatga tgattgttac cttaacttct taatgtctga ggctaaaaca     900
   ttgactaagg aacagatcgc aatccttgtc tgggaaacaa tcattgaaac agcagatact     960
   accttagtca caactgaatg ggccatatac gagctagcca aacatccatc tgtgcaagat    1020
   aggttgtgta aggagatcca gaacgtgtgt ggtggagaga aattcaagga agagcagttg    1080
   tcacaagttc cttaccttaa cggcgttttc catgaaacct tgagaaaata ctcacctgca    1140
   ccattagttc ctattagata cgcccacgaa gatacacaaa tcggtggcta ccatgttcca    1200
   gctgggtccg aaattgctat aaacatctac gggtgcaaca tggacaaaaa gagatgggaa    1260
   agaccagaag attggtggcc agaaagattc ttagatgatg gcaaatatga aacatctgat    1320
   ttgcataaaa caatggcttt cggagctggc aaaagagtgt gtgccggtgc tctacaagcc    1380
   tccctaatgg ctggtatcgc tattggtaga ttggtccaag agttcgaatg gaaacttaga    1440
   gatggtgaag aggaaaatgt cgatacttat gggttaacat ctcaaaagtt atacccacta    1500
   atggcaatca tcaatcctag aagatcctaa                                     1530
<210> 76
   <211> 509
   <212> PRT
   <213> Arabidopsis thaliana
<400> 76
   Met Ala Phe Phe Ser Met Ile Ser Ile Leu Leu Gly Phe Val Ile Ser
   1               5                   10                  15
   Ser Phe Ile Phe Ile Phe Phe Phe Lys Lys Leu Leu Ser Phe Ser Arg
               20                  25                  30
   Lys Asn Met Ser Glu Val Ser Thr Leu Pro Ser Val Pro Val Val Pro
           35                  40                  45
   Gly Phe Pro Val Ile Gly Asn Leu Leu Gln Leu Lys Glu Lys Lys Pro
       50                  55                  60
   His Lys Thr Phe Thr Arg Trp Ser Glu Ile Tyr Gly Pro Ile Tyr Ser
   65                  70                  75                  80
   Ile Lys Met Gly Ser Ser Ser Leu Ile Val Leu Asn Ser Thr Glu Thr
                   85                  90                  95
   Ala Lys Glu Ala Met Val Thr Arg Phe Ser Ser Ile Ser Thr Arg Lys
               100                 105                 110
   Leu Ser Asn Ala Leu Thr Val Leu Thr Cys Asp Lys Ser Met Val Ala
           115                 120                 125
   Thr Ser Asp Tyr Asp Asp Phe His Lys Leu Val Lys Arg Cys Leu Leu
       130                 135                 140
   Asn Gly Leu Leu Gly Ala Asn Ala Gln Lys Arg Lys Arg His Tyr Arg
   145                 150                 155                 160
   Asp Ala Leu Ile Glu Asn Val Ser Ser Lys Leu His Ala His Ala Arg
                   165                 170                 175
   Asp His Pro Gln Glu Pro Val Asn Phe Arg Ala Ile Phe Glu His Glu
               180                 185                 190
   Leu Phe Gly Val Ala Leu Lys Gln Ala Phe Gly Lys Asp Val Glu Ser
           195                 200                 205
   Ile Tyr Val Lys Glu Leu Gly Val Thr Leu Ser Lys Asp Glu Ile Phe
       210                 215                 220
   Lys Val Leu Val His Asp Met Met Glu Gly Ala Ile Asp Val Asp Trp
   225                 230                 235                 240
   Arg Asp Phe Phe Pro Tyr Leu Lys Trp Ile Pro Asn Lys Ser Phe Glu
                   245                 250                 255
   Ala Arg Ile Gln Gln Lys His Lys Arg Arg Leu Ala Val Met Asn Ala
               260                 265                 270
   Leu Ile Gln Asp Arg Leu Lys Gln Asn Gly Ser Glu Ser Asp Asp Asp
           275                 280                 285
   Cys Tyr Leu Asn Phe Leu Met Ser Glu Ala Lys Thr Leu Thr Lys Glu
       290                 295                 300
   Gln Ile Ala Ile Leu Val Trp Glu Thr Ile Ile Glu Thr Ala Asp Thr
   305                 310                 315                 320
   Thr Leu Val Thr Thr Glu Trp Ala Ile Tyr Glu Leu Ala Lys His Pro
                   325                 330                 335
   Ser Val Gln Asp Arg Leu Cys Lys Glu Ile Gln Asn Val Cys Gly Gly
               340                 345                 350
   Glu Lys Phe Lys Glu Glu Gln Leu Ser Gln Val Pro Tyr Leu Asn Gly
           355                 360                 365
   Val Phe His Glu Thr Leu Arg Lys Tyr Ser Pro Ala Pro Leu Val Pro
       370                 375                 380
   Ile Arg Tyr Ala His Glu Asp Thr Gln Ile Gly Gly Tyr His Val Pro
   385                 390                 395                 400
   Ala Gly Ser Glu Ile Ala Ile Asn Ile Tyr Gly Cys Asn Met Asp Lys
                   405                 410                 415
   Lys Arg Trp Glu Arg Pro Glu Asp Trp Trp Pro Glu Arg Phe Leu Asp
               420                 425                 430
   Asp Gly Lys Tyr Glu Thr Ser Asp Leu His Lys Thr Met Ala Phe Gly
           435                 440                 445
   Ala Gly Lys Arg Val Cys Ala Gly Ala Leu Gln Ala Ser Leu Met Ala
   450 455 460
   Gly Ile Ala Ile Gly Arg Leu Val Gln Glu Phe Glu Trp Lys Leu Arg
   465 470 475 480
   Asp Gly Glu Glu Glu Asn Val Asp Thr Tyr Gly Leu Thr Ser Gln Lys
   485 490 495
   Leu Tyr Pro Leu Met Ala Ile Ile Asn Pro Arg Arg Ser
   500 505
<210> 77
   <211> 2133
   <212> DNA
   <213> Artificial Sequence
<220>
   <223> Codon-optimized CPR
<400> 77
   atgcaatcag attcagtcaa agtctctcca tttgatttgg tttccgctgc tatgaatggc        60
   aaggcaatgg aaaagttgaa cgctagtgaa tctgaagatc caacaacatt gcctgcacta       120
   aagatgctag ttgaaaatag agaattgttg acactgttca caacttcctt cgcagttctt       180
   attgggtgtc ttgtatttct aatgtggaga cgttcatcct ctaaaaagct ggtacaagat       240
   ccagttccac aagttatcgt tgtaaagaag aaagagaagg agtcagaggt tgatgacggg       300
   aaaaagaaag tttctatttt ctacggcaca caaacaggaa ctgccgaagg ttttgctaaa       360
   gcattagtcg aggaagcaaa agtgagatat gaaaagacct ctttcaaggt tatcgatcta       420
   gatgactacg ctgcagatga tgatgaatat gaggaaaaac tgaaaaagga atccttagcc       480
   ttcttcttct tggccacata cggtgatggt gaacctactg ataatgctgc taacttctac       540
   aagtggttca cagaaggcga cgataaaggt gaatggctga aaaagttaca atacggagta       600
   tttggtttag gtaacagaca atatgaacat ttcaacaaga tcgctattgt agttgatgat       660
   aaacttactg aaatgggagc caaaagatta gtaccagtag gattagggga tgatgatcag       720
   tgtatagaag atgacttcac cgcctggaag gaattggtat ggccagaatt ggatcaactt       780
   ttaagggacg aagatgatac ttctgtgact accccataca ctgcagccgt attggagtac       840
   agagtggttt accatgataa accagcagac tcatatgctg aagatcaaac ccatacaaac       900
   ggtcatgttg ttcatgatgc acagcatcct tcaagatcta atgtggcttt caaaaaggaa       960
   ctacacacct ctcaatcaga taggtcttgt actcacttag aattcgatat ttctcacaca      1020
   ggactgtctt acgaaactgg cgatcacgtt ggcgtttatt ccgagaactt gtccgaagtt      1080
   gtcgatgaag cactaaaact gttagggtta tcaccagaca catacttctc agtccatgct      1140
   gataaggagg atgggacacc tatcggtggt gcttcactac caccaccttt tcctccttgc 1200
   acattgagag acgctctaac cagatacgca gatgtcttat cctcacctaa aaaggtagct 1260
   ttgctggcat tggctgctca tgctagtgat cctagtgaag ccgataggtt aaagttcctg 1320
   gcttcaccag ccggaaaaga tgaatatgca caatggatcg tcgccaacca acgttctttg 1380
   ctagaagtga tgcaaagttt tccatctgcc aagcctccat taggtgtgtt cttcgcagca 1440
   gtagctccac gtttacaacc aagatactac tctatcagtt catctcctaa gatgtctcct 1500
   aacagaatac atgttacatg tgctttggtg tacgagacta ctccagcagg cagaattcac 1560
   agaggattgt gttcaacctg gatgaaaaat gctgtccctt taacagagtc acctgattgc 1620
   tctcaagcat ccattttcgt tagaacatca aatttcagac ttccagtgga tccaaaagtt 1680
   ccagtcatta tgataggacc aggcactggt cttgccccat tcaggggctt tcttcaagag 1740
   agattggcct tgaaggaatc tggtacagaa ttgggttctt ctatcttttt ctttggttgc 1800
   cgtaatagaa aagttgactt tatctacgag gacgagctta acaattttgt tgagacagga 1860
   gcattgtcag aattgatcgt cgcattttca agagaaggga ctgccaaaga gtacgttcag 1920
   cacaagatga gtcaaaaagc ctccgatata tggaaacttc taagtgaagg tgcctatctt 1980
   tatgtctgtg gcgatgcaaa gggcatggcc aaggatgtcc atagaactct gcatacaatt 2040
   gttcaggaac aagggagtct ggattcttcc aaggctgaat tgtacgtcaa aaacttacag 2100
   atgtctggaa gatacttaag agatgtttgg taa 2133
<210> 78
   <211> 710
   <212> PRT
   <213> Stevia rebaudiana
<400> 78
   Met Gln Ser Asp Ser Val Lys Val Ser Pro Phe Asp Leu Val Ser Ala
   1               5                   10                  15
   Ala Met Asn Gly Lys Ala Met Glu Lys Leu Asn Ala Ser Glu Ser Glu
               20                  25                  30
   Asp Pro Thr Thr Leu Pro Ala Leu Lys Met Leu Val Glu Asn Arg Glu
           35                  40                  45
   Leu Leu Thr Leu Phe Thr Thr Ser Phe Ala Val Leu Ile Gly Cys Leu
       50                  55                  60
   Val Phe Leu Met Trp Arg Arg Ser Ser Ser Lys Lys Leu Val Gln Asp
   65                  70                  75                  80
   Pro Val Pro Gln Val Ile Val Val Lys Lys Lys Glu Lys Glu Ser Glu
                   85                  90                  95
   Val Asp Asp Gly Lys Lys Lys Val Ser Ile Phe Tyr Gly Thr Gln Thr
               100                 105                 110
   Gly Thr Ala Glu Gly Phe Ala Lys Ala Leu Val Glu Glu Ala Lys Val
           115                 120                 125
   Arg Tyr Glu Lys Thr Ser Phe Lys Val Ile Asp Leu Asp Asp Tyr Ala
       130                 135                 140
   Ala Asp Asp Asp Glu Tyr Glu Glu Lys Leu Lys Lys Glu Ser Leu Ala
   145                 150                 155                 160
   Phe Phe Phe Leu Ala Thr Tyr Gly Asp Gly Glu Pro Thr Asp Asn Ala
                   165                 170                 175
   Ala Asn Phe Tyr Lys Trp Phe Thr Glu Gly Asp Asp Lys Gly Glu Trp
               180                 185                 190
   Leu Lys Lys Leu Gln Tyr Gly Val Phe Gly Leu Gly Asn Arg Gln Tyr
           195                 200                 205
   Glu His Phe Asn Lys Ile Ala Ile Val Val Asp Asp Lys Leu Thr Glu
       210                 215                 220
   Met Gly Ala Lys Arg Leu Val Pro Val Gly Leu Gly Asp Asp Asp Gln
   225                 230                 235                 240
   Cys Ile Glu Asp Asp Phe Thr Ala Trp Lys Glu Leu Val Trp Pro Glu
                   245                 250                 255
   Leu Asp Gln Leu Leu Arg Asp Glu Asp Asp Thr Ser Val Thr Thr Pro
               260                 265                 270
   Tyr Thr Ala Ala Val Leu Glu Tyr Arg Val Val Tyr His Asp Lys Pro
           275                 280                 285
   Ala Asp Ser Tyr Ala Glu Asp Gln Thr His Thr Asn Gly His Val Val
       290                 295                 300
   His Asp Ala Gln His Pro Ser Arg Ser Asn Val Ala Phe Lys Lys Glu
   305                 310                 315                 320
   Leu His Thr Ser Gln Ser Asp Arg Ser Cys Thr His Leu Glu Phe Asp
                   325                 330                 335
   Ile Ser His Thr Gly Leu Ser Tyr Glu Thr Gly Asp His Val Gly Val
               340                 345                 350
   Tyr Ser Glu Asn Leu Ser Glu Val Val Asp Glu Ala Leu Lys Leu Leu
           355                 360                 365
   Gly Leu Ser Pro Asp Thr Tyr Phe Ser Val His Ala Asp Lys Glu Asp
       370                 375                 380
   Gly Thr Pro Ile Gly Gly Ala Ser Leu Pro Pro Pro Phe Pro Pro Cys
   385                 390                 395                 400
   Thr Leu Arg Asp Ala Leu Thr Arg Tyr Ala Asp Val Leu Ser Ser Pro
                   405                 410                 415
   Lys Lys Val Ala Leu Leu Ala Leu Ala Ala His Ala Ser Asp Pro Ser
               420                 425                 430
   Glu Ala Asp Arg Leu Lys Phe Leu Ala Ser Pro Ala Gly Lys Asp Glu
           435                 440                 445
   Tyr Ala Gln Trp Ile Val Ala Asn Gln Arg Ser Leu Leu Glu Val Met
       450                 455                 460
   Gln Ser Phe Pro Ser Ala Lys Pro Pro Leu Gly Val Phe Phe Ala Ala
   465                 470                 475                 480
   Val Ala Pro Arg Leu Gln Pro Arg Tyr Tyr Ser Ile Ser Ser Ser Pro
                   485                 490                 495
   Lys Met Ser Pro Asn Arg Ile His Val Thr Cys Ala Leu Val Tyr Glu
               500                 505                 510
   Thr Thr Pro Ala Gly Arg Ile His Arg Gly Leu Cys Ser Thr Trp Met
           515                 520                 525
   Lys Asn Ala Val Pro Leu Thr Glu Ser Pro Asp Cys Ser Gln Ala Ser
       530                 535                 540
   Ile Phe Val Arg Thr Ser Asn Phe Arg Leu Pro Val Asp Pro Lys Val
   545                 550                 555                 560
   Pro Val Ile Met Ile Gly Pro Gly Thr Gly Leu Ala Pro Phe Arg Gly
                   565                 570                 575
   Phe Leu Gln Glu Arg Leu Ala Leu Lys Glu Ser Gly Thr Glu Leu Gly
               580                 585                 590
   Ser Ser Ile Phe Phe Phe Gly Cys Arg Asn Arg Lys Val Asp Phe Ile
           595                 600                 605
   Tyr Glu Asp Glu Leu Asn Asn Phe Val Glu Thr Gly Ala Leu Ser Glu
       610                 615                 620
   Leu Ile Val Ala Phe Ser Arg Glu Gly Thr Ala Lys Glu Tyr Val Gln
   625                 630                 635                 640
   His Lys Met Ser Gln Lys Ala Ser Asp Ile Trp Lys Leu Leu Ser Glu
                   645                 650                 655
   Gly Ala Tyr Leu Tyr Val Cys Gly Asp Ala Lys Gly Met Ala Lys Asp
               660                 665                 670
   Val His Arg Thr Leu His Thr Ile Val Gln Glu Gln Gly Ser Leu Asp
           675                 680                 685
   Ser Ser Lys Ala Glu Leu Tyr Val Lys Asn Leu Gln Met Ser Gly Arg
       690                 695                 700
   Tyr Leu Arg Asp Val Trp
   705                 710
<210> 79
   <211> 2106
   <212> DNA
   <213> Siraitia grosvenorii
<400> 79
   atgaaggtca gtccattcga attcatgtcc gctattatca agggtagaat ggacccatct 60
   aactcctcat ttgaatctac tggtgaagtt gcctccgtta tctttgaaaa cagagaattg 120
   gttgccatct tgaccacttc tattgctgtt atgattggtt gcttcgttgt cttgatgtgg 180
   agaagagctg gttctagaaa ggttaagaat gtcgaattgc caaagccatt gattgtccat 240
   gaaccagaac ctgaagttga agatggtaag aagaaggttt ccatcttctt cggtactcaa 300
   actggtactg ctgaaggttt tgctaaggct ttggctgatg aagctaaagc tagatacgaa 360
   aaggctacct tcagagttgt tgatttggat gattatgctg ccgatgatga ccaatacgaa 420
   gaaaaattga agaacgaatc cttcgccgtt ttcttgttgg ctacttatgg tgatggtgaa 480
   cctactgata atgctgctag attttacaag tggttcgccg aaggtaaaga aagaggtgaa 540
   tggttgcaaa acttgcacta tgctgttttt ggtttgggta acagacaata cgaacacttc 600
   aacaagattg ctaaggttgc cgacgaatta ttggaagctc aaggtggtaa tagattggtt 660
   aaggttggtt taggtgatga cgatcaatgc atcgaagatg atttttctgc ttggagagaa 720
   tctttgtggc cagaattgga tatgttgttg agagatgaag atgatgctac tactgttact      780
   actccatata ctgctgctgt cttggaatac agagttgtct ttcatgattc tgctgatgtt      840
   gctgctgaag ataagtcttg gattaacgct aatggtcatg ctgttcatga tgctcaacat      900
   ccattcagat ctaacgttgt cgtcagaaaa gaattgcata cttctgcctc tgatagatcc      960
   tgttctcatt tggaattcaa catttccggt tccgctttga attacgaaac tggtgatcat     1020
   gttggtgtct actgtgaaaa cttgactgaa actgttgatg aagccttgaa cttgttgggt     1080
   ttgtctccag aaacttactt ctctatctac accgataacg aagatggtac tccattgggt     1140
   ggttcttcat tgccaccacc atttccatca tgtactttga gaactgcttt gaccagatac     1200
   gctgatttgt tgaactctcc aaaaaagtct gctttgttgg ctttagctgc tcatgcttct     1260
   aatccagttg aagctgatag attgagatac ttggcttctc cagctggtaa agatgaatat     1320
   gcccaatctg ttatcggttc ccaaaagtct ttgttggaag ttatggctga attcccatct     1380
   gctaaaccac cattaggtgt tttttttgct gctgttgctc caagattgca acctagattc     1440
   tactccattt catcctctcc aagaatggct ccatctagaa tccatgttac ttgtgctttg     1500
   gtttacgata agatgccaac tggtagaatt cataagggtg tttgttctac ctggatgaag     1560
   aattctgttc caatggaaaa gtcccatgaa tgttcttggg ctccaatttt cgttagacaa     1620
   tccaatttta agttgccagc cgaatccaag gttccaatta tcatggttgg tccaggtact     1680
   ggtttggctc cttttagagg ttttttacaa gaaagattgg ccttgaaaga atccggtgtt     1740
   gaattgggtc catccatttt gtttttcggt tgcagaaaca gaagaatgga ttacatctac     1800
   gaagatgaat tgaacaactt cgttgaaacc ggtgctttgt ccgaattggt tattgctttt     1860
   tctagagaag gtcctaccaa agaatacgtc caacataaga tggctgaaaa ggcttctgat     1920
   atctggaact tgatttctga aggtgcttac ttgtacgttt gtggtgatgc taaaggtatg     1980
   gctaaggatg ttcatagaac cttgcatacc atcatgcaag aacaaggttc tttggattct     2040
   tccaaagctg aatccatggt caagaacttg caaatgaatg gtagatactt aagagatgtt     2100
   tggtaa                                                                2106
<210> 80
   <211> 701
   <212> PRT
   <213> Siraitia grosvenorii
<400> 80
   Met Lys Val Ser Pro Phe Glu Phe Met Ser Ala Ile Ile Lys Gly Arg
   1               5                   10                  15
   Met Asp Pro Ser Asn Ser Ser Phe Glu Ser Thr Gly Glu Val Ala Ser
               20                  25                  30
   Val Ile Phe Glu Asn Arg Glu Leu Val Ala Ile Leu Thr Thr Ser Ile
           35                  40                  45
   Ala Val Met Ile Gly Cys Phe Val Val Leu Met Trp Arg Arg Ala Gly
       50                  55                  60
   Ser Arg Lys Val Lys Asn Val Glu Leu Pro Lys Pro Leu Ile Val His
   65                  70                  75                  80
   Glu Pro Glu Pro Glu Val Glu Asp Gly Lys Lys Lys Val Ser Ile Phe
                   85                  90                  95
   Phe Gly Thr Gln Thr Gly Thr Ala Glu Gly Phe Ala Lys Ala Leu Ala
               100                 105                 110
   Asp Glu Ala Lys Ala Arg Tyr Glu Lys Ala Thr Phe Arg Val Val Asp
           115                 120                 125
   Leu Asp Asp Tyr Ala Ala Asp Asp Asp Gln Tyr Glu Glu Lys Leu Lys
       130                 135                 140
   Asn Glu Ser Phe Ala Val Phe Leu Leu Ala Thr Tyr Gly Asp Gly Glu
   145                 150                 155                 160
   Pro Thr Asp Asn Ala Ala Arg Phe Tyr Lys Trp Phe Ala Glu Gly Lys
                   165                 170                 175
   Glu Arg Gly Glu Trp Leu Gln Asn Leu His Tyr Ala Val Phe Gly Leu
               180                 185                 190
   Gly Asn Arg Gln Tyr Glu His Phe Asn Lys Ile Ala Lys Val Ala Asp
           195                 200                 205
   Glu Leu Leu Glu Ala Gln Gly Gly Asn Arg Leu Val Lys Val Gly Leu
       210                 215                 220
   Gly Asp Asp Asp Gln Cys Ile Glu Asp Asp Phe Ser Ala Trp Arg Glu
   225                 230                 235                 240
   Ser Leu Trp Pro Glu Leu Asp Met Leu Leu Arg Asp Glu Asp Asp Ala
                   245                 250                 255
   Thr Thr Val Thr Thr Pro Tyr Thr Ala Ala Val Leu Glu Tyr Arg Val
               260                 265                 270
   Val Phe His Asp Ser Ala Asp Val Ala Ala Glu Asp Lys Ser Trp Ile
   275 280 285
   Asn Ala Asn Gly His Ala Val His Asp Ala Gln His Pro Phe Arg Ser
   290 295 300
   Asn Val Val Val Arg Lys Glu Leu His Thr Ser Ala Ser Asp Arg Ser
   305 310 315 320
   Cys Ser His Leu Glu Phe Asn Ile Ser Gly Ser Ala Leu Asn Tyr Glu
   325 330 335
   Thr Gly Asp His Val Gly Val Tyr Cys Glu Asn Leu Thr Glu Thr Val
   340 345 350
   Asp Glu Ala Leu Asn Leu Leu Gly Leu Ser Pro Glu Thr Tyr Phe Ser
   355 360 365
   Ile Tyr Thr Asp Asn Glu Asp Gly Thr Pro Leu Gly Gly Ser Ser Leu
   370 375 380
   Pro Pro Pro Phe Pro Ser Cys Thr Leu Arg Thr Ala Leu Thr Arg Tyr
   385 390 395 400
   Ala Asp Leu Leu Asn Ser Pro Lys Lys Ser Ala Leu Leu Ala Leu Ala
   405 410 415
   Ala His Ala Ser Asn Pro Val Glu Ala Asp Arg Leu Arg Tyr Leu Ala
   420 425 430
   Ser Pro Ala Gly Lys Asp Glu Tyr Ala Gln Ser Val Ile Gly Ser Gln
   435 440 445
   Lys Ser Leu Leu Glu Val Met Ala Glu Phe Pro Ser Ala Lys Pro Pro
   450 455 460
   Leu Gly Val Phe Phe Ala Ala Val Ala Pro Arg Leu Gln Pro Arg Phe
   465 470 475 480
   Tyr Ser Ile Ser Ser Ser Pro Arg Met Ala Pro Ser Arg Ile His Val
   485 490 495
   Thr Cys Ala Leu Val Tyr Asp Lys Met Pro Thr Gly Arg Ile His Lys
   500 505 510
   Gly Val Cys Ser Thr Trp Met Lys Asn Ser Val Pro Met Glu Lys Ser
   515 520 525
   His Glu Cys Ser Trp Ala Pro Ile Phe Val Arg Gln Ser Asn Phe Lys
       530                 535                 540
   Leu Pro Ala Glu Ser Lys Val Pro Ile Ile Met Val Gly Pro Gly Thr
   545                 550                 555                 560
   Gly Leu Ala Pro Phe Arg Gly Phe Leu Gln Glu Arg Leu Ala Leu Lys
                   565                 570                 575
   Glu Ser Gly Val Glu Leu Gly Pro Ser Ile Leu Phe Phe Gly Cys Arg
               580                 585                 590
   Asn Arg Arg Met Asp Tyr Ile Tyr Glu Asp Glu Leu Asn Asn Phe Val
           595                 600                 605
   Glu Thr Gly Ala Leu Ser Glu Leu Val Ile Ala Phe Ser Arg Glu Gly
       610                 615                 620
   Pro Thr Lys Glu Tyr Val Gln His Lys Met Ala Glu Lys Ala Ser Asp
   625                 630                 635                 640
   Ile Trp Asn Leu Ile Ser Glu Gly Ala Tyr Leu Tyr Val Cys Gly Asp
                   645                 650                 655
   Ala Lys Gly Met Ala Lys Asp Val His Arg Thr Leu His Thr Ile Met
               660                 665                 670
   Gln Glu Gln Gly Ser Leu Asp Ser Ser Lys Ala Glu Ser Met Val Lys
           675                 680                 685
   Asn Leu Gln Met Asn Gly Arg Tyr Leu Arg Asp Val Trp
       690                 695                 700
<210> 81
   <211> 2142
   <212> DNA
   <213> Artificial Sequence
<220>
   <223> Codon-optimized CPR
<400> 81
   atggcagaat tagatacact tgatatagta gtattaggtg ttatcttttt gggtactgtg      60
   gcatacttta ctaagggtaa attgtggggt gttaccaagg atccatacgc taacggattc     120
   gctgcaggtg gtgcttccaa gcctggcaga actagaaaca tcgtcgaagc tatggaggaa     180
   tcaggtaaaa actgtgttgt tttctacggc agtcaaacag gtacagcgga ggattacgca     240
   tcaagacttg caaaggaagg aaagtccaga ttcggtttga acactatgat cgccgatcta     300
   gaagattatg acttcgataa cttagacact gttccatctg ataacatcgt tatgtttgta 360
   ttggctactt acggtgaagg cgaaccaaca gataacgccg tggatttcta tgagttcatt 420
   actggcgaag atgcctcttt caatgagggc aacgatcctc cactaggtaa cttgaattac 480
   gttgcgttcg gtctgggcaa caatacctac gaacactaca actcaatggt caggaacgtt 540
   aacaaggctc tagaaaagtt aggagctcat agaattggag aagcaggtga gggtgacgac 600
   ggagctggaa ctatggaaga ggacttttta gcttggaaag atccaatgtg ggaagccttg 660
   gctaaaaaga tgggcttgga ggaaagagaa gctgtatatg aacctatttt cgctatcaat 720
   gagagagatg atttgacccc tgaagcgaat gaggtatact tgggagaacc taataagcta 780
   cacttggaag gtacagcgaa aggtccattc aactcccaca acccatatat cgcaccaatt 840
   gcagaatcat acgaactttt ctcagctaag gatagaaatt gtctgcatat ggaaattgat 900
   atttctggta gtaatctaaa gtatgaaaca ggcgaccata tcgcgatctg gcctaccaac 960
   ccaggtgaag aggtcaacaa atttcttgac attctagatc tgtctggtaa gcaacattcc 1020
   gtcgtaacag tgaaagcctt agaacctaca gccaaagttc cttttccaaa tccaactacc 1080
   tacgatgcta tattgagata ccatctggaa atatgcgctc cagtttctag acagtttgtc 1140
   tcaactttag cagcattcgc ccctaatgat gatatcaaag ctgagatgaa ccgtttggga 1200
   tcagacaaag attacttcca cgaaaagaca ggaccacatt actacaatat cgctagattt 1260
   ttggcctcag tctctaaagg tgaaaaatgg acaaagatac cattttctgc tttcatagaa 1320
   ggccttacaa aactacaacc aagatactat tctatctctt cctctagttt agttcagcct 1380
   aaaaagatta gtattactgc tgttgtcgaa tctcagcaaa ttccaggtag agatgaccca 1440
   ttcagaggtg tagcgactaa ctacttgttc gctttgaagc agaaacaaaa cggtgatcca 1500
   aatccagctc cttttggcca atcatacgag ttgacaggac caaggaataa gtatgatggt 1560
   atacatgttc cagtccatgt aagacattct aactttaagc taccatctga tccaggcaaa 1620
   cctattatca tgatcggtcc aggtaccggt gttgcccctt ttagaggctt cgtccaagag 1680
   agggcaaaac aagccagaga tggtgtagaa gttggtaaaa cactgctgtt ctttggatgt 1740
   agaaagagta cagaagattt catgtatcaa aaagagtggc aagagtacaa ggaagctctt 1800
   ggcgacaaat tcgaaatgat tacagctttt tcaagagaag gatctaaaaa ggtttatgtt 1860
   caacacagac tgaaggaaag atcaaaggaa gtttctgatc ttctatccca aaaagcatac 1920
   ttctacgttt gcggagacgc cgcacatatg gcacgtgaag tgaacactgt gttagcacag 1980
   atcatagcag aaggccgtgg tgtatcagaa gccaagggtg aggaaattgt caaaaacatg 2040
   agatcagcaa atcaatacca agtgtgttct gatttcgtaa ctttacactg taaagagaca 2100
   acatacgcga attcagaatt gcaagaggat gtctggagtt aa 2142
<210> 82
   <211> 713
   <212> PRT
   <213> Gibberella fujikuroi
<400> 82
   Met Ala Glu Leu Asp Thr Leu Asp Ile Val Val Leu Gly Val Ile Phe
   1               5                   10                  15
   Leu Gly Thr Val Ala Tyr Phe Thr Lys Gly Lys Leu Trp Gly Val Thr
               20                  25                  30
   Lys Asp Pro Tyr Ala Asn Gly Phe Ala Ala Gly Gly Ala Ser Lys Pro
           35                  40                  45
   Gly Arg Thr Arg Asn Ile Val Glu Ala Met Glu Glu Ser Gly Lys Asn
       50                  55                  60
   Cys Val Val Phe Tyr Gly Ser Gln Thr Gly Thr Ala Glu Asp Tyr Ala
   65                  70                  75                  80
   Ser Arg Leu Ala Lys Glu Gly Lys Ser Arg Phe Gly Leu Asn Thr Met
                   85                  90                  95
   Ile Ala Asp Leu Glu Asp Tyr Asp Phe Asp Asn Leu Asp Thr Val Pro
               100                 105                 110
   Ser Asp Asn Ile Val Met Phe Val Leu Ala Thr Tyr Gly Glu Gly Glu
           115                 120                 125
   Pro Thr Asp Asn Ala Val Asp Phe Tyr Glu Phe Ile Thr Gly Glu Asp
       130                 135                 140
   Ala Ser Phe Asn Glu Gly Asn Asp Pro Pro Leu Gly Asn Leu Asn Tyr
   145                 150                 155                 160
   Val Ala Phe Gly Leu Gly Asn Asn Thr Tyr Glu His Tyr Asn Ser Met
                   165                 170                 175
   Val Arg Asn Val Asn Lys Ala Leu Glu Lys Leu Gly Ala His Arg Ile
               180                 185                 190
   Gly Glu Ala Gly Glu Gly Asp Asp Gly Ala Gly Thr Met Glu Glu Asp
           195                 200                 205
   Phe Leu Ala Trp Lys Asp Pro Met Trp Glu Ala Leu Ala Lys Lys Met
       210                 215                 220
   Gly Leu Glu Glu Arg Glu Ala Val Tyr Glu Pro Ile Phe Ala Ile Asn
   225 230 235 240
   Glu Arg Asp Asp Leu Thr Pro Glu Ala Asn Glu Val Tyr Leu Gly Glu
   245 250 255
   Pro Asn Lys Leu His Leu Glu Gly Thr Ala Lys Gly Pro Phe Asn Ser
   260 265 270
   His Asn Pro Tyr Ile Ala Pro Ile Ala Glu Ser Tyr Glu Leu Phe Ser
   275 280 285
   Ala Lys Asp Arg Asn Cys Leu His Met Glu Ile Asp Ile Ser Gly Ser
   290 295 300
   Asn Leu Lys Tyr Glu Thr Gly Asp His Ile Ala Ile Trp Pro Thr Asn
   305 310 315 320
   Pro Gly Glu Glu Val Asn Lys Phe Leu Asp Ile Leu Asp Leu Ser Gly
   325 330 335
   Lys Gln His Ser Val Val Thr Val Lys Ala Leu Glu Pro Thr Ala Lys
   340 345 350
   Val Pro Phe Pro Asn Pro Thr Thr Tyr Asp Ala Ile Leu Arg Tyr His
   355 360 365
   Leu Glu Ile Cys Ala Pro Val Ser Arg Gln Phe Val Ser Thr Leu Ala
   370 375 380
   Ala Phe Ala Pro Asn Asp Asp Ile Lys Ala Glu Met Asn Arg Leu Gly
   385 390 395 400
   Ser Asp Lys Asp Tyr Phe His Glu Lys Thr Gly Pro His Tyr Tyr Asn
   405 410 415
   Ile Ala Arg Phe Leu Ala Ser Val Ser Lys Gly Glu Lys Trp Thr Lys
   420 425 430
   Ile Pro Phe Ser Ala Phe Ile Glu Gly Leu Thr Lys Leu Gln Pro Arg
   435 440 445
   Tyr Tyr Ser Ile Ser Ser Ser Ser Leu Val Gln Pro Lys Lys Ile Ser
   450 455 460
   Ile Thr Ala Val Val Glu Ser Gln Gln Ile Pro Gly Arg Asp Asp Pro
   465 470 475 480
   Phe Arg Gly Val Ala Thr Asn Tyr Leu Phe Ala Leu Lys Gln Lys Gln
                   485                 490                 495
   Asn Gly Asp Pro Asn Pro Ala Pro Phe Gly Gln Ser Tyr Glu Leu Thr
               500                 505                 510
   Gly Pro Arg Asn Lys Tyr Asp Gly Ile His Val Pro Val His Val Arg
           515                 520                 525
   His Ser Asn Phe Lys Leu Pro Ser Asp Pro Gly Lys Pro Ile Ile Met
       530                 535                 540
   Ile Gly Pro Gly Thr Gly Val Ala Pro Phe Arg Gly Phe Val Gln Glu
   545                 550                 555                 560
   Arg Ala Lys Gln Ala Arg Asp Gly Val Glu Val Gly Lys Thr Leu Leu
                   565                 570                 575
   Phe Phe Gly Cys Arg Lys Ser Thr Glu Asp Phe Met Tyr Gln Lys Glu
               580                 585                 590
   Trp Gln Glu Tyr Lys Glu Ala Leu Gly Asp Lys Phe Glu Met Ile Thr
           595                 600                 605
   Ala Phe Ser Arg Glu Gly Ser Lys Lys Val Tyr Val Gln His Arg Leu
       610                 615                 620
   Lys Glu Arg Ser Lys Glu Val Ser Asp Leu Leu Ser Gln Lys Ala Tyr
   625                 630                 635                 640
   Phe Tyr Val Cys Gly Asp Ala Ala His Met Ala Arg Glu Val Asn Thr
                   645                 650                 655
   Val Leu Ala Gln Ile Ile Ala Glu Gly Arg Gly Val Ser Glu Ala Lys
               660                 665                 670
   Gly Glu Glu Ile Val Lys Asn Met Arg Ser Ala Asn Gln Tyr Gln Val
           675                 680                 685
   Cys Ser Asp Phe Val Thr Leu His Cys Lys Glu Thr Thr Tyr Ala Asn
       690                 695                 700
   Ser Glu Leu Gln Glu Asp Val Trp Ser
   705                 710
<210> 83
   <211> 2130
   <212> DNA
   <213> Stevia rebaudiana
<400> 83
   atgcaatcgg aatccgttga agcatcgacg attgatttga tgactgctgt tttgaaggac 60
   acagtgatcg atacagcgaa cgcatctgat aacggagact caaagatgcc gccggcgttg 120
   gcgatgatgt tcgaaattcg tgatctgttg ctgattttga ctacgtcagt tgctgttttg 180
   gtcggatgtt tcgttgtttt ggtgtggaag agatcgtccg ggaagaagtc cggcaaggaa 240
   ttggagccgc cgaagatcgt tgtgccgaag aggcggctgg agcaggaggt tgatgatggt 300
   aagaagaagg ttacgatttt cttcggaaca caaactggaa cggctgaagg tttcgctaag 360
   gcacttttcg aagaagcgaa agcgcgatat gaaaaggcag cgtttaaagt gattgatttg 420
   gatgattatg ctgctgattt ggatgagtat gcagagaagc tgaagaagga aacatatgct 480
   ttcttcttct tggctacata tggagatggt gagccaactg ataatgctgc caaattttat 540
   aaatggttta ctgagggaga cgagaaaggc gtttggcttc aaaaacttca atatggagta 600
   tttggtcttg gcaacagaca atatgaacat ttcaacaaga ttggaatagt ggttgatgat 660
   ggtctcaccg agcagggtgc aaaacgcatt gttcccgttg gtcttggaga cgacgatcaa 720
   tcaattgaag acgatttttc ggcatggaaa gagttagtgt ggcccgaatt ggatctattg 780
   cttcgcgatg aagatgacaa agctgctgca actccttaca cagctgcaat ccctgaatac 840
   cgcgtcgtat ttcatgacaa acccgatgcg ttttctgatg atcatactca aaccaatggt 900
   catgctgttc atgatgctca acatccatgc agatccaatg tggctgttaa aaaagagctt 960
   catactcctg aatccgatcg ttcatgcaca catcttgaat ttgacatttc tcacactgga 1020
   ttatcttatg aaactgggga tcatgttggt gtatactgtg aaaacctaat tgaagtagtg 1080
   gaagaagctg ggaaattgtt aggattatca acagatactt atttctcgtt acatattgat 1140
   aacgaagatg gttcaccact tggtggacct tcattacaac ctccttttcc tccttgtact 1200
   ttaagaaaag cattgactaa ttatgcagat ctgttaagct ctcccaaaaa gtcaactttg 1260
   cttgctctag ctgctcatgc ttccgatccc actgaagctg atcgtttaag atttcttgca 1320
   tctcgcgagg gcaaggatga atatgctgaa tgggttgttg caaaccaaag aagtcttctt 1380
   gaagtcatgg aagctttccc gtcagctaga ccgccacttg gtgttttctt tgcagcggtt 1440
   gcaccgcgtt tacagcctcg ttactactct atttcttcct ccccaaagat ggaaccaaac 1500
   aggattcatg ttacttgcgc gttggtttat gaaaaaactc ccgcaggtcg tatccacaaa 1560
   ggaatctgct caacctggat gaagaacgct gtacctttga ccgaaagtca agattgcagt 1620
   tgggcaccga tttttgttag aacatcaaac ttcagacttc caattgaccc gaaagtcccg 1680
   gttatcatga ttggtcctgg aaccgggttg gctccattta ggggttttct tcaagaaaga 1740
   ttggctctta aagaatccgg aaccgaactc gggtcatcta ttttattctt cggttgtaga 1800
   aaccgcaaag tggattacat atatgagaat gaactcaaca actttgttga aaatggtgcg     1860
   ctttctgagc ttgatgttgc tttctcccgc gatggcccga cgaaagaata cgtgcaacat     1920
   aaaatgaccc aaaaggcttc tgaaatatgg aatatgcttt ctgagggagc atatttatat     1980
   gtatgtggtg atgctaaagg catggctaaa gatgtacacc gtacacttca caccattgtg     2040
   caagaacagg gaagtttgga ctcgtctaaa gcggagttgt atgtgaagaa tctacaaatg     2100
   tcaggaagat acctccgtga tgtttggtaa                                      2130
<210> 84
   <211> 709
   <212> PRT
   <213> Stevia rebaudiana
<400> 84
   Met Gln Ser Glu Ser Val Glu Ala Ser Thr Ile Asp Leu Met Thr Ala
   1               5                   10                  15
   Val Leu Lys Asp Thr Val Ile Asp Thr Ala Asn Ala Ser Asp Asn Gly
               20                  25                  30
   Asp Ser Lys Met Pro Pro Ala Leu Ala Met Met Phe Glu Ile Arg Asp
           35                  40                  45
   Leu Leu Leu Ile Leu Thr Thr Ser Val Ala Val Leu Val Gly Cys Phe
       50                  55                  60
   Val Val Leu Val Trp Lys Arg Ser Ser Gly Lys Lys Ser Gly Lys Glu
   65                  70                  75                  80
   Leu Glu Pro Pro Lys Ile Val Val Pro Lys Arg Arg Leu Glu Gln Glu
                   85                  90                  95
   Val Asp Asp Gly Lys Lys Lys Val Thr Ile Phe Phe Gly Thr Gln Thr
               100                 105                 110
   Gly Thr Ala Glu Gly Phe Ala Lys Ala Leu Phe Glu Glu Ala Lys Ala
           115                 120                 125
   Arg Tyr Glu Lys Ala Ala Phe Lys Val Ile Asp Leu Asp Asp Tyr Ala
       130                 135                 140
   Ala Asp Leu Asp Glu Tyr Ala Glu Lys Leu Lys Lys Glu Thr Tyr Ala
   145                 150                 155                 160
   Phe Phe Phe Leu Ala Thr Tyr Gly Asp Gly Glu Pro Thr Asp Asn Ala
                   165                 170                 175
   Ala Lys Phe Tyr Lys Trp Phe Thr Glu Gly Asp Glu Lys Gly Val Trp
               180                 185                 190
   Leu Gln Lys Leu Gln Tyr Gly Val Phe Gly Leu Gly Asn Arg Gln Tyr
           195                 200                 205
   Glu His Phe Asn Lys Ile Gly Ile Val Val Asp Asp Gly Leu Thr Glu
       210                 215                 220
   Gln Gly Ala Lys Arg Ile Val Pro Val Gly Leu Gly Asp Asp Asp Gln
   225                 230                 235                 240
   Ser Ile Glu Asp Asp Phe Ser Ala Trp Lys Glu Leu Val Trp Pro Glu
                   245                 250                 255
   Leu Asp Leu Leu Leu Arg Asp Glu Asp Asp Lys Ala Ala Ala Thr Pro
               260                 265                 270
   Tyr Thr Ala Ala Ile Pro Glu Tyr Arg Val Val Phe His Asp Lys Pro
           275                 280                 285
   Asp Ala Phe Ser Asp Asp His Thr Gln Thr Asn Gly His Ala Val His
       290                 295                 300
   Asp Ala Gln His Pro Cys Arg Ser Asn Val Ala Val Lys Lys Glu Leu
   305                 310                 315                 320
   His Thr Pro Glu Ser Asp Arg Ser Cys Thr His Leu Glu Phe Asp Ile
                   325                 330                 335
   Ser His Thr Gly Leu Ser Tyr Glu Thr Gly Asp His Val Gly Val Tyr
               340                 345                 350
   Cys Glu Asn Leu Ile Glu Val Val Glu Glu Ala Gly Lys Leu Leu Gly
           355                 360                 365
   Leu Ser Thr Asp Thr Tyr Phe Ser Leu His Ile Asp Asn Glu Asp Gly
       370                 375                 380
   Ser Pro Leu Gly Gly Pro Ser Leu Gln Pro Pro Phe Pro Pro Cys Thr
   385                 390                 395                 400
   Leu Arg Lys Ala Leu Thr Asn Tyr Ala Asp Leu Leu Ser Ser Pro Lys
                   405                 410                 415
   Lys Ser Thr Leu Leu Ala Leu Ala Ala His Ala Ser Asp Pro Thr Glu
   420 425 430
   Ala Asp Arg Leu Arg Phe Leu Ala Ser Arg Glu Gly Lys Asp Glu Tyr
   435 440 445
   Ala Glu Trp Val Val Ala Asn Gln Arg Ser Leu Leu Glu Val Met Glu
   450 455 460
   Ala Phe Pro Ser Ala Arg Pro Pro Leu Gly Val Phe Phe Ala Ala Val
   465 470 475 480
   Ala Pro Arg Leu Gln Pro Arg Tyr Tyr Ser Ile Ser Ser Ser Pro Lys
   485 490 495
   Met Glu Pro Asn Arg Ile His Val Thr Cys Ala Leu Val Tyr Glu Lys
   500 505 510
   Thr Pro Ala Gly Arg Ile His Lys Gly Ile Cys Ser Thr Trp Met Lys
   515 520 525
   Asn Ala Val Pro Leu Thr Glu Ser Gln Asp Cys Ser Trp Ala Pro Ile
   530 535 540
   Phe Val Arg Thr Ser Asn Phe Arg Leu Pro Ile Asp Pro Lys Val Pro
   545 550 555 560
   Val Ile Met Ile Gly Pro Gly Thr Gly Leu Ala Pro Phe Arg Gly Phe
   565 570 575
   Leu Gln Glu Arg Leu Ala Leu Lys Glu Ser Gly Thr Glu Leu Gly Ser
   580 585 590
   Ser Ile Leu Phe Phe Gly Cys Arg Asn Arg Lys Val Asp Tyr Ile Tyr
   595 600 605
   Glu Asn Glu Leu Asn Asn Phe Val Glu Asn Gly Ala Leu Ser Glu Leu
   610 615 620
   Asp Val Ala Phe Ser Arg Asp Gly Pro Thr Lys Glu Tyr Val Gln His
   625 630 635 640
   Lys Met Thr Gln Lys Ala Ser Glu Ile Trp Asn Met Leu Ser Glu Gly
   645 650 655
   Ala Tyr Leu Tyr Val Cys Gly Asp Ala Lys Gly Met Ala Lys Asp Val
   660 665 670
   His Arg Thr Leu His Thr Ile Val Gln Glu Gln Gly Ser Leu Asp Ser
   675 680 685
   Ser Lys Ala Glu Leu Tyr Val Lys Asn Leu Gln Met Ser Gly Arg Tyr
   690 695 700
   Leu Arg Asp Val Trp
   705
<210> 85
   <211> 2124
   <212> DNA
   <213> Artificial Sequence
<220>
   <223> Codon-optimized CPR
<400> 85
   atgcaatcta actccgtgaa gatttcgccg cttgatctgg taactgcgct gtttagcggc 60
   aaggttttgg acacatcgaa cgcatcggaa tcgggagaat ctgctatgct gccgactata 120
   gcgatgatta tggagaatcg tgagctgttg atgatactca caacgtcggt tgctgtattg 180
   atcggatgcg ttgtcgtttt ggtgtggcgg agatcgtcta cgaagaagtc ggcgttggag 240
   ccaccggtga ttgtggttcc gaagagagtg caagaggagg aagttgatga tggtaagaag 300
   aaagttacgg ttttcttcgg cacccaaact ggaacagctg aaggcttcgc taaggcactt 360
   gttgaggaag ctaaagctcg atatgaaaag gctgtcttta aagtaattga tttggatgat 420
   tatgctgctg atgacgatga gtatgaggag aaactaaaga aagaatcttt ggcctttttc 480
   tttttggcta cgtatggaga tggtgagcca acagataatg ctgccagatt ttataaatgg 540
   tttactgagg gagatgcgaa aggagaatgg cttaataagc ttcaatatgg agtatttggt 600
   ttgggtaaca gacaatatga acattttaac aagatcgcaa aagtggttga tgatggtctt 660
   gtagaacagg gtgcaaagcg tcttgttcct gttggacttg gagatgatga tcaatgtatt 720
   gaagatgact tcaccgcatg gaaagagtta gtatggccgg agttggatca attacttcgt 780
   gatgaggatg acacaactgt tgctactcca tacacagctg ctgttgcaga atatcgcgtt 840
   gtttttcatg aaaaaccaga cgcgctttct gaagattata gttatacaaa tggccatgct 900
   gttcatgatg ctcaacatcc atgcagatcc aacgtggctg tcaaaaagga acttcatagt 960
   cctgaatctg accggtcttg cactcatctt gaatttgaca tctcgaacac cggactatca 1020
   tatgaaactg gggaccatgt tggagtttac tgtgaaaact tgagtgaagt tgtgaatgat 1080
   gctgaaagat tagtaggatt accaccagac acttactcct ccatccacac tgatagtgaa 1140
   gacgggtcgc cacttggcgg agcctcattg ccgcctcctt tcccgccatg cactttaagg 1200
   aaagcattga cgtgttatgc tgatgttttg agttctccca agaagtcggc tttgcttgca 1260
   ctagctgctc atgccaccga tcccagtgaa gctgatagat tgaaatttct tgcatccccc 1320
   gccggaaagg atgaatattc tcaatggata gttgcaagcc aaagaagtct ccttgaagtc 1380
   atggaagcat tcccgtcagc taagccttca cttggtgttt tctttgcatc tgttgccccg 1440
   cgcttacaac caagatacta ctctatttct tcctcaccca agatggcacc ggataggatt 1500
   catgttacat gtgcattagt ctatgagaaa acacctgcag gccgcatcca caaaggagtt 1560
   tgttcaactt ggatgaagaa cgcagtgcct atgaccgaga gtcaagattg cagttgggcc 1620
   ccaatatacg tccgaacatc caatttcaga ctaccatctg accctaaggt cccggttatc 1680
   atgattggac ctggcactgg tttggctcct tttagaggtt tccttcaaga gcggttagct 1740
   ttaaaggaag ccggaactga cctcggttta tccattttat tcttcggatg taggaatcgc 1800
   aaagtggatt tcatatatga aaacgagctt aacaactttg tggagactgg tgctctttct 1860
   gagcttattg ttgctttctc ccgtgaaggc ccgactaagg aatatgtgca acacaagatg 1920
   agtgagaagg cttcggatat ctggaacttg ctttctgaag gagcatattt atacgtatgt 1980
   ggtgatgcca aaggcatggc caaagatgta catcgaaccc tccacacaat tgtgcaagaa 2040
   cagggatctc ttgactcgtc aaaggcagaa ctctacgtga agaatctaca aatgtcagga 2100
   agatacctcc gtgacgtttg gtaa 2124
<210> 86
   <211> 707
   <212> PRT
   <213> Stevia rebaudiana
<400> 86
   Met Gln Ser Asn Ser Val Lys Ile Ser Pro Leu Asp Leu Val Thr Ala
   1               5                   10                  15
   Leu Phe Ser Gly Lys Val Leu Asp Thr Ser Asn Ala Ser Glu Ser Gly
               20                  25                  30
   Glu Ser Ala Met Leu Pro Thr Ile Ala Met Ile Met Glu Asn Arg Glu
           35                  40                  45
   Leu Leu Met Ile Leu Thr Thr Ser Val Ala Val Leu Ile Gly Cys Val
       50                  55                  60
   Val Val Leu Val Trp Arg Arg Ser Ser Thr Lys Lys Ser Ala Leu Glu
   65                  70                  75                  80
   Pro Pro Val Ile Val Val Pro Lys Arg Val Gln Glu Glu Glu Val Asp
                   85                  90                  95
   Asp Gly Lys Lys Lys Val Thr Val Phe Phe Gly Thr Gln Thr Gly Thr
   100 105 110
   Ala Glu Gly Phe Ala Lys Ala Leu Val Glu Glu Ala Lys Ala Arg Tyr
   115 120 125
   Glu Lys Ala Val Phe Lys Val Ile Asp Leu Asp Asp Tyr Ala Ala Asp
   130 135 140
   Asp Asp Glu Tyr Glu Glu Lys Leu Lys Lys Glu Ser Leu Ala Phe Phe
   145 150 155 160
   Phe Leu Ala Thr Tyr Gly Asp Gly Glu Pro Thr Asp Asn Ala Ala Arg
   165 170 175
   Phe Tyr Lys Trp Phe Thr Glu Gly Asp Ala Lys Gly Glu Trp Leu Asn
   180 185 190
   Lys Leu Gln Tyr Gly Val Phe Gly Leu Gly Asn Arg Gln Tyr Glu His
   195 200 205
   Phe Asn Lys Ile Ala Lys Val Val Asp Asp Gly Leu Val Glu Gln Gly
   210 215 220
   Ala Lys Arg Leu Val Pro Val Gly Leu Gly Asp Asp Asp Gln Cys Ile
   225 230 235 240
   Glu Asp Asp Phe Thr Ala Trp Lys Glu Leu Val Trp Pro Glu Leu Asp
   245 250 255
   Gln Leu Leu Arg Asp Glu Asp Asp Thr Thr Val Ala Thr Pro Tyr Thr
   260 265 270
   Ala Ala Val Ala Glu Tyr Arg Val Val Phe His Glu Lys Pro Asp Ala
   275 280 285
   Leu Ser Glu Asp Tyr Ser Tyr Thr Asn Gly His Ala Val His Asp Ala
   290 295 300
   Gln His Pro Cys Arg Ser Asn Val Ala Val Lys Lys Glu Leu His Ser
   305 310 315 320
   Pro Glu Ser Asp Arg Ser Cys Thr His Leu Glu Phe Asp Ile Ser Asn
   325 330 335
   Thr Gly Leu Ser Tyr Glu Thr Gly Asp His Val Gly Val Tyr Cys Glu
   340 345 350
   Asn Leu Ser Glu Val Val Asn Asp Ala Glu Arg Leu Val Gly Leu Pro
   355 360 365
   Pro Asp Thr Tyr Ser Ser Ile His Thr Asp Ser Glu Asp Gly Ser Pro
   370 375 380
   Leu Gly Gly Ala Ser Leu Pro Pro Pro Phe Pro Pro Cys Thr Leu Arg
   385 390 395 400
   Lys Ala Leu Thr Cys Tyr Ala Asp Val Leu Ser Ser Pro Lys Lys Ser
   405 410 415
   Ala Leu Leu Ala Leu Ala Ala His Ala Thr Asp Pro Ser Glu Ala Asp
   420 425 430
   Arg Leu Lys Phe Leu Ala Ser Pro Ala Gly Lys Asp Glu Tyr Ser Gln
   435 440 445
   Trp Ile Val Ala Ser Gln Arg Ser Leu Leu Glu Val Met Glu Ala Phe
   450 455 460
   Pro Ser Ala Lys Pro Ser Leu Gly Val Phe Phe Ala Ser Val Ala Pro
   465 470 475 480
   Arg Leu Gln Pro Arg Tyr Tyr Ser Ile Ser Ser Ser Pro Lys Met Ala
   485 490 495
   Pro Asp Arg Ile His Val Thr Cys Ala Leu Val Tyr Glu Lys Thr Pro
   500 505 510
   Ala Gly Arg Ile His Lys Gly Val Cys Ser Thr Trp Met Lys Asn Ala
   515 520 525
   Val Pro Met Thr Glu Ser Gln Asp Cys Ser Trp Ala Pro Ile Tyr Val
   530 535 540
   Arg Thr Ser Asn Phe Arg Leu Pro Ser Asp Pro Lys Val Pro Val Ile
   545 550 555 560
   Met Ile Gly Pro Gly Thr Gly Leu Ala Pro Phe Arg Gly Phe Leu Gln
   565 570 575
   Glu Arg Leu Ala Leu Lys Glu Ala Gly Thr Asp Leu Gly Leu Ser Ile
   580 585 590
   Leu Phe Phe Gly Cys Arg Asn Arg Lys Val Asp Phe Ile Tyr Glu Asn
   595 600 605
   Glu Leu Asn Asn Phe Val Glu Thr Gly Ala Leu Ser Glu Leu Ile Val
   610 615 620
   Ala Phe Ser Arg Glu Gly Pro Thr Lys Glu Tyr Val Gln His Lys Met
   625 630 635 640
   Ser Glu Lys Ala Ser Asp Ile Trp Asn Leu Leu Ser Glu Gly Ala Tyr
   645 650 655
   Leu Tyr Val Cys Gly Asp Ala Lys Gly Met Ala Lys Asp Val His Arg
   660 665 670
   Thr Leu His Thr Ile Val Gln Glu Gln Gly Ser Leu Asp Ser Ser Lys
   675 680 685
   Ala Glu Leu Tyr Val Lys Asn Leu Gln Met Ser Gly Arg Tyr Leu Arg
   690 695 700
   Asp Val Trp
   705
<210> 87
   <211> 2070
   <212> DNA
   <213> Artificial Sequence
<220>
   <223> Codon-optimized CPR
<400> 87
   atgtcctcca actccgattt ggtcagaaga ttggaatctg ttttgggtgt ttctttcggt 60
   ggttctgtta ctgattccgt tgttgttatt gctaccacct ctattgcttt ggttatcggt 120
   gttttggttt tgttgtggag aagatcctct gacagatcta gagaagttaa gcaattggct 180
   gttccaaagc cagttactat cgttgaagaa gaagatgaat tcgaagttgc ttctggtaag 240
   accagagttt ctattttcta cggtactcaa actggtactg ctgaaggttt tgctaaggct 300
   ttggctgaag aaatcaaagc cagatacgaa aaagctgccg ttaaggttat tgatttggat 360
   gattacacag ccgaagatga caaatacggt gaaaagttga agaaagaaac tatggccttc 420
   ttcatgttgg ctacttatgg tgatggtgaa cctactgata atgctgctag attttacaag 480
   tggttcaccg aaggtactga tagaggtgtt tggttggaac atttgagata cggtgtattc 540
   ggtttgggta acagacaata cgaacacttc aacaagattg ccaaggttgt tgatgatttg 600
   ttggttgaac aaggtgccaa gagattggtt actgttggtt tgggtgatga tgatcaatgc 660
   atcgaagatg atttctccgc ttggaaagaa gccttgtggc cagaattgga tcaattattg 720
   caagatgata ccaacaccgt ttctactcca tacactgctg ttattccaga atacagagtt 780
   gttatccacg atccatctgt tacctcttat gaagatccat actctaacat ggctaacggt      840
   aatgcctctt acgatattca tcatccatgt agagctaacg ttgccgtcca aaaagaattg      900
   cataagccag aatctgacag aagttgcatc catttggaat tcgatatttt cgctactggt      960
   ttgacttacg aaaccggtga tcatgttggt gtttacgctg ataattgtga tgatactgta     1020
   gaagaagccg ctaagttgtt gggtcaacca ttggatttgt tgttctccat tcataccgat     1080
   aacaacgacg gtacttcttt gggttcttct ttgccaccac catttccagg tccatgtact     1140
   ttgagaactg ctttggctag atatgccgat ttgttgaatc caccaaaaaa ggctgctttg     1200
   attgctttag ctgctcatgc tgatgaacca tctgaagctg aaagattgaa gttcttgtca     1260
   tctccacaag gtaaggacga atattctaaa tgggttgtcg gttcccaaag atccttggtt     1320
   gaagttatgg ctgaatttcc atctgctaaa ccaccattgg gtgtattttt tgctgctgtt     1380
   gttcctagat tgcaacctag atattactcc atctcttcca gtccaagatt tgctccacat     1440
   agagttcatg ttacttgcgc tttggtttat ggtccaactc caactggtag aattcacaga     1500
   ggtgtatgtt cattctggat gaagaatgtt gtcccattgg aaaagtctca aaactgttct     1560
   tgggccccaa ttttcatcag acaatctaat ttcaagttgc cagccgatca ttctgttcca     1620
   atagttatgg ttggtccagg tactggttta gctcctttta gaggtttctt acaagaaaga     1680
   ttggccttga aagaagaagg tgctcaagtt ggtcctgctt tgttgttttt tggttgcaga     1740
   aacagacaaa tggacttcat ctacgaagtc gaattgaaca actttgtcga acaaggtgct     1800
   ttgtccgaat tgatcgttgc tttttcaaga gaaggtccat ccaaagaata cgtccaacat     1860
   aagatggttg aaaaggcagc ttacatgtgg aacttgattt ctcaaggtgg ttacttctac     1920
   gtttgtggtg atgctaaagg tatggctaga gatgttcata gaacattgca taccatcgtc     1980
   caacaagaag aaaaggttga ttctaccaag gccgaatcca tcgttaagaa attgcaaatg     2040
   gacggtagat acttgagaga tgtttggtga                                     2070
<210> 88
   <211> 689
   <212> PRT
   <213> Rubus suavissimus
<400> 88
   Met Ser Ser Asn Ser Asp Leu Val Arg Arg Leu Glu Ser Val Leu Gly
   1               5                   10                  15
   Val Ser Phe Gly Gly Ser Val Thr Asp Ser Val Val Val Ile Ala Thr
               20                  25                  30
   Thr Ser Ile Ala Leu Val Ile Gly Val Leu Val Leu Leu Trp Arg Arg
           35                  40                  45
   Ser Ser Asp Arg Ser Arg Glu Val Lys Gln Leu Ala Val Pro Lys Pro
       50                  55                  60
   Val Thr Ile Val Glu Glu Glu Asp Glu Phe Glu Val Ala Ser Gly Lys
   65                  70                  75                  80
   Thr Arg Val Ser Ile Phe Tyr Gly Thr Gln Thr Gly Thr Ala Glu Gly
                   85                  90                  95
   Phe Ala Lys Ala Leu Ala Glu Glu Ile Lys Ala Arg Tyr Glu Lys Ala
               100                 105                 110
   Ala Val Lys Val Ile Asp Leu Asp Asp Tyr Thr Ala Glu Asp Asp Lys
           115                 120                 125
   Tyr Gly Glu Lys Leu Lys Lys Glu Thr Met Ala Phe Phe Met Leu Ala
       130                 135                 140
   Thr Tyr Gly Asp Gly Glu Pro Thr Asp Asn Ala Ala Arg Phe Tyr Lys
   145                 150                 155                 160
   Trp Phe Thr Glu Gly Thr Asp Arg Gly Val Trp Leu Glu His Leu Arg
                   165                 170                 175
   Tyr Gly Val Phe Gly Leu Gly Asn Arg Gln Tyr Glu His Phe Asn Lys
               180                 185                 190
   Ile Ala Lys Val Val Asp Asp Leu Leu Val Glu Gln Gly Ala Lys Arg
           195                 200                 205
   Leu Val Thr Val Gly Leu Gly Asp Asp Asp Gln Cys Ile Glu Asp Asp
       210                 215                 220
   Phe Ser Ala Trp Lys Glu Ala Leu Trp Pro Glu Leu Asp Gln Leu Leu
   225                 230                 235                 240
   Gln Asp Asp Thr Asn Thr Val Ser Thr Pro Tyr Thr Ala Val Ile Pro
                   245                 250                 255
   Glu Tyr Arg Val Val Ile His Asp Pro Ser Val Thr Ser Tyr Glu Asp
               260                 265                 270
   Pro Tyr Ser Asn Met Ala Asn Gly Asn Ala Ser Tyr Asp Ile His His
           275                 280                 285
   Pro Cys Arg Ala Asn Val Ala Val Gln Lys Glu Leu His Lys Pro Glu
   290 295 300
   Ser Asp Arg Ser Cys Ile His Leu Glu Phe Asp Ile Phe Ala Thr Gly
   305 310 315 320
   Leu Thr Tyr Glu Thr Gly Asp His Val Gly Val Tyr Ala Asp Asn Cys
   325 330 335
   Asp Asp Thr Val Glu Glu Ala Ala Lys Leu Leu Gly Gln Pro Leu Asp
   340 345 350
   Leu Leu Phe Ser Ile His Thr Asp Asn Asn Asp Gly Thr Ser Leu Gly
   355 360 365
   Ser Ser Leu Pro Pro Pro Phe Pro Gly Pro Cys Thr Leu Arg Thr Ala
   370 375 380
   Leu Ala Arg Tyr Ala Asp Leu Leu Asn Pro Pro Lys Lys Ala Ala Leu
   385 390 395 400
   Ile Ala Leu Ala Ala His Ala Asp Glu Pro Ser Glu Ala Glu Arg Leu
   405 410 415
   Lys Phe Leu Ser Ser Pro Gln Gly Lys Asp Glu Tyr Ser Lys Trp Val
   420 425 430
   Val Gly Ser Gln Arg Ser Leu Val Glu Val Met Ala Glu Phe Pro Ser
   435 440 445
   Ala Lys Pro Pro Leu Gly Val Phe Phe Ala Ala Val Val Pro Arg Leu
   450 455 460
   Gln Pro Arg Tyr Tyr Ser Ile Ser Ser Ser Pro Arg Phe Ala Pro His
   465 470 475 480
   Arg Val His Val Thr Cys Ala Leu Val Tyr Gly Pro Thr Pro Thr Gly
   485 490 495
   Arg Ile His Arg Gly Val Cys Ser Phe Trp Met Lys Asn Val Val Pro
   500 505 510
   Leu Glu Lys Ser Gln Asn Cys Ser Trp Ala Pro Ile Phe Ile Arg Gln
   515 520 525
   Ser Asn Phe Lys Leu Pro Ala Asp His Ser Val Pro Ile Val Met Val
   530 535 540
   Gly Pro Gly Thr Gly Leu Ala Pro Phe Arg Gly Phe Leu Gln Glu Arg
   545                 550                 555                 560
   Leu Ala Leu Lys Glu Glu Gly Ala Gln Val Gly Pro Ala Leu Leu Phe
                   565                 570                 575
   Phe Gly Cys Arg Asn Arg Gln Met Asp Phe Ile Tyr Glu Val Glu Leu
               580                 585                 590
   Asn Asn Phe Val Glu Gln Gly Ala Leu Ser Glu Leu Ile Val Ala Phe
           595                 600                 605
   Ser Arg Glu Gly Pro Ser Lys Glu Tyr Val Gln His Lys Met Val Glu
       610                 615                 620
   Lys Ala Ala Tyr Met Trp Asn Leu Ile Ser Gln Gly Gly Tyr Phe Tyr
   625                 630                 635                 640
   Val Cys Gly Asp Ala Lys Gly Met Ala Arg Asp Val His Arg Thr Leu
                   645                 650                 655
   His Thr Ile Val Gln Gln Glu Glu Lys Val Asp Ser Thr Lys Ala Glu
               660                 665                 670
   Ser Ile Val Lys Lys Leu Gln Met Asp Gly Arg Tyr Leu Arg Asp Val
           675                 680                 685
   Trp
<210> 89
   <211> 2079
   <212> DNA
   <213> Artificial Sequence
<220>
   <223> Codon-optimized CPR
<400> 89
   atgacttctg cactttatgc ctccgatctt ttcaaacaat tgaaaagtat catgggaacg 60
   gattctttgt ccgatgatgt tgtattagtt attgctacaa cttctctggc actggttgct 120
   ggtttcgttg tcttattgtg gaaaaagacc acggcagatc gttccggcga gctaaagcca 180
   ctaatgatcc ctaagtctct gatggcgaaa gatgaggatg atgacttaga tctaggttct 240
   ggaaaaacga gagtctctat cttcttcggc acacaaaccg gaacagccga aggattcgct 300
   aaagcacttt cagaagagat caaagcaaga tacgaaaagg cggctgtaaa agtaatcgat 360
   ttggatgatt acgctgccga tgatgaccaa tatgaggaaa agttgaaaaa ggaaacattg 420
   gctttctttt gtgtagccac gtatggtgat ggtgaaccaa ccgataacgc cgcaagattc 480
   tacaagtggt ttactgaaga gaacgaaaga gatatcaagt tgcagcaact tgcttacggc 540
   gtttttgcct taggtaacag acaatacgag cactttaaca agataggtat tgtcttagat 600
   gaagagttat gcaaaaaggg tgcgaagaga ttgattgaag tcggtttagg agatgatgat 660
   caatctatcg aggatgactt taatgcatgg aaggaatctt tgtggtctga attagataag 720
   ttacttaagg acgaagatga taaatccgtt gccactccat acacagccgt cattccagaa 780
   tatagagtag ttactcatga tccaagattc acaacacaga aatcaatgga aagtaatgtg 840
   gctaatggta atactaccat cgatattcat catccatgta gagtagacgt tgcagttcaa 900
   aaggaattgc acactcatga atcagacaga tcttgcatac atcttgaatt tgatatatca 960
   cgtactggta tcacttacga aacaggtgat cacgtgggtg tctacgctga aaaccatgtt 1020
   gaaattgtag aggaagctgg aaagttgttg ggccatagtt tagatcttgt tttctcaatt 1080
   catgccgata aagaggatgg ctcaccacta gaaagtgcag tgcctccacc atttccagga 1140
   ccatgcaccc taggtaccgg tttagctcgt tacgcggatc tgttaaatcc tccacgtaaa 1200
   tcagctctag tggccttggc tgcgtacgcc acagaacctt ctgaggcaga aaaactgaaa 1260
   catctaactt caccagatgg taaggatgaa tactcacaat ggatagtagc tagtcaacgt 1320
   tctttactag aagttatggc tgctttccca tccgctaaac ctcctttggg tgttttcttc 1380
   gccgcaatag cgcctagact gcaaccaaga tactattcaa tttcatcctc acctagactg 1440
   gcaccatcaa gagttcatgt cacatccgct ttagtgtacg gtccaactcc tactggtaga 1500
   atccataagg gcgtttgttc aacatggatg aaaaacgcgg ttccagcaga gaagtctcac 1560
   gaatgttctg gtgctccaat ctttatcaga gcctccaact tcaaactgcc ttccaatcct 1620
   tctactccta ttgtcatggt cggtcctggt acaggtcttg ctccattcag aggtttctta 1680
   caagagagaa tggccttaaa ggaggatggt gaagagttgg gatcttcttt gttgtttttc 1740
   ggctgtagaa acagacaaat ggatttcatc tacgaagatg aactgaataa ctttgtagat 1800
   caaggagtta tttcagagtt gataatggct ttttctagag aaggtgctca gaaggagtac 1860
   gtccaacaca aaatgatgga aaaggccgca caagtttggg acttaatcaa agaggaaggc 1920
   tatctatatg tctgtggtga tgcaaagggt atggcaagag atgttcacag aacacttcat 1980
   actatagtcc aggaacagga aggcgttagt tcttctgaag cggaagcaat tgtgaaaaag 2040
   ttacaaacag agggaagata cttgagagat gtgtggtaa 2079
<210> 90
   <211> 692
   <212> PRT
   <213> Arabidopsis thaliana
<400> 90
<210> 91
   <211> 2139
   <212> DNA
   <213> Artificial Sequence
<220>
   <223> Codon-optimized CPR
<400> 91
<210> 92
   <211> 712
   <212> PRT
   <213> Arabidopsis thaliana
<400> 92
   Met Ser Ser Ser Ser Ser Ser Ser Thr Ser Met Ile Asp Leu Met Ala
   1               5                   10                  15
   Ala Ile Ile Lys Gly Glu Pro Val Ile Val Ser Asp Pro Ala Asn Ala
               20                  25                  30
   Ser Ala Tyr Glu Ser Val Ala Ala Glu Leu Ser Ser Met Leu Ile Glu
           35                  40                  45
   Asn Arg Gln Phe Ala Met Ile Val Thr Thr Ser Ile Ala Val Leu Ile
       50                  55                  60
   Gly Cys Ile Val Met Leu Val Trp Arg Arg Ser Gly Ser Gly Asn Ser
   65                  70                  75                  80
   Lys Arg Val Glu Pro Leu Lys Pro Leu Val Ile Lys Pro Arg Glu Glu
                   85                  90                  95
   Glu Ile Asp Asp Gly Arg Lys Lys Val Thr Ile Phe Phe Gly Thr Gln
               100                 105                 110
   Thr Gly Thr Ala Glu Gly Phe Ala Lys Ala Leu Gly Glu Glu Ala Lys
           115                 120                 125
   Ala Arg Tyr Glu Lys Thr Arg Phe Lys Ile Val Asp Leu Asp Asp Tyr
       130                 135                 140
   Ala Ala Asp Asp Asp Glu Tyr Glu Glu Lys Leu Lys Lys Glu Asp Val
   145                 150                 155                 160
   Ala Phe Phe Phe Leu Ala Thr Tyr Gly Asp Gly Glu Pro Thr Asp Asn
                   165                 170                 175
   Ala Ala Arg Phe Tyr Lys Trp Phe Thr Glu Gly Asn Asp Arg Gly Glu
               180                 185                 190
   Trp Leu Lys Asn Leu Lys Tyr Gly Val Phe Gly Leu Gly Asn Arg Gln
   195 200 205
   Tyr Glu His Phe Asn Lys Val Ala Lys Val Val Asp Asp Ile Leu Val
   210 215 220
   Glu Gln Gly Ala Gln Arg Leu Val Gln Val Gly Leu Gly Asp Asp Asp
   225 230 235 240
   Gln Cys Ile Glu Asp Asp Phe Thr Ala Trp Arg Glu Ala Leu Trp Pro
   245 250 255
   Glu Leu Asp Thr Ile Leu Arg Glu Glu Gly Asp Thr Ala Val Ala Thr
   260 265 270
   Pro Tyr Thr Ala Ala Val Leu Glu Tyr Arg Val Ser Ile His Asp Ser
   275 280 285
   Glu Asp Ala Lys Phe Asn Asp Ile Thr Leu Ala Asn Gly Asn Gly Tyr
   290 295 300
   Thr Val Phe Asp Ala Gln His Pro Tyr Lys Ala Asn Val Ala Val Lys
   305 310 315 320
   Arg Glu Leu His Thr Pro Glu Ser Asp Arg Ser Cys Ile His Leu Glu
   325 330 335
   Phe Asp Ile Ala Gly Ser Gly Leu Thr Met Lys Leu Gly Asp His Val
   340 345 350
   Gly Val Leu Cys Asp Asn Leu Ser Glu Thr Val Asp Glu Ala Leu Arg
   355 360 365
   Leu Leu Asp Met Ser Pro Asp Thr Tyr Phe Ser Leu His Ala Glu Lys
   370 375 380
   Glu Asp Gly Thr Pro Ile Ser Ser Ser Leu Pro Pro Pro Phe Pro Pro
   385 390 395 400
   Cys Asn Leu Arg Thr Ala Leu Thr Arg Tyr Ala Cys Leu Leu Ser Ser
   405 410 415
   Pro Lys Lys Ser Ala Leu Val Ala Leu Ala Ala His Ala Ser Asp Pro
   420 425 430
   Thr Glu Ala Glu Arg Leu Lys His Leu Ala Ser Pro Ala Gly Lys Asp
   435 440 445
   Glu Tyr Ser Lys Trp Val Val Glu Ser Gln Arg Ser Leu Leu Glu Val
   450 455 460
   Met Ala Glu Phe Pro Ser Ala Lys Pro Pro Leu Gly Val Phe Phe Ala
   465 470 475 480
   Gly Val Ala Pro Arg Leu Gln Pro Arg Phe Tyr Ser Ile Ser Ser Ser
   485 490 495
   Pro Lys Ile Ala Glu Thr Arg Ile His Val Thr Cys Ala Leu Val Tyr
   500 505 510
   Glu Lys Met Pro Thr Gly Arg Ile His Lys Gly Val Cys Ser Thr Trp
   515 520 525
   Met Lys Asn Ala Val Pro Tyr Glu Lys Ser Glu Lys Leu Phe Leu Gly
   530 535 540
   Arg Pro Ile Phe Val Arg Gln Ser Asn Phe Lys Leu Pro Ser Asp Ser
   545 550 555 560
   Lys Val Pro Ile Ile Met Ile Gly Pro Gly Thr Gly Leu Ala Pro Phe
   565 570 575
   Arg Gly Phe Leu Gln Glu Arg Leu Ala Leu Val Glu Ser Gly Val Glu
   580 585 590
   Leu Gly Pro Ser Val Leu Phe Phe Gly Cys Arg Asn Arg Arg Met Asp
   595 600 605
   Phe Ile Tyr Glu Glu Glu Leu Gln Arg Phe Val Glu Ser Gly Ala Leu
   610 615 620
   Ala Glu Leu Ser Val Ala Phe Ser Arg Glu Gly Pro Thr Lys Glu Tyr
   625 630 635 640
   Val Gln His Lys Met Met Asp Lys Ala Ser Asp Ile Trp Asn Met Ile
   645 650 655
   Ser Gln Gly Ala Tyr Leu Tyr Val Cys Gly Asp Ala Lys Gly Met Ala
   660 665 670
   Arg Asp Val His Arg Ser Leu His Thr Ile Ala Gln Glu Gln Gly Ser
   675 680 685
   Met Asp Ser Thr Lys Ala Glu Gly Phe Val Lys Asn Leu Gln Thr Ser
   690 695 700
   Gly Arg Tyr Leu Arg Asp Val Trp
   705 710
<210> 93
   <211> 1503
   <212> DNA
   <213> Artificial Sequence
<220>
   <223> Codon-optimized KAH
<400> 93
   atggaagcct cttacctata catttctatt ttgcttttac tggcatcata cctgttcacc      60
   actcaactta gaaggaagag cgctaatcta ccaccaaccg tgtttccatc aataccaatc     120
   attggacact tatacttact caaaaagcct ctttatagaa ctttagcaaa aattgccgct     180
   aagtacggac caatactgca attacaactc ggctacagac gtgttctggt gatttcctca     240
   ccatcagcag cagaagagtg ctttaccaat aacgatgtaa tcttcgcaaa tagacctaag     300
   acattgtttg gcaaaatagt gggtggaaca tcccttggca gtttatccta cggcgatcaa     360
   tggcgtaatc taaggagagt agcttctatc gaaatcctat cagttcatag gttgaacgaa     420
   tttcatgata tcagagtgga tgagaacaga ttgttaatta gaaaacttag aagttcatct     480
   tctcctgtta ctcttataac agtcttttat gctctaacat tgaacgtcat tatgagaatg     540
   atctctggca aaagatattt cgacagtggg gatagagaat tggaggagga aggtaagaga     600
   tttcgagaaa tcttagacga aacgttgctt ctagccggtg cttctaatgt tggcgactac     660
   ttaccaatat tgaactggtt gggagttaag tctcttgaaa agaaattgat cgctttgcag     720
   aaaaagagag atgacttttt ccagggtttg attgaacagg ttagaaaatc tcgtggtgct     780
   aaagtaggca aaggtagaaa aacgatgatc gaactcttat tatctttgca agagtcagaa     840
   cctgagtact atacagatgc tatgataaga tcttttgtcc taggtctgct ggctgcaggt     900
   agtgatactt cagcgggcac tatggaatgg gccatgagct tactggtcaa tcacccacat     960
   gtattgaaga aagctcaagc tgaaatcgat agagttatcg gtaataacag attgattgac    1020
   gagtcagaca ttggaaatat cccttacatc gggtgtatta tcaatgaaac tctaagactc    1080
   tatccagcag ggccattgtt gttcccacat gaaagttctg ccgactgcgt tatttccggt    1140
   tacaatatac ctagaggtac aatgttaatc gtaaaccaat gggcgattca tcacgatcct    1200
   aaagtctggg atgatcctga aacctttaaa cctgaaagat ttcaaggatt agaaggaact    1260
   agagatggtt tcaaacttat gccattcggt tctgggagaa gaggatgtcc aggtgaaggt    1320
   ttggcaataa ggctgttagg gatgacacta ggctcagtga tccaatgttt tgattgggag    1380
   agagtaggag atgagatggt tgacatgaca gaaggtttgg gtgtcacact tcctaaggcc    1440
   gttccattag ttgccaaatg taagccacgt tccgaaatga ctaatctcct atccgaactt    1500
   taa                                                                  1503
<210> 94
   <211> 500
   <212> PRT
   <213> Stevia rebaudiana
<400> 94
   Met Glu Ala Ser Tyr Leu Tyr Ile Ser Ile Leu Leu Leu Leu Ala Ser
   1               5                   10                  15
   Tyr Leu Phe Thr Thr Gln Leu Arg Arg Lys Ser Ala Asn Leu Pro Pro
               20                  25                  30
   Thr Val Phe Pro Ser Ile Pro Ile Ile Gly His Leu Tyr Leu Leu Lys
           35                  40                  45
   Lys Pro Leu Tyr Arg Thr Leu Ala Lys Ile Ala Ala Lys Tyr Gly Pro
       50                  55                  60
   Ile Leu Gln Leu Gln Leu Gly Tyr Arg Arg Val Leu Val Ile Ser Ser
   65                  70                  75                  80
   Pro Ser Ala Ala Glu Glu Cys Phe Thr Asn Asn Asp Val Ile Phe Ala
                   85                  90                  95
   Asn Arg Pro Lys Thr Leu Phe Gly Lys Ile Val Gly Gly Thr Ser Leu
               100                 105                 110
   Gly Ser Leu Ser Tyr Gly Asp Gln Trp Arg Asn Leu Arg Arg Val Ala
           115                 120                 125
   Ser Ile Glu Ile Leu Ser Val His Arg Leu Asn Glu Phe His Asp Ile
       130                 135                 140
   Arg Val Asp Glu Asn Arg Leu Leu Ile Arg Lys Leu Arg Ser Ser Ser
   145                 150                 155                 160
   Ser Pro Val Thr Leu Ile Thr Val Phe Tyr Ala Leu Thr Leu Asn Val
                   165                 170                 175
   Ile Met Arg Met Ile Ser Gly Lys Arg Tyr Phe Asp Ser Gly Asp Arg
               180                 185                 190
   Glu Leu Glu Glu Glu Gly Lys Arg Phe Arg Glu Ile Leu Asp Glu Thr
           195                 200                 205
   Leu Leu Leu Ala Gly Ala Ser Asn Val Gly Asp Tyr Leu Pro Ile Leu
       210                 215                 220
   Asn Trp Leu Gly Val Lys Ser Leu Glu Lys Lys Leu Ile Ala Leu Gln
   225                 230                 235                 240
   Lys Lys Arg Asp Asp Phe Phe Gln Gly Leu Ile Glu Gln Val Arg Lys
                   245                 250                 255
   Ser Arg Gly Ala Lys Val Gly Lys Gly Arg Lys Thr Met Ile Glu Leu
               260                 265                 270
   Leu Leu Ser Leu Gln Glu Ser Glu Pro Glu Tyr Tyr Thr Asp Ala Met
           275                 280                 285
   Ile Arg Ser Phe Val Leu Gly Leu Leu Ala Ala Gly Ser Asp Thr Ser
       290                 295                 300
   Ala Gly Thr Met Glu Trp Ala Met Ser Leu Leu Val Asn His Pro His
   305                 310                 315                 320
   Val Leu Lys Lys Ala Gln Ala Glu Ile Asp Arg Val Ile Gly Asn Asn
                   325                 330                 335
   Arg Leu Ile Asp Glu Ser Asp Ile Gly Asn Ile Pro Tyr Ile Gly Cys
               340                 345                 350
   Ile Ile Asn Glu Thr Leu Arg Leu Tyr Pro Ala Gly Pro Leu Leu Phe
           355                 360                 365
   Pro His Glu Ser Ser Ala Asp Cys Val Ile Ser Gly Tyr Asn Ile Pro
       370                 375                 380
   Arg Gly Thr Met Leu Ile Val Asn Gln Trp Ala Ile His His Asp Pro
   385                 390                 395                 400
   Lys Val Trp Asp Asp Pro Glu Thr Phe Lys Pro Glu Arg Phe Gln Gly
                   405                 410                 415
   Leu Glu Gly Thr Arg Asp Gly Phe Lys Leu Met Pro Phe Gly Ser Gly
               420                 425                 430
   Arg Arg Gly Cys Pro Gly Glu Gly Leu Ala Ile Arg Leu Leu Gly Met
           435                 440                 445
   Thr Leu Gly Ser Val Ile Gln Cys Phe Asp Trp Glu Arg Val Gly Asp
       450                 455                 460
   Glu Met Val Asp Met Thr Glu Gly Leu Gly Val Thr Leu Pro Lys Ala
   465 470 475 480
   Val Pro Leu Val Ala Lys Cys Lys Pro Arg Ser Glu Met Thr Asn Leu
   485 490 495
   Leu Ser Glu Leu
   500
<210> 95
   <211> 1572
   <212> DNA
   <213> Rubus suavissimus
<400> 95
   atggaagtaa cagtagctag tagtgtagcc ctgagcctgg tctttattag catagtagta 60
   agatgggcat ggagtgtggt gaattgggtg tggtttaagc cgaagaagct ggaaagattt 120
   ttgagggagc aaggccttaa aggcaattcc tacaggtttt tatatggaga catgaaggag 180
   aactctatcc tgctcaaaca agcaagatcc aaacccatga acctctccac ctcccatgac 240
   atagcacctc aagtcacccc ttttgtcgac caaaccgtga aagcttacgg taagaactct 300
   tttaattggg ttggccccat accaagggtg aacataatga atccagaaga tttgaaggac 360
   gtcttaacaa aaaatgttga ctttgttaag ccaatatcaa acccacttat caagttgcta 420
   gctacaggta ttgcaatcta tgaaggtgag aaatggacta aacacagaag gattatcaac 480
   ccaacattcc attcggagag gctaaagcgt atgttacctt catttcacca aagttgtaat 540
   gagatggtca aggaatggga gagcttggtg tcaaaagagg gttcatcatg tgagttggat 600
   gtctggcctt ttcttgaaaa tatgtcggca gatgtgatct cgagaacagc atttggaact 660
   agctacaaaa aaggacagaa aatctttgaa ctcttgagag agcaagtaat atatgtaacg 720
   aaaggctttc aaagttttta cattccagga tggaggtttc tcccaactaa gatgaacaag 780
   aggatgaatg agattaacga agaaataaaa ggattaatca ggggtattat aattgacaga 840
   gagcaaatca ttaaggcagg tgaagaaacc aacgatgact tattaggtgc acttatggag 900
   tcaaacttga aggacattcg ggaacatggg aaaaacaaca aaaatgttgg gatgagtatt 960
   gaagatgtaa ttcaggagtg taagctgttt tactttgctg ggcaagaaac cacttcagtg 1020
   ttgctggctt ggacaatggt tttacttggt caaaatcaga actggcaaga tcgagcaaga 1080
   caagaggttt tgcaagtctt tggaagcagc aagccagatt ttgatggtct agctcacctt 1140
   aaagtcgtaa ccatgatttt gcttgaagtt cttcgattat acccaccagt cattgaactt 1200
   attcgaacca ttcacaagaa aacacaactt gggaagctct cactaccaga aggagttgaa 1260
   gtccgcttac caacactgct cattcaccat gacaaggaac tgtggggtga tgatgcaaac 1320
   cagttcaatc cagagaggtt ttcggaagga gtttccaaag caacaaagaa ccgactctca 1380
   ttcttcccct tcggagccgg tccacgcatt tgcattggac agaacttttc tatgatggaa 1440
   gcaaagttgg ccttagcatt gatcttgcaa cacttcacct ttgagctttc tccatctcat 1500
   gcacatgctc cttcccatcg tataaccctt caaccacagt atggtgttcg tatcatttta 1560
   catcgacgtt ag 1572
<210> 96
   <211> 1572
   <212> DNA
   <213> Artificial Sequence
<220>
   <223> Codon-optimized KAH
<400> 96
   atggaagtca ctgtcgcctc ttctgtcgct ttatccttag tcttcatttc cattgtcgtc      60
   agatgggctt ggtccgttgt caactgggtt tggttcaaac caaagaagtt ggaaagattc     120
   ttgagagagc aaggtttgaa gggtaattct tatagattct tgtacggtga catgaaggaa     180
   aattctattt tgttgaagca agccagatcc aaaccaatga acttgtctac ctctcatgat     240
   attgctccac aagttactcc attcgtcgat caaactgtta aagcctacgg taagaactct     300
   ttcaattggg ttggtccaat tcctagagtt aacatcatga acccagaaga tttgaaggat     360
   gtcttgacca agaacgttga cttcgttaag ccaatttcca acccattgat taaattgttg     420
   gctactggta ttgccattta cgaaggtgaa aagtggacta agcatagaag aatcatcaac     480
   cctaccttcc actctgaaag attgaagaga atgttaccat ctttccatca atcctgtaat     540
   gaaatggtta aggaatggga atccttggtt tctaaagaag gttcttcttg cgaattggat     600
   gtttggccat tcttggaaaa tatgtctgct gatgtcattt ccagaaccgc tttcggtacc     660
   tcctacaaga agggtcaaaa gattttcgaa ttgttgagag agcaagttat ttacgttacc     720
   aagggtttcc aatccttcta catcccaggt tggagattct tgccaactaa aatgaacaag     780
   cgtatgaacg agatcaacga agaaattaaa ggtttgatca gaggtattat tatcgacaga     840
   gaacaaatta ttaaagctgg tgaagaaacc aacgatgatt tgttgggtgc tttgatggag     900
   tccaacttga aggatattag agaacatggt aagaacaaca agaatgttgg tatgtctatt     960
   gaagatgtta ttcaagaatg taagttattc tacttcgctg gtcaagagac cacttctgtt    1020
   ttgttagcct ggactatggt cttgttaggt caaaaccaaa attggcaaga tagagctaga    1080
   caagaagttt tgcaagtctt cggttcttcc aagccagact ttgatggttt ggcccacttg    1140
   aaggttgtta ctatgatttt gttagaagtt ttgagattgt acccaccagt cattgagtta    1200
   atcagaacca ttcataaaaa gactcaattg ggtaaattat ctttgccaga aggtgttgaa    1260
   gtcagattac caaccttgtt gattcaccac gataaggaat tatggggtga cgacgctaat    1320
   caatttaatc cagaaagatt ttccgaaggt gtttccaagg ctaccaaaaa ccgtttgtcc    1380
   ttcttcccat ttggtgctgg tccacgtatt tgtatcggtc aaaacttttc catgatggaa    1440
   gccaagttgg ctttggcttt aatcttgcaa cacttcactt tcgaattgtc tccatcccat    1500
   gcccacgctc cttctcatag aatcacttta caaccacaat acggtgtcag aatcatctta    1560
   cacagaagat aa                                                        1572
<210> 97
   <211> 523
   <212> PRT
   <213> Rubus suavissimus
<400> 97
   Met Glu Val Thr Val Ala Ser Ser Val Ala Leu Ser Leu Val Phe Ile
   1               5                   10                  15
   Ser Ile Val Val Arg Trp Ala Trp Ser Val Val Asn Trp Val Trp Phe
               20                  25                  30
   Lys Pro Lys Lys Leu Glu Arg Phe Leu Arg Glu Gln Gly Leu Lys Gly
           35                  40                  45
   Asn Ser Tyr Arg Phe Leu Tyr Gly Asp Met Lys Glu Asn Ser Ile Leu
       50                  55                  60
   Leu Lys Gln Ala Arg Ser Lys Pro Met Asn Leu Ser Thr Ser His Asp
   65                  70                  75                  80
   Ile Ala Pro Gln Val Thr Pro Phe Val Asp Gln Thr Val Lys Ala Tyr
                   85                  90                  95
   Gly Lys Asn Ser Phe Asn Trp Val Gly Pro Ile Pro Arg Val Asn Ile
               100                 105                 110
   Met Asn Pro Glu Asp Leu Lys Asp Val Leu Thr Lys Asn Val Asp Phe
           115                 120                 125
   Val Lys Pro Ile Ser Asn Pro Leu Ile Lys Leu Leu Ala Thr Gly Ile
       130                 135                 140
   Ala Ile Tyr Glu Gly Glu Lys Trp Thr Lys His Arg Arg Ile Ile Asn
   145                 150                 155                 160
   Pro Thr Phe His Ser Glu Arg Leu Lys Arg Met Leu Pro Ser Phe His
                   165                 170                 175
   Gln Ser Cys Asn Glu Met Val Lys Glu Trp Glu Ser Leu Val Ser Lys
               180                 185                 190
   Glu Gly Ser Ser Cys Glu Leu Asp Val Trp Pro Phe Leu Glu Asn Met
           195                 200                 205
   Ser Ala Asp Val Ile Ser Arg Thr Ala Phe Gly Thr Ser Tyr Lys Lys
       210                 215                 220
   Gly Gln Lys Ile Phe Glu Leu Leu Arg Glu Gln Val Ile Tyr Val Thr
   225                 230                 235                 240
   Lys Gly Phe Gln Ser Phe Tyr Ile Pro Gly Trp Arg Phe Leu Pro Thr
                   245                 250                 255
   Lys Met Asn Lys Arg Met Asn Glu Ile Asn Glu Glu Ile Lys Gly Leu
               260                 265                 270
   Ile Arg Gly Ile Ile Ile Asp Arg Glu Gln Ile Ile Lys Ala Gly Glu
           275                 280                 285
   Glu Thr Asn Asp Asp Leu Leu Gly Ala Leu Met Glu Ser Asn Leu Lys
       290                 295                 300
   Asp Ile Arg Glu His Gly Lys Asn Asn Lys Asn Val Gly Met Ser Ile
   305                 310                 315                 320
   Glu Asp Val Ile Gln Glu Cys Lys Leu Phe Tyr Phe Ala Gly Gln Glu
                   325                 330                 335
   Thr Thr Ser Val Leu Leu Ala Trp Thr Met Val Leu Leu Gly Gln Asn
               340                 345                 350
   Gln Asn Trp Gln Asp Arg Ala Arg Gln Glu Val Leu Gln Val Phe Gly
           355                 360                 365
   Ser Ser Lys Pro Asp Phe Asp Gly Leu Ala His Leu Lys Val Val Thr
       370                 375                 380
   Met Ile Leu Leu Glu Val Leu Arg Leu Tyr Pro Pro Val Ile Glu Leu
   385                 390                 395                 400
   Ile Arg Thr Ile His Lys Lys Thr Gln Leu Gly Lys Leu Ser Leu Pro
                   405                 410                 415
   Glu Gly Val Glu Val Arg Leu Pro Thr Leu Leu Ile His His Asp Lys
               420                 425                 430
   Glu Leu Trp Gly Asp Asp Ala Asn Gln Phe Asn Pro Glu Arg Phe Ser
           435                 440                 445
   Glu Gly Val Ser Lys Ala Thr Lys Asn Arg Leu Ser Phe Phe Pro Phe
       450                 455                 460
   Gly Ala Gly Pro Arg Ile Cys Ile Gly Gln Asn Phe Ser Met Met Glu
   465                 470                 475                 480
   Ala Lys Leu Ala Leu Ala Leu Ile Leu Gln His Phe Thr Phe Glu Leu
                   485                 490                 495
   Ser Pro Ser His Ala His Ala Pro Ser His Arg Ile Thr Leu Gln Pro
               500                 505                 510
   Gln Tyr Gly Val Arg Ile Ile Leu His Arg Arg
           515                 520
<210> 98
   <211> 1566
   <212> DNA
   <213> Prunus avium
<400> 98
   atggaagcat caagggctag ttgtgttgcg ctatgtgttg tttgggtgag catagtaatt 60
   acattggcat ggagggtgct gaattgggtg tggttgaggc caaagaaact agaaagatgc 120
   ttgagggagc aaggccttac aggcaattct tacaggcttt tgtttggaga caccaaggat 180
   ctctcgaaga tgctggaaca aacacaatcc aaacccatca aactctccac ctcccatgat 240
   atagcgccac gagtcacccc atttttccat cgaactgtga actctaatgg caagaattct 300
   tttgtttgga tgggccctat accaagagtg cacatcatga atccagaaga tttgaaagat 360
   gccttcaaca gacatgatga ttttcataag acagtaaaaa atcctatcat gaagtctcca 420
   ccaccgggca ttgtaggcat tgaaggtgag caatgggcta aacacagaaa gattatcaac 480
   ccagcattcc atttagagaa gctaaagggt atggtaccaa tattttacca aagttgtagc 540
   gagatgatta acaaatggga gagcttggtg tccaaagaga gttcatgtga gttggatgtg 600
   tggccttatc ttgaaaattt taccagcgat gtgatttccc gagctgcatt tggaagtagc 660
   tatgaagagg gaaggaaaat atttcaacta ctaagagagg aagcaaaagt ttattcggta 720
   gctctacgaa gtgtttacat tccaggatgg aggtttctac caaccaagca gaacaagaag 780
   acgaaggaaa ttcacaatga aattaaaggc ttacttaagg gcattataaa taaaagggaa 840
   gaggcgatga aggcagggga agccactaaa gatgacttac taggaatact tatggagtcc 900
   aacttcaggg aaattcagga acatgggaac aacaaaaatg ctggaatgag tattgaagat 960
   gtaattggag agtgtaagtt gttttacttt gctgggcaag agaccacttc ggtgttgctt 1020
   gtttggacaa tgattttact aagccaaaat caggattggc aagctcgtgc aagagaagag 1080
   gtcttgaaag tctttggaag caacatccca acctatgaag agctaagtca cctaaaagtt 1140
   gtgaccatga ttttacttga agttcttcga ttatacccat cagtcgttgc gcttcctcga 1200
   accactcaca agaaaacaca gcttggaaaa ttatcattac cagctggagt ggaagtctcc 1260
   ttgcccatac tgcttgttca ccatgacaaa gagttgtggg gtgaggatgc aaatgagttc 1320
   aagccagaga ggttttcaga gggagtttca aaggcaacaa agaacaaatt tacatactta 1380
   cctttcggag gggtccaagg gatttgcatt ggacaaaact ttgccatggt ggaagctaaa 1440
   ttggccttgg ccctgatttt acaacacttt gcctttgagc tttctccatc ctatgctcat 1500
   gctccttctg cagttataac ccttcaacct caatttggtg ctcatatcat tttgcataaa 1560
   cgttga 1566
<210> 99
   <211> 1567
   <212> DNA
   <213> Artificial Sequence
<220>
   <223> Codon-optimized KAH
<400> 99
   atggaagctt ctagagcatc ttgtgttgct ttgtgtgttg tttgggtttc catcgttatt      60
   actttggctt ggagagtttt gaattgggtc tggttaagac caaaaaagtt ggaaagatgc     120
   ttgagagaac aaggtttgac tggtaactct tacagattgt tgttcggtga taccaaggac     180
   ttgtctaaga tgttggaaca aactcaatcc aagcctatca agttgtctac ctctcatgat     240
   attgctccaa gagttactcc attcttccat agaactgtta actccaacgg taagaactct     300
   tttgtttgga tgggtccaat tccaagagtc catattatga accctgaaga tttgaaggac     360
   gctttcaaca gacatgatga tttccataag accgtcaaga acccaattat gaagtctcca     420
   ccaccaggta tagttggtat tgaaggtgaa caatgggcca aacatagaaa gattattaac     480
   ccagccttcc acttggaaaa gttgaaaggt atggttccaa tcttctacca atcctgctct     540
   gaaatgatta acaagtggga atccttggtt tccaaagaat cttcctgtga attggatgtc     600
   tggccatatt tggaaaactt cacctccgat gttatttcca gagctgcttt tggttcttct     660
   tacgaagaag gtagaaagat cttccaatta ttgagagaag aagccaaggt ttactccgtt     720
   gctttgagat ctgtttacat tccaggttgg agattcttgc caactaagca aaacaaaaag     780
   accaaagaaa tccacaacga aatcaagggt ttgttgaagg gtatcatcaa caagagagaa     840
   gaagctatga aggctggtga agctacaaaa gatgatttgt tgggtatctt gatggaatcc     900
   aacttcagag aaatccaaga acacggtaac aacaagaatg ccggtatgtc tattgaagat     960
   gttatcggtg aatgcaagtt gttctacttt gctggtcaag aaactacctc cgttttgttg    1020
   gtttggacca tgattttgtt gtcccaaaat caagattggc aagctagagc tagagaagaa    1080
   gtcttgaaag ttttcggttc taacatccca acctacgaag aattgtctca cttgaaggtt    1140
   gtcactatga tcttgttgga agtattgaga ttatacccat ccgttgttgc attgccaaga    1200
   actactcata agaaaactca attgggtaaa ttgtccttgc cagctggtgt tgaagtttct    1260
   ttgccaattt tgttagtcca ccacgacaaa gaattgtggg gtgaagatgc taatgaattc    1320
   aagccagaaa gattctccga aggtgtttct aaagctacca agaacaagtt cacttacttg    1380
   ccatttggtg gtggtccaag aatatgtatt ggtcaaaatt tcgctatggt cgaagctaaa    1440
   ttggctttgg ctttgatctt gcaacatttc gctttcgaat tgtcaccatc ttatgctcat    1500
   gctccatctg ctgttattac attgcaacca caatttggtg cccatatcat cttgcataag    1560
   agataac                                                              1567
<210> 100
   <211> 521
   <212> PRT
   <213> Prunus avium
<400> 100
   Met Glu Ala Ser Arg Ala Ser Cys Val Ala Leu Cys Val Val Trp Val
   1               5                   10                  15
   Ser Ile Val Ile Thr Leu Ala Trp Arg Val Leu Asn Trp Val Trp Leu
               20                  25                  30
   Arg Pro Lys Lys Leu Glu Arg Cys Leu Arg Glu Gln Gly Leu Thr Gly
           35                  40                  45
   Asn Ser Tyr Arg Leu Leu Phe Gly Asp Thr Lys Asp Leu Ser Lys Met
       50                  55                  60
   Leu Glu Gln Thr Gln Ser Lys Pro Ile Lys Leu Ser Thr Ser His Asp
   65                  70                  75                  80
   Ile Ala Pro Arg Val Thr Pro Phe Phe His Arg Thr Val Asn Ser Asn
                   85                  90                  95
   Gly Lys Asn Ser Phe Val Trp Met Gly Pro Ile Pro Arg Val His Ile
               100                 105                 110
   Met Asn Pro Glu Asp Leu Lys Asp Ala Phe Asn Arg His Asp Asp Phe
           115                 120                 125
   His Lys Thr Val Lys Asn Pro Ile Met Lys Ser Pro Pro Pro Gly Ile
       130                 135                 140
   Val Gly Ile Glu Gly Glu Gln Trp Ala Lys His Arg Lys Ile Ile Asn
   145                 150                 155                 160
   Pro Ala Phe His Leu Glu Lys Leu Lys Gly Met Val Pro Ile Phe Tyr
                   165                 170                 175
   Gln Ser Cys Ser Glu Met Ile Asn Lys Trp Glu Ser Leu Val Ser Lys
               180                 185                 190
   Glu Ser Ser Cys Glu Leu Asp Val Trp Pro Tyr Leu Glu Asn Phe Thr
           195                 200                 205
   Ser Asp Val Ile Ser Arg Ala Ala Phe Gly Ser Ser Tyr Glu Glu Gly
       210                 215                 220
   Arg Lys Ile Phe Gln Leu Leu Arg Glu Glu Ala Lys Val Tyr Ser Val
   225                 230                 235                 240
   Ala Leu Arg Ser Val Tyr Ile Pro Gly Trp Arg Phe Leu Pro Thr Lys
                   245                 250                 255
   Gln Asn Lys Lys Thr Lys Glu Ile His Asn Glu Ile Lys Gly Leu Leu
               260                 265                 270
   Lys Gly Ile Ile Asn Lys Arg Glu Glu Ala Met Lys Ala Gly Glu Ala
           275                 280                 285
   Thr Lys Asp Asp Leu Leu Gly Ile Leu Met Glu Ser Asn Phe Arg Glu
       290                 295                 300
   Ile Gln Glu His Gly Asn Asn Lys Asn Ala Gly Met Ser Ile Glu Asp
   305                 310                 315                 320
   Val Ile Gly Glu Cys Lys Leu Phe Tyr Phe Ala Gly Gln Glu Thr Thr
                   325                 330                 335
   Ser Val Leu Leu Val Trp Thr Met Ile Leu Leu Ser Gln Asn Gln Asp
               340                 345                 350
   Trp Gln Ala Arg Ala Arg Glu Glu Val Leu Lys Val Phe Gly Ser Asn
           355                 360                 365
   Ile Pro Thr Tyr Glu Glu Leu Ser His Leu Lys Val Val Thr Met Ile
       370                 375                 380
   Leu Leu Glu Val Leu Arg Leu Tyr Pro Ser Val Val Ala Leu Pro Arg
   385 390 395 400
   Thr Thr His Lys Lys Thr Gln Leu Gly Lys Leu Ser Leu Pro Ala Gly
   405 410 415
   Val Glu Val Ser Leu Pro Ile Leu Leu Val His His Asp Lys Glu Leu
   420 425 430
   Trp Gly Glu Asp Ala Asn Glu Phe Lys Pro Glu Arg Phe Ser Glu Gly
   435 440 445
   Val Ser Lys Ala Thr Lys Asn Lys Phe Thr Tyr Leu Pro Phe Gly Gly
   450 455 460
   Gly Pro Arg Ile Cys Ile Gly Gln Asn Phe Ala Met Val Glu Ala Lys
   465 470 475 480
   Leu Ala Leu Ala Leu Ile Leu Gln His Phe Ala Phe Glu Leu Ser Pro
   485 490 495
   Ser Tyr Ala His Ala Pro Ser Ala Val Ile Thr Leu Gln Pro Gln Phe
   500 505 510
   Gly Ala His Ile Ile Leu His Lys Arg
   515 520
<210> 101
   <211> 517
   <212> PRT
   <213> Prunus mume
<400> 101
   Ala Ser Trp Val Ala Val Leu Ser Val Val Trp Val Ser Met Val Ile
   1               5                   10                  15
   Ala Trp Ala Trp Arg Val Leu Asn Trp Val Trp Leu Arg Pro Lys Lys
               20                  25                  30
   Leu Glu Lys Cys Leu Arg Glu Gln Gly Leu Ala Gly Asn Ser Tyr Arg
           35                  40                  45
   Leu Leu Phe Gly Asp Thr Lys Asp Leu Ser Lys Met Leu Glu Gln Thr
       50                  55                  60
   Gln Ser Lys Pro Ile Lys Leu Ser Thr Ser His Asp Ile Ala Pro His
   65                  70                  75                  80
   Val Thr Pro Phe Phe His Gln Thr Val Asn Ser Tyr Gly Lys Asn Ser
                   85                  90                  95
   Phe Val Trp Met Gly Pro Ile Pro Arg Val His Ile Met Asn Pro Glu
               100                 105                 110
   Asp Leu Lys Asp Thr Phe Asn Arg His Asp Asp Phe His Lys Val Val
           115                 120                 125
   Lys Asn Pro Ile Met Lys Ser Leu Pro Gln Gly Ile Val Gly Ile Glu
       130                 135                 140
   Gly Glu Gln Trp Ala Lys His Arg Lys Ile Ile Asn Pro Ala Phe His
   145                 150                 155                 160
   Leu Glu Lys Leu Lys Gly Met Val Pro Ile Phe Tyr Arg Ser Cys Ser
                   165                 170                 175
   Glu Met Ile Asn Lys Trp Glu Ser Leu Val Ser Lys Glu Ser Ser Cys
               180                 185                 190
   Glu Leu Asp Val Trp Pro Tyr Leu Glu Asn Phe Thr Ser Asp Val Ile
           195                 200                 205
   Ser Arg Ala Ala Phe Gly Ser Ser Tyr Glu Glu Gly Arg Lys Ile Phe
       210                 215                 220
   Gln Leu Leu Arg Glu Glu Ala Lys Ile Tyr Thr Val Ala Met Arg Ser
   225                 230                 235                 240
   Val Tyr Ile Pro Gly Trp Arg Phe Leu Pro Thr Lys Gln Asn Lys Lys
                   245                 250                 255
   Ala Lys Glu Ile His Asn Glu Ile Lys Gly Leu Leu Lys Gly Ile Ile
               260                 265                 270
   Asn Lys Arg Glu Glu Ala Met Lys Ala Gly Glu Ala Thr Lys Asp Asp
           275                 280                 285
   Leu Leu Gly Ile Leu Met Glu Ser Asn Phe Arg Glu Ile Gln Glu His
       290                 295                 300
   Gly Asn Asn Lys Asn Ala Gly Met Ser Ile Glu Asp Val Ile Gly Glu
   305                 310                 315                 320
   Cys Lys Leu Phe Tyr Phe Ala Gly Gln Glu Thr Thr Ser Val Leu Leu
                   325                 330                 335
   Val Trp Thr Met Val Leu Leu Ser Gln Asn Gln Asp Trp Gln Ala Arg
   340 345 350
   Ala Arg Glu Glu Val Leu Gln Val Phe Gly Ser Asn Ile Pro Thr Tyr
   355 360 365
   Glu Glu Leu Ser Gln Leu Lys Val Val Thr Met Ile Leu Leu Glu Val
   370 375 380
   Leu Arg Leu Tyr Pro Ser Val Val Ala Leu Pro Arg Thr Thr His Lys
   385 390 395 400
   Lys Thr Gln Leu Gly Lys Leu Ser Leu Pro Ala Gly Val Glu Val Ser
   405 410 415
   Leu Pro Ile Leu Leu Val His His Asp Lys Glu Leu Trp Gly Glu Asp
   420 425 430
   Ala Asn Glu Phe Lys Pro Glu Arg Phe Ser Glu Gly Val Ser Lys Ala
   435 440 445
   Thr Lys Asn Gln Phe Thr Tyr Phe Pro Phe Gly Gly Gly Pro Arg Ile
   450 455 460
   Cys Ile Gly Gln Asn Phe Ala Met Met Glu Ala Lys Leu Ala Leu Ser
   465 470 475 480
   Leu Ile Leu Arg His Phe Ala Leu Glu Leu Ser Pro Leu Tyr Ala His
   485 490 495
   Ala Pro Ser Val Thr Ile Thr Leu Gln Pro Gln Tyr Gly Ala His Ile
   500 505 510
   Ile Leu His Lys Arg
   515
<210> 102
   <211> 521
   <212> PRT
   <213> Prunus mume
<400> 102
   Met Glu Ala Ser Arg Pro Ser Cys Val Ala Leu Ser Val Val Leu Val
   1               5                   10                  15
   Ser Ile Val Ile Ala Trp Ala Trp Arg Val Leu Asn Trp Val Trp Leu
               20                  25                  30
   Arg Pro Asn Lys Leu Glu Arg Cys Leu Arg Glu Gln Gly Leu Thr Gly
   35 40 45
   Asn Ser Tyr Arg Leu Leu Phe Gly Asp Thr Lys Glu Ile Ser Met Met
   50 55 60
   Val Glu Gln Ala Gln Ser Lys Pro Ile Lys Leu Ser Thr Thr His Asp
   65 70 75 80
   Ile Ala Pro Arg Val Ile Pro Phe Ser His Gln Ile Val Tyr Thr Tyr
   85 90 95
   Gly Arg Asn Ser Phe Val Trp Met Gly Pro Thr Pro Arg Val Thr Ile
   100 105 110
   Met Asn Pro Glu Asp Leu Lys Asp Ala Phe Asn Lys Ser Asp Glu Phe
   115 120 125
   Gln Arg Ala Ile Ser Asn Pro Ile Val Lys Ser Ile Ser Gln Gly Leu
   130 135 140
   Ser Ser Leu Glu Gly Glu Lys Trp Ala Lys His Arg Lys Ile Ile Asn
   145 150 155 160
   Pro Ala Phe His Leu Glu Lys Leu Lys Gly Met Leu Pro Thr Phe Tyr
   165 170 175
   Gln Ser Cys Ser Glu Met Ile Asn Lys Trp Glu Ser Leu Val Phe Lys
   180 185 190
   Glu Gly Ser Arg Glu Met Asp Val Trp Pro Tyr Leu Glu Asn Leu Thr
   195 200 205
   Ser Asp Val Ile Ser Arg Ala Ala Phe Gly Ser Ser Tyr Glu Glu Gly
   210 215 220
   Arg Lys Ile Phe Gln Leu Leu Arg Glu Glu Ala Lys Phe Tyr Thr Ile
   225 230 235 240
   Ala Ala Arg Ser Val Tyr Ile Pro Gly Trp Arg Phe Leu Pro Thr Lys
   245 250 255
   Gln Asn Lys Arg Met Lys Glu Ile His Lys Glu Val Arg Gly Leu Leu
   260 265 270
   Lys Gly Ile Ile Asn Lys Arg Glu Asp Ala Ile Lys Ala Gly Glu Ala
   275 280 285
   Ala Lys Gly Asn Leu Leu Gly Ile Leu Met Glu Ser Asn Phe Arg Glu
   290 295 300
   Ile Gln Glu His Gly Asn Asn Lys Asn Ala Gly Met Ser Ile Glu Asp
   305 310 315 320
   Val Ile Gly Glu Cys Lys Leu Phe Tyr Phe Ala Gly Gln Glu Thr Thr
   325 330 335
   Ser Val Leu Leu Val Trp Thr Leu Val Leu Leu Ser Gln Asn Gln Asp
   340 345 350
   Trp Gln Ala Arg Ala Arg Glu Glu Val Leu Gln Val Phe Gly Thr Asn
   355 360 365
   Ile Pro Thr Tyr Asp Gln Leu Ser His Leu Lys Val Val Thr Met Ile
   370 375 380
   Leu Leu Glu Val Leu Arg Leu Tyr Pro Ala Val Val Glu Leu Pro Arg
   385 390 395 400
   Thr Thr Tyr Lys Lys Thr Gln Leu Gly Lys Phe Leu Leu Pro Ala Gly
   405 410 415
   Val Glu Val Ser Leu His Ile Met Leu Ala His His Asp Lys Glu Leu
   420 425 430
   Trp Gly Glu Asp Ala Lys Glu Phe Lys Pro Glu Arg Phe Ser Glu Gly
   435 440 445
   Val Ser Lys Ala Thr Lys Asn Gln Phe Thr Tyr Phe Pro Phe Gly Ala
   450 455 460
   Gly Pro Arg Ile Cys Ile Gly Gln Asn Phe Ala Met Leu Glu Ala Lys
   465 470 475 480
   Leu Ala Leu Ser Leu Ile Leu Gln His Phe Thr Phe Glu Leu Ser Pro
   485 490 495
   Ser Tyr Ala His Ala Pro Ser Val Thr Ile Thr Leu His Pro Gln Phe
   500 505 510
   Gly Ala His Phe Ile Leu His Lys Arg
   515 520
<210> 103
   <211> 514
   <212> PRT
   <213> Prunus mume
<400> 103
   Cys Val Ala Leu Ser Val Val Leu Val Ser Ile Val Ile Ala Trp Ala
   1               5                   10                  15
   Trp Arg Val Leu Asn Trp Val Trp Leu Arg Pro Asn Lys Leu Glu Arg
               20                  25                  30
   Cys Leu Arg Glu Gln Gly Leu Thr Gly Asn Ser Tyr Arg Leu Leu Phe
           35                  40                  45
   Gly Asp Thr Lys Glu Ile Ser Met Met Val Glu Gln Ala Gln Ser Lys
       50                  55                  60
   Pro Ile Lys Leu Ser Thr Thr His Asp Ile Ala Pro Arg Val Ile Pro
   65                  70                  75                  80
   Phe Ser His Gln Ile Val Tyr Thr Tyr Gly Arg Asn Ser Phe Val Trp
                   85                  90                  95
   Met Gly Pro Thr Pro Arg Val Thr Ile Met Asn Pro Glu Asp Leu Lys
               100                 105                 110
   Asp Ala Phe Asn Lys Ser Asp Glu Phe Gln Arg Ala Ile Ser Asn Pro
           115                 120                 125
   Ile Val Lys Ser Ile Ser Gln Gly Leu Ser Ser Leu Glu Gly Glu Lys
       130                 135                 140
   Trp Ala Lys His Arg Lys Ile Ile Asn Pro Ala Phe His Leu Glu Lys
   145                 150                 155                 160
   Leu Lys Gly Met Leu Pro Thr Phe Tyr Gln Ser Cys Ser Glu Met Ile
                   165                 170                 175
   Asn Lys Trp Glu Ser Leu Val Phe Lys Glu Gly Ser Arg Glu Met Asp
               180                 185                 190
   Val Trp Pro Tyr Leu Glu Asn Leu Thr Ser Asp Val Ile Ser Arg Ala
           195                 200                 205
   Ala Phe Gly Ser Ser Tyr Glu Glu Gly Arg Lys Ile Phe Gln Leu Leu
       210                 215                 220
   Arg Glu Glu Ala Lys Phe Tyr Thr Ile Ala Ala Arg Ser Val Tyr Ile
   225                 230                 235                 240
   Pro Gly Trp Arg Phe Leu Pro Thr Lys Gln Asn Lys Arg Met Lys Glu
                   245                 250                 255
   Ile His Lys Glu Val Arg Gly Leu Leu Lys Gly Ile Ile Asn Lys Arg
               260                 265                 270
   Glu Asp Ala Ile Lys Ala Gly Glu Ala Ala Lys Gly Asn Leu Leu Gly
           275                 280                 285
   Ile Leu Met Glu Ser Asn Phe Arg Glu Ile Gln Glu His Gly Asn Asn
       290                 295                 300
   Lys Asn Ala Gly Met Ser Ile Glu Asp Val Ile Gly Glu Cys Lys Leu
   305                 310                 315                 320
   Phe Tyr Phe Ala Gly Gln Glu Thr Thr Ser Val Leu Leu Val Trp Thr
                   325                 330                 335
   Leu Val Leu Leu Ser Gln Asn Gln Asp Trp Gln Ala Arg Ala Arg Glu
               340                 345                 350
   Glu Val Leu Gln Val Phe Gly Thr Asn Ile Pro Thr Tyr Asp Gln Leu
           355                 360                 365
   Ser His Leu Lys Val Val Thr Met Ile Leu Leu Glu Val Leu Arg Leu
       370                 375                 380
   Tyr Pro Ala Val Val Glu Leu Pro Arg Thr Thr Tyr Lys Lys Thr Gln
   385                 390                 395                 400
   Leu Gly Lys Phe Leu Leu Pro Ala Gly Val Glu Val Ser Leu His Ile
                   405                 410                 415
   Met Leu Ala His His Asp Lys Glu Leu Trp Gly Glu Asp Ala Lys Glu
               420                 425                 430
   Phe Lys Pro Glu Arg Phe Ser Glu Gly Val Ser Lys Ala Thr Lys Asn
           435                 440                 445
   Gln Phe Thr Tyr Phe Pro Phe Gly Ala Gly Pro Arg Ile Cys Ile Gly
       450                 455                 460
   Gln Asn Phe Ala Met Leu Glu Ala Lys Leu Ala Leu Ser Leu Ile Leu
   465                 470                 475                 480
   Gln His Phe Thr Phe Glu Leu Ser Pro Ser Tyr Ala His Ala Pro Ser
                   485                 490                 495
   Val Thr Ile Thr Leu His Pro Gln Phe Gly Ala His Phe Ile Leu His
               500                 505                 510
   Lys Arg
<210> 104
   <211> 418
   <212> PRT
   <213> Prunus persica
<400> 104
   Met Gly Pro Ile Pro Arg Val His Ile Met Asn Pro Glu Asp Leu Lys
   1               5                   10                  15
   Asp Thr Phe Asn Arg His Asp Asp Phe His Lys Val Val Lys Asn Pro
               20                  25                  30
   Ile Met Lys Ser Leu Pro Gln Gly Ile Val Gly Ile Glu Gly Asp Gln
           35                  40                  45
   Trp Ala Lys His Arg Lys Ile Ile Asn Pro Ala Phe His Leu Glu Lys
       50                  55                  60
   Leu Lys Gly Met Val Pro Ile Phe Tyr Gln Ser Cys Ser Glu Met Ile
   65                  70                  75                  80
   Asn Ile Trp Lys Ser Leu Val Ser Lys Glu Ser Ser Cys Glu Leu Asp
                   85                  90                  95
   Val Trp Pro Tyr Leu Glu Asn Phe Thr Ser Asp Val Ile Ser Arg Ala
               100                 105                 110
   Ala Phe Gly Ser Ser Tyr Glu Glu Gly Arg Lys Ile Phe Gln Leu Leu
           115                 120                 125
   Arg Glu Glu Ala Lys Val Tyr Thr Val Ala Val Arg Ser Val Tyr Ile
       130                 135                 140
   Pro Gly Trp Arg Phe Leu Pro Thr Lys Gln Asn Lys Lys Thr Lys Glu
   145                 150                 155                 160
   Ile His Asn Glu Ile Lys Gly Leu Leu Lys Gly Ile Ile Asn Lys Arg
                   165                 170                 175
   Glu Glu Ala Met Lys Ala Gly Glu Ala Thr Lys Asp Asp Leu Leu Gly
               180                 185                 190
   Ile Leu Met Glu Ser Asn Phe Arg Glu Ile Gln Glu His Gly Asn Asn
           195                 200                 205
   Lys Asn Ala Gly Met Ser Ile Glu Asp Val Ile Gly Glu Cys Lys Leu
       210                 215                 220
   Phe Tyr Phe Ala Gly Gln Glu Thr Thr Ser Val Leu Leu Val Trp Thr
   225                 230                 235                 240
   Met Val Leu Leu Ser Gln Asn Gln Asp Trp Gln Ala Arg Ala Arg Glu
                   245                 250                 255
   Glu Val Leu Gln Val Phe Gly Ser Asn Ile Pro Thr Tyr Glu Glu Leu
               260                 265                 270
   Ser His Leu Lys Val Val Thr Met Ile Leu Leu Glu Val Leu Arg Leu
           275                 280                 285
   Tyr Pro Ser Val Val Ala Leu Pro Arg Thr Thr His Lys Lys Thr Gln
       290                 295                 300
   Leu Gly Lys Leu Ser Leu Pro Ala Gly Val Glu Val Ser Leu Pro Ile
   305                 310                 315                 320
   Leu Leu Val His His Asp Lys Glu Leu Trp Gly Glu Asp Ala Asn Glu
                   325                 330                 335
   Phe Lys Pro Glu Arg Phe Ser Glu Gly Val Ser Lys Ala Thr Lys Asn
               340                 345                 350
   Gln Phe Thr Tyr Phe Pro Phe Gly Gly Gly Pro Arg Ile Cys Ile Gly
           355                 360                 365
   Gln Asn Phe Ala Met Met Glu Ala Lys Leu Ala Leu Ser Leu Ile Leu
       370                 375                 380
   Gln His Phe Thr Phe Glu Leu Ser Pro Gln Tyr Ser His Ala Pro Ser
   385                 390                 395                 400
   Val Thr Ile Thr Leu Gln Pro Gln Tyr Gly Ala His Leu Ile Leu His
                   405                 410                 415
   Lys Arg
<210> 105
   <211> 1578
   <212> DNA
   <213> Artificial Sequence
<220>
   <223> Codon-optimized KAH
<400> 105
   atgggtttgt tcccattaga ggattcctac gcgctggtct ttgaaggact agcaataaca      60
   ctggctttgt actatctact gtctttcatc tacaaaacat ctaaaaagac atgtacacct     120
   cctaaagcat ctggtgaaat cattccaatt acaggaatca tattgaatct gctatctggc     180
   tcaagtggtc tacctattat cttagcactt gcctctttag cagacagatg tggtcctatt     240
   ttcaccatta ggctgggtat taggagagtg ctagtagtat caaattggga aatcgctaag     300
   gagattttca ctacccacga tttgatagtt tctaatagac caaaatactt agccgctaag     360
   attcttggtt tcaattatgt ttcattctct ttcgctccat acggcccata ttgggtcgga     420
   atcagaaaga ttattgctac aaaactaatg tcttcttcca gacttcagaa gttgcaattt     480
   gtaagagttt ttgaactaga aaactctatg aaatctatca gagaatcatg gaaggagaaa     540
   aaggatgaag agggaaaggt attagttgag atgaaaaagt ggttctggga actgaatatg     600
   aacatagtgt taaggacagt tgctggtaaa caatacactg gtacagttga tgatgccgat     660
   gcaaagcgta tctccgagtt attcagagaa tggtttcact acactggcag atttgtcgtt     720
   ggagacgctt ttccttttct aggttggttg gacctgggcg gatacaaaaa gacaatggaa     780
   ttagttgcta gtagattgga ctcaatggtc agtaaatggt tagatgagca tcgtaaaaag     840
   caagctaacg atgacaaaaa ggaggatatg gatttcatgg atatcatgat ctccatgaca     900
   gaagcaaatt caccacttga aggatacggc actgatacta ttatcaagac cacatgtatg     960
   actttgattg tttcaggagt tgatacaacc tcaatcgtac ttacttgggc cttatcactt    1020
   ttgttaaaca acagagatac tttgaaaaag gcacaagagg aattagatat gtgcgtaggt    1080
   aaaggaagac aagtcaacga gtctgatctt gttaacttga tatacttgga agcagtgctt    1140
   aaagaggctt taagacttta cccagcagcg ttcttaggcg gaccaagagc attcttggaa    1200
   gattgtactg ttgctggtta tagaattcca aagggcacct gcttgttgat taacatgtgg    1260
   aaactgcata gagatccaaa catttggagt gatccttgcg aattcaagcc agaaagattt    1320
   ttgacaccta atcaaaagga tgttgatgtg atcggtatgg atttcgaatt gataccattt    1380
   ggtgccggca gaagatattg tccaggtact agattggctt tacagatgtt gcatatcgta    1440
   ttagcgacat tgctgcaaaa cttcgaaatg tcaacaccaa acgatgcgcc agtcgatatg    1500
   actgcttctg ttggcatgac aaatgccaaa gcatcacctt tagaagtctt gctatcacct    1560
   cgtgttaaat ggtcctaa 1578
<210> 106
   <211> 522
   <212> PRT
   <213> Stevia rebaudiana
<400> 106
   Met Gly Leu Phe Pro Leu Glu Asp Ser Tyr Ala Leu Val Phe Glu Gly
   1               5                   10                  15
   Leu Ala Ile Thr Leu Ala Leu Tyr Tyr Leu Leu Ser Phe Ile Tyr Lys
               20                  25                  30
   Thr Ser Lys Lys Thr Cys Thr Pro Pro Lys Ala Ser Gly Glu His Pro
           35                  40                  45
   Ile Thr Gly His Leu Asn Leu Leu Ser Gly Ser Ser Gly Leu Pro His
       50                  55                  60
   Leu Ala Leu Ala Ser Leu Ala Asp Arg Cys Gly Pro Ile Phe Thr Ile
   65                  70                  75                  80
   Arg Leu Gly Ile Arg Arg Val Leu Val Val Ser Asn Trp Glu Ile Ala
                   85                  90                  95
   Lys Glu Ile Phe Thr Thr His Asp Leu Ile Val Ser Asn Arg Pro Lys
               100                 105                 110
   Tyr Leu Ala Ala Lys Ile Leu Gly Phe Asn Tyr Val Ser Phe Ser Phe
           115                 120                 125
   Ala Pro Tyr Gly Pro Tyr Trp Val Gly Ile Arg Lys Ile Ile Ala Thr
       130                 135                 140
   Lys Leu Met Ser Ser Ser Arg Leu Gln Lys Leu Gln Phe Val Arg Val
   145                 150                 155                 160
   Phe Glu Leu Glu Asn Ser Met Lys Ser Ile Arg Glu Ser Trp Lys Glu
                   165                 170                 175
   Lys Lys Asp Glu Glu Gly Lys Val Leu Val Glu Met Lys Lys Trp Phe
               180                 185                 190
   Trp Glu Leu Asn Met Asn Ile Val Leu Arg Thr Val Ala Gly Lys Gln
           195                 200                 205
   Tyr Thr Gly Thr Val Asp Asp Ala Asp Ala Lys Arg Ile Ser Glu Leu
   210 215 220
   Phe Arg Glu Trp Phe His Tyr Thr Gly Arg Phe Val Val Gly Asp Ala
   225 230 235 240
   Phe Pro Phe Leu Gly Trp Leu Asp Leu Gly Gly Tyr Lys Lys Thr Met
   245 250 255
   Glu Leu Val Ala Ser Arg Leu Asp Ser Met Val Ser Lys Trp Leu Asp
   260 265 270
   Glu His Arg Lys Lys Gln Ala Asn Asp Asp Lys Lys Glu Asp Met Asp
   275 280 285
   Phe Met Asp Ile Met Ile Ser Met Thr Glu Ala Asn Ser Pro Leu Glu
   290 295 300
   Gly Tyr Gly Thr Asp Thr Ile Ile Lys Thr Thr Cys Met Thr Leu Ile
   305 310 315 320
   Val Ser Gly Val Asp Thr Thr Ser Ile Val Leu Thr Trp Ala Leu Ser
   325 330 335
   Leu Leu Leu Asn Asn Arg Asp Thr Leu Lys Lys Ala Gln Glu Glu Leu
   340 345 350
   Asp Met Cys Val Gly Lys Gly Arg Gln Val Asn Glu Ser Asp Leu Val
   355 360 365
   Asn Leu Ile Tyr Leu Glu Ala Val Leu Lys Glu Ala Leu Arg Leu Tyr
   370 375 380
   Pro Ala Ala Phe Leu Gly Gly Pro Arg Ala Phe Leu Glu Asp Cys Thr
   385 390 395 400
   Val Ala Gly Tyr Arg Ile Pro Lys Gly Thr Cys Leu Leu Ile Asn Met
   405 410 415
   Trp Lys Leu His Arg Asp Pro Asn Ile Trp Ser Asp Pro Cys Glu Phe
   420 425 430
   Lys Pro Glu Arg Phe Leu Thr Pro Asn Gln Lys Asp Val Asp Val Ile
   435 440 445
   Gly Met Asp Phe Glu Leu Ile Pro Phe Gly Ala Gly Arg Arg Tyr Cys
   450 455 460
   Pro Gly Thr Arg Leu Ala Leu Gln Met Leu His Ile Val Leu Ala Thr
   465 470 475 480
   Leu Leu Gln Asn Phe Glu Met Ser Thr Pro Asn Asp Ala Pro Val Asp
   485 490 495
   Met Thr Ala Ser Val Gly Met Thr Asn Ala Lys Ala Ser Pro Leu Glu
   500 505 510
   Val Leu Leu Ser Pro Arg Val Lys Trp Ser
   515 520
<210> 107
   <211> 1431
   <212> DNA
   <213> Artificial Sequence
<220>
   <223> Codon-optimized KAH
<400> 107
   atgatacaag ttttaactcc aattctactc ttcctcatct tcttcgtttt ctggaaagtc      60
   tacaaacatc aaaagactaa aatcaatcta ccaccaggtt ccttcggctg gccatttttg     120
   ggtgaaacct tagccttact tagagcaggc tgggattctg agccagaaag attcgtaaga     180
   gagcgtatca aaaagcatgg atctccactt gttttcaaga catcactatt tggagacaga     240
   ttcgctgttc tttgcggtcc agctggtaat aagtttttgt tctgcaacga aaacaaatta     300
   gtggcatctt ggtggccagt ccctgtaagg aagttgttcg gtaaaagttt actcacaata     360
   agaggagatg aagcaaaatg gatgagaaaa atgctattgt cttacttggg tccagatgca     420
   tttgccacac attatgccgt tactatggat gttgtaacac gtagacatat tgatgtccat     480
   tggaggggca aggaggaagt taatgtattt caaacagtta agttgtacgc attcgaatta     540
   gcttgtagat tattcatgaa cctagatgac ccaaaccaca tcgcgaaact cggtagtctt     600
   ttcaacattt tcctcaaagg gatcatcgag cttcctatag acgttcctgg aactagattt     660
   tactccagta aaaaggccgc agctgccatt agaattgaat tgaaaaagct cattaaagct     720
   agaaaactcg aattgaagga gggtaaggcg tcttcttcac aggacttgct ttctcatcta     780
   ttaacatcac ctgatgagaa tgggatgttc ttgacagaag aggaaatagt cgataacatt     840
   ctacttttgt tattcgctgg tcacgatacc tctgcactat caataacact tttgatgaaa     900
   accttaggtg aacacagtga tgtgtacgac aaggttttga aggaacaatt agaaatttcc     960
   aaaacaaagg aggcttggga atcactaaag tgggaagata tccagaagat gaagtactca    1020
   tggtcagtaa tctgtgaagt catgagattg aatcctcctg tcatagggac atacagagag    1080
   gcgttggttg atatcgacta tgctggttac actatcccaa aaggatggaa gttgcattgg    1140
   tcagctgttt ctactcaaag agacgaagcc aatttcgaag atgtaactag attcgatcca    1200
   tccagatttg aaggggcagg ccctactcca ttcacatttg tgcctttcgg tggaggtcct    1260
   agaatgtgtt taggcaaaga gtttgccagg ttagaagtgt tagcatttct ccacaacatt    1320
   gttaccaact ttaagtggga tcttctaatc cctgatgaga agatcgaata tgatccaatg    1380
   gctactccag ctaagggctt gccaattaga cttcatccac accaagtcta a             1431
<210> 108
   <211> 476
   <212> PRT
   <213> Stevia rebaudiana
<400> 108
   Met Ile Gln Val Leu Thr Pro Ile Leu Leu Phe Leu Ile Phe Phe Val
   1               5                   10                  15
   Phe Trp Lys Val Tyr Lys His Gln Lys Thr Lys Ile Asn Leu Pro Pro
               20                  25                  30
   Gly Ser Phe Gly Trp Pro Phe Leu Gly Glu Thr Leu Ala Leu Leu Arg
           35                  40                  45
   Ala Gly Trp Asp Ser Glu Pro Glu Arg Phe Val Arg Glu Arg Ile Lys
       50                  55                  60
   Lys His Gly Ser Pro Leu Val Phe Lys Thr Ser Leu Phe Gly Asp Arg
   65                  70                  75                  80
   Phe Ala Val Leu Cys Gly Pro Ala Gly Asn Lys Phe Leu Phe Cys Asn
                   85                  90                  95
   Glu Asn Lys Leu Val Ala Ser Trp Trp Pro Val Pro Val Arg Lys Leu
               100                 105                 110
   Phe Gly Lys Ser Leu Leu Thr Ile Arg Gly Asp Glu Ala Lys Trp Met
           115                 120                 125
   Arg Lys Met Leu Leu Ser Tyr Leu Gly Pro Asp Ala Phe Ala Thr His
       130                 135                 140
   Tyr Ala Val Thr Met Asp Val Val Thr Arg Arg His Ile Asp Val His
   145                 150                 155                 160
   Trp Arg Gly Lys Glu Glu Val Asn Val Phe Gln Thr Val Lys Leu Tyr
                   165                 170                 175
   Ala Phe Glu Leu Ala Cys Arg Leu Phe Met Asn Leu Asp Asp Pro Asn
   180 185 190
   His Ile Ala Lys Leu Gly Ser Leu Phe Asn Ile Phe Leu Lys Gly Ile
   195 200 205
   Ile Glu Leu Pro Ile Asp Val Pro Gly Thr Arg Phe Tyr Ser Ser Lys
   210 215 220
   Lys Ala Ala Ala Ala Ile Arg Ile Glu Leu Lys Lys Leu Ile Lys Ala
   225 230 235 240
   Arg Lys Leu Glu Leu Lys Glu Gly Lys Ala Ser Ser Ser Gln Asp Leu
   245 250 255
   Leu Ser His Leu Leu Thr Ser Pro Asp Glu Asn Gly Met Phe Leu Thr
   260 265 270
   Glu Glu Glu Ile Val Asp Asn Ile Leu Leu Leu Leu Phe Ala Gly His
   275 280 285
   Asp Thr Ser Ala Leu Ser Ile Thr Leu Leu Met Lys Thr Leu Gly Glu
   290 295 300
   His Ser Asp Val Tyr Asp Lys Val Leu Lys Glu Gln Leu Glu Ile Ser
   305 310 315 320
   Lys Thr Lys Glu Ala Trp Glu Ser Leu Lys Trp Glu Asp Ile Gln Lys
   325 330 335
   Met Lys Tyr Ser Trp Ser Val Ile Cys Glu Val Met Arg Leu Asn Pro
   340 345 350
   Pro Val Ile Gly Thr Tyr Arg Glu Ala Leu Val Asp Ile Asp Tyr Ala
   355 360 365
   Gly Tyr Thr Ile Pro Lys Gly Trp Lys Leu His Trp Ser Ala Val Ser
   370 375 380
   Thr Gln Arg Asp Glu Ala Asn Phe Glu Asp Val Thr Arg Phe Asp Pro
   385 390 395 400
   Ser Arg Phe Glu Gly Ala Gly Pro Thr Pro Phe Thr Phe Val Pro Phe
   405 410 415
   Gly Gly Gly Pro Arg Met Cys Leu Gly Lys Glu Phe Ala Arg Leu Glu
   420 425 430
   Val Leu Ala Phe Leu His Asn Ile Val Thr Asn Phe Lys Trp Asp Leu
   435 440 445
   Leu Ile Pro Asp Glu Lys Ile Glu Tyr Asp Pro Met Ala Thr Pro Ala
   450 455 460
   Lys Gly Leu Pro Ile Arg Leu His Pro His Gln Val
   465 470 475
<210> 109
   <211> 1578
   <212> DNA
   <213> Artificial Sequence
<220>
   <223> Codon-optimized KAH
<400> 109
   atggagtctt tagtggttca tacagtaaat gctatctggt gtattgtaat cgtcgggatt      60
   ttctcagttg gttatcacgt ttacggtaga gctgtggtcg aacaatggag aatgagaaga     120
   tcactgaagc tacaaggtgt taaaggccca ccaccatcca tcttcaatgg taacgtctca     180
   gaaatgcaac gtatccaatc cgaagctaaa cactgctctg gcgataacat tatctcacat     240
   gattattctt cttcattatt cccacacttc gatcactgga gaaaacagta cggcagaatc     300
   tacacatact ctactggatt aaagcaacac ttgtacatca atcatccaga aatggtgaag     360
   gagctatctc agactaacac attgaacttg ggtagaatca cccatataac caaaagattg     420
   aatcctatct taggtaacgg aatcataacc tctaatggtc ctcattgggc ccatcagcgt     480
   agaattatcg cctacgagtt tactcatgat aagatcaagg gtatggttgg tttgatggtt     540
   gagtctgcta tgcctatgtt gaataagtgg gaggagatgg taaagagagg cggagaaatg     600
   ggatgcgaca taagagttga tgaggacttg aaagatgttt cagcagatgt gattgcaaaa     660
   gcctgtttcg gatcctcatt ttctaaaggt aaggctattt tctctatgat aagagatttg     720
   cttacagcta tcacaaagag aagtgttcta ttcagattca acggattcac tgatatggtc     780
   tttgggagta aaaagcatgg tgacgttgat atagacgctt tagaaatgga attggaatca     840
   tccatttggg aaactgtcaa ggaacgtgaa atagaatgta aagatactca caaaaaggat     900
   ctgatgcaat tgattttgga aggggcaatg cgttcatgtg acggtaacct ttgggataaa     960
   tcagcatata gaagatttgt tgtagataat tgtaaatcta tctacttcgc agggcatgat    1020
   agtacagctg tctcagtgtc atggtgtttg atgttactgg ccctaaaccc atcatggcaa    1080
   gttaagatcc gtgatgaaat tctgtcttct tgcaaaaatg gtattccaga tgccgaaagt    1140
   atcccaaacc ttaaaacagt gactatggtt attcaagaga caatgagatt ataccctcca    1200
   gcaccaatcg tcgggagaga agcctctaaa gatatcagat tgggcgatct agttgttcct    1260
   aaaggcgtct gtatatggac actaatacca gctttacaca gagatcctga gatttgggga    1320
   ccagatgcaa acgatttcaa accagaaaga ttttctgaag gaatttcaaa ggcttgtaag    1380
   tatcctcaaa gttacattcc atttggtctg ggtcctagaa catgcgttgg taaaaacttt    1440
   ggcatgatgg aagtaaaggt tcttgtttcc ctgattgtct ccaagttctc tttcactcta    1500
   tctcctacct accaacatag tcctagtcac aaacttttag tagaaccaca acatggggtg    1560
   gtaattagag tggtttaa                                                  1578
<210> 110
   <211> 525
   <212> PRT
   <213> Arabidopsis thaliana
<400> 110
   Met Glu Ser Leu Val Val His Thr Val Asn Ala Ile Trp Cys Ile Val
   1               5                   10                  15
   Ile Val Gly Ile Phe Ser Val Gly Tyr His Val Tyr Gly Arg Ala Val
               20                  25                  30
   Val Glu Gln Trp Arg Met Arg Arg Ser Leu Lys Leu Gln Gly Val Lys
           35                  40                  45
   Gly Pro Pro Pro Ser Ile Phe Asn Gly Asn Val Ser Glu Met Gln Arg
       50                  55                  60
   Ile Gln Ser Glu Ala Lys His Cys Ser Gly Asp Asn Ile Ile Ser His
   65                  70                  75                  80
   Asp Tyr Ser Ser Ser Leu Phe Pro His Phe Asp His Trp Arg Lys Gln
                   85                  90                  95
   Tyr Gly Arg Ile Tyr Thr Tyr Ser Thr Gly Leu Lys Gln His Leu Tyr
               100                 105                 110
   Ile Asn His Pro Glu Met Val Lys Glu Leu Ser Gln Thr Asn Thr Leu
           115                 120                 125
   Asn Leu Gly Arg Ile Thr His Ile Thr Lys Arg Leu Asn Pro Ile Leu
       130                 135                 140
   Gly Asn Gly Ile Ile Thr Ser Asn Gly Pro His Trp Ala His Gln Arg
   145                 150                 155                 160
   Arg Ile Ile Ala Tyr Glu Phe Thr His Asp Lys Ile Lys Gly Met Val
                   165                 170                 175
   Gly Leu Met Val Glu Ser Ala Met Pro Met Leu Asn Lys Trp Glu Glu
   180 185 190
   Met Val Lys Arg Gly Gly Glu Met Gly Cys Asp Ile Arg Val Asp Glu
   195 200 205
   Asp Leu Lys Asp Val Ser Ala Asp Val Ile Ala Lys Ala Cys Phe Gly
   210 215 220
   Ser Ser Phe Ser Lys Gly Lys Ala Ile Phe Ser Met Ile Arg Asp Leu
   225 230 235 240
   Leu Thr Ala Ile Thr Lys Arg Ser Val Leu Phe Arg Phe Asn Gly Phe
   245 250 255
   Thr Asp Met Val Phe Gly Ser Lys Lys His Gly Asp Val Asp Ile Asp
   260 265 270
   Ala Leu Glu Met Glu Leu Glu Ser Ser Ile Trp Glu Thr Val Lys Glu
   275 280 285
   Arg Glu Ile Glu Cys Lys Asp Thr His Lys Lys Asp Leu Met Gln Leu
   290 295 300
   Ile Leu Glu Gly Ala Met Arg Ser Cys Asp Gly Asn Leu Trp Asp Lys
   305 310 315 320
   Ser Ala Tyr Arg Arg Phe Val Val Asp Asn Cys Lys Ser Ile Tyr Phe
   325 330 335
   Ala Gly His Asp Ser Thr Ala Val Ser Val Ser Trp Cys Leu Met Leu
   340 345 350
   Leu Ala Leu Asn Pro Ser Trp Gln Val Lys Ile Arg Asp Glu Ile Leu
   355 360 365
   Ser Ser Cys Lys Asn Gly Ile Pro Asp Ala Glu Ser Ile Pro Asn Leu
   370 375 380
   Lys Thr Val Thr Met Val Ile Gln Glu Thr Met Arg Leu Tyr Pro Pro
   385 390 395 400
   Ala Pro Ile Val Gly Arg Glu Ala Ser Lys Asp Ile Arg Leu Gly Asp
   405 410 415
   Leu Val Val Pro Lys Gly Val Cys Ile Trp Thr Leu Ile Pro Ala Leu
   420 425 430
   His Arg Asp Pro Glu Ile Trp Gly Pro Asp Ala Asn Asp Phe Lys Pro
           435                 440                 445
   Glu Arg Phe Ser Glu Gly Ile Ser Lys Ala Cys Lys Tyr Pro Gln Ser
       450                 455                 460
   Tyr Ile Pro Phe Gly Leu Gly Pro Arg Thr Cys Val Gly Lys Asn Phe
   465                 470                 475                 480
   Gly Met Met Glu Val Lys Val Leu Val Ser Leu Ile Val Ser Lys Phe
                   485                 490                 495
   Ser Phe Thr Leu Ser Pro Thr Tyr Gln His Ser Pro Ser His Lys Leu
               500                 505                 510
   Leu Val Glu Pro Gln His Gly Val Val Ile Arg Val Val
           515                 520                 525
<210> 111
   <211> 1590
   <212> DNA
   <213> Artificial Sequence
<220>
   <223> Codon-optimized KAH
<400> 111
   atgtacttcc tactacaata cctcaacatc acaaccgttg gtgtctttgc cacattgttt      60
   ctctcttatt gtttacttct ctggagaagt agagcgggta acaaaaagat tgccccagaa     120
   gctgccgctg catggcctat tatcggccac ctccacttac ttgcaggtgg atcccatcaa     180
   ctaccacata ttacattggg taacatggca gataagtacg gtcctgtatt cacaatcaga     240
   ataggcttgc atagagctgt agttgtctca tcttgggaaa tggcaaagga atgttcaaca     300
   gctaatgatc aagtgtcttc ttcaagacct gaactattag cttctaagtt gttgggttat     360
   aactacgcca tgtttggttt ttcaccatac ggttcatact ggagagaaat gagaaagatc     420
   atctctctcg aattactatc taattccaga ttggaactat tgaaagatgt tagagcctca     480
   gaagttgtca catctattaa ggaactatac aaattgtggg cggaaaagaa gaatgagtca     540
   ggattggttt ctgtcgagat gaaacaatgg ttcggagatt tgactttaaa cgtgatcttg     600
   agaatggtgg ctggtaaaag atacttctcc gcgagtgacg cttcagaaaa caaacaggcc     660
   cagcgttgta gaagagtctt cagagaattc ttccatctct ccggcttgtt tgtggttgct     720
   gatgctatac cttttcttgg atggctcgat tggggaagac acgagaagac cttgaaaaag     780
   accgccatag aaatggattc catcgcccag gagtggcttg aggaacatag acgtagaaaa     840
   gattctggag atgataattc tacccaagat ttcatggacg ttatgcaatc tgtgctagat 900
   ggcaaaaatc taggcggata cgatgctgat acgattaaca aggctacatg cttaactctt 960
   atatcaggtg gcagtgatac tactgtagtt tctttgacat gggctcttag tcttgtgtta 1020
   aacaatagag atactttgaa aaaggcacag gaagagttag acatccaagt cggtaaggaa 1080
   agattggtta acgagcaaga catcagtaag ttagtttact tgcaagcaat agtaaaagag 1140
   acactcagac tttatccacc aggtcctttg ggtggtttga gacaattcac tgaagattgt 1200
   acactaggtg gctatcacgt ttcaaaagga actagattaa tcatgaactt atccaagatt 1260
   caaaaagatc cacgtatttg gtctgatcct actgaattcc aaccagagag attccttacg 1320
   actcataaag atgtcgatcc acgtggtaaa cactttgaat tcattccatt cggtgcagga 1380
   agacgtgcat gtcctggtat cacattcgga ttacaagtac tacatctaac attggcatct 1440
   ttcttgcatg cgtttgaatt ttcaacacca tcaaatgagc aggttaacat gagagaatca 1500
   ttaggtctta cgaatatgaa atctacccca ttagaagttt tgatttctcc aagactatcc 1560
   cttaattgct tcaaccttat gaaaatttga 1590
<210> 112
   <211> 526
   <212> PRT
   <213> Vitis vinifera
<400> 112
   Met Tyr Phe Leu Leu Gln Tyr Leu Asn Ile Thr Thr Val Gly Val Phe
   1               5                   10                  15
   Ala Thr Leu Phe Leu Ser Tyr Cys Leu Leu Leu Trp Arg Ser Arg Ala
               20                  25                  30
   Gly Asn Lys Lys Ile Ala Pro Glu Ala Ala Ala Ala Trp Pro Ile Ile
           35                  40                  45
   Gly His Leu His Leu Leu Ala Gly Gly Ser His Gln Leu Pro His Ile
       50                  55                  60
   Thr Leu Gly Asn Met Ala Asp Lys Tyr Gly Pro Val Phe Thr Ile Arg
   65                  70                  75                  80
   Ile Gly Leu His Arg Ala Val Val Val Ser Ser Trp Glu Met Ala Lys
                   85                  90                  95
   Glu Cys Ser Thr Ala Asn Asp Gln Val Ser Ser Ser Arg Pro Glu Leu
               100                 105                 110
   Leu Ala Ser Lys Leu Leu Gly Tyr Asn Tyr Ala Met Phe Gly Phe Ser
           115                 120                 125
   Pro Tyr Gly Ser Tyr Trp Arg Glu Met Arg Lys Ile Ile Ser Leu Glu
       130                 135                 140
   Leu Leu Ser Asn Ser Arg Leu Glu Leu Leu Lys Asp Val Arg Ala Ser
   145                 150                 155                 160
   Glu Val Val Thr Ser Ile Lys Glu Leu Tyr Lys Leu Trp Ala Glu Lys
                   165                 170                 175
   Lys Asn Glu Ser Gly Leu Val Ser Val Glu Met Lys Gln Trp Phe Gly
               180                 185                 190
   Asp Leu Thr Leu Asn Val Ile Leu Arg Met Val Ala Gly Lys Arg Tyr
           195                 200                 205
   Phe Ser Ala Ser Asp Ala Ser Glu Asn Lys Gln Ala Gln Arg Cys Arg
       210                 215                 220
   Arg Val Phe Arg Glu Phe Phe His Leu Ser Gly Leu Phe Val Val Ala
   225                 230                 235                 240
   Asp Ala Ile Pro Phe Leu Gly Trp Leu Asp Trp Gly Arg His Glu Lys
                   245                 250                 255
   Thr Leu Lys Lys Thr Ala Ile Glu Met Asp Ser Ile Ala Gln Glu Trp
               260                 265                 270
   Leu Glu Glu His Arg Arg Arg Lys Asp Ser Gly Asp Asp Asn Ser Thr
           275                 280                 285
   Gln Asp Phe Met Asp Val Met Gln Ser Val Leu Asp Gly Lys Asn Leu
       290                 295                 300
   Gly Gly Tyr Asp Ala Asp Thr Ile Asn Lys Ala Thr Cys Leu Thr Leu
   305                 310                 315                 320
   Ile Ser Gly Gly Ser Asp Thr Thr Val Val Ser Leu Thr Trp Ala Leu
                   325                 330                 335
   Ser Leu Val Leu Asn Asn Arg Asp Thr Leu Lys Lys Ala Gln Glu Glu
               340                 345                 350
   Leu Asp Ile Gln Val Gly Lys Glu Arg Leu Val Asn Glu Gln Asp Ile
           355                 360                 365
   Ser Lys Leu Val Tyr Leu Gln Ala Ile Val Lys Glu Thr Leu Arg Leu
       370                 375                 380
   Tyr Pro Pro Gly Pro Leu Gly Gly Leu Arg Gln Phe Thr Glu Asp Cys
   385                 390                 395                 400
   Thr Leu Gly Gly Tyr His Val Ser Lys Gly Thr Arg Leu Ile Met Asn
                   405                 410                 415
   Leu Ser Lys Ile Gln Lys Asp Pro Arg Ile Trp Ser Asp Pro Thr Glu
               420                 425                 430
   Phe Gln Pro Glu Arg Phe Leu Thr Thr His Lys Asp Val Asp Pro Arg
           435                 440                 445
   Gly Lys His Phe Glu Phe Ile Pro Phe Gly Ala Gly Arg Arg Ala Cys
       450                 455                 460
   Pro Gly Ile Thr Phe Gly Leu Gln Val Leu His Leu Thr Leu Ala Ser
   465                 470                 475                 480
   Phe Leu His Ala Phe Glu Phe Ser Thr Pro Ser Asn Glu Gln Val Asn
                   485                 490                 495
   Met Arg Glu Ser Leu Gly Leu Thr Asn Met Lys Ser Thr Pro Leu Glu
               500                 505                 510
   Val Leu Ile Ser Pro Arg Leu Ser Ser Cys Ser Leu Tyr Asn
           515                 520                 525
<210> 113
   <211> 1440
   <212> DNA
   <213> Artificial Sequence
<220>
   <223> Codon-optimized KAH
<400> 113
   atggaaccta acttttactt gtcattacta ttgttgttcg tgaccttcat ttctttaagt        60
   ctgtttttca tcttttacaa acaaaagtcc ccattgaatt tgccaccagg gaaaatgggt       120
   taccctatca taggtgaaag tttagaattc ctatccacag gctggaaggg acatcctgaa       180
   aagttcatat ttgatagaat gcgtaagtac agtagtgagt tattcaagac ttctattgta       240
   ggcgaatcca cagttgtttg ctgtggggca gctagtaaca aattcctatt ctctaacgaa       300
   aacaaactgg taactgcctg gtggccagat tctgttaaca aaatcttccc aacaacttca       360
   ctggattcta atttgaagga ggaatctata aagatgagaa agttgctgcc acagttcttc       420
   aaaccagaag cacttcaaag atacgtcggc gttatggatg taatcgcaca aagacatttt       480
   gtcactcact gggacaacaa aaatgagatc acagtttatc cacttgctaa aagatacact       540
   ttcttgcttg cgtgtagact gttcatgtct gttgaggatg aaaatcatgt ggcgaaattc       600
   tcagacccat tccaactaat cgctgcaggc atcatttcac ttcctatcga tcttcctggt       660
   actccattca acaaggccat aaaggcttca aatttcatta gaaaagagct gataaagatt       720
   atcaaacaaa gacgtgttga tctggcagag ggtacagcat ctccaaccca ggatatcttg       780
   tcacatatgc tattaacatc tgatgaaaac ggtaaatcta tgaacgagtt gaacattgcc       840
   gacaagattc ttggactatt gataggaggc cacgatacag cttcagtagc ttgcacattt       900
   ctagtgaagt acttaggaga attaccacat atctacgata aagtctacca agagcaaatg       960
   gaaattgcca agtccaaacc tgctggggaa ttgttgaatt gggatgactt gaaaaagatg      1020
   aagtattcat ggaatgtggc atgtgaggta atgagattgt caccaccttt acaaggtggt      1080
   tttagagagg ctataactga ctttatgttt aacggtttct ctattccaaa agggtggaag      1140
   ttatactggt ccgccaactc tacacacaaa aatgcagaat gtttcccaat gcctgagaaa      1200
   ttcgatccta ccagatttga aggtaatggt ccagcgcctt atacatttgt accattcggt      1260
   ggaggcccta gaatgtgtcc tggaaaggaa tacgctagat tagaaatctt ggttttcatg      1320
   cataatctgg tcaaacgttt taagtgggaa aaggttattc cagacgaaaa gattattgtc      1380
   gatccattcc caatcccagc taaagatctt ccaatccgtt tgtatcctca caaagcttaa      1440
<210> 114
   <211> 479
   <212> PRT
   <213> Medicago truncatula
<400> 114
   Met Glu Pro Asn Phe Tyr Leu Ser Leu Leu Leu Leu Phe Val Thr Phe
   1               5                   10                  15
   Ile Ser Leu Ser Leu Phe Phe Ile Phe Tyr Lys Gln Lys Ser Pro Leu
               20                  25                  30
   Asn Leu Pro Pro Gly Lys Met Gly Tyr Pro Ile Ile Gly Glu Ser Leu
           35                  40                  45
   Glu Phe Leu Ser Thr Gly Trp Lys Gly His Pro Glu Lys Phe Ile Phe
       50                  55                  60
   Asp Arg Met Arg Lys Tyr Ser Ser Glu Leu Phe Lys Thr Ser Ile Val
   65                  70                  75                  80
   Gly Glu Ser Thr Val Val Cys Cys Gly Ala Ala Ser Asn Lys Phe Leu
   85 90 95
   Phe Ser Asn Glu Asn Lys Leu Val Thr Ala Trp Trp Pro Asp Ser Val
   100 105 110
   Asn Lys Ile Phe Pro Thr Thr Ser Leu Asp Ser Asn Leu Lys Glu Glu
   115 120 125
   Ser Ile Lys Met Arg Lys Leu Leu Pro Gln Phe Phe Lys Pro Glu Ala
   130 135 140
   Leu Gln Arg Tyr Val Gly Val Met Asp Val Ile Ala Gln Arg His Phe
   145 150 155 160
   Val Thr His Trp Asp Asn Lys Asn Glu Ile Thr Val Tyr Pro Leu Ala
   165 170 175
   Lys Arg Tyr Thr Phe Leu Leu Ala Cys Arg Leu Phe Met Ser Val Glu
   180 185 190
   Asp Glu Asn His Val Ala Lys Phe Ser Asp Pro Phe Gln Leu Ile Ala
   195 200 205
   Ala Gly Ile Ile Ser Leu Pro Ile Asp Leu Pro Gly Thr Pro Phe Asn
   210 215 220
   Lys Ala Ile Lys Ala Ser Asn Phe Ile Arg Lys Glu Leu Ile Lys Ile
   225 230 235 240
   Ile Lys Gln Arg Arg Val Asp Leu Ala Glu Gly Thr Ala Ser Pro Thr
   245 250 255
   Gln Asp Ile Leu Ser His Met Leu Leu Thr Ser Asp Glu Asn Gly Lys
   260 265 270
   Ser Met Asn Glu Leu Asn Ile Ala Asp Lys Ile Leu Gly Leu Leu Ile
   275 280 285
   Gly Gly His Asp Thr Ala Ser Val Ala Cys Thr Phe Leu Val Lys Tyr
   290 295 300
   Leu Gly Glu Leu Pro His Ile Tyr Asp Lys Val Tyr Gln Glu Gln Met
   305 310 315 320
   Glu Ile Ala Lys Ser Lys Pro Ala Gly Glu Leu Leu Asn Trp Asp Asp
   325 330 335
   Leu Lys Lys Met Lys Tyr Ser Trp Asn Val Ala Cys Glu Val Met Arg
               340                 345                 350
   Leu Ser Pro Pro Leu Gln Gly Gly Phe Arg Glu Ala Ile Thr Asp Phe
           355                 360                 365
   Met Phe Asn Gly Phe Ser Ile Pro Lys Gly Trp Lys Leu Tyr Trp Ser
       370                 375                 380
   Ala Asn Ser Thr His Lys Asn Ala Glu Cys Phe Pro Met Pro Glu Lys
   385                 390                 395                 400
   Phe Asp Pro Thr Arg Phe Glu Gly Asn Gly Pro Ala Pro Tyr Thr Phe
                   405                 410                 415
   Val Pro Phe Gly Gly Gly Pro Arg Met Cys Pro Gly Lys Glu Tyr Ala
               420                 425                 430
   Arg Leu Glu Ile Leu Val Phe Met His Asn Leu Val Lys Arg Phe Lys
           435                 440                 445
   Trp Glu Lys Val Ile Pro Asp Glu Lys Ile Ile Val Asp Pro Phe Pro
       450                 455                 460
   Ile Pro Ala Lys Asp Leu Pro Ile Arg Leu Tyr Pro His Lys Ala
   465                 470                 475
<210> 115
   <211> 1116
   <212> DNA
   <213> Artificial Sequence
<220>
   <223> Codon-optimized GGPPS
<400> 115
   atggcctctg ttactttggg ttcctggatc gtcgtccacc accataacca tcaccatcca      60
   tcatctatcc taactaaatc tcgttcaaga tcctgtccta ttacactaac caaaccaatc     120
   tcttttcgtt caaagagaac agtttcctct agtagttcta tcgtgtcctc tagtgtcgtc     180
   actaaggaag acaatctgag acagtctgaa ccttcttcct ttgatttcat gtcatatatc     240
   attactaagg cagaactagt gaataaggct cttgattcag cagttccatt aagagagcca     300
   ttgaaaatcc atgaagcaat gagatactct cttctagctg gcgggaagag agtcagacct     360
   gtactctgca tagcagcgtg cgaattagtt ggtggcgagg aatcaaccgc tatgcctgcc     420
   gcttgtgctg tagaaatgat tcatacaatg tcactgatac acgatgattt gccatgtatg     480
   gataacgatg atctgagaag gggtaagcca actaaccata aggttttcgg cgaagatgtt     540
   gccgtcttag ctggtgatgc tttgttatct ttcgcgttcg aacatttggc atccgcaaca     600
   tcaagtgatg ttgtgtcacc agtaagagta gttagagcag ttggagaact ggctaaagct     660
   attggaactg agggtttagt tgcaggtcaa gtcgtcgata tctcttccga aggtcttgat     720
   ttgaatgatg taggtcttga acatctcgaa ttcatccatc ttcacaagac agctgcactt     780
   ttagaagcca gtgcggttct cggcgcaatt gttggcggag ggagtgatga cgaaattgag     840
   agattgagga agtttgctag atgtatagga ttactgttcc aagtagtaga cgatatacta     900
   gatgtgacaa agtcttccaa agagttggga aaaacagctg gtaaagattt gattgccgac     960
   aaattgacct accctaagat tatggggcta gaaaaatcaa gagaatttgc cgagaaactc    1020
   aatagagagg cgcgtgatca actgttgggt ttcgattctg ataaagttgc accactctta    1080
   gccttagcca actacatcgc ttacagacaa aactaa                              1116
<210> 116
   <211> 371
   <212> PRT
   <213> Arabidopsis thaliana
<400> 116
   Met Ala Ser Val Thr Leu Gly Ser Trp Ile Val Val His His His Asn
   1               5                   10                  15
   His His His Pro Ser Ser Ile Leu Thr Lys Ser Arg Ser Arg Ser Cys
               20                  25                  30
   Pro Ile Thr Leu Thr Lys Pro Ile Ser Phe Arg Ser Lys Arg Thr Val
           35                  40                  45
   Ser Ser Ser Ser Ser Ile Val Ser Ser Ser Val Val Thr Lys Glu Asp
       50                  55                  60
   Asn Leu Arg Gln Ser Glu Pro Ser Ser Phe Asp Phe Met Ser Tyr Ile
   65                  70                  75                  80
   Ile Thr Lys Ala Glu Leu Val Asn Lys Ala Leu Asp Ser Ala Val Pro
                   85                  90                  95
   Leu Arg Glu Pro Leu Lys Ile His Glu Ala Met Arg Tyr Ser Leu Leu
               100                 105                 110
   Ala Gly Gly Lys Arg Val Arg Pro Val Leu Cys Ile Ala Ala Cys Glu
           115                 120                 125
   Leu Val Gly Gly Glu Glu Ser Thr Ala Met Pro Ala Ala Cys Ala Val
       130                 135                 140
   Glu Met Ile His Thr Met Ser Leu Ile His Asp Asp Leu Pro Cys Met
   145                 150                 155                 160
   Asp Asn Asp Asp Leu Arg Arg Gly Lys Pro Thr Asn His Lys Val Phe
                   165                 170                 175
   Gly Glu Asp Val Ala Val Leu Ala Gly Asp Ala Leu Leu Ser Phe Ala
               180                 185                 190
   Phe Glu His Leu Ala Ser Ala Thr Ser Ser Asp Val Val Ser Pro Val
           195                 200                 205
   Arg Val Val Arg Ala Val Gly Glu Leu Ala Lys Ala Ile Gly Thr Glu
       210                 215                 220
   Gly Leu Val Ala Gly Gln Val Val Asp Ile Ser Ser Glu Gly Leu Asp
   225                 230                 235                 240
   Leu Asn Asp Val Gly Leu Glu His Leu Glu Phe Ile His Leu His Lys
                   245                 250                 255
   Thr Ala Ala Leu Leu Glu Ala Ser Ala Val Leu Gly Ala Ile Val Gly
               260                 265                 270
   Gly Gly Ser Asp Asp Glu Ile Glu Arg Leu Arg Lys Phe Ala Arg Cys
           275                 280                 285
   Ile Gly Leu Leu Phe Gln Val Val Asp Asp Ile Leu Asp Val Thr Lys
       290                 295                 300
   Ser Ser Lys Glu Leu Gly Lys Thr Ala Gly Lys Asp Leu Ile Ala Asp
   305                 310                 315                 320
   Lys Leu Thr Tyr Pro Lys Ile Met Gly Leu Glu Lys Ser Arg Glu Phe
                   325                 330                 335
   Ala Glu Lys Leu Asn Arg Glu Ala Arg Asp Gln Leu Leu Gly Phe Asp
               340                 345                 350
   Ser Asp Lys Val Ala Pro Leu Leu Ala Leu Ala Asn Tyr Ile Ala Tyr
           355                 360                 365
   Arg Gln Asn
       370
<210> 117
   <211> 511
   <212> PRT
   <213> Rubus suavissimus
<400> 117
   Met Ala Thr Leu Leu Glu His Phe Gln Ala Met Pro Phe Ala Ile Pro
   1               5                   10                  15
   Ile Ala Leu Ala Ala Leu Ser Trp Leu Phe Leu Phe Tyr Ile Lys Val
               20                  25                  30
   Ser Phe Phe Ser Asn Lys Ser Ala Gln Ala Lys Leu Pro Pro Val Pro
           35                  40                  45
   Val Val Pro Gly Leu Pro Val Ile Gly Asn Leu Leu Gln Leu Lys Glu
       50                  55                  60
   Lys Lys Pro Tyr Gln Thr Phe Thr Arg Trp Ala Glu Glu Tyr Gly Pro
   65                  70                  75                  80
   Ile Tyr Ser Ile Arg Thr Gly Ala Ser Thr Met Val Val Leu Asn Thr
                   85                  90                  95
   Thr Gln Val Ala Lys Glu Ala Met Val Thr Arg Tyr Leu Ser Ile Ser
               100                 105                 110
   Thr Arg Lys Leu Ser Asn Ala Leu Lys Ile Leu Thr Ala Asp Lys Cys
           115                 120                 125
   Met Val Ala Ile Ser Asp Tyr Asn Asp Phe His Lys Met Ile Lys Arg
       130                 135                 140
   Tyr Ile Leu Ser Asn Val Leu Gly Pro Ser Ala Gln Lys Arg His Arg
   145                 150                 155                 160
   Ser Asn Arg Asp Thr Leu Arg Ala Asn Val Cys Ser Arg Leu His Ser
                   165                 170                 175
   Gln Val Lys Asn Ser Pro Arg Glu Ala Val Asn Phe Arg Arg Val Phe
               180                 185                 190
   Glu Trp Glu Leu Phe Gly Ile Ala Leu Lys Gln Ala Phe Gly Lys Asp
           195                 200                 205
   Ile Glu Lys Pro Ile Tyr Val Glu Glu Leu Gly Thr Thr Leu Ser Arg
       210                 215                 220
   Asp Glu Ile Phe Lys Val Leu Val Leu Asp Ile Met Glu Gly Ala Ile
   225                 230                 235                 240
   Glu Val Asp Trp Arg Asp Phe Phe Pro Tyr Leu Arg Trp Ile Pro Asn
                   245                 250                 255
   Thr Arg Met Glu Thr Lys Ile Gln Arg Leu Tyr Phe Arg Arg Lys Ala
               260                 265                 270
   Val Met Thr Ala Leu Ile Asn Glu Gln Lys Lys Arg Ile Ala Ser Gly
           275                 280                 285
   Glu Glu Ile Asn Cys Tyr Ile Asp Phe Leu Leu Lys Glu Gly Lys Thr
       290                 295                 300
   Leu Thr Met Asp Gln Ile Ser Met Leu Leu Trp Glu Thr Val Ile Glu
   305                 310                 315                 320
   Thr Ala Asp Thr Thr Met Val Thr Thr Glu Trp Ala Met Tyr Glu Val
                   325                 330                 335
   Ala Lys Asp Ser Lys Arg Gln Asp Arg Leu Tyr Gln Glu Ile Gln Lys
               340                 345                 350
   Val Cys Gly Ser Glu Met Val Thr Glu Glu Tyr Leu Ser Gln Leu Pro
           355                 360                 365
   Tyr Leu Asn Ala Val Phe His Glu Thr Leu Arg Lys His Ser Pro Ala
       370                 375                 380
   Ala Leu Val Pro Leu Arg Tyr Ala His Glu Asp Thr Gln Leu Gly Gly
   385                 390                 395                 400
   Tyr Tyr Ile Pro Ala Gly Thr Glu Ile Ala Ile Asn Ile Tyr Gly Cys
                   405                 410                 415
   Asn Met Asp Lys His Gln Trp Glu Ser Pro Glu Glu Trp Lys Pro Glu
               420                 425                 430
   Arg Phe Leu Asp Pro Lys Phe Asp Pro Met Asp Leu Tyr Lys Thr Met
           435                 440                 445
   Ala Phe Gly Ala Gly Lys Arg Val Cys Ala Gly Ser Leu Gln Ala Met
       450                 455                 460
   Leu Ile Ala Cys Pro Thr Ile Gly Arg Leu Val Gln Glu Phe Glu Trp
   465                 470                 475                 480
   Lys Leu Arg Asp Gly Glu Glu Glu Asn Val Asp Thr Val Gly Leu Thr
   485 490 495
   Thr His Lys Arg Tyr Pro Met His Ala Ile Leu Lys Pro Arg Ser
   500 505 510
<210> 118
   <211> 1449
   <212> DNA
   <213> Rubus suavissimus
<400> 118
   atggattcca aggccttgaa gaagccacat gcagtttgtg taccattccc tactcaaggt      60
   cacataaacc cgttgctaaa attggcaaaa ctccttcact acagaggctt ccatataacc     120
   tttgtcaaca cagagtacaa ccataagcgc ctgcttcaat cccgaggtac caactctctt     180
   gatggcctgc cgtcctttag gttcaaatcg attcccgatg gcctccctcc tactgatgcc     240
   aacgccaccc aagaagtaac atccctagtc gattccacta gaaaaaactg cttagcaccc     300
   ttcaggtgcc ttctttcaga actcaacaca tccccaaatt cccctcctgt tactctcata     360
   gtttctgatg gtggcatgtt cttcactctt gatgcagccc aagaacttgg tcttccagaa     420
   gttatattca agccaacttg tgcttcttca ttcatgtgct accatcattg tgcccatgtc     480
   attgaaaagg gtctcgttcc tcttaaagat gccagttatg tgacaaatgg ctatttggac     540
   actgtcatag attggatacc aggcataagc agtatccgtt tgagggatat gccgagcttc     600
   attagaacca cagacccaaa tgacatcttg ctgaaattta tgatggcaga gacggaaaga     660
   acccaaagag cgtctgcaat cattattaac acatttgatg cgttggagca tgaagtttta     720
   gatgcacttt caacttttct accacctatt tattccattg gacctctaca tctacaactc     780
   agtcagatcc cagaggatga ggactcaagg tcgattgggt caaatctgtg gagagaagaa     840
   ccagagtgtc ttgaatggct cgactctaaa gcaccaaatt ctgtggttta tgtaaacttc     900
   ggaagtatca cagtcatgac aaacgagcag ctgattgagt ttgcttgggg tcttgcaaac     960
   agtaacatga cctttttatg ggtgattagg cccgaccttg ttgctgggaa atcagctgtt    1020
   gttcctccag agtttgcgga agtgaccaag ggaaggagtt tattggcgag ttggtgccct    1080
   caagaacaag tgttgaacca cccggctgta ggaggattct tgacacacag tggatggaac    1140
   tctacaattg aaagtatatg cggtggagtt cccatgatct gttggccctt ctttgctgat    1200
   caacagacaa attgtaggtt ctgttgcaaa gaatggggta taggcctgga gatagagggt    1260
   gatgttaaaa ggaataacat agaagacctt gtaagaaagt taatggaagg aaaggaaggc    1320
   caagaaatga ggacgaaagc cttggaatgg aagaagttgg cagaggaggc tactactgct    1380
   cccaatggtt catctttctt gaatttggac aagatggtta accaggtgct tcttcgtcct    1440
   aagaattag                                                             1449
<210> 119
   <211> 482
   <212> PRT
   <213> Rubus suavissimus
<400> 119
   Met Asp Ser Lys Ala Leu Lys Lys Pro His Ala Val Cys Val Pro Phe
   1               5                   10                  15
   Pro Thr Gln Gly His Ile Asn Pro Leu Leu Lys Leu Ala Lys Leu Leu
               20                  25                  30
   His Tyr Arg Gly Phe His Ile Thr Phe Val Asn Thr Glu Tyr Asn His
           35                  40                  45
   Lys Arg Leu Leu Gln Ser Arg Gly Thr Asn Ser Leu Asp Gly Leu Pro
       50                  55                  60
   Ser Phe Arg Phe Lys Ser Ile Pro Asp Gly Leu Pro Pro Thr Asp Ala
   65                  70                  75                  80
   Asn Ala Thr Gln Glu Val Thr Ser Leu Val Asp Ser Thr Arg Lys Asn
                   85                  90                  95
   Cys Leu Ala Pro Phe Arg Cys Leu Leu Ser Glu Leu Asn Thr Ser Pro
               100                 105                 110
   Asn Ser Pro Pro Val Thr Leu Ile Val Ser Asp Gly Gly Met Phe Phe
           115                 120                 125
   Thr Leu Asp Ala Ala Gln Glu Leu Gly Leu Pro Glu Val Ile Phe Lys
       130                 135                 140
   Pro Thr Cys Ala Ser Ser Phe Met Cys Tyr His His Cys Ala His Val
   145                 150                 155                 160
   Ile Glu Lys Gly Leu Val Pro Leu Lys Asp Ala Ser Tyr Val Thr Asn
                   165                 170                 175
   Gly Tyr Leu Asp Thr Val Ile Asp Trp Ile Pro Gly Ile Ser Ser Ile
               180                 185                 190
   Arg Leu Arg Asp Met Pro Ser Phe Ile Arg Thr Thr Asp Pro Asn Asp
           195                 200                 205
   Ile Leu Leu Lys Phe Met Met Ala Glu Thr Glu Arg Thr Gln Arg Ala
       210                 215                 220
   Ser Ala Ile Ile Ile Asn Thr Phe Asp Ala Leu Glu His Glu Val Leu
   225 230 235 240
   Asp Ala Leu Ser Thr Phe Leu Pro Pro Ile Tyr Ser Ile Gly Pro Leu
   245 250 255
   His Leu Gln Leu Ser Gln Ile Pro Glu Asp Glu Asp Ser Arg Ser Ile
   260 265 270
   Gly Ser Asn Leu Trp Arg Glu Glu Pro Glu Cys Leu Glu Trp Leu Asp
   275 280 285
   Ser Lys Ala Pro Asn Ser Val Val Tyr Val Asn Phe Gly Ser Ile Thr
   290 295 300
   Val Met Thr Asn Glu Gln Leu Ile Glu Phe Ala Trp Gly Leu Ala Asn
   305 310 315 320
   Ser Asn Met Thr Phe Leu Trp Val Ile Arg Pro Asp Leu Val Ala Gly
   325 330 335
   Lys Ser Ala Val Val Pro Pro Glu Phe Ala Glu Val Thr Lys Gly Arg
   340 345 350
   Ser Leu Leu Ala Ser Trp Cys Pro Gln Glu Gln Val Leu Asn His Pro
   355 360 365
   Ala Val Gly Gly Phe Leu Thr His Ser Gly Trp Asn Ser Thr Ile Glu
   370 375 380
   Ser Ile Cys Gly Gly Val Pro Met Ile Cys Trp Pro Phe Phe Ala Asp
   385 390 395 400
   Gln Gln Thr Asn Cys Arg Phe Cys Cys Lys Glu Trp Gly Ile Gly Leu
   405 410 415
   Glu Ile Glu Gly Asp Val Lys Arg Asn Asn Ile Glu Asp Leu Val Arg
   420 425 430
   Lys Leu Met Glu Gly Lys Glu Gly Gln Glu Met Arg Thr Lys Ala Leu
   435 440 445
   Glu Trp Lys Lys Leu Ala Glu Glu Ala Thr Thr Ala Pro Asn Gly Ser
   450 455 460
   Ser Phe Leu Asn Leu Asp Lys Met Val Asn Gln Val Leu Leu Arg Pro
   465 470 475 480
   Lys Asn